# EUROPEAN PATENT APPLICATION

(11) **EP 4 831 279 A2**
(43) Date of publication of application: **28.10.2026**
(21) Application number: 26182715.8
(22) Date of filing: 11.06.2012
(51) Int. Cl.: C12N 5/074

(54) **CULTURE MEDIA FOR STEM CELLS**

(30) Priority: 10.06.2011 US 201161520569 P; 30.06.2011 GB 201111244; 30.06.2011 US 201161571663 P; 29.07.2011 US 201161513461 P; 29.07.2011 US 201113194866; 02.02.2012 US 201261594295 P
(62) Document number(s) of the earlier application(s) in accordance with Art. 76 EPC: 23205567.3 / 4 324 913; 17189470.2 / 3 318 627; 12729741.4 / 2 718 422
(71) Applicant: Koninklijke Nederlandse Akademie van Wetenschappen (KNAW), 3584 CT Utrecht (NL)
(72) Inventor: CLEVERS, Johannes Carolus, 3584 CT Utrecht (NL); SATO, Toshiro, 3584 CT Utrecht (NL); HUCH ORTEGA, Meritxell, 3584 CT Utrecht (NL); KARTHAUS, Wouter Richard, 3584 CT Utrecht (NL)
(74) Representative: Carpmaels & Ransford LLP

(57) **Abstract**

Culture media and methods for expanding and differentiating populations of stem cells and for obtaining organoids. Expanded cell populations and organoids obtainable by methods of the invention and their use in drug screening, toxicity assays and regenerative medicine.

## Description

All documents cited herein are incorporated by reference in their entirety.

### TECHNICAL FIELD

The invention is in the field of stem cell culture media and methods, in particular culture media and methods for expanding populations of stem cells, e.g. human epithelial stem cells.

### BACKGROUND

There is great interest in culture media and methods for expanding populations of stem cells. Populations of stem cells have many uses. For example, stem cells and their differentiated progeny can be used in cellular assays, drug screening, and toxicity assays. Stem cells also show promise for cell-based therapies, such as in regenerative medicine for the treatment of damaged tissue. They can also act as a source of differentiated cells for transplantation purposes e.g. transplantation of pancreatic beta-cells for treatment of diabetes etc. Furthermore, efficient cell culture media are important for providing and maintaining populations of cells for research purposes.

There is also interest in culture media and methods for culturing stem cells for the formation, maintenance and expansion of organoids, such as intestinal crypt-villus, gastric or pancreatic organoids. An organoid comprises stem cells, such as epithelial stem cells, which retain their undifferentiated phenotype and self-renewal properties but also have differentiating progeny that grow into tissue-like structures. Similarly to populations of related or identical cells, crypt-villus, gastric or pancreatic organoids, which more closely mimic the basic physiology of their tissue of origin, may be used in toxicity assays, or assays for drugs or food supplements. They may also be useful for culturing pathogens which currently lack suitable tissue culture or animal models. Furthermore, such organoids may be useful in regenerative medicine, for example in post-radiation and/or post-surgery repair of the intestinal epithelium, or in the repair of the intestinal epithelium in patients suffering from inflammatory bowel disease.

It is clear that there are many clinical and research applications for stem cells and their differentiated progeny. For all these applications, reproducible stem cell culture methods are of the utmost importance for providing adequate numbers of cells of suitable quality. For example, for effective drug screening, conditions must be carefully controlled requiring precise culture methods for controlling differentiation and proliferation of cells, so that pure populations of phenotypically and karyotypically identical cells can be generated. Similarly, for cell-based therapies, wherein cultured cells may be directly provided to patients, the cells must be genetically and phenotypically sound so as to avoid undesirable immune responses or cell fates when provided to the patient.

Although a variety of culture systems have been described for culturing primary epithelial stem cells, including intestinal epithelial stem cells (Bjerknes and Cheng, 2006. Methods Enzymol. 419: 337-83), to date, no long-term culture system has been established which maintains the differentiation potential and phenotypic and genomic integrity of human epithelial stem cells.

International patent application WO2010/090513 discloses a method for culturing epithelial stem cells or isolated tissue fragments. The method is optimised for the culturing of human colon and intestinal crypts by the addition of Wnt-3a to the medium. This was the first time that human intestinal stem cell cultures had been cultured for a prolonged period of time (up to 3 months) and provided the first reproducible human intestinal stem cell culture system. However, there is still a need for improved stem cell culture media and methods, in particular human stem cell culture media and methods, that improve proliferation rates, survival time and phenotypic and genomic integrity of stem cells grown in culture.

### SUMMARY OF THE INVENTION

The invention provides improved culture media and methods for stem cells, in particular human epithelial stem cells, and organoids comprising said stem cells, which provide significant advantages over known culture media and methods. The invention also provides related culture medium supplements, compositions and uses.

Accordingly, the invention provides a culture medium for expanding a population of stem cells, wherein the culture medium comprises at least one or more inhibitors that bind to and reduce the activity of one or more serine/threonine protein kinase targets. This has the effect of allowing continual growth for at least 3 months at an expansion rate of approximately five-fold expansion per week. The serine/threonine protein kinase is preferably selected from the group comprising: TGFbeta receptor kinase 1, ALK4, ALK5, ALK7, p38. Surprisingly, the inventors have found that the inclusion of inhibitors of certain serine/threonine kinases in culture media significantly improved the performance of the culture media in expanding a population of stem cells. The population of stem cells may be normal (healthy) cells or diseased cells (for example, cancer stem cells). Specifically, inhibitors of p38 and ALK were shown to provide the greatest improvement out of all the compounds tested. This is unexpected because there is no known mechanism predicting how these particular inhibitors might work. Indeed, several of the small molecule inhibitors that were chosen to be tested and function in similar pathways, had no effect on the method. Therefore, the skilled person could not have predicted that inhibitors of these particular kinases would have such a marked improvement on the culture medium. A still further improvement was observed when two inhibitors, for example a p38 inhibitor, such as SB202190 and an ALK inhibitor, such as A83-01, were added to the culture medium together.

To arrive at this realisation, the inventors investigated signalling pathways that are known to be subverted in certain cancers e.g. colorectal cancer. They hypothesised that these pathways, which affect cell fate in cancer, may also play a role in determining cell fate in culture conditions. It should be emphasised, however, that this hypothesis was entirely new; given the state of the art, there was no way to predict the effect of any of these additional compounds on the culture medium, and no particular expectation that any of these compounds might in fact have a beneficial effect.

In a first screening experiment, a series of vitamins, hormones and growth factors were tested in combination with standard stem cell culture media. Gastrin and nicotinamide were initially identified as resulting in significantly improved culture conditions. Incorporating these factors into the standard culture conditions, a second screening experiment was performed, in which small molecule inhibitors related to relevant signalling pathways, such as ERK, p38, JNK, PTEN, ROCK, and Hedgehog, were tested. These pathways were chosen because they were known to be subverted in certain cancers.

Previous attempts to culture human intestinal stem cells with previously described stem cell culture medium (comprising Epidermal Growth Factor (EGF or ("E"), Noggin ("N") and R-spondin ("R"), referred to herein as "ENR" medium) optimised with Wnt-3A ("W") (referred to herein as "WENR" medium), have resulted in the disintegration of most cells within 7 days, with very few cells surviving beyond 1 month. Such attempts have also been subject to slow proliferation times, chromosome irregularities and morphological changes from budding to cystic structures. By "cystic" it is meant that the organoid is mostly spherical. By "budding" it is meant that the organoid has multiple regions growing out of the basic structure. It is not necessarily always an advantage to have budding structures, although budding structures typically have a larger surface area and typically resemble the corresponding *in vivo* tissue more closely.

The inventors showed that the improved method allowed continual growth of the stem cells for at least seven months.

The new method also increased the speed of proliferation of the cells in the expanded population. This is clearly of great utility when growing cells for commercial and therapeutic purposes.

The new method also increased the quality of the cells in the expanded population. This is a great advantage because clinical and research applications for stem cells and their differentiated progeny require reproducible stem cell culture methods that provide populations of cells of high quality. Generally, *in vitro* expansion of stem cells aims to provide a population of cells which resemble their *in vivo* counterparts as closely as possible. This property is herein referred to as the "genomic and phenotypic integrity" of the cells.

For the first time, the inventors have discovered that it is possible to expand human epithelial stem cells in culture, without loss of genomic and phenotypic integrity, for at least 7 months (see Example 1). Under the improved culture conditions of the invention, human intestinal organoids displayed budding organoid structures, rather than the cystic structures seen under previous culture conditions. Metaphase spreads of organoids more than 3 months old consistently revealed 46 chromosomes in each of the 20 cells taken from three different donors. Furthermore, microarray analysis revealed that the stem cells in culture possessed similar molecular signatures to intestinal crypt cells including intestinal stem cell genes.

The inventors also demonstrated that the human intestinal organoids generated by media and methods of the present invention, mimicked *in vivo* cell fate decisions in response to external factors. For example, it has previously been shown that Notch inhibition in intestinal stem cells, terminates intestinal epithelial proliferation and induces goblet cell hyperplasia *in vivo.* The inventors were able to show that the intestinal organoids of the invention, when treated with a Notch inhibitor, ceased proliferation and most cells converted into goblet cells within 3 days.

Similar advantages were observed when including a TGF-beta inhibitor and/or a p38 inhibitor in culture media for expanding stem cells or organoids from other epithelial tissues, such as stomach, pancreas, liver and prostate (see the Examples). The tissues may be normal (healthy) tissues or diseased tissues, for example cancerous tissues or tissues showing a cystic fibrosis phenotype.

These results show the dramatic improvement in the genomic and phenotypic integrity of the stem cells and organoids produced by the methods and media of the present invention compared to previous methods and media.

Thus, the invention provides a culture medium for expanding and/or differentiating a population of adult stem cells, wherein said culture medium comprises:
i. any one of Rspondin 1-4 and/or an Rspondin mimic; and
ii. one or more inhibitor that directly or indirectly negatively regulates TGF-beta signalling.

The invention also provides a composition comprising a culture medium according to the invention and an extracellular matrix or a 3D matrix that mimics the extracellular matrix by its interaction with the cellular membrane proteins such as integrins, for example, a laminin-containing extracellular matrix such as Matrigel^{™} (BD Biosciences).

The invention also provides a hermetically-sealed vessel containing a culture medium or composition according to the invention.

The invention also provides the use of a culture medium according to the invention for expanding and/or differentiating a stem cell, population of stem cells, tissue fragment or organoid.

The invention also provides methods for expanding a single stem cell, a population of stem cells or a tissue fragment, preferably to generate an organoid, wherein the method comprises culturing the single stem cell or population of stem cells in a culture medium according to the invention.

The invention also provides organoids or populations of cells obtainable by the methods of the invention.

The invention also provides an organoid, preferably obtainable by the methods of the invention, which is a three-dimensional organoid comprising epithelial cells surrounding a central lumen, wherein optionally the epithelial cells exist in distinct dividing domains and differentiating domains.

The invention also provides an organoid, preferably obtainable by the methods of the invention, which is a three-dimensional organoid comprising epithelial cells arranged in regions of monolayers, optionally folded monolayers and regions of stratified cells, and preferably which is a three-dimensional organoid comprising epithelial cells surrounding a central lumen, wherein optionally the epithelial cells exist in distinct dividing domains and differentiating domains.

The invention also provides a composition comprising:
i) one or more organoids or population of cells of the invention; and
ii) a culture medium of the invention and/or an extracellular matrix.

The invention also provides an organoid, a population of cells or a composition according to the invention for use in drug screening, target validation, target discovery, toxicology, toxicology screens, personalized medicine, regenerative medicine or *ex vivo* cell/organ models, for example for use as a disease model.

The invention also provides an organoid, apopulation of cells or a composition according to the invention, for use in transplantation of said organoid, population of cells or composition into a mammal, preferably into a human.

The invention also provides a population of stem cells, or organoids comprising said stem cells, that have been obtained or are obtainable using the culture medium of the invention. The stem cells or organoids comprising said stem cells may be used, for example, for transplantation purposes or other therapeutic applications. For example,the stem cells or organoids comprising said stem cells may be used for drug screening, target validation, target discovery, toxicology and toxicology screens, personalized medicine, regenerative medicine and *ex vivo* cell/organ models, for example disease models.

The invention also provides compositions comprising a culture medium of the invention.

The invention also provides culture medium supplements comprising an inhibitor according to the invention.

The invention also provides a hermetically-sealed vessel comprising a culture medium and/or a culture medium supplement according to the invention.

The specific ingredients of the culture media, supplements and compositions of the invention can be varied according to particular needs and applications. Likewise, the precise steps of the methods of the invention can vary according to particular needs and applications.

The culture media, supplements, methods, compositions and uses according to this invention may also be optimised by routine experimentation. For example, if a culture medium, supplement or composition fails to give the desired level of stem cell expansion, variables such as the amount of each ingredient in the culture medium or supplement, seeding densities, culture conditions, culture periods, *etc.* can be altered in further experiments. The amount of each of the ingredients described herein can be optimised independently of the other ingredients by routine optimisation or one or more ingredients can be added or removed. A culture medium can be tested for its ability to support expansion of stem cells by testing it alongside or in place of a known culture medium or method.

The culture media, supplements, methods, compositions and uses of the invention are described in more detail below. The practice of the present invention will employ, unless otherwise indicated, conventional techniques of cell culture, molecular biology and microbiology, which are within the skill of those working in the art.

Numerous textbooks are available that provide guidance on mammalian cell culture media and methods, including textbooks dedicated to culture media and methods for culturing stem cells. Such textbooks include 'Basic Cell Culture Protocols' by J. Pollard and J. M. Walker (1997), 'Mammalian Cell Culture: Essential Techniques' by A. Doyle and J. B. Griffiths (1997), 'Culture of Animal Cells: A Manual of Basic Technique' by R. I. Freshney (2005), 'Basic Cell Culture Protocols' by C. Helgason and C. L. Miller (2005), 'Stem Cells: From Bench to Bedside' by A. Bongso (2005), 'Human Stem Cell Manual: A Laboratory Guide' by J. F. Loring, R. L. Wesselschmidt and P. H. Schwartz (2007).

Stem cells and cell culture reagents and apparatus for use in the invention are available commercially, e.g. from Cellartis AB (Göteborg, Sweden), VitroLife AB (Kungsbacka, Sweden), GIBCO^{®} (Invitrogen), Millipore Corporation (Billerica, Massachusetts), Sigma^{®} (St. Louis, Missouri) and Biomol International L.P. (Exeter, UK).

### DETAILED DESCRIPTION

According to the invention, there is provided a culture medium for expanding a population of stem cells, wherein the culture medium comprises at least one or more inhibitors that bind to and reduce the activity of one or more serine/threonine protein kinase targets, wherein the culture medium has the effect of allowing continual growth of the population of stem cells for at least 3 months, preferably at least 4 months, at least 5 months, at least 6 months, at least 7 months, at least 9 months, or at least 12 months or more.

### Inhibitors

A culture medium used according to a first aspect of the invention comprises any inhibitor that, directly or indirectly, negatively regulates TGF-beta or p38 signalling. In a preferred embodiment the culture medium of the invention comprises an inhibitor that directly or indirectly negatively regulates TGF-beta signalling. In some embodiments the culture medium of the invention comprises an inhibitor that directly or indirectly negatively regulates TGF-beta and an inhibitor that directly or indirectly negatively regulates p38 signalling. In a further embodiment, the culture medium of the invention additionally comprises Rspondin or an Rspondin mimic.

The one or more inhibitor preferably targets a serine/threonine protein kinase selected from the group comprising: TGF-beta receptor kinase 1, ALK4, ALK5, ALK7, p38. An inhibitor of any one of these kinases is one that effects a reduction in the enzymatic activity of any one (or more) of these molecules. Inhibition of ALK and p38 kinase has previously been shown to be linked in B-cell lymphoma (Bakkebø M Huse K, Hilden VI, Smeland EB, Oksvold MP, "TGF-beta-induced growth inhibition in B-cell lymphoma correlates with Smad1/5 signalling and constitutively active p38 MAPK", BMC Immunol. 11:57, 2010). In this publication, it was found that TGF-beta sensitive cell lines expressed higher cell surface levels of ALK-5 and that constitutive phosphorylation of p38 was restricted to the TGF-beta sensitive cell lines. Inhibition of p38 MAPK led to reduced sensitivity to TGF-beta suggesting that phosphorylation of Smad1/5 is important for the anti-proliferative effects of TGF-beta in B-cell lymphoma. The results indicate a role for p38 MAPK in the regulation of TGF-beta-induced anti-proliferative effects.

Without wishing to be bound by theory, the present inventors propose that ALK and p38 belong to a pathway that negatively regulates long-term maintenance of stem cells, in particular, human epithelial stem cells. The inventors hypothesise that inhibitors that act at any level on this pathway, including, for example, by inhibiting Smad1/5 signalling, would also be beneficial for stem cell culture. Smads play a key role in TGF-beta signalling.

In some embodiments an inhibitor of the invention binds to and reduces the activity serine/threonine protein kinase selected from the group comprising: TGF-beta receptor kinase 1, ALK4, ALK5, ALK7, p38.

In some embodiments of the invention, the culture medium comprises a TGF-beta inhibitor, meaning any inhibitor that, directly or indirectly, negatively regulates TGF-beta signalling. In some embodiments, a culture medium of the invention comprises one or more TGF-beta inhibitor that binds to and reduces the activity of one or more serine/threonine protein kinases selected from the group consisting of ALK5, ALK4, TGF-beta receptor kinase 1 and ALK7.

ALK4, ALK5 and ALK7 are all closely related receptors of the TGF-beta superfamily. ALK4 has GI number 91; ALK5 (also known as TGF-beta receptor kinase 1) has GI number 7046; and ALK7 has GI number 658. In one embodiment, an inhibitor according to the invention binds to and reduces the activity of ALK4, ALK5 (TGF-beta receptor kinase 1) and/or ALK7. In another embodiment, the TGF-beta receptor binds to and reduces the activity of a Smad protein, for example R-SMAD or SMAD1-5 (i.e. SMAD 1, SMAD 2, SMAD 3, SMAD 4 or SMAD 5). In a preferred embodiment, the culture medium of the invention comprises an inhibitor of ALK5.

Various methods for determining if a substance is a TGF-beta inhibitor are known. For example, a cellular assay may be used, in which cells are stably transfected with a reporter construct comprising the human PAI-1 promoter or Smad binding sites, driving a luciferase reporter gene. Inhibition of luciferase activity relative to control groups can be used as a measure of compound activity (De Gouville et al., Br J Pharmacol. 2005 May; 145(2): 166-177). Another example is the AlphaScreen^{®} phosphosensor assay for measurement of kinase activity (Drew A E et al., Comparison of 2 Cell-Based Phosphoprotein Assays to Support Screening and Development of an ALK Inhibitor J Biomol Screen. 16(2) 164-173, 2011).

Various TGF-beta inhibitors are known in the art (for example, see Table 1). In some embodiments the inhibitor that directly or indirectly negatively regulates TGF-beta signalling is selected from the group consisting of A83-01, SB-431542, SB-505124, SB-525334, SD-208, LY-36494 and SJN-2511.

In some embodiments of the invention, the culture medium comprises a p38 inhibitor, meaning any inhibitor that, directly or indirectly, negatively regulates p38 signalling. In some embodiments, an inhibitor according to the invention binds to and reduces the activity of p38 (GI number 1432). p38 protein kinases are part of the family of mitogen-activated protein kinases (MAPKs). MAPKs are serine/threonine-specific protein kinases that respond to extracellular stimuli, such as environmental stress and inflammatory cytokines, and regulate various cellular activities, such as gene expression, mitosis, differentiation, proliferation, and cell survival/apoptosis. The p38 MAPKs exist as α, β, β2, γ and δ isoforms. A p38 inhibitor is an agent that binds to and reduces the activity of at least one p38 isoform. Various methods for determining if a substance is a p38 inhibitor are known, and might be used in conjunction with the invention. Examples include: phospho-specific antibody detection of phosphorylation at Thr180/Tyr182, which provides a well-established measure of cellular p38 activation or inhibition; biochemical recombinant kinase assays; tumor necrosis factor alpha (TNFα) secretion assays; and DiscoverRx high throughput screening platform for p38 inhbitors (see http://www.discoverx.com/kinases/literature/biochemical/collaterals/DRx_poster_p38%20KBA. pdf). Several p38 activity assay kits also exist (e.g. Millipore, Sigma-Aldrich).

The inventors hypothesise that in some embodiments, high concentrations (e.g. more than 100 nM, or more than 1uM, more than 10 uM, or more than 100 uM) of a p38 inhibitor may have the effect of inhibiting TGF-beta. However, the inventors do not wish to be constrained by this hypothethis and in other emobodiments, the p38 inhibitor does not inhibit TGF-beta signalling.

Various p38 inhibitors are known in the art (for example, see Table 1). In some embodiments, the inhibitor that directly or indirectly negatively regulates p38 signalling is selected from the group consisting of SB-202190, SB-203580, VX-702, VX-745, PD-169316, RO-4402257 and BIRB-796.In a further embodiment of the invention, the culture medium comprises both: a) an inhibitor that binds to and reduces the activity of any one or more of the kinases from the group consisting of: ALK4, ALK5 and ALK7; and b) an inhibitor that binds to and reduces the activity of p38. In a preferred embodiment, the culture medium comprises an inhibitor that binds to and reduces the activity of ALK5 and an inhibitor that binds to and reduces the activity of p38.

In one embodiment, an inhibitor according to the invention binds to and reduces the activity of its target (for example, TGF-beta or p38) by more than 10%; more than 30%; more than 60%; more than 80%; more than 90%; more than 95%; or more than 99% compared to a control, as assessed by a cellular assay. Examples of cellular assays for measuring target inhibition are well known in the art as described above.

An inhibitor according to the invention may have an IC50 value equal to or less than 2000nM; less than 1000nM; less than 100nM; less than 50nM; less than 30nM; less than 20nM or less than 10nM. The IC50 value refers to the effectiveness of an inhibitor in inhibiting its target's biological or biochemical function. The IC50 indicates how much of a particular inhibitor is required to inhibit a kinase by 50%. IC50 values can be calculated in accordance with the assay methods set out above.

An inhibitor according to the invention may act competitively, non-competitively, uncompetitively or by mixed inhibition. For example, in certain embodiments, an inhibitor may be a competitive inhibitor of the ATP binding pocket of the target kinase.

Inhibitors according to the invention may exist in various forms, including natural or modified substrates, enzymes, receptors, small organic molecules, such as small natural or synthetic organic molecules of up to 2000Da, preferably 800Da or less, peptidomimetics, inorganic molecules, peptides, polypeptides, antisense oligonucleotides aptamers, and structural or functional mimetics of these including small molecules. The inhibitor according to the invention may also be an aptamer. As used herein, the term "aptamer" refers to strands of oligonucleotides (DNA or RNA) that can adopt highly specific three-dimensional conformations. Aptamers are designed to have high binding affinities and specificities towards certain target molecules, including extracellular and intracellular proteins.

For example, the inhibitor may be a small synthetic molecule with a molecular weight of between 50 and 800 Da, between 80 and 700 Da, between 100 and 600 Da or between 150 and 500 Da.

In some embodiments, the small-molecule inhibitor comprises a pyridinylimidazole or a 2,4-disubstituted pteridine or a quinazoline, for example comprises:

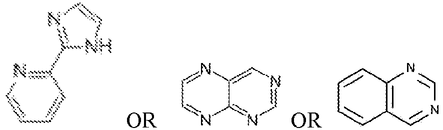

Particular examples of inhibitors that may be used in accordance with the invention include, but are not limited to: SB-202190, SB-203580, SB-206718, SB-227931, VX-702, VX-745, PD-169316, RO-4402257, BIRB-796, A83-01 SB-431542, SB-505124, SB-525334, LY 364947, SD-208, SJN 2511 (see table 1). A culture medium of the invention may comprise one or more of any of the inhibitors listed in table 1. A culture medium of the invention may comprise any combination of one inhibitor with another inhibitor listed. For example, a culture medium of the invention may comprise SB-202190 or SB-203580 or A83-01; or a culture medium of the invention may comprise SB-202190 and A83-01; or a culture medium of the invention may comprise SB-203580 and A83-01. The skilled person will appreciate that other inhibitors and combinations of inhibitors which bind to and reduce the activity of the targets according to the invention, may be included in a culture medium or a culture medium supplement in accordance with the invention.

Inhibitors according to the invention may be added to the culture medium to a final concentration that is appropriate, taking into account the IC50 value of the inhibitor.

For example, SB-202190 may be added to the culture medium at a concentration of between 50 nM and 100 uM, or between 100 nM and 50 uM, or between 1 uM and 50 uM. For example, SB-202190 may be added to the culture medium at approximately 10 uM.

SB-203580 may be added to the culture medium at a concentration of between 50 nM and 100 uM, or between 100 nM and 50 uM, or between 1 uM and 50 uM. For example, SB-203580 may be added to the culture medium at approximately 10 uM.

VX-702 may be added to the culture medium at a concentration of between 50 nM and 100 uM, or between 100 nM and 50 uM, or between 1 uM and 25 uM. For example, VX-702 may be added to the culture medium at approximately 5 uM.

VX-745 may be added to the culture medium at a concentration of between 10 nM and 50 uM, or between 50 nM and 50 uM, or between 250 nM and 10 uM. For example, VX-745 may be added to the culture medium at approximately 1 uM.

PD-169316 may be added to the culture medium at a concentration of between 100 nM and 200 uM, or between 200 nM and 100 uM, or between 1 uM and 50 uM. For example, PD-169316 may be added to the culture medium at approximately 20 uM.

RO-4402257 may be added to the culture medium at a concentration of between 10 nM and 50 uM, or between 50 nM and 50 uM, or between 500 nM and 10 uM. For example, RO-4402257 may be added to the culture medium at approximately 1 uM.

BIRB-796 may be added to the culture medium at a concentration of between 10 nM and 50 uM, or between 50 nM and 50 uM, or between 500 nM and 10 uM. For example, BIRB-796 may be added to the culture medium at approximately 1 uM.

A83-01 may be added to the culture medium at a concentration of between 10 nM and 10 uM, or between 20 nM and 5 uM, or between 50 nM and 1 uM. For example, A83-01 may be added to the culture medium at approximately 500 nM.

SB-431542 may be added to the culture medium at a concentration of between 80 nM and 80 uM, or between 100 nM and 40 uM, or between 500 nM and 10 uM. For example, SB-431542 may be added to the culture medium at approximately 1 uM.

SB-505124 may be added to the culture medium at a concentration of between 40 nM and 40 uM, or between 80 nM and 20 uM, or between 200 nM and 1 uM. For example, SB-505124 may be added to the culture medium at approximately 500 nM.

SB-525334 may be added to the culture medium at a concentration of between 10 nM and 10 uM, or between 20 nM and 5 uM, or between 50 nM and 1 uM. For example, SB-525334 may be added to the culture medium at approximately 100 nM.

LY 36494 may be added to the culture medium at a concentration of between 40 nM and 40 uM, or between 80 nM and 20 uM, or between 200 nM and 1 uM. For example, LY 36494 may be added to the culture medium at approximately 500 nM.

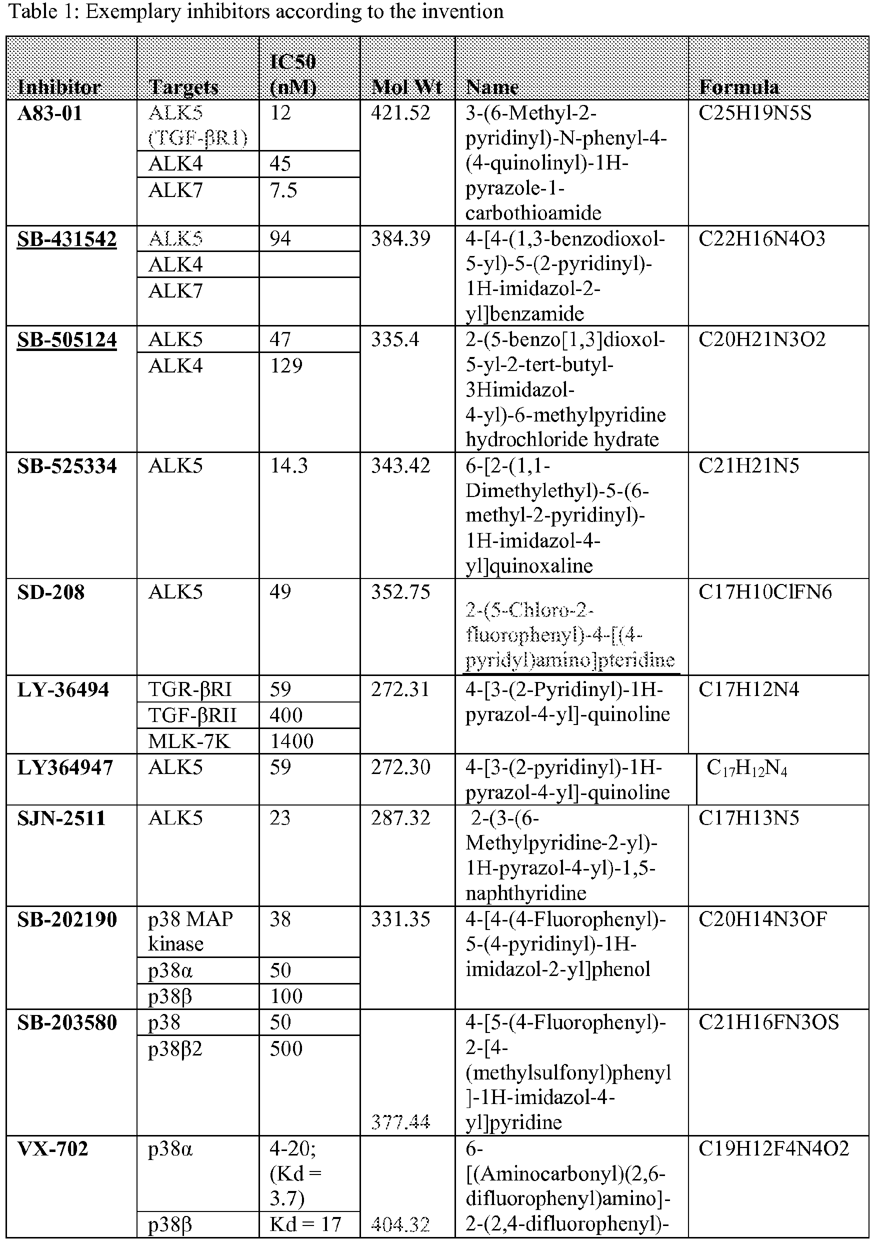

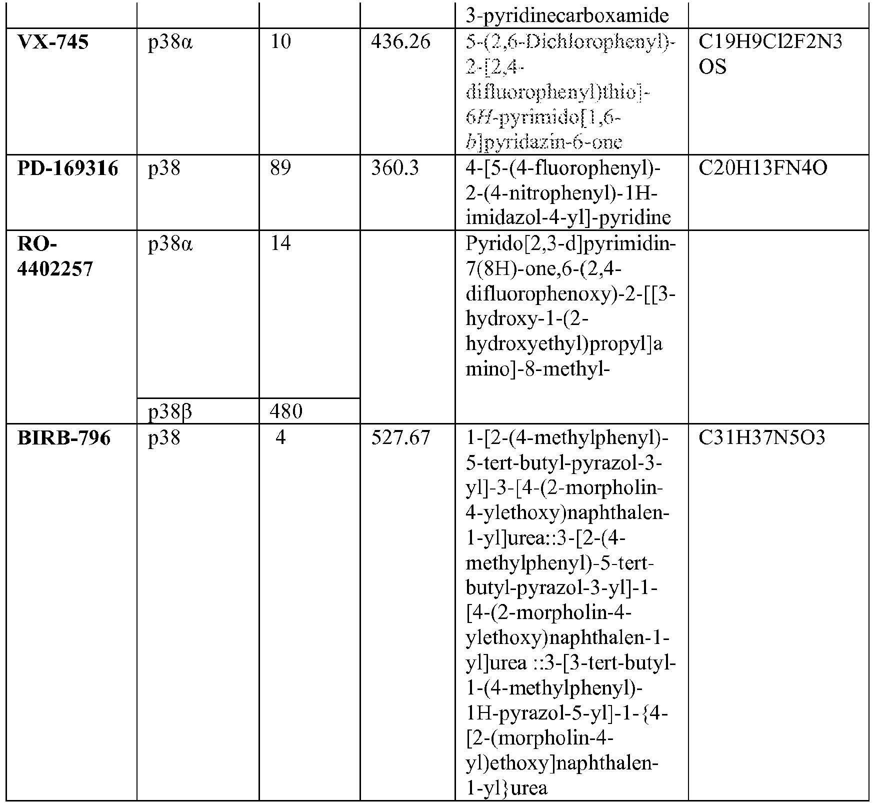

SD-208 may be added to the culture medium at a concentration of between 40 nM and 40 uM, or between 80 nM and 20 uM, or between 200 nM and 1 uM. For example, SD-208 may be added to the culture medium at approximately 500 nM.

LY364947 may be added to the culture medium at a concentration of between 40 nM and 40 uM, or between 80 nM and 20 uM, or between 200 nM and 1 uM. For example, LY364947 may be added to the culture medium at approximately 500 nM.

SJN 2511 may be added to the culture medium at a concentration of between 20 nM and 20 uM, or between 40 nM and 10 uM, or between 100 nM and 1 uM. For example, SJN 2511 may be added to the culture medium at approximately 200 nM.

Thus, in some embodiments the inhibitor that directly or indirectly, negatively regulates TGF-beta or p38 signalling is added to the culture medium at a concentration of between 1nM and 100 µM, between 10 nM and 100 µM, between 100 nM and 10 µM, or approximately 1 µM, for example, wherein the total concentration of the one or more inhibitor is between 10 nM and 100 µM, between 100 nM and 10 µM, or approximately 1 µM.

Additionally to the inhibitor, cell culture media generally contain a number of components which are necessary to support maintenance and/or expansion of the cultured cells. A cell culture medium of the invention will therefore normally contain many other components in addition to an inhibitor according to the invention. Suitable combinations of components can readily be formulated by the skilled person, taking into account the following disclosure. A culture medium according to the invention will generally be a nutrient solution comprising standard cell culture components, such as amino acids, vitamins, inorganic salts, a carbon energy source, and a buffer as described in more detail below. Other standard cell culture components that may be included in the culture include hormones, such as progesterone, proteins, such as albumin, catalase, insulin and transferrin. These other standard cell culture components make up the "basal" culture medium.

A culture medium according to the invention may be generated by modification of an existing cell culture medium. The skilled person will understand from common general knowledge the types of culture media that might be used for stem cell culture. Potentially suitable cell culture media are available commercially, and include, but are not limited to, Dulbecco's Modified Eagle Media (DMEM), Minimal Essential Medium (MEM), Knockout-DMEM (KO-DMEM), Glasgow Minimal Essential Medium (G-MEM), Basal Medium Eagle (BME), DMEM/Ham's F12, Advanced DMEM/Ham's F12, Iscove's Modified Dulbecco's Media and Minimal Essential Media (MEM), Ham's F-10, Ham's F-12, Medium 199, and RPMI 1640 Media. Thus, in some embodiments, one of these pre-existing cell culture media is used as the basal culture medium to which is added the inhibitor that, directly or indirectly, negatively regulates TGF-beta or p38 signalling, and, optionally, to which is added one or more other components as described herein.

In some embodiments, the culture medium of the invention comprises one or more additional components selected from: a BMP inhibitor, a Wnt agonist, a receptor tyrosine kinase ligand, a Rock inhibitor, nicotinamide and gastrin. In some embodiments, the culture medium of the invention comprises any one of Rspondin 1-4 and/or an Rspondin mimic, a TGF-beta inhibitor, a BMP inhibitor (for example, Noggin) and a Wnt agonist (for example, Wnt(3a)).

In some embodiments, the culture medium of the invention comprises any one of Rspondin 1-4 and/or an Rspondin mimic, a BMP inhibitor (for example, Noggin), a TGF-beta inhibitor, a receptor tyrosine kinase ligand (for example, EGF), Nicotinamide, a Wnt agonist (for example, Wnt(3a)), and optionally one or more additional components selected from: a p38 inhibitor, gastrin, FGF10, HGF and a Rock inhibitor. The optional additional components may be added for optimisation of the culture medium for culturing cells originating from particular tissues, as explained in more detail later on.

The culture media of the invention may comprise one or more bone morphogenetic protein (BMP) inhibitor. BMP ligands signal as dimers by assembling a quadripartite transmembrane serine/threonine kinase receptor complex consisting of two type I and two type II receptors. Complex assembly initiates a phosphorylation cascade activating the BMP responsive Smads1/5/8 and resulting in changes in transcriptional activity. Advantageously, the present inventors show that BMP inhibitors promote expression of Lgr5, and so the presence of a BMP inhibitor in a culture medium of the invention will likely result in more proliferative organoids than if the BMP inhibitor is absent (for example, see Example 3). Thus, BMP inhibitors are an advantageous component of expansion media of the invention. Thus, the use of a BMP inhibitor is advantageous in the use of an expansion medium when it is desirable to culture the cells for at least 3 months (e.g. at least 4, 5, 6, 7, 8 or 9 months) without the cells differentiating.

Several classes of natural BMP-binding proteins are known, including Noggin (Peprotech), Chordin and chordin-like proteins (R&D systems) comprising chordin domains, Follistatin and follistatin-related protines (R&D systems) comprising a follistatin domain, DAN and DAN-like proteins (R&D systems) comprising a DAN cystein-knot domain, sclerostin/ SOST (R&D systems) and apha-2 macroglobulin (R&D systems). A BMP inhibitor is an agent that binds to a BMP molecule to form a complex wherein the BMP activity is reduced, for example by preventing or inhibiting the binding of the BMP molecule to a BMP receptor. Alternatively, the inhibitor may be an agent that binds to a BMP receptor and prevents binding of a BMP ligand to the receptor, for example, an antibody that binds the receptor. A BMP inhibitor may be a protein or small molecule and may be naturally occurring, modified, and/or partially or entirely synthetic. A BMP inhibitor of a culture medium of the invention may be Noggin, DAN, or DAN-like proteins including Cerberus and Gremlin (R&D systems). These diffusible proteins are able to bind a BMP ligand with varying degrees of affinity and inhibit their access to signalling receptors. A preferred BMP inhibitor for use in a culture medium of the invention is Noggin. Noggin may be used at any suitable concentration. In some embodiments, a basal medium of the culture medium of the invention may comprise between about 10 ng/ml and about 100 ng/ml of Noggin. For example, a culture medium may comprise at least 10 ng/ml of Noggin, at least 20 ng/ml of Noggin, at least 50 ng/ml of Noggin, at least 100 ng/ml of Noggin, approximately 100 ng/ml of Noggin or 100 ng/ml of Noggin. In some embodiments, a culture medium may comprise less than 200 ng/ml of Noggin, less than 150 ng/ml of Noggin, less than 100 ng/ml of Noggin, less than 75 ng/ml of Noggin, less than 50 ng/ml of Noggin or less than 30 ng/ml of Noggin. The BMP inhibitor may be added to the culture medium every second day during culturing, or every day during culturing, or every third day, every fourth day, every fifth day or as required. BMP inhibitors are particularly advantageous components of the expansion media, for example for expanding pancreas, small intestine, colon, liver, prostate stem cells. However, Noggin has been shown to prevent some differentiation (for example, see example 3). Therefore, in some embodiments a BMP inhibitor is excluded from a differentiation medium of the invention.

In some embodiments, cells cultured with a BMP inhibitor have upregulated expression of Lgr5 compared to cells cultured without a BMP inhibitor. Therefore, addition of a BMP inhibitor typically results in more proliferative organoids. This is surprising, because in the literature it is described that BMP activity is useful for the differentiation of pancreatic cells into both the ductal (see keratin7 and 19 expression) and endocrine cells. Thus, the skilled person would expect the inclusion of a BMP inhibitor, such as Noggin, to decrease proliferation and to increase differentiation. However, the inventors surprisingly found that the use of a BMP inhibitor was advantageous because it resulted in more proliferative organoids and higher expression of Lgr5.The culture media of the invention may comprise one or more Wnt agonist. The Wnt signalling pathway is defined by a series of events that occur when a Wnt protein binds to a cell-surface receptor of a Frizzled receptor family member. This results in the activation of Dishevelled family proteins which inhibit a complex of proteins that includes axin, GSK-3, and the protein APC to degrade intracellular beta-catenin. The resulting enriched nuclear beta-catenin enhances transcription by TCF/LEF family transcription factors. A Wnt agonist is defined as an agent that activates TCF/LEF-mediated transcription in a cell. Wnt agonists are therefore selected from true Wnt agonists that bind and activate a Frizzled receptor family member including any and all of the Wnt family proteins, an inhibitor of intracellular beta-catenin degradation, and activators of TCF/LEF. Said Wnt agonist stimulates a Wnt activity in a cell by at least 10%, more preferred at least 20%, more preferred at least 30%, more preferred at least 50%, more preferred at least 70%, more preferred at least 90%, more preferred at least 100%, relative to a level of said Wnt activity in the absence of said molecule. As is known to a skilled person, a Wnt activity can be determined by measuring the transcriptional activity of Wnt, for example by pTOPFLASH and pFOPFLASH Tcf luciferase reporter constructs (Korinek et al, 1997 Science 275 1784-1787).

In some embodiments, a Wnt agonist comprises a secreted glycoprotein including Wnt- l/Int-1, Wnt- 2/Irp (InM -related Protein), Wnt-2b/13, Wnt-3/Int-4, Wnt-3a (R&D sytems), Wnt- 4, Wnt-5a, Wnt-5b, Wnt-6 (Kirikoshi H et al 2001 Biochem Biophys Res Com 283 798-805), Wnt-7a (R&D systems), Wnt-7b, Wnt-8a/8d, Wnt-8b, Wnt-9a/14, Wnt- 9b/14b/15, Wnt-10a, Wnt-10b/12, WnM 1, and Wnt-16. An overview of human Wnt proteins is provided in "THE WNT FAMILY OF SECRETED PROTEINS", R&D Systems Catalog, 2004. Further Wnt agonists include the R-spondin family of secreted proteins, which is implicated in the activation and regulation of Wnt signaling pathway and which is comprised of 4 members (R-spondin 1 (NU206, Nuvelo, San Carlos, CA), R-spondin 2 (R&D systems), R-spondin 3, and R-spondin-4), and Norrin (also called Nome Disease Protein or NDP) (R&D systems), which is a secreted regulatory protein that functions like a Wnt protein in that it binds with high affinity to the Frizzled-4 receptor and induces activation of the Wnt signaling pathway (Kestutis Planutis et al (2007) BMC Cell Biol 8 12). In some embodiments, one or more Wnt agonists for use in the invention is an R-spondin mimic, for example an agonist of Lgr5 such as an anti-Lgr5 antibody. A small-molecule agonist of the Wnt signaling pathway, an aminopyrimidine derivative, was recently identified and is also expressly included as a Wnt agonist (Lm et al (2005) Angew Chem Int Ed Engl 44, 1987-90).

In some embodiments, the Wnt agonist is a GSK-inhibitor. Known GSK-inhibitors comprise small-interfering RNAs (siRNA, Cell Signaling), lithium (Sigma), kenpaullone (Biomol International, Leost, M et al (2000) Eur J Biochem 267, 5983-5994), 6-Bromoindirubin-30-acetoxime (Meyer, L et al (2003) Chem Biol 10, 1255-1266), SB 216763 and SB 415286 (Sigma-Aldrich), and FRAT-family members and FRAT-derived peptides that prevent interaction of GSK-3 with axin. An overview is provided by Meijer et al , (2004) Trends in Pharmacological Sciences 25, 471-480, which is hereby incorporated by reference. Methods and assays for determining a level of GSK-3 inhibition are known to a skilled person and comprise, for example, the methods and assay as described in Liao et al 2004, Endocrinology, 145(6) 2941-2949.

In some embodiments, the Wnt agonist is an inhibitor of RNF43 or ZNRF3. The inventors have discovered that RNF43 and ZNRF3 reside in the cell membrane and negatively regulate levels of the Wnt receptor complex in the membrane, probably by ubiquitination of Frizzled. Therefore, the inventors hypothesise that inhibition of RNF43 or ZNRF3 with antagonistic antibodies, RNAi or small molecule inhibitors would indirectly stimulate the Wnt pathway. RNF43 and ZNRF3 have a catalytic ring domain (with ubiquitination activity), which can be targeted in small molecule inhibitor design. Several anti-RNF43 antibodies and several anti-ZNRF3 antibodies are available commercially. In some embodiments, such antibodies are suitable Wnt agonists in the context of the invention.

In some embodiments, said Wnt agonist is selected from the group consisting of Wnt-3a, a GSK-inhibitor (such as CHIR99021), Wnt 5, Wnt-6a, Norrin, and any other Wnt family protein.

In some embodiments, said Wnt agonist comprises or consists of any one of Rspondin 1, Rspondin 2, Rspondin 3 or Rspondin 4. In a preferred embodiment, said Wnt agonist is selected from one or more of a Wnt family member, R-spondin 1-4, Norrin, and a GSK-inhibitor. In some embodiments, said Wnt agonist is a GSK-3 inhibitor, such as CHIR99021 (Stemgent 04-0004).

In some embodiments, CHIR99021 is added to the culture medium to a final concentration of between 50 nM and 100 uM, for example between 100 nM and 50 uM, between 1 uM and 10 uM, between 1 uM and 5 uM, or 3 uM. In some embodiments in which a GSK-3 inhibitor is used, the GSK-3 inhibitor is not BIO (6-bromoindirubin-3'-oxime, Stemgent 04-0003). It was found by the inventors that the addition of at least one Wnt agonist to the basal culture medium is essential for proliferation of the epithelial stem cells or isolated crypts.

In a further preferred embodiment, said Wnt agonist comprises or consists of R-spondin 1 or R-spondin-4. R-spondin 1, R-spondin 2, R-spondin 3 or R-spondin 4 is preferably added to the basal culture medium at a concentration of at least 50 ng/ml, more preferred at least 100 ng/ml, more preferred at least 200 ng/ml, more preferred at least 300 ng/ml, more preferred at least 500 ng/ml. A most preferred concentration of R-spondin 1, R-spondin 2, R-spondin 3 or R-spondin 4 is approximately 500 ng/ml or 500 ng/ml. In some embodiments, R-spondin 1, R-spondin 2, R-spondin 3 or R-spondin 4 is added to the culture medium at a concentration of at least 500 ng/ml, at least 600 ng/ml, at least 700 ng/ml, at least 800 ng/ml, at least 900 ng/ml, at least 1 ug/ml, at least 1.5 ug/ml or at least 2 ug/ml. In another preferred embodiment, R-spondin 1, R-spondin 2, R-spondin 3 or R-spondin 4 is added to the culture medium at a concentration of approximately 1 ug/ml or 1 ug/ml. In some embodiments, R-spondin 1, R-spondin 2, R-spondin 3 or R-spondin 4 is added to the basal culture medium at a concentration of less than 1000 ng/ml, for example, less than 800 ng/ml, less than 600 ng/ml, less than 550 ng/ml, less than 500 ng/ml, less than 400 ng/ml, less than 300 ng/ml or less than 200 ng/ml, or less than 100 ng/ml. In some embodiments, two or more (e.g. 2, 3 or 4) of Rspondin 1, Rspondin 2, Rspondin 3 and Rspondin 4 ("Rspondin 1-4") are added to the medium. Preferably, when two or more of Rspondin 1-4 are added, the total concentration of Rspondin amounts to the concentrations described above. Where culture media described herein are said to comprise "Rspondin 1-4", it is meant that the medium comprises any one or more of Rspondin 1, Rspondin 2, Rspondin 3 and Rspondin 4.Where culture media described herein are said to comprise "Rspondin", it is meant that the medium comprises any one or more of Rspondin 1, Rspondin 2, Rspondin 3, Rspondin 4 and an Rspondin mimic.

During culturing of stem cells, said Wnt family member is preferably added to the culture medium every second day, while the culture medium is refreshed preferably every fourth day.

In a preferred embodiment, a Wnt agonist is selected from the group consisting of R-spondin, Wnt-3a and Wnt-6. More preferably, R-spondin and Wnt-3a are both used as Wnt agonist. This combination is particularly preferred since this combination surprisingly has a synergistic effect on organoid formation. Preferred concentrations are approximately 500 ng/ml or 500 ng/ml for R-spondin and approximately 100 ng/ml or 100 ng/ml for Wnt3a.

The culture media of the invention may comprise one or more receptor tyrosine kinase ligands. An example of a receptor tyrosine kinase ligand for use in the invention is EGF, which is the ligand for the receptor tyrosine kinase EGFR. Many receptor tyrosine kinase ligands are also mitogenic growth factors.

The culture media of the invention may comprise one or more mitogenic growth factor. The one or more mitogenic growth factor may be selected from a family of growth factors comprising epidermal growth factor (EGF, Peprotech), Transforming Growth Factor-alpha (TGF-alpha, Peprotech), basic Fibroblast Growth Factor (bFGF, Peprotech), brain-derived neurotrophic factor (BDNF, R&D Systems), and Keratinocyte Growth Factor (KGF, Peprotech). EGF is a potent mitogenic factor for a variety of cultured ectodermal and mesodermal cells and has a profound effect on the differentiation of specific cells *in vivo* and *in vitro* and of some fibroblasts in cell culture. The EGF precursor exists as a membrane-bound molecule which is proteolytically cleaved to generate the 53-amino acid peptide hormone that stimulates cells. A preferred mitogenic growth factor is EGF. EGF is preferably added to the basal culture medium at a concentration of between 5 and 500 ng/ml or of at least 5 and not higher than 500 ng/ml. A preferred concentration is at least 10, 20, 25, 30, 40, 45, or 50 ng/ml and not higher than 500, 450, 400, 350, 300, 250, 200, 150, or 100 ng/ml. A more preferred concentration is at least 50 and not higher than 100 ng/ml. An even more preferred concentration is about 50 ng/ml or 50 ng/ml. The same concentrations could be used for a FGF, preferably for FGF10 or FGF7. If more than one FGF is used, for example FGF7 and FGF10, the concentration of a FGF is as defined above and refers to the total concentration of FGF used. During culturing of stem cells, said mitogenic growth factor is preferably added to the culture medium every second day, while the culture medium is refreshed preferably every fourth day. Any member of the FGF family may be used. Preferably, FGF7 and/or FGF10 is used FGF7 is also known as KGF (Keratinocyte Growth Factor). In a further preferred embodiment, a combination of mitogenic growth factors such as, for example, EGF and KGF, or EGF and BDNF, is added to the basal culture medium. In a further preferred embodiment, a combination of mitogenic growth factors such as, for example, EGF and KGF, or EGF and FGF10, is added to the basal culture medium. The mitogenic growth factor may be added to a culture media at a concentration of between 5 and 500 nanogram/ml or at least 5 and not more than 500 nanogram/ml, for example at least 10, 20, 25, 30, 40, 45, or 50 ng/ml and not higher than 500, 450, 400, 350, 300, 250, 200, 150, or 100 ng/ml. The mitogenic growth factor may be selected from the group consisting of EGF, TGF-alpha, KGF, FGF7 and FGF. Preferably, a mitogenic factor is selected from the groups consisting of EGF, TGF-alpha and KGF or from EGF, TGF-alpha and FGF7 or from EGF, TGF-alpha and FGF or from EGF and KGF or from EGF and FGF7 or from EGF and a FGF or from TGF-alpha and KGF or from TGF-alpha and FGF7 or from TGF-alpha and a FGF. EGF may be replaced by TGF-alpha. In some embodiments, the mitogenic growth factor is hepatocyte growth factor (HGF). In some embodiments, HGF is added to the culture medium.

In some embodiments, the receptor tyrosine kinase ligand is a mitogenic growth factor, for example selected from a family of growth factors consisting of epidermal growth factor (EGF), Transforming Growth Factor-alpha (TGF-alpha), basic Fibroblast Growth Factor (bFGF), brain-derived neurotrophic factor (BDNF), Hepatocyte growth factor (HGF) and Keratinocyte Growth Factor (KGF).

ROCK inhibitors, such as Y-27632 (10 µM; Sigma), can be included in any of the media described, in particular in the first few days of culture before performing cell sorting experiments, because it is known to avoid anoikis (a form of programmed cell death which is induced by anchorage-dependent cells detaching from the surrounding extracellular matrix). Therefore, any of the media defined herein, may additionally comprise a ROCK inhibitor for the first few days. In some embodiments, the culture media of the invention additionally comprises a ROCK inhibitor, such as Y-27632, for example for the first few days of culture before performing cell sorting experiments.

A further embodiment of a method according to the invention comprises a culture medium comprising a Rock (Rho-kinase) inhibitor. The addition of a Rock inhibitor was found to prevent anoikis, especially when culturing single stem cells. Said Rock inhibitor is preferably selected from R-(+)-trans-4-(1-aminoethyl)-N-(4-Pyridyl)cyclohexanecarboxamide dihydrochloride monohydrate (Y-27632, Sigma- Aldrich), 5-(l ,4-diazepan- l-ylsulfonyl)isoquinoline (fasudil or HA1077, Cayman Chemical), and (S)-(+)-2-methyl- l-[(4-methyl-5-isoquinolinyl)sulfonyl] - hexahydro-1H- 1,4-diazepine dihydrochloride (H-1 152, Tocris Bioschience). Said Rho-kinase inhibitor, for example Y-27632, is preferably added to the culture medium every second day during the first seven days of culturing said stem cells. A Rock inhibitor is preferably included in the medium in the first few days e.g. for the first 1, 2, 3, 4, 5, 6 or 7 days of culture after single cell seeding or after a split. Any suitable concentration of the Rock inhibitor may be used, for example, 1-200 uM, 1-100 uM, 5-50 uM or approximately 10uM. A preferred concentration for Y27632 is 10uM. Therefore, in some embodiments, the invention provides a method for culturing stem cells and/or a method for obtaining an organoid wherein a Rock inhibitor is added to the culture medium for the first 1, 2, 3, 4, 5, 6 or 7 days, optionally every second day. In some embodiments, the Rock inhibitor is not added to the culture medium after the first 2, 3, 4, 5, 6, 7, 8, 9 or 10 days.

Addition of a Rock inhibitor is particularly important when culturing single stem cells (as mentioned above), i.e. when the starting material for an organoid is a single stem cell. Therefore, in some embodiments the invention provides a method for obtaining an organoid, wherein the method comprises culturing stem cells, optionally single stem cells, wherein a Rock inhibitor is added to the culture medium for the first 1, 2, 3, 4, 5, 6 or 7 days, optionally every second day, and optionally not adding the Rock inhibitor to the culture medium after the first 2, 3, 4, 5, 6, 7, 8, 9 or 10 days.

The Rock inhibitor is less important, and sometimes not necessary, when culturing multiple cells, for example when the starting material for an organoid is a tissue fragment. Therefore, in some embodiments, the invention provides a method for obtaining an organoid, wherein the method comprises culturing stem cells, optionally a tissue fragment, wherein the Rock inhibitor is not added to the culture medium either at all or after the first 2, 3, 4, 5, 6, 7, 8, 9 or 10 days.

After the cells are split into multiple cultures, a Rock inhibitor may be added to the culture medium in the same way, meaning for the first 1, 2, 3, 4, 5, 6 or 7 days, optionally every second day, after the split, particularly when the split involves taking single stem cells from a first culture and placing these into a second culture. If the split involves taking multiple stem cells from the first culture and placing these into a second culture then addition of a Rock inhibitor is less important, and sometimes not necessary. Therefore, in some embodiments, wherein the method for obtaining organoids or for culturing stem cells involves a split, optionally where a single cell is involved in the split, a Rock inhibitor is added to the new culture medium for the first 1, 2, 3, 4, 5, 6 or 7 days, optionally every second day, after the split. In some embodiments, wherein the method for obtaining organoids or for culturing stem cells involves a split, optionally where multiple cells are involved in the split, is not added to the culture medium either at all or after the first 2, 3, 4, 5, 6, 7, 8, 9 or 10 days.

In yet a further embodiment, a method according to the invention comprises a culture medium further comprising a Notch agonist. Notch signaling has been shown to play an important role in cell-fate determination, as well as in cell survival and proliferation. Notch receptor proteins can interact with a number of surface-bound or secreted ligands, including but not limited to Delta 1, Jagged 1 and 2, and Delta-like 1, Delta-like 3, Delta-like 4. Upon ligand binding, Notch receptors are activated by serial cleavage events involving members of the ADAM protease family, as well as an intramembranous cleavage regulated by the gamma secretase presenilin. The result is a translocation of the intracellular domain of Notch to the nucleus where it transcriptionally activates downstream genes. A preferred Notch agonist is selected from Jagged 1 and Delta 1, or an active fragment or derivative thereof. A most preferred Notch agonist is DSL peptide (Dontu et al., 2004. Breast Cancer Res 6. R605-R615) with the sequence CDDYYYGFGCNKFCRPR. Said DSL peptide is preferably used at a concentration between 10µM and 100nM or at least 10µM and not higher than 100nM. The addition of a Notch agonist, especially during the first week of culturing, increases the culture efficiency by a factor of 2-3.

Said Notch agonist is preferably added to the culture medium every second day during the first seven days of culturing said stem cells. Therefore, in some embodiments, the invention provides a method for culturing stem cells and/or a method for obtaining an organoid wherein a Notch agonist is added to the culture medium for the first 1, 2, 3, 4, 5, 6 or 7 days, optionally every second day. In some embodiments, the Notch agonist is not added to the culture medium after the first 2, 3, 4, 5, 6, 7, 8, 9 or 10 days.

A Notch agonist is defined as a molecule that stimulates a Notch activity in a cell by at least 10%, more preferred at least 20%, more preferred at least 30%, more preferred at least 50%, more preferred at least 70%, more preferred at least 90%, more preferred at least 100%, relative to a level of a Notch activity in the absence of said molecule. As is known to a skilled person, a Notch activity can be determined by measuring the transcriptional activity of Notch, for example by a 4xwtCBFl-luciferase reporter construct as described (Hsieh et al, 1996 Mol Cell. Biol. 16, 952-959).

In a further embodiment, the cell culture medium is supplemented with a gamma-secretase inhibitor, such as DAPT or DBZ. Gamma-secretase inhibitors can influence cell fate decisions during differentiation. For example, in some embodiments, gamma-secretase inhibitors can influence cell fate towards secretory cells, such as goblet cells.. Any suitable concentration of the gamma-secretase inhibitor may be used, for example, between 1 nM and 10 uM, 1 nM and 1 uM, between 1 and 100 nM, or preferably between 1 and 20nM. For example, a gamma-secretase inhibitor may be added to the culture medium to a final concentration of approximately 1 nM.

In a further embodiment, the cell culture medium is supplemented with gastrin (or a suitable alternative such as Leu15-gastrin). Gastrin (or a suitable alternative) may be added to the culture medium to a final concentration of between 1 nM and 10 uM, 1 nM and 1 uM, between 5 and 100 nM, or preferably between 10 and 50nM. For example, Leu15-gastrin may be added to the culture medium to a final concentration of approximately 10 nM. Gastrin is not necessary for some culture media of the invention. Therefore, in some embodiments the culture medium of the invention does not comprise gastrin. In particular, gastrin is not required for culturing intestinal stem cells or for obtaining intestinal (crypt-villus or colon crypt) organoids. However, even where gastrin is not required, it may still be added to the culture medium without negative effects.

In a further embodiment, the culture medium of the invention is supplemented with nicotinamide. Addition of nicotinamide has been found to improve culture efficiency and lifespan of human colon organoids. Nicotinamide may be added to the culture medium to a final concentration of between 1 and 100 mM, between 5 and 50 mM, or preferably between 5 and 20mM. For example, nicotinamide may be added to the culture medium to a final concentration of approximately 10 mM.

In a preferred embodiment of the invention, the culture medium is supplemented with nicotinamide and gastrin (or a suitable alternative, such as Leu15-gastrin), wherein nicotinamide and gastrin are added to the culture medium at any of the concentrations described above.

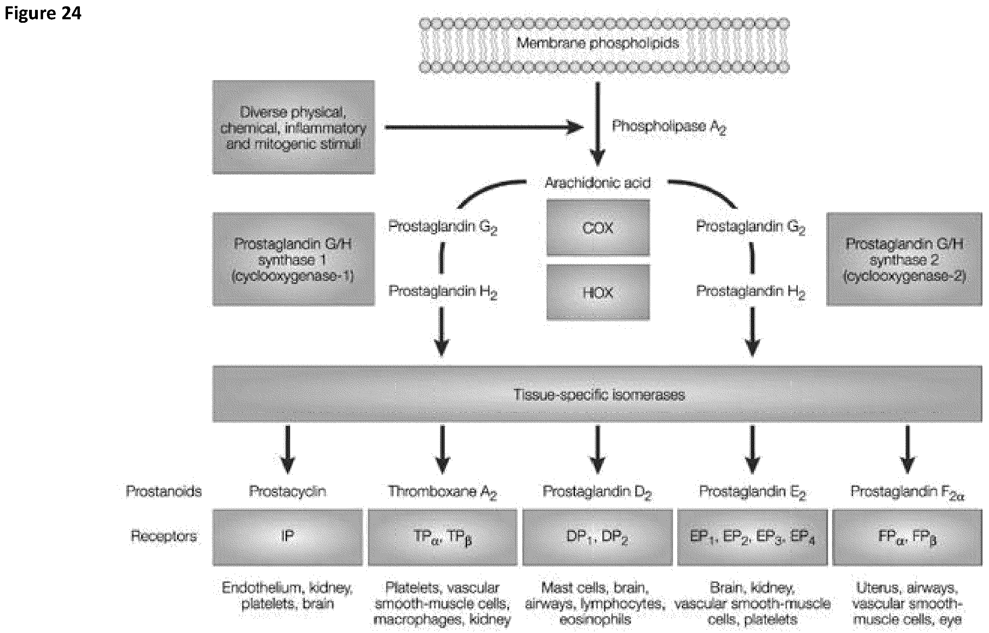

In some embodiments, the culture medium is supplemented with an activator of the prostaglandin signalling pathway (see Figure 24, Antagonism of the prostaglandin D₂ receptors DP₁ and CRTH2 as an approach to treat allergic diseases. Roy Pettipher, Trevor T. Hansel & Richard Armer Nature Reviews Drug Discovery 6, 313-325 (April 2007)). For example, the culture medium is supplemented with any one or more of the compounds selected from the list comprising: Phospholipids, Arachidonic acid (AA), prostaglandin E2 (PGE2), prostaglandin G2 (PGG2), prostaglandin F2 (PGF2), prostaglandin H2 (PGH2), prostaglandin D2 (PGD2). For example, in some embodiments, the culture medium is supplemented with PGE2 and/or AA. In some embodiments, PGE2 is added to the medium to a final concentration of at least 10 nM, for example at least 20nM, at least 30nM, at least 40nM, at least 45nM, between 10 nM and 500 nM, between 10 nM, and 400 nM, between 10 nM and 300 nM, between 10 nM and 200 nM, between 10 nM and 100 nM, between 20 nM and 50 nM. In a preferred embodiment, PGE2 is added to the medium to a final concentration of 50 nM. In some embodiments, AA is added to the medium to a final concentration of at least 1 ug/ml, at least 5 ug/ml , at least 8 ug/ml, at least 9 ug/ml, at least 10 ug/ml, for example between 1 ug/ml and 1000 ug/ml, between 1 ug/ ml and 500 ug/ml, between 1 ug/ml and 100 ug/ml, between 1 ug/ml and 50 ug/ml, or between 5 ug/ml and 20 ug/ml. In a preferred embodiment, AA is added to the medium to a final concentration of 10 ug/ml. AA and PGE2 are interchangeable in the context of the culture media of the invention. Therefore, where a culture medium described herein is said to include PGE2, it may alternatively include AA (at an appropriate concentration) instead of PGE2. Conversely, where a culture medium described herein is said to include AA, it may alternatively include PGE2 (at an appropriate concentration) instead of AA. Furthermore, the skilled person would understand that where PGE2 and/or AA are included in a culture medium of the invention, the culture medium could instead comprise any one or more of the compounds selected from the following list in replacement or in addition to PGE2 and/or AA: Phospholipids, prostaglandin G2 (PGG2), prostaglandin F2 (PGF2), prostaglandin H2 (PGH2), and prostaglandin D2 (PGD2).

In a futher embodiment, the culture medium of the invention is supplemented with RANK ligand (also referred to herein as RANKL). RANK ligand can be useful for directing differentiation towards particular cell fates. For example, when RANK ligand is included in the culture medium for small intestinal cells, preferably in the medium for differentiating small intestinal cells, it results in a greater proportion of the cells being differentiated into M cells. Therefore, in some embodiments, the invention provides a culture medium comprising RANKL. In particular, the invention provides a culture medium for culturing, preferably for differentiating small intestinal cells, wherein the culture medium comprises RANKL. Any suitable concentration of the RANKL may be used, for example, between 10ng/ml and 1000ng/ml, between 10 and 500 ng/ml, or between 50 and 100ng/ml. For example, RANKL may be added to the culture medium to a final concentration of approximately 100 ng/ml.

A culture medium comprising EGF, Noggin and R-spondin is referred to herein as the "ENR medium". A culture medium comprising the ENR medium and a Wnt agonist such as Wnt-3a is referred to herein as the "WENR medium". In a preferred embodiment of the invention, the culture medium comprises a WENR medium. In a most preferred embodiment of the invention, the culture medium comprises a WENR medium supplemented with gastrin and/or nicotinamide (i.e., WENRg or WENR+nicotinamide or WENRg+nicotinamide).

The pH of the medium may be in the range from about 7.0 to 7.8, in the range from about 7.2 to 7.6, or about 7.4. The pH may be maintained using a buffer. A suitable buffer can readily be selected by the skilled person. Buffers that may be used include carbonate buffers (e.g. NaHCO₃), and phosphates (e.g. NaH₂PO₄). These buffers are generally used at about 50 to about 500 mg/l. Other buffers such as N-[2-hydroxyethyl]-piperazine-N'-[2-ethanesul-phonic acid] (HEPES) and 3-[N-morpholino]-propanesulfonic acid (MOPS) may also be used, normally at around 1000 to around 10,000 mg/l. A culture medium may comprise a pH indicator, such as phenol red, to enable the pH status of the medium to be easily monitored (e.g. at about 5 to about 50 mg/litre).

A culture medium for use in the invention may comprise one or more amino acids. The skilled person understands the appropriate types and amounts of amino acids for use in stem cell culture media. Amino acids which may be present include L-alanine, L-arginine, L-asparagine, L-aspartic acid, L-cysteine, L-cystine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, L-valine and combinations thereof. Some culture media will contain all of these amino acids. Generally, each amino acid when present is present at about 0.001 to about 1 g/L of medium (usually at about 0.01 to about 0.15 g/L), except for L-glutamine which is present at about 0.05 to about 1 g/L (usually about 0.1 to about 0.75 g/L). The amino acids may be of synthetic origin.

A culture medium for use in the invention may comprise one or more vitamins. The skilled person understands the appropriate types and amounts of vitamins for use in stem cell culture media. Vitamins which may be present include thiamine (vitamin B1), riboflavin (vitamin B2), niacin (vitamin B3), D-calcium pantothenate (vitamin B5), pyridoxal/pyridoxamine/pyridoxine (vitamin B6), folic acid (vitamin B9), cyanocobalamin (vitamin B12), ascorbic acid (vitamin C), calciferol (vitamin D2), DL-alpha tocopherol (vitamin E), biotin (vitamin H) and menadione (vitamin K).

A culture medium for use in the invention may comprise one or more inorganic salts. The skilled person understands the appropriate types and amounts of inorganic salts for use in stem cell culture media. Inorganic salts are typically included in culture media to aid maintenance of the osmotic balance of the cells and to help regulate membrane potential. Inorganic salts which may be present include salts of calcium, copper, iron, magnesium, potassium, sodium, zinc. The salts are normally used in the form of chlorides, phosphates, sulphates, nitrates and bicarbonates. Specific salts that may be used include CaCl₂, CuSO₄-5H₂O, Fe(NO₃)-9H₂O, FeSO₄-7H₂O, MgCl, MgSO₄, KCl, NaHCO₃, NaCl, Na₂HPO₄, Na₂HPO₄-H₂O and ZnSO₄-7H₂O.

The osmolarity of the medium may be in the range from about 200 to about 400 mOsm/kg, in the range from about 290 to about 350 mOsm/kg, or in the range from about 280 to about 310 mOsm/kg. The osmolarity of the medium may be less than about 300 mOsm/kg (e.g. about 280 mOsm/kg).

A culture medium for use in the invention may comprise a carbon energy source, in the form of one or more sugars. The skilled person understands the appropriate types and amounts of sugars to use in stem cell culture media. Sugars which may be present include glucose, galactose, maltose and fructose. The sugar is preferably glucose, particularly D-glucose (dextrose). A carbon energy source will normally be present at between about 1 and about 10 g/L.

A culture medium of the invention may contain serum. Serum obtained from any appropriate source may be used, including fetal bovine serum (FBS), goat serum or human serum. Preferably, human serum is used. Serum may be used at between about 1% and about 30% by volume of the medium, according to conventional techniques.

In other embodiments, a culture medium of the invention may contain a serum replacement. Various different serum replacement formulations are commercially available and are known to the skilled person. Where a serum replacement is used, it may be used at between about 1% and about 30% by volume of the medium, according to conventional techniques.

In other embodiments, a culture medium of the invention may be serum-free and/or serum replacement-free. A serum-free medium is one that contains no animal serum of any type. Serum-free media may be preferred to avoid possible xeno-contamination of the stem cells. A serum replacement-free medium is one that has not been supplemented with any commercial serum replacement formulation.

In a preferred embodiment, the cell culture medium is supplemented with a purified, natural, semi-synthetic and/or synthetic growth factor and does not comprise an undefined component, such as fetal bovine serum or fetal calf serum. For example, supplements such as B27 (Invitrogen), N-Acetylcysteine (Sigma) and N2 (Invitrogen) stimulate proliferation of some cells. In some embodiments, the cell culture medium is supplemented with one or more of these supplements, for example one, any two or all three of these supplements.

In other embodiments, the cell culture medium is supplemented with Exendin-4. Exendin-4, a 39 amino acid peptide, activates GLP-1 (glucagon-like peptide-1) receptors to increase intracellular cAMP in pancreatic acinar cells and has no effect on VIP (vasoactive intestinal peptide) receptors.

A culture medium for use in the invention may comprise one or more trace elements, such as ions of barium, bromium, cobalt, iodine, manganese, chromium, copper, nickel, selenium, vanadium, titanium, germanium, molybdenum, silicon, iron, fluorine, silver, rubidium, tin, zirconium, cadmium, zinc and/or aluminium.

The medium may comprise a reducing agent, such as beta-mercaptoethanol at a concentration of about 0.1 mM.

A culture medium of the invention may comprise one or more additional agents, such as nutrients or growth factors previously reported to improve stem cell culture, such as cholesterol/transferrin/albumin/insulin/progesterone, putrescine, selenite/other factors.

A culture medium of the invention may be diffused into an extracellular matrix (ECM). In a preferred method of the invention, isolated tissue fragments or isolated epithelial stem cells are attached to an ECM. ECM is composed of a variety of polysaccharides, water, elastin, and glycoproteins, wherein the glycoproteins comprise collagen, entactin (nidogen), fibronectin, and laminin. ECM is secreted by connective tissue cells. Different types of ECM are known, comprising different compositions including different types of glycoproteins and/or different combination of glycoproteins. Said ECM can be provided by culturing ECM-producing cells, such as for example fibroblast cells, in a receptacle, prior to the removal of these cells and the addition of isolated tissue fragments or isolated epithelial stem cells. Examples of extracellular matrix-producing cells are chondrocytes, producing mainly collagen and proteoglycans, fibroblast cells, producing mainly type IV collagen, laminin, interstitial procollagens, and fibronectin, and colonic myofibroblasts producing mainly collagens (type I, III, and V), chondroitin sulfate proteoglycan, hyaluronic acid, fibronectin, and tenascin-C. Alternatively, said ECM is commercially provided. Examples of commercially available extracellular matrices are extracellular matrix proteins (Invitrogen) and basement membrane preparations from Engelbreth-Holm-Swarm (EHS) mouse sarcoma cells (e.g. Matrigel^{™} (BD Biosciences)). A synthetic extracellular matrix material, such as ProNectin (Sigma Z378666) may be used. Mixtures of extracellular matrix materials may be used, if desired. The use of an ECM for culturing stem cells enhanced long-term survival of the stem cells and the continued presence of undifferentiated stem cells. In the absence of an ECM, stem cell cultures could not be cultured for longer periods and no continued presence of undifferentiated stem cells was observed. In addition, the presence of an ECM allowed culturing of three-dimensional tissue organoids, which could not be cultured in the absence of an ECM. The extracellular matrix material will normally be a drop on the bottom of the dish in which cells are suspended. Typically, when the matrix solidifies at 37°C, the medium is added and diffuses into the ECM. The cells in the medium stick to the ECM by interaction with its surface structure, for example interaction with integrins. A fibronectin solution of about 1mg/ml (stock solution) used at approximately 1 µg/cm² may be used to coat a cell culture vessel, or between about 1 µg/cm² to about 250 µg/cm², or at about 1 µg/cm² to about 150 µg/cm². In some embodiments, a cell culture vessel is coated with fibronectin at between 8 µg/cm² and 125 µg/cm².

An example of an ECM for use in a method of the invention comprises at least one glycoprotein, such as laminin.

A preferred ECM for use in a method of the invention comprises at least two distinct glycoproteins, such as two different types of collagen or a collagen and laminin. The ECM can be a synthetic hydrogel extracellular matrix or a naturally occurring ECM. A further preferred ECM is provided by Matrigel^{™} (BD Biosciences), which comprises laminin, entactin, and collagen IV. In some embodiments the extracellular matrix is a laminin-containing extracellular matrix such as Matrigel^{™} (BD Biosciences).

In some embodiments, the single stem cell, population of cells, or tissue fragment is embedded in matrigel, which is optionally growth factor reduced and/or phenol red-free.

In some embodiments, the culture medium is placed on top of the ECM. The culture medium can then be removed and replenished as and when required. In some embodiments, the culture medium is replenished every 1, 2, 3, 4, 5, 6 or 7 days. If components are "added" or "removed" from the media, then this can in some embodiments mean that the media itself is removed from the ECM and then a new media containing the "added" component or with the "removed" component excluded is placed on the ECM.

In some embodiments the culture medium of the invention is in contact with an extracellular matrix or a 3D matrix that mimics the extracellular matrix by its interaction with the cellular membrane proteins, such as integrins.

In some embodiments, the basal culture medium comprises or consists of Advanced DMEM/F12 supplemented with penicillin/streptomycin, 10mM HEPES, Glutamax, 1× N2, 1× B27 (all from Invitrogen) and 1 mM N-acetylcysteine (Sigma)).

### Examples of culture media of the invention

In one embodiment, the cell culture medium comprises a TGF-beta inhibitor that binds to and reduces the activity of ALK5 and a p38 inhibitor that binds to and reduces the activity of p38. For example, in one embodiment the cell culture media comprises A83-01 and/or SB202190, preferably A83-01+ SB202190. The use of A83-01+SB202190 together in a culture medium of the invention has surprisingly be found to synergistically increase the number of passages of human colon organoids. In one embodiment, the cell culture media comprises WENR+A83-01+SB202190. In one embodiment, the cell culture media comprise WENR+A83-01+SB202190+nicotinamide. In one embodiment, the cell culture media comprises WENRg+nicotinamide +A83-01+SB202190 (where "g" is gastrin). In one embodiment, the cell culture medium comprises WENR+A83-01+Nicotinamide+ FGF10. In one embodiment, the cell culture medium comprises WENRg+A83-01+Nicotinamide+ FGF10. In one embodiment, the cell culture medium comprises WENRg+A83-01+Nicotinamide+FGF10+SB202190. In one embodiment, the cell culture media is used for obtaining colon organoids. A colon organoid obtainable by culturing epithelial cells using a cell culture media as described in this embodiment is also provided.

For example, in one embodiment the cell culture media comprises WENRg+A83-01+FGF10, wherein the Wnt agonist is R-Spondin but no other Wnt agonist is present and no nicotinamide is present. For example, in some embodiments, the cell culture media comprises EGF (e.g. 50 ng/ml), R-Spondin (e.g. 10% or 1 ug/ml), Noggin (e.g. 100ng/ml), FGF10 (e.g. 100ng/ml), A8301 (e.g. 500 nM) and Gastrin (e.g. 10uM) and optionally SB202190. These components may be added to a basal medium, such as DMEM/F12 media. In some embodiments, the basal medium is further supplemented with any one or more (for example, 1, 2, 3, 4 or 5) or all, of the components selected from the list comprising: P/S, Glutamax, 10nmM Hepes, B27, N2 and N-Acetylcysteine. The use of such a cell culture media has been found to be useful for obtaining pancreatic organoids. A pancreatic organoid obtained by culturing epithelial cells using a cell culture media as described in this embodiment is also provided. In some embodiments, gastrin or nicotinamide or gastrin and nicotinamide are excluded from the culture medium.

### Tissue-specific culture media of the invention

Particularly preferred culture media are described in the Examples herein. The culture medium of the invention can be adapted for use with different tissues, for example as described below.

### Intestinal culture media

In some embodiments, the culture medium for small intestinal crypts, such as murine small intestinal crypts, comprises or consists of a basal medium, for example as described above, additionally comprising: EGF, such as murine EGF; a BMP inhibitor, such as murine Noggin; and Rspondin, such as human Rspondin-1 or 4. In some embodiments, this culture medium further comprises a TGF-beta inhibitor (such as A83-01) and/or a p38 inhibitor (such as SB202190). In some embodiments, the culture medium for colonic crypts, such as murine colonic crypts, comprises or consists of a basal medium, for example as described above, additionally comprising: a Wnt agonist, such as recombinant human Wnt-3A or Wnt-3A conditioned medium; EGF, such as murine EGF; a BMP inhibitor, such as murine Noggin; and Rspondin, such as human Rspondin-1 or 4. In some embodiments, this culture medium further comprises a TGF-beta inhibitor (such as A83-01) and/or a p38 inhibitor (such as SB202190).

In some embodiments, the culture medium for human intestinal stem cells, human small intestinal crypts or human colonic crypts (also known as the HISC culture medium), comprises or consists of a basal medium, for example as described above, additionally comprising: a Wnt agonist, such as recombinant human Wnt-3A or Wnt-3A conditioned medium; EGF; a BMP inhibitor, such as Noggin; Rspondin, such as human Rspondin-1; a TGF-beta inhibitor, such as A83-01; a p38 inhibitor, such as SB202190; gastrin; and nicotinamide. In some embodiments, the p38 inhibitor and/or gastrin can be excluded from the HISC culture medium.

In some embodiments the invention provides a culture medium for culturing intestinal cells, comprising or consisting of a basal medium, Wnt-3a, EGF, Noggin, any one of Rspondin 1-4, a TGF-beta inhibitor, nicotinamide, and preferably a p38 inhibitor.

In some embodiments, the culture medium for expanding small intestine or colon stem cells, for example human small intestine or colon cells, comprises or consists of a basal medium (for example comprising Advanced DMEM/F12, B27 (50x), n-Acetyleysteine (1 mM) and glutamin/glutamax), Wnt3A (optionally conditioned medium), any one of Rspondin 1-4 (preferably 1 ug/ml), Noggin (preferably 50-100 ng/ml), nicotinamide (preferably 10 mM), EGF (preferably 10-50 ng/ml), gastrin (preferably 10 nM), a TGF-beta inhibitor, for example A83-01 (preferably 500 nM). In a further embodiment, this culture medium additionally comprises a p38 inhibitor, for example SB202190 (preferably 100 nM). In a further embodiment, this culture medium additionally comprises a Rock inhibitor, for example LY2157299.

In some embodiments, the invention provides a culture medium for differentiating intestinal cells, comprising or consisting of a basal medium, EGF, Noggin, a TGF-beta inhibitor and a p38 inhibitor.

In some embodiments, the culture medium for differentiating small intestine or colon stem cells, for example human small intestine or colon cells, comprises or consists of a basal medium (for example comprising Advanced DMEM/F12, B27 (50x), n-Acetylcysteine (1 mM) and glutamin/glutamax), Noggin (preferably 50-100 ng/ml), EGF (preferably 10-50 ng/ml), gastrin (preferably 10 nM), a TGF-beta inhibitor, for example A83-01 (preferably 500 nM) and a p38 inhibitor, for example SB202190 (preferably 100 nM). In some embodiments, gastrin can be excluded from this differentiation medium. In some embodiments, a gamma-secretase inhibitor may be added to the differentiation medium (preferably at a concentration of 1 uM). Gamma-secretase inhibitors can influence cell fate decisions during differentiation e.g. towards secretory cells, such as goblet cells. In some embodiments, a RANKL may be added to the differentiation medium (for example at a concentration of 100 ng/ml). RANKL can influence cell fate decisions during differentiation e.g. towards M-cells.

### Cancer culture media

In some embodiments, the culture medium for colon cancer cells, comprises or consists of a basal medium, for example as described above, additionally comprising: a Wnt agonist, such as recombinant human Wnt-3A or Wnt-3A conditioned medium; EGF; a BMP inhibitor, such as Noggin; Rspondin, such as human Rspondin-1; a TGF-beta inhibitor, such as A83-01; a p38 inhibitor, such as SB202190; gastrin; and nicotinamide.

In one embodiment, the culture medium for colon carcinoma, for example human colon carcinoma, comprises a basal medium (for example comprising Advanced DMEM/F12, B27 (50x), n-Acetylcysteine (1 mM), primocin and/or P/S (antibiotics) (500x) and hepes), Rspondin (optionally conditioned medium) (preferably 1 ug/ml), Noggin (preferably 100 ng/ml), Nicotinamide (preferably 10 mM), EGF (preferably 50 ng/ml), gastrin (preferably 50 nM), a TGF-beta inhibitor, for example A83-01 (preferably 500 nM), a p38 inhibitor, such as SB202190 (preferably 10 uM), optionally PGE2 (preferably 10 nM) and/or a Rock inhibitor (preferably 10 uM).

In some embodiments, colon cancer cells can also be grown in the HISC culture medium. In some embodiments, colon cancer cells can be cultured in the HISC culture medium, wherein one or more or all of the following are excluded from the medium: EGF, Noggin, Rspondin, TGF-beta inhibitor and p38 inhibitor. Cancer cells may have mutations that consitutively activate or deactivate certain growth pathways. For example, many colon cancers result in constitutive activation of the Wnt pathway. In such cases, a culture medium would not require a Wnt agonist. Other mutations would allow other factors to be left out of the medium as described above. Other epithelial cancers (carcinomas) can also be grown in culture media of the invention. In a preferred embodiment, a cancer organoid obtained from cancer stem cells is grown in a culture medium that is suitable for growth of the corresponding normal tissue organoid obtained from normal stem cells, optionally with certain factors excluded from the medium. For example, a stomach cancer organoid obtained by culturing stomach cancer stem cells may be grown in the same culture conditions as a normal gastric organoid obtained by culturing gastric stem cells, optionally with certain factors excluded from the medium. In another example, a pancreatic cancer organoid obtained by culturing pancreatic cancer stem cells may be grown in the same culture conditions as a normal pancreatic organoid obtained by culturing pancreatic stem cells, optionally with certain factors excluded from the medium. In another example, a prostate cancer organoid obtained by culturing prostatic cancer stem cells may be grown in the same culture conditions as a normal prostate organoid obtained by culturing prostatic stem cells, optionally with certain factors excluded from the medium. In another example, a liver cancer organoid obtained by culturing liver cancer stem cells may be grown in the same culture conditions as a normal liver organoid obtained by culturing liver stem cells, optionally with certain factors excluded from the medium. In many situations it may be preferable (or at least more convenient) to grow cancer organoids in the normal tissue medium (without any factors excluded). The normal tissue medium should allow cancers with all genetic backgrounds to grow, without excluding any particular cancer mutations.

Therefore, in some embodiments, the invention provides a culture medium for culturing cancer cells, for example cancer stem cells, such as adenocarcinoma or carcinoma cells from a tissue type of interest, wherein the culture medium comprises or consists of the components of the culture medium used for culturing the cells from the corresponding non-cancerous tissue type of interest, optionally wherein one or more of the following are excluded from the medium that is used to culture the non-cancerous cells of the tissue type of interest: Wnt-3a, EGF, Noggin, Rspondin, TGF-beta inhibitor, p38 inhibitor, nicotinamide, gastrin, FGF10 and HGF.

### Adenoma culture medium

In some embodiments, the culture medium for intestinal adenomas, such as murine intestinal adenomas comprises a basal medium, for example as described above, additionally comprising EGF, such as murine EGF.

### Gastric culture media

In some embodiments, the invention provides a culture medium for culturing gastric cells, comprising or consisting of a basal medium, Wnt-3a, EGF, Noggin, any one of Rspondin 1-4, a TGF-beta inhibitor, gastrin, nicotinamide, FGF-10, and preferably a p38 inhibitor.

In some embodiments, the culture medium for gastric stem cells, for example human gastric stem cells comprises or consists of a basal medium (for example comprising Advanced DMEM/F12, B27 (50x), n-Acetylcysteine (1 mM), primocin and/or P/S (antibiotics) (500x) and glutamin/glutamax), any one of Rspondin 1-4 (optionally conditioned medium) (preferably 1ug/ml), Noggin (optionally conditioned medium) (preferably 100ng/ml), Wnt3A (optionally conditioned medium), nicotinamide (preferably 5 mM), EGF (preferably 50 ng/ml), FGF10 (preferably 200 ng/ml), gastrin (preferably 1 nM), a TGF-beta inhibitor, for example A83-01 (preferably 2 uM). The culture medium for gastric stem cells optionally further comprises a p38 inhibitor, for example SB202190 (preferably 10 nM). The culture medium for gastric stem cells optionally further comprises PGE2 (preferably 500 nM). The culture medium for gastric stem cells optionally further comprises a Rock inhibitor (preferably 10 uM).

In some embodiments, the culture medium for gastric stem cells, for example murine gastric cells comprises or consists of a basal medium (for example comprising Advanced DMEM/F12, B27 (50x), n-Acetylcysteine (1 mM), primocin and/or P/S (antibiotics) (500x) and glutamin/glutamax), any one of Rspondin 1-4 (optionally conditioned medium) (preferably 1ug/ml), Noggin (optionally conditioned medium) (preferably 100ng/ml), Wnt3A (optionally conditioned medium), EGF (preferably 50 ng/ml), FGF10 (preferably 200 ng/ml), gastrin (preferably 1 nM) and a Rock inhibitor (preferably 10 uM). In some embodiments, this culture medium further comprises a TGF-beta inhibitor (such as A83-01) and/or a p38 inhibitor (such as SB202190).

### Prostate culture media

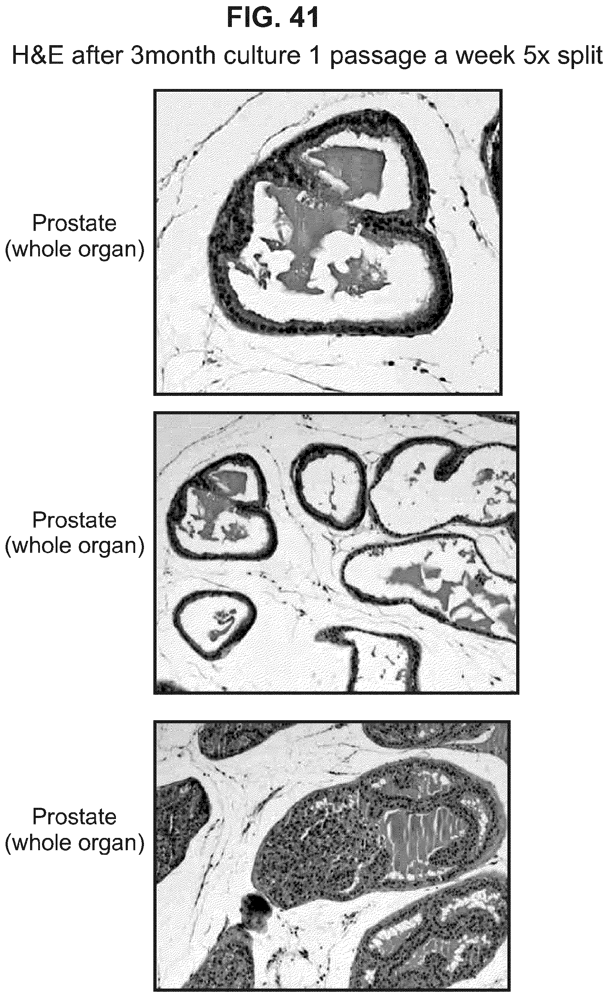

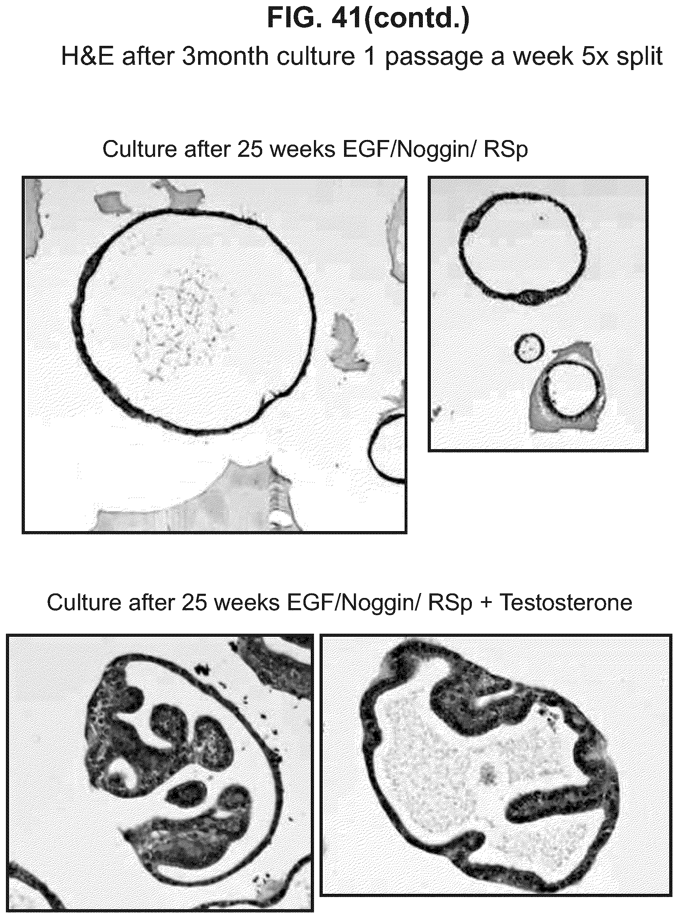

In some embodiments, the culture medium for expanding prostate stem cells comprises testosterone, optionally dihydrotestosterone (also referred to herein as DHT). Testosterone is a steroid hormone from the androgen group. In humans, a large percentage of testosterone undergoes 5α-reduction to form the more potent androgen, dihydrotestosterone. Testosterone, dihydrotestosterone or a testosterone mimic (for example, a molecule that mimics the activity of testosterone binding to an androgen receptor) can be added to a culture medium of the invention. Therefore, where the term testosterone is used, it can always be replaced by dihydrotestosterone or a testosterone mimic. The inventors have shown that addition of testosterone to a culture medium for prostate stem cells, results in increased differentiation but also in continued expansion of the stem cell population (for example, see Figures 41 - 45). This is highly surprising because the literature teaches that testosterone plays an important role in the differentiation of cells by acting to suppress proliferation and maintain terminal differentiation (Mirochnik et al. PLoS One, 7(3), e31052, 2012; Niu et al. Oncogene 29, 3593-3604, 2010). The skilled person would have expected that addition of testosterone to a culture medium for prostate would result in completely differentiated organoids with no further expansion potential. This would be similar to what is observed when the colon, pancreas and liver organoids are differentiated in a differentiation medium. However, by contrast, the present inventors have found that although testosterone increases differentiation, it also allows stem cell expansion to continue. Therefore, organoids grown in a culture medium comprising testosterone surprisingly comprise stem cells and differentiated cells i.e. luminal cells and basal cells.

In some embodiments, the culture medium for obtaining a prostate organoid comprises a basal medium and testosterone, optionally dihydrotestosterone and anyone of Rspondin 1-4 or an Rspondin mimic. In some embodiments, the culture medium further comprises a BMP inhibitor, for example Noggin. In some embodiments, the culture medium further comprises a tyrosine receptor kinase ligand, optionally wherein the tyrosine receptor kinase ligand is a mitogenic growth factor, such as EGF, FGF, KGF or HGF. In some embodiments, the culture medium for obtaining a prostate organoid comprises EGF, Noggin, any one or Rspondin 1-4 and testosterone.

In a preferred embodiment, the culture medium for prostate cells comprises a TGF-beta inhibitor. In some embodiments, the culture medium for prostate cells comprises EGF, Noggin, any one of Rspondin 1-4, a TGF-beta inhibitor and testosterone. In some embodiments, the culture medium for prostate cells further comprises a p38 inhibitor. In some embodiments, a culture medium for obtaining a prostate organoid does not comprise an inhibitor of the invention, for example a TGF-beta inhibitor and/or a p38 inhibitor. In some embodiments the culture medium for prostate stem cells does not comprise testosterone. In some embodiments, the culture medium comprises a basal medium, EGF, Noggin and any one of Rspondin 1-4 and optionally a TGF-beta inhibitor, and does not comprise testosterone.

In some embodiments, the invention provides a culture medium for culturing prostate cells, comprising or consisting of a basal medium, EGF, any one of Rspondin 1-4, Noggin, nicotinamide a TGF-beta inhibitor, and preferably Wnt-3a and FGF-10. In some embodiments, the culture medium for culturing prostate cells further comprises testosterone, for example (dihydro)testosterone. In some embodiments the culture medium further comprises a p38 inhibitor.In some embodiments, the culture medium for prostate cells, for example mouse, human, normal or carcinoma, comprises a basal medium (for example comprising Advanced DMEM/F12, B27 (50x), n-Acetylcysteine (1 mM) and glutamin/glutamax), any one of Rspondin 1-4 (optionally conditioned medium) (preferably 1 ug/ml), Noggin (optionally conditioned medium) (preferably 100 ng/ml), nicotinamide (preferably 10mM), EGF (preferably 50 ng/ml), FGF10 (preferably 100 ng/ml), a TGF-beta inhibitor, for example A83-01 (preferably 500 nM), (Dihydro)testosterone (preferably 1 nM) 10 nM and optionally Wnt-3a. In some embodiments, this culture medium further comprises a Rock inhibitor (preferably 10 uM). In some embodiments, the culture medium for prostate cells further comprises a p38 inhibitor, for example SB202190. In some embodiments, wherein mouse prostate cells are cultured, the TGF-beta inhibitor can be excluded from the culture medium. In other embodiments, nicotinamide, FGF10 and/or the Rock inhibitor can be excluded from the culture medium.

### Pancreatic culture media

In some embodiments, the invention provides a culture medium for expanding pancreas cells, comprising or consisting of a basal medium, any one of Rspondin 1-4, Noggin, EGF, FGF10, gastrin, a TGF-beta inhibitor, and preferably exendin 4 and Wnt-3a.

In some embodiments, the culture medium for expanding pancreatic stem cells, for example human pancreatic stem cells comprises or consists of a basal medium (for example comprising Advanced DMEM/F12, B27 (50x), n-Acetylcysteine (1 mM) and glutamin/glutamax), any one of Rspondin 1-4 (optionally conditioned medium) (preferably 1ug/ml), Noggin (optionally conditioned medium) (preferably 100ng/ml), nicotinamide (preferably 10 mM), EGF (preferably 50 ng/ml), FGF10 (preferably 100 ng/ml), gastrin (preferably 100 nM), and a TGF-beta inhibitor, for example A83-01 (preferably 2 uM). In a further embodiment, this culture medium additionally comprises Wnt-3a. In a further embodiment, this culture medium additionally comprises a p38 inhibitor, for example SB202190 (preferably 100 nM). In a further embodiment, this culture medium additionally comprises a Rock inhibitor, for example LY2157299 (preferably 10 uM). In a further embodiment, this culture medium additionally comprises Exendin 4 (preferably 50 ng/ml).

In some embodiments, the culture medium for expanding pancreatic stem cells, for example mouse pancreatic stem cells comprises or consists of a basal medium (for example comprising Advanced DMEM/F12, B27 (50x), n-Acetylcysteine (1 mM), primocin and/or P/S (antibiotics), Hepes and glutamin/glutamax), any one of Rspondin 1-4 (optionally conditioned medium) (preferably 1ug/ml), Noggin (optionally conditioned medium) (preferably 100ng/ml), nicotinamide (preferably 10 mM), EGF (preferably 50 ng/ml), FGF10 (preferably 100 ng/ml), gastrin (preferably 100 nM), and a TGF-beta inhibitor, for example A83-01 (preferably 2 uM). In a further embodiment, this culture medium additionally comprises a Rock inhibitor, for example LY2157299 (preferably 10 uM). In some embodiments, the culture medium for pancreatic cells further comprises a p38 inhibitor, for example SB202190.

In some embodiments, the invention provides a culture medium for differentiating pancreas cells comprising or consisting of a basal medium, Noggin, EGF, FGF10, gastrin, a TGF-beta inhibitor, gamma-secretase inhibitor and preferably exendin 4.

In some embodiments, the culture medium for differentiating pancreatic stem cells, for example human pancreatic stem cells comprises or consists of a basal medium (for example comprising Advanced DMEM/F12, B27 (50x), n-Acetylcysteine (1 mM) and glutamin/glutamax), Noggin (preferably 100 ng/ml), EGF (preferably 50 ng/ml), FGF10 (preferably 10 nM), gastrin (preferably 100 nM), a TGF-beta inhibitor, for example A83-01 (preferably 50nM) and gamma-secretase inhibitor (DAPT/DBZ) (preferably 10 uM). In a further embodiment, this culture medium additionally comprises Exendin 4 (preferably 50 ng/ml). In some embodiments, the culture medium for pancreatic cells further comprises a p38 inhibitor, for example SB202190.

In some embodiments, the culture medium for differentiating pancreatic stem cells, for example mouse pancreatic stem cells comprises or consists of a basal medium (for example comprising Advanced DMEM/F12, B27 (50x), n-Acetylcysteine (1 mM) and glutamin/glutamax), EGF (preferably 50 ng/ml) and gamma-secretase inhibitor (for example DAPT/DBZ) (preferably 10 uM).

### Barrett's Esophagus culture medium

In some embodiments, the culture medium for Barrett's Esophagus, comprises or consists of a basal medium, for example as described above, additionally comprising: a Wnt agonist, such as recombinant human Wnt-3A or Wnt-3A conditioned medium; EGF; a BMP inhibitor, such as Noggin; Rspondin, such as human Rspondin-1; a TGF-beta inhibitor, such as A83-01; a p38 inhibitor, such as SB202190; gastrin; nicotinamide; and an FGF, such as human FGF10 (i.e. HISC+FGF). In some embodiments, gastrin is excluded from this culture medium.

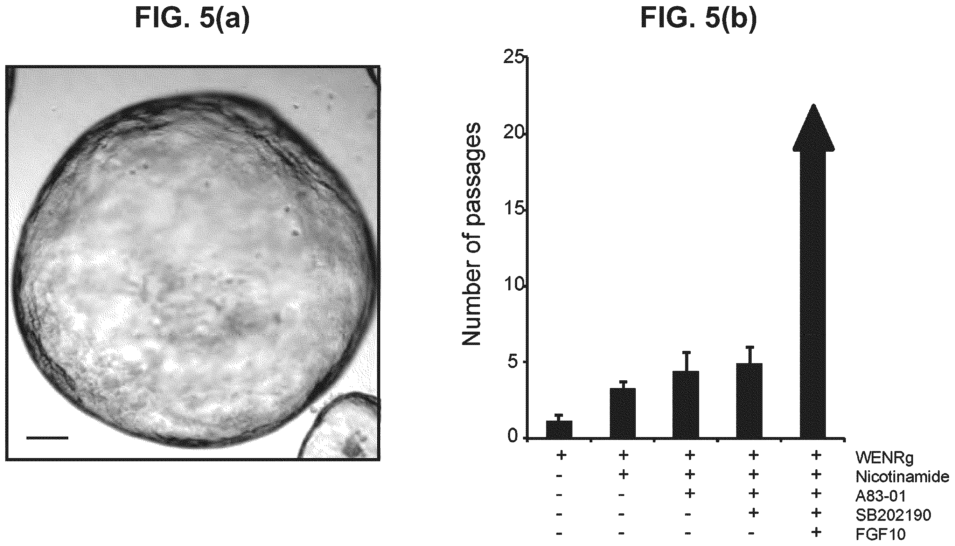

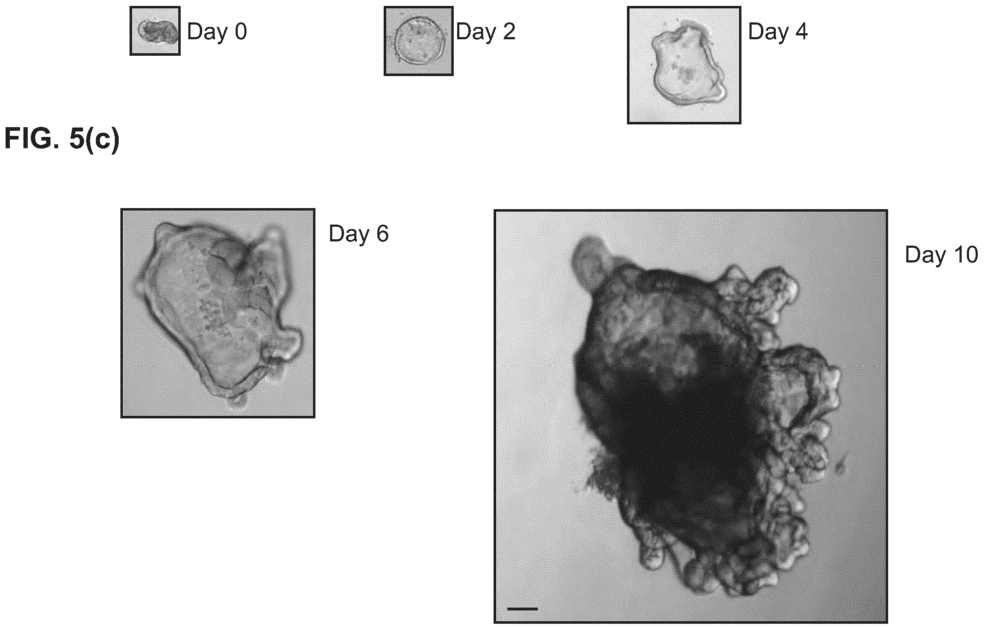

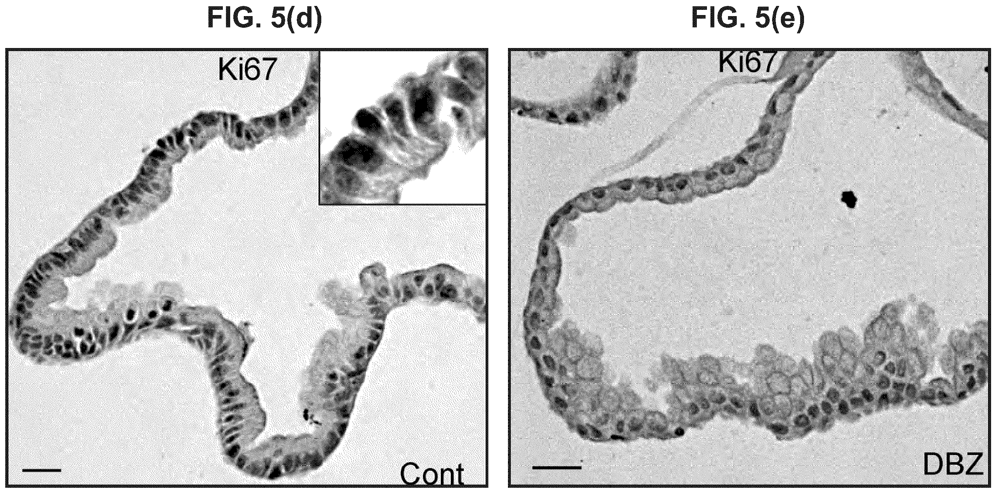

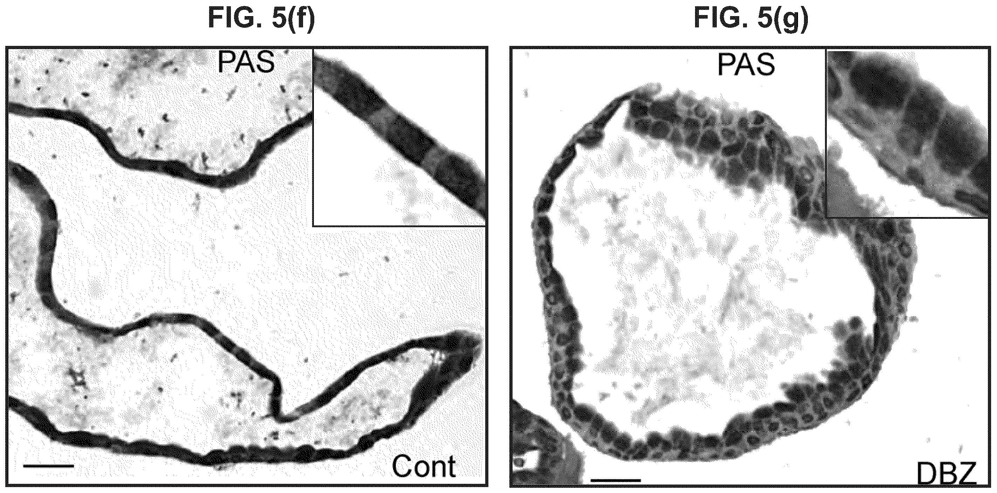

In some embodiments, the invention provides a method for obtaining a Barrett's Esophagus organoid, wherein the method comprises culturing isolated epithelium from Barrett's Esophagus for 1, 2, 3, 4, 5, 6, 7 or more days in HISC culture medium, optionally additionally comprising FGF10; and withdrawing nicotinamide and SB202190 after the first 1, 2, 3, 4 or more days. In some embodiments the culture medium additionally comprises a Notich inhibitor, such as DBZ. In some embodiments, a Barrett's Esophagus organoid cultured in the presence of a Notch inhibitor comprises almost no or no proliferating cells, and comprises more goblet cells relative to an organoid cultured in the absence of a Notch inhibitor (see figure 5).

### Liver culture media

In some embodiments, liver cells can be grown in a first "expansion" culture medium (also referred to herein as EM), preferably followed by culturing the cells in a second "differentiation" culture medium (also referred to herein as DM). However, in some embodiments, the step of differentiating in DM media is not carried out, for example in some methods, cells are transplanted and allowed to differentiate in vivo. Similarly, there are expansion culture media and differentiation culture media for other tissues, such as the pancreas, small intestine and colon (see above).

In one embodiment, the liver expansion medium comprises EGF, a Wnt agonist, FGF, and Nicotinamide. Preferably, the Wnt agonist is R-spondin 1-4 (for example any one or more of Rspondin 1, 2, 3, and 4) and so the expansion medium is referred to as "ERFNic". A particularly preferred expansion medium additionally comprises HGF and is referred to as "ERFHNic".

In some embodiments, the liver expansion medium is supplemented with a TGF beta inhibitor. In some embodiments, TGF beta is present at at least 5 nM, for example, at least 50nM, at least 100nM, at least 300nM, at least 450nM, at least 475nM, for example 5nM-500mM, 10nM-100mM, 50nM-700uM, 50nM-10uM, 100nM-1000nM, 350-650nM or more preferably 500nM. The presence of a TGF beta inhibitor in the expansion media is particularly preferred for human cell embodiments.

In some embodiments, the invention provides a culture medium for expanding liver cells, comprising or consisting of a basal medium, any one of Rspondin 1-4, Noggin, nicotinamide, EGF, FGF10, HGF, gastrin, a TGF-beta inhibitor and PGE2, and preferably Wnt-3a.

In some embodiments, the liver expansion medium further comprises a p38 inhibitor.

In some embodiments, the liver expansion medium is supplemented with an activator of the prostaglandin signalling pathway (also called a prostaglandin pathway activator) (see Figure 24). For example, the liver expansion medium may be supplemented with any one or more of the compounds selected from the list comprising: Phospholipids, Arachidonic acid (AA), prostaglandin E2 (PGE2), prostaglandin G2 (PGG2), prostaglandin F2 (PGF2), prostaglandin H2 (PGH2), prostaglandin D2 (PGD2). For example, in some embodiments, the liver expansion medium is supplemented with PGE2 and/or AA. In some embodiments, PGE2 is added to the liver expansion medium to a final concentration of at least 10 nM, at least 30nM, at least 40nM, at least 45 nM, at least 50nM, for example between 10 nM and 500 nM, between 10 nM and 400 nM, between 10 nM and 300 nM, between 10 nM and 200 nM, between 10 nM and 100 nM, between 20 nM and 50 nM. In a preferred embodiment, PGE2 is added to the liver expansion medium to a final concentration of 50 nM. In some embodiments, AA is added to the liver expansion medium to a final concentration of at least 1 ug/ml, for example at least 3 ug/ml, at least 5 ug/ml, at least 8 ug/ml, at least 9 ug/ml, at least 10 ug/ml, between 1 ug/ml and 1000 ug/ml, between 1 ug/ ml and 500 ug/ml, between 1 ug/ml and 100 ug/ml, between 1 ug/ml and 50 ug/ml, or between 5 ug/ml and 10 ug/ml. In a preferred embodiment, AA is added to the medium to a final concentration of 10 ug/ml.

In a preferred embodiment, the liver expansion medium is supplemented with both a TGF-beta inhibitor and an activator of the prostaglandin signalling pathway (for example, PGE2 and/or AA) and optionally a p38 inhibitor.

In preferred embodiments, the liver expansion medium additionally comprises gastrin.

In one embodiment, the liver differentiation medium comprises EGF, a TGF-beta inhibitor, FGF (for example, FGF10, FGF2 or any other suitable FGF family member) and a Notch inhibitor. In one embodiment, the TGF-beta inhibitor is A83-01 and/or the Notch inhibitor is DAPT. This differentiation medium is referred to herein as "EAFD" and is a preferred differentiation medium of the invention. FGF may optionally be replaced by HGF or alternatively both FGF and HGF may be present or absent in the differentiation medium. In some embodiments, EGF might be replaced by HGF or another receptor tyrosine kinase ligand. Dexamethasone may also be added, for example at a concentration of between 10nM to 10uM. The liver differentiation medium may optionally include a prostaglandin pathway activator, such as PGE2 or AA. However, this component may also be excluded from the differentiation medium. In some embodiments, oncostatin M may also be added, for example at a concentration range between 1ng/ml to 1mg/ml, to help differentiation to hepatocyte fate.

In some embodiments, the invention provides a culture medium for differentiating liver cells comprising or consisting of a basal medium, Noggin, EGF, gastrin, a TGF-beta inhibitor, a gamma-secretase inhibitor such as DAPT or DBZ, and preferably Wnt-3a.

In some embodiments, the liver cells may initially be cultured in an expansion medium that additionally contains Wnt and Noggin, for example an "ENRW" medium containing EGF, Noggin, R-spondin and Wnt (for example, Wnt-3A), optionally a prostaglandin pathway activator, such as PGE2 or AA, optionally a TGF beta inhibitor and optionally FGF, HGF, Nicotinamide. In a preferred embodiment, the liver expansion medium is supplemented with one of or more preferably both of a TGF-beta inhibitor and an activator of the prostaglandin signalling pathway (for example, PGE2 and/or AA).

In some embodiments, the expansion media for liver comprises EGF, Noggin, Gastrin, FGF, Nicotinamide, a TGF-beta inhibitor such as A83-01, HGF, RSpondin 1-4 (e.g. any one or more of Rspondin 1, 2, 3 and 4) and PGE2.

In a preferred embodiment, the liver cells may initially be cultured in an expansion media that contains EGF, noggin, gastrin, FGF10, nicotinamide, A8301, HGF and any one of Rspondin 1-4 supplemented with PGE2 and/or AA. Rspondin 1-4 may be provided in the form of Rspo conditioned media. For example, the expansion media may contain EGF (100ng/ml, Invitrogen); noggin (25ng/ml, peprotech); gastrin (10nM, sigma); FGF10 (e.g. 100ng/ml, peprotech); nicotinamide (10mM, sigma); A8301 (500nM, Tocris); HGF (50ng/ml, peprotech); Rspo conditioned media (10%, e.g. 1ug/ml) supplemented with PGE2 (50nM) and/or AA (10 ug/ml). The expansion medium may also contain a Rock inhibitor.

When expanding mouse liver cells, one or more of the following components may be excluded from the culture medium described above: TGF-beta inhibitor (e.g. A83-01) and PGE2.

The inventors have found that this medium is optimal for stimulating initial expansion of cells for the first few days. Therefore, this first expansion medium is sometimes referred to herein as EM1. In some embodiments, the Wnt and Noggin are removed after approximately 1 day, 2 days, 3 days, 4 days, 5 days, 6 days, 7 days or more, for example 2 weeks, 1 month, 5 weeks, 8 weeks, 2 months 3, 4, 5, 6, 7, 8, 9, 10, 11, 12 months or more, 14 months or more. In some embodiments, the cells may then be expanded in an expansion medium of the invention, as described above that does not contain Wnt or Noggin. This second expansion medium is sometimes referred to herein as EM2. In some embodiments, the cells are cultured in EM2 for approximately 1 day, 2 days, 3 days, 4 days, 5 days, 6 days, 7 days or a longer time period, such as 3, 4, 5, 10, 20 or more weeks. The culture medium may then be changed to an optimised differentiation medium, as described above, that contains a TGF-beta inhibitor and a Notch inhibitor. Typically, the differentiation medium does not contain a Wnt agonist, R-spondin or Nicotinamide. In some embodiments, the differentiation medium does not contain a prostaglandin pathway activator, such as PGE2 or AA. This encourages the differentiation of the cells towards mature hepatocytes and cholangyocytes. These cells are suitable for transplantation into humans or animals.

### Expansion Medium (EM2) for liver:

In one aspect of the present invention there is provided a cell culture medium which comprises or consists of a basal medium for animal or human cells to which is added: Epidermal Growth Factor, an FGF able to bind to FGFR2 or FGFR4, preferably FGF10 as a mitogenic growth factor, Nicotinamide, and preferably, a Wnt agonist, preferably R-spondin 1-4. This medium is referred to as EM2. This "EM2" medium is preferred for expanding liver cells.

In some embodiments, EM2 comprises a prostaglandin pathway activator such as PGE2 and/or AA.

In some embodiments, EM2 comprises a TGF-beta inhibitor such as A83-01.

Preferably, the Wnt agonist is R-spondin 1-4. A medium comprising EGF, R-spondin 1-4, FGF and Nicotinamide is referred to herein as ERFNic.

In some embodiments, the EM2 medium does not comprise noggin, and more preferably does not comprise a BMP-inhibitor. In some embodiments, the EM2 medium does not comprise Wnt, for example Wnt-3a.

In some embodiments, HGF is present in addition to FGF. A preferred medium comprising HGF in addition to FGF is ERFHNic (EGF + R-spondin (preferably R-spondin1-4) + FGF (preferably FGF10) + HGF + Nicotinamide + a prostaglandin pathway inhibitor such as PGE2 and/or AA and a TGF-beta inhibitor. The inventors have found that the ERFHNic medium containing the TGF-beta inhibitor and the prostaglandin pathway activator is the optimal medium for long-term expansion of cells. In the absence of HGF, cells did not remain viable in culture for longer than three months. Further, in the absence of HGF, after 10 passages, cells showed a growth disadvantage compared to cells cultured in the presence of HGF as evidenced by a lower proliferation ratio. In particular, after 15 passages, the cells were not growing organoids at the same speed ratio in the absence of HGF as in the presence of HGF. Thus, HGF was found to be essential for maintaining a good proliferation rate during long-term culture. Thus the invention provides the use of an ERFHNic medium of the invention for culturing cells for at least 2 weeks, at least 1 month, at least 2 months, more preferably at least 3, at least 4, at least 5, at least 6, at least 7, at least 8, at least 9, at least 10, at least 15, at least 20, at least 24, at least 25, at least 30 or more months, for example 3 or more years. In practice, some embodiments of the invention comprise the use of EM2 for around 20-50 passages of the cells. For example, the cells may be split 4-8 times once a week for 7-10 weeks in a row. Preferably the cells will expand at a rate of about 4-5 fold per week or more than two population doublings a week. The invention further provides the use of an ERFHNic medium of the invention for culturing cells for at least 8 passages, for example, at least 9, at least 10, at least 11, at least 12, at least 15, at least 20, at least 25, at least 30, at least 40, at least 50, at least 60 passages or for between 15-35 passages, for example approximately 20-30 passages. In some embodiments, a TGF-beta inhibitor such as A83-01 is additionally present in the EM2 medium. This is particularly useful when human cells or organoids are being cultured. In some embodiments, the A83-01 is present at a concentration of between 400-600 nM, for example 450-550 nM, 470-530 nM or approximately 500 nM. In embodiments in which a TGF-beta inhibitor is present in EM2, a Notch inhibitor is preferably not present. In some embodiments, EM2 additionally comprises a p38 inhibitor.

### Expansion Medium (EM1) for liver:

In one aspect, the invention provides a cell culture medium comprising or consisting of a basal medium for animal or human cells to which is added EGF, a BMP inhibitor, R-spondin Wnt. Preferably, the BMP inhibitor is Noggin and the EM1 medium is termed "ENRW" (EGF, Noggin, R-spondin and Wnt (e.g. Wnt3A)). This medium is referred to as EM1. In some embodiments, EM1 additionally comprises a prostaglandin pathway activator such as PGE2 and/or AA. In some embodiments, EM1 comprises a TGF-beta inhibitor such as A83-01. More preferably, the EM1 additionally comprises a prostaglandin pathway activator and a TGF-beta inhibitor. The inventors have found that a medium containing Wnt and Noggin is ideal for stimulating initial expansion of cells. Thus, in some embodiments, the EM1 medium is used for just 1 passage or 1 week but it is also envisaged that EM1 medium can be used for around a year because it is not harmful for the cells. Thus, in some embodiments, an EM1 medium is used for culturing cells from day 0 to day 10, for example from days 0-7, days 0- 6, days 0-5, days 0-4, days 0-3, days 0-2, days 0-1, wherein day 0 is the day that the cells are isolated from their tissue of origin and day 1 is the subsequent day or is used for 1 or more weeks for example 2, 3, 4 or more weeks or 1, 2, 3, 4, 5, 6, 7, 8, 9, 10, 11, 12 or more months. In some embodiments, the EM1 medium is used only for the first day or first two days of culture. In some embodiments, EM1 medium is used for 1 or more passages, for example, 1, 2, 3, 4, 5, 6, 7, 8, 9, 10, 15, 20, 25, 30 or more passages, for example, 20-30 passages, 30-35 passages, 32-40 passages or more. In some embodiments, the EM1 medium is used subsequent to a freezing step or any other transportation step involving a medium or temperature change that does not combine with optimal growth. This "EM1" medium is preferred for expanding liver cells.

The EM1 medium is supplemented with one or more of the compounds selected from the group consisting of FGF, HGF, Nicotinamide, gastrin, B27, N-acetyleystein and N2. In the case of starting the cultures from a frozen stock or from a single cell, the EM1 media is preferably supplemented with a ROCK inhibitor. Y27632 is the preferred ROCK inhibitor for use in the invention.

Thus, in one embodiment there is provided a cell culture medium which comprises or consists of a basal medium for animal or human cells to which is added: Epidermal Growth Factor, an FGF (for example, an FGF able to bind to FGFR2 or FGFR4), preferably FGF10 and HGF as mitogenic growth factors,
a prostaglandin pathway activator, such as PGE2 and/or AA;
a TGF-beta inhibitor;
gastrin, Nicotinamide, B27, N2 and N-Acetylcysteine, and preferably;
a BMP inhibitor, preferably Noggin; and
a Wnt agonist, preferably R-spondin 1 and/or Wnt-3a.

B27 (Invitrogen), N-Acetyleysteine (Sigma) and N2 (Invitrogen), Gastrin (Sigma) and Nicotinamide (Sigma) are also added to the medium defined above and are believed to control proliferation of the cells and assist with DNA stability. In the context of the invention, Nicotinamide is also referred to herein as "Nic".

'N2 Supplement' is available from Invitrogen, Carlsbad, CA; www.invitrogen.com; catalog no. 17502-048; and from PAA Laboratories GmbH, Pasching, Austria; www.paa.com; catalog no. F005-004; Bottenstein & Sato, PNAS, 76(1):514-517, 1979. N2 Supplement is supplied by PAA Laboratories GmbH as a 100x liquid concentrate, containing 500µg/ml human transferrin, 500µg/ml bovine insulin, 0.63µg/ml progesterone, 1611µg/ml putrescine, and 0.52µg/ml sodium selenite. N2 Supplement may be added to a culture medium as a concentrate or diluted before addition to a culture medium. It may be used at a 1x final concentration or at other final concentrations. Use of N2 Supplement is a convenient way to incorporate transferrin, insulin, progesterone, putrescine and sodium selenite into a culture medium of the invention. In some embodiments in which the medium comprises B27, it does not also comprise N2. The embodiments of the present invention can therefore be adapted to exclude N2 when B27 is present, if desired.

'B27 Supplement' (available from Invitrogen, Carlsbad, CA; www.invitrogen.com; currently catalog no. 17504-044; and from PAA Laboratories GmbH, Pasching, Austria; www.paa.com; catalog no. F01-002; Brewer et al., J Neurosci Res., 35(5):567-76, 1993) may be used to formulate a culture medium that comprises biotin, cholesterol, linoleic acid, linolenic acid, progesterone, putrescine, retinol, retinyl acetate, sodium selenite, tri-iodothyronine (T3), DL-alpha tocopherol (vitamin E), albumin, insulin and transferrin. B27 Supplement is supplied by PAA Laboratories GmbH as a liquid 50x concentrate, containing amongst other ingredients biotin, cholesterol, linoleic acid, linolenic acid, progesterone, putrescine, retinol, retinyl acetate, sodium selenite, tri-iodothyronine (T3), DL-alpha tocopherol (vitamin E), albumin, insulin and transferrin. Of these ingredients at least linolenic acid, retinol, retinyl acetate and tri-iodothyronine (T3) are nuclear hormone receptor agonists. B27 Supplement may be added to a culture medium as a concentrate or diluted before addition to a culture medium. It may be used at a 1x final concentration or at other final concentrations. Use of B27 Supplement is a convenient way to incorporate biotin, cholesterol, linoleic acid, linolenic acid, progesterone, putrescine, retinol, retinyl acetate, sodium selenite, tri-iodothyronine (T3), DL-alpha tocopherol (vitamin E), albumin, insulin and transferrin into a culture medium of the invention.

For example, a cell culture medium may comprise or consist of a basal medium to which is added: EGF and R-spondin 1 supplemented with FGF10, HGF and Nicotinamide; for example, EGF (50 ng/ml) and R-spondin 1 (1ug/ml) supplemented with FGF10 (100ng/ml), HGF (25-50ng/ml), Nicotinamide (1-10mM), a prostaglandin pathway activator, such as PGE2 (50 nM) and/or AA (10 ug/ml) and a TGF-beta inhibitor such as A83-01 (500nM). In some embodiments the medium additionally comprises a p38 inhibitor. The inventors have found that this medium may be used for long-term expansion of cells. Thus, this cell culture medium is preferred for use as an EM2 of the invention. The basal medium is preferably supplemented with B27, N2 and 200ng/ml N-Acetylcysteine. In some embodiments, the basal medium is Advanced-DMEM/F12. However, any other suitable basal medium may be used.

Another example of a cell culture medium, and method of using this medium comprises or consists of Advanced-DMEM/F12 preferably supplemented with B27, N2, 200ng/ml N-Acetylcysteine, 50ng/ml EGF, 1 µg/ml R-spondin1, 10 nM gastrin, 100 ng/ml FGF10, 10mM Nicotinamide, 50ng/ml HGF, 50% Wnt conditioned media, a prostaglandin pathway activator, such as PGE2 (50 nM) and/or AA (10 ug/ml) and a TGF-beta inhibitor such as A83-01 (500nM) and, preferably 10-100ng/ml Noggin. Wnt conditioned media comprises Advanced DMEM, P/S, B27, N2 and also FCS. 293T cells transfected with Wnt3A expression plasmid produce Wnt. The whole medium is taken after a few days (i.e. with secreted Wnt) and used as the Wnt source.

The invention therefore provides a cell culture medium, comprising or consisting of a basal medium for animal or human cells to which is added:
Epidermal Growth Factor, an FGF able to bind to FGFR2 or FGFR4, preferably FGF10 and HGF as mitogenic growth factors,
a prostaglandin pathway activator, such as PGE2 and/or AA,
a TGF-beta inhibitor;
gastrin, Nicotinamide, B27, N2 and N-Acetylcystein, and preferably
a BMP inhibitor more preferably Noggin and
a Wnt agonist, more preferably R-spondin 1 and/or Wnt-3a.

The invention thus encompasses a first preferred culture medium comprising or consisting of a basal medium for animal or human cells to which is added:
Epidermal Growth Factor, FGF10 and HGF as mitogenic growth factors,
a prostaglandin pathway activator, such as PGE2 and/or AA,
a TGF-beta inhibitor;
gastrin, Nicotinamide, B27, N2 and N-Acetylcysteine,
a BMP inhibitor more preferably Noggin and
a Wnt agonist, more preferably R-spondin 1 and Wnt-3a.

In some embodiments, a p38 inhibitor is added to the expansion medium.

This medium may be used as an EM1 cell culture medium of the invention to stimulate initial expansion of cells. In some embodiments, the medium used as an EM1 cell culture medium comprises all the components of an EM2 culture medium of the invention and additionally comprises Wnt-3a and Noggin.

In embodiments in which the basal medium is supplemented with N-Acetylcysteine, B27 and N2, the following are preferably added to the culture media: EGF, R-spondin1, gastrin, FGF10, Nicotinamide and HGF and Wnt-conditioned media and a prostaglandin pathway activator, such as PGE2 and/or AA. Preferably, the basal medium is supplemented with N-Acetyleysteine, EGF, R-spondin1, gastrin, FGF10, Nicotinamide and HGF and Wnt-conditioned media and a prostaglandin pathway activator, such as PGE2 and/or AA in accordance with the quantities described hereinabove. Preferably, a TGF-beta inhibitor is also present at the quantities described herein.

For example, in some embodiments the basal medium may be supplemented with 150ng/ml to 250 ng/ml N-Acetylcysteine; preferably, the basal medium is supplemented with, about or exactly 200ng/ml N-Acetyleysteine. For example, in some embodiments the basal medium may be supplemented with 40ng/ml to 60ng/ml EGF; preferably, the basal medium is supplemented with about or exactly 50ng/ml EGF. For example, in some embodiments the basal medium may be supplemented with 0.5 µg/ml to 1.5 µg/ml R-spondin1; preferably, the basal medium is supplemented with about or exactly 1 µg/ml R-spondin1. For example, **in** some embodiments the basal medium may be supplemented with 5nM to 15nM gastrin; preferably, the basal medium is supplemented with about or exactly 10nM gastrin. For example, in some embodiments the basal medium may be supplemented with 25-200ng/ml FGF10, for example 70 ng/ml to 130 ng/ml FGF10; preferably, the basal medium is supplemented with about or exactly 100 ng/ml FGF10. For example, in some embodiments the basal medium may be supplemented with 5mM to 15mM Nicotinamide; preferably, the basal medium is supplemented with about or exactly 10mM Nicotinamide. For example, in some embodiments the basal medium may be supplemented with 25ng/ml to 100 ng/ml HGF, for example 35ng/ml to 65ng/ml HGF; preferably, the basal medium is supplemented with about or exactly and 50ng/ml HGF. For example, in some embodiments the basal medium may be supplemented with 35% to 65% Wnt-conditioned media; preferably, the basal medium is supplemented with about or exactly 50% Wnt-conditioned media.

For example, in some embodiments, the liver expansion medium is supplemented with an activator of the prostaglandin signalling pathway (see Figure 24). For example, the liver expansion medium may be supplemented with any one or more of the compounds selected from the list comprising: Phospholipids, Arachidonic acid (AA), prostaglandin E2 (PGE2), prostaglandin G2 (PGG2), prostaglandin F2 (PGF2), prostaglandin H2 (PGH2), prostaglandin D2 (PGD2). For example, in some embodiments, the liver expansion medium is supplemented with PGE2 and/or AA. In some embodiments, PGE2 is added to the liver expansion medium to a final concentration of at least 10 nM, for example between 10 nM and 500 nM, between 10 nM, and 400 nM, between 10 nM and 300 nM, between 10 nM and 200 nM, between 10 nM and 100 nM, between 20 nM and 50 nM. In a preferred embodiment, PGE2 is added to the liver expansion medium to a final concentration of 50 nM. In some embodiments, AA is added to the liver expansion medium to a final concentration of at least 1 ug/ml, for example between 1 ug/ml and 1000 ug/ml, between 1 ug/ ml and 500 ug/ml, between 1 ug/ml and 100 ug/ml, between 1 ug/ml and 50 ug/ml, or between 5 ug/ml and 10 ug/ml. In a preferred embodiment, AA is added to the medium to a final concentration of 10 ug/ml.

In some embodiments one or both of gastrin and N2 are not present in the cell culture medium.

Preferably, the basal medium is advanced-DMEM/F12.

This first culture medium (for example, EM1, EM2 or both EM1 and EM2) is preferably used during the first two weeks of the culture method of the invention. However, it may be used for a shorter time period, such as for 1, 2, 3, 5, 7, or 10 days, or a longer time period, such as 3, 4, 5, 10, 20 or more weeks, 5 months or more, for example, 6, 7, 8, 9, 10, 11, 12 months or more.

### Differentiation Medium (DM) for liver:

In another aspect, there is provided a second cell culture medium which comprises or consists of a basal medium for animal or human cells to which is added: EGF, a TGF-beta inhibitor, a Notch inhibitor and a prostaglandin pathway activator, such as PGE2 and/or AA. The inventors have found that this medium is useful for differentiating cells. The medium used for differentiating the cells may be referred to herein as DM.

Preferably, the second cell culture medium also comprises FGF and/or HGF.

In one embodiment, the second culture medium comprises or consists of a basal medium for animal or human cells to which is added:
Epidermal Growth Factor, FGF10 and HGF as mitogenic growth factors;
a Notch inhibitor;
a TGF-beta inhibitor; and
a prostaglandin pathway activator, such as PGE2 and/or AA.

In one embodiment, the TGF-beta inhibitor is A83-01 and/or the Notch inhibitor is DAPT. In another embodiment, the DM cell culture medium additionally comprises Dexamethasone. In another embodiment, the DM cell culture medium additionally comprises Oncostatin M. In another embodiment, the DM cell culture medium additionally comprises gastrin.

A preferred second cell culture medium, and method of using this medium, is described in the examples, and comprises or consists of a basal medium to which is added: 50ng/ml EGF, 100 ng/ml FGF10, 50 nM A8301 and 10 uM DAPT. Advanced-DMEM/F12 may be used as the basal medium as may any other suitable basal medium.

In some embodiments, the differentiation medium for liver cells, for example for human liver cells, comprises or consists of a basal medium (for example comprising Advanced DMEM/F12, B27 (50x), n-Acetylcystein (1 mM) glutamin/glutamax), Noggin (preferably 100 ng/ml), EGF (preferably 50 ng/ml), gastrin (preferably 10nM), TGF-beta inhibitor, such as A83-01 (preferably 50 nM) and a gamma-secretase inhibitor (for example DAPT/DBZ) (preferably 10 uM).

In some embodiments, the differentiation medium for liver cells, for example for mouse liver cells, comprises or consists of a basal medium (for example comprising Advanced DMEM/F12, B27 (50x), n-Acetylcystein (preferably 1mM) glutamin/glutamax), EGF (preferably 50 ng/ml), FGF10 (preferably 100 ng/ml) gastrin (preferably 10nM), TGF-beta inhibitor, such as A83-01 (preferably 50 nM) and a gamma-secretase inhibitor (for example DAPT/DBZ) (preferably 10 uM).

In some embodiments, the second cell culture medium does not comprise R-spondin or Wnt. In some embodiments, the second cell culture medium does not comprise a Wnt agonist. In some embodiments, the second cell culture medium does not comprise Nicotinamide. In some embodiments, the second cell culture medium does not comprise a BMP inhibitor. In some embodiments, the second cell culture medium does not comprise a prostaglandin pathway activator, such as PGE2 and/or AA.

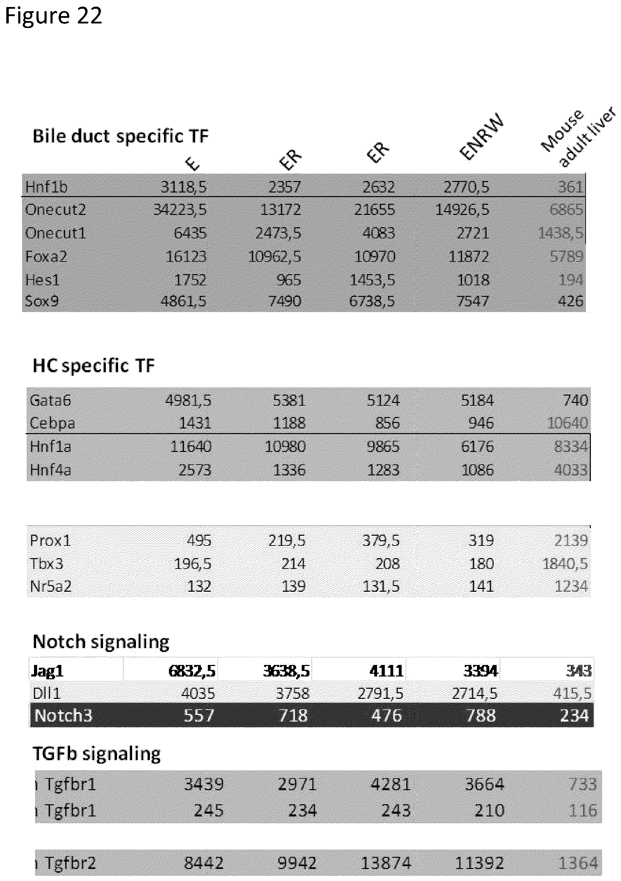

The inventors have discovered that R-spondin1 and Nicotinamide both inhibit the expression of the mature hepatocyte marker CYP3A11 and yet promote the expression of the hepatoblast marker albumin. Therefore, to increase differentiation of the cells to more mature liver fates, the inventors removed R-spondin and Nicotinamide from the cell culture. The inventors have also discovered that the expression of specific biliary transcription factors is highly upregulated in expansion cultures containing R-spondin1, indicating that the culture gene expression was unbalanced towards a more biliary cell fate. Notch and TGF-beta signaling pathways have been implicated in biliary cell fate *in vivo.* In fact, deletion of Rbpj (essential to achieve active Notch signalling) results in abnormal tubulogenesis (Zong Y. Development 2009) and the addition of TGF-beta to liver explants facilitates the biliary differentiation *in vitro* (Clotman F. Genes and Development 2005). Since both Notch and TGF-beta signalling pathways were highly upregulated in the liver cultures (Figure 22) the inventors reasoned that inhibition of biliary duct cell-fate might trigger the differentiation of the cells towards a more hepatocytic phenotype. It was found that addition of a TGF-beta inhibitor (such as A8301) and a Notch inhibitor (such as DAPT) to a differentiation medium that preferably does not contain R-spondin or Wnt, enhances the expression of mature hepatocyte markers and increases the number of hepatocyte-like cells (for example, see example 5).

### General culture media

A cell culture medium according to the invention allows the survival and/or proliferation and/or differentiation of epithelial stem cells or isolated crypts on an extracellular matrix. In some embodiments, a cell culture medium according to the invention allows the survival and/or proliferation and/or differentiation of an organoid of the invention, such as a crypt-villus organoid, a colon organoid, a pancreatic organoid, a gastric organoid, a Barret's Esophagus organoid, an adenocarcinoma organoid or a colon carcinoma organoid on an extracellular matrix. In some embodiments, a cell culture medium according to the invention allows the survival and/or proliferation and/or differentiation of an organoid of the invention, such as a small intestinal (crypt-villus) organoid, a colon organoid, a pancreatic organoid, a gastric organoid, a Barret's Esophagus organoid, an adenocarcinoma organoid, a carcinoma organoid, a colon carcinoma organoid, a prostate organoid or a prostate carcinoma organoid on an extracellular matrix. Preferably, in embodiments in which a TGF-beta inhibitor and/or p38 inhibitor is present the cell culture medium allows the survival and/or proliferation, preferably the survival and proliferation of a population of cells or organoid of the invention. Preferably, embodiments in which a TGF-beta inhibitor and/or p38 inhibitor is initially present in a cell culture medium but is then removed from the medium (e.g. by failing to add it when the medium is refreshed), allow the survival and/or differentiation, preferably the survival and differentiation of a population of cells or organoid of the invention.

In some embodiments, a p38 inhibitor is added to any of the media described herein.

The term cell culture medium is synonymous with medium, culture medium or cell medium.

### Uses of culture media of the invention

The invention also provides the use of a culture medium of the invention for expanding and/or differentiating a stem cell, population of stem cells, tissue fragment or organoid.

In some embodiments, the stem cell, population of stem cells, tissue fragment or organoid is selected from the group consisting of one or more intestinal stem cells, small intestinal crypts, colonic crypts, gastric stem cells, liver stem cells, pancreas stem cells and prostate stem cells.

In some embodiments, the stem cell, population of stem cells, tissue fragment or organoid is obtainable from a normal tissue.

In some embodiments, the stem cell, population of stem cells, tissue fragment or organoid is obtainable from a diseased tissue, for example from an adenoma, a carcinoma, an adenocarcinoma, an intestine of a patient having cystic fibrosis or an intestine of a patient having inflammatory bowel disease.

### Stem cells cultured according to the invention

Stem cells are found in many organs of adult humans and mice. Although there may be great variation in the exact characteristics of adult stem cells in individual tissues, adult stem cells share at least the following characteristics: they retain an undifferentiated phenotype; their offspring can differentiate towards all lineages present in the pertinent tissue; they retain self-maintenance capabilities throughout life; and they are able to regenerate the pertinent tissue after injury. Stem cells reside in a specialised location, the stem cell niche, which supplies the appropriate cell-cell contacts and signals for maintenance of said stem cell population. The stem cells according to the invention preferably express Lgr5.

In one embodiment, the invention provides a population of cells or one or more organoids comprising said stem cells that have been generated or obtained by culturing stem cells or tissue fragments according to the invention, which have been cultured for at least 3 months, preferably at least 4 months, at least 5 months, at least 6 months, at least 7 months, at least 9 months, or at least 12 months or more.

A 'population' of cells is any number of cells greater than 1, but is preferably at least 1x10³ cells, at least 1x10⁴ cells, at least 1x10⁵ cells, at least 1x10⁶ cells, at least 1x10⁷ cells, at least 1x10⁸ cells, or at least 1x10⁹ cells.

The stem cells of the invention cultured according to the invention may be human stem cells. The stem cells of the invention cultured according to the invention may be epithelial stem cells.

In some embodiments, the stem cells of the invention and/or cultured according to the invention are not embryonic stem cells. In some embodiments the stem cells of the invention and/or cultured according to the invention are not human embryonic stem cells. Preferably, the stem cells of the invention are adult stem cells.

In a preferred embodiment, the stem cells may be human epithelial stem cells. Human epithelial stem cells include stem cells of human epithelial tissue origin. These include, but are not limited to pancreatic, small intestinal, large intestinal, corneal, olfactory, respiratory tissues, gastric tissues, liver and skin, mammary and/or prostatic tissues, for example, a tissue selected from the group consisting of pancreatic, small intestinal, large intestinal, corneal, olfactory, and respiratory tissues. Epithelial stem cells are able to form the distinct cell types of which the epithelium is composed. Some epithelia, such as skin or intestine, show rapid cell turnover, indicating that the residing stem cells must be continuously proliferating. Other epithelia, such as the liver or pancreas, show a very slow turnover under normal conditions.

### Intestinal stem cells

The self-renewing epithelium of the small intestine is ordered into crypts and villi (Gregoreff and Clevers, 2005 Genes Dev 19, 877-90). Each cell along the crypt-villus axis is polarized, whereby cells on the top of the intestinal villi, or in the upper positions of colonic crypts, are the most differentiated and are continuously lost into the lumen by apoptosis. Continuous proliferation of stem cells residing in the base of the crypts, and massive proliferation of progenitor cells residing in the middle of the crypts, ensures proper replacement of the shed cells. There is a resulting epithelial turnover time of 5 days in the mouse. Self-renewing stem cells have long been known to reside near the crypt bottom and to produce the rapidly proliferating transit amplifying (TA) cells capable of differentiating towards all lineages. The estimated number of stem cells is between 4 and 6 per crypt (Bjerknes and Cheng, 1999 Gastroenterology 1 16, 7- 14). Three differentiated cell types, enterocytes, goblet cells and enteroendocrine cells, form from TA cells and continue their migration in coherent bands along the crypt-villus axis. Each villus receives cells from multiple different crypts. The fourth major differentiated cell-type, the Paneth cell, resides at the crypt bottom.

The colon resembles the small intestine but with a flat surface epithelium rather than villi. Colon crypts are organized like small intestinal crypts. Paneth cells are not present in colon crypts; instead there are so-called "Deep Crypt Secretory" cells. The flat surface of the epithelium contains the differentiated cells (colonocytes and secretory cells). Differentiated goblet cells occur throughout the crypt, also intermingled with transit amplifying cells.

### Isolation of tissue fragments and stem cells

Crypts can be isolated from the small and large intestine, including the duodenum, jejunum, ileum and colon, and the pyloric and corpus region of the stomach by protocols that are known to the skilled person. For example, crypts can be isolated by incubation of isolated tissue with chelating agents that release cells from their calcium- and magnesium-dependent interactions with the basement membrane and stromal cell types. After washing the tissue, the epithelial cell layer is scraped from the submucosa with a glass slide and minced. This is followed by incubation in trypsin or, more preferred, EDTA and/or EGTA and separation of undigested tissue fragments and single cells from crypts using, for example, filtration and/or centrifugations steps. Other proteolytic enzymes, such as collagenase and/or dispase I, can be used instead of trypsin. Similar methods are used to isolate fragments of the pancreas and stomach. Similar methods may be used to isolated fragments of other tissues described herein. The culture media of the invention are suitable for culturing such tissue fragments (see Example 1).

A culture medium according to the invention allows the establishment of long-term culture conditions under which single crypts undergo multiple crypt fission events, while simultaneously generating villus-like epithelial domains in which all differentiated cell types are present. Cultured crypts undergo dramatic morphological changes after taking them into culture. The upper opening of freshly isolated crypts becomes sealed and this region gradually balloons out and becomes filled with apoptotic cells, much like apoptotic cells are pinched off at the villus tip. The crypt region undergoes continuous budding events which create additional crypts, a process reminiscent of crypt fission. In a preferred embodiment of the invention, the organoids comprise crypt-like extensions which comprise all differentiated epithelial cell types, including proliferative cells, Paneth cells, enterocytes and goblet cells. No myofibroblasts or other non-epithelial cells were identified in the organoids at any stage.

Expansion of the budding crypt structures creates organoids, comprising crypt-like structures surrounding a central lumen lined by a villus-like epithelium and filled with apoptotic cell bodies. The crypt-villus organoids comprise a central lumen lined by a villus-like epithelium. The lumen is opened at consecutive time intervals to release the content into the medium.

A similar crypt-villus organoid structure is formed when single epithelial stem cells are cultured. After about one week, structures are formed that strongly resemble the crypt-villus organoid structures that are obtained with intact crypts.

Methods to isolate stem cells are known and suitable methods for use with this invention can be selected by the skilled person depending on the stem cell type that is used. For example, isolation of epithelial stem cells may be performed using compounds that bind to Lgr5 and/or Lgr6, which are unique cell surface markers on epithelial stem cells. Examples of such compounds are anti-Lgr5 and anti-Lgr6 antibodies.

In some embodiments of the invention, single Lgr5+ epithelial stem cells, for example from the colon, small intestine, or pancreas, may be used to form organoids, such as colonic, crypt-villus or pancreatic organoids respectively.

In a further example, single Lgr5+ epithelial stem cells from the liver, prostate or stomach may be used to obtain organoids, such as liver, prostate or gastric organoids respectively.

In an alternative embodiment, tissue fragments, such as cultured crypts from the intestinal tract, comprising Lgr5+ stem cells may be used to obtain organoids using methods and culture media described herein.

In some embodiments the single Lgr5+ epithelial stem cell or tissue fragment may be a cancer stem cell or cancer tissue fragment, for example from a carcinoma or adenocarcinoma. In some embodiments the single Lgr5+ epithelial stem cell may be a stem cell or tissue fragment from a neoplastic pathology or diseased tissue, for example Barrett's esophagus, cystic fibrosis or adenoma. Organoids obtained from cancerous, neoplastic or diseased starting material have characterisitics resembling the *in vivo* starting material and therefore are useful as a research tool for drug screening, target validation, target discovery, toxicology and toxicology screens, personalized medicine, regenerative medicine and *ex vivo* cell/organ models, for example disease models.In one embodiment, the invention provides organoids generated or obtained by culturing human stem cells or tissue fragments according to a method of the invention. In one embodiment, the invention provides crypt-villus organoids or gastric organoids or pancreatic organoids or colon organoids or Barrett's Esophagus organoids or adenocarcinoma organoids or colon carcinoma organoids generated or obtained by culturing human stem cells or tissue fragments according to a method of the invention. In one embodiment, the invention provides prostate organoids generated or obtained by culturing human stem cells or tissue fragments according to a method of the invention. Such a population of organoids, for example, crypt-villus, gastric or pancreatic organoids, generated or obtained by culturing human stem cells or tissue fragments according to a method of the invention, may each comprise more than 10, preferably more than 20, more preferably more than 40 organoids. Said collection of organoids preferably comprises at least 10% viable cells, more preferred at least 20% viable cells, more preferred at least 50% viable cells, more preferred at least 60% viable cells, more preferred at least 70% viable cells, more preferred at least 80% viable cells, more preferred at least 90% viable cells. Viability of cells may be assessed using Hoechst staining or Propidium Iodide staining in FACS.

The inventors have shown that the culture media and methods of the invention may be used for culture of cancer cell lines, including colorectal cancer and adenocarcinoma (see Example 1). As explained in Example 1, the culture technology is widely applicable as a research tool for infectious, inflammatory and neoplastic pathologies. Accordingly, the stem cells according to the invention may be cancer stem cells. In some embodiments of the invention, cancer stem cells can form adenoma or colon cancer organoids. In some embodiments, these organoids comprise cells which are Ki67+ (Thermo Scientific* Cellomics, Millipore).

Similarly, the inventors have shown that the culture media and methods of the invention may be used for culturing stem cells with other diseased genotypes and/or phenotypes. For example, intestinal stem cells taken from patients with cystic fibrosis can be expanded using the culture media and methods of the invention. These stem cells maintain the cystic fibrosis genotype and phenotype. Therefore, in some embodiments of the invention, the stem cells are taken from a patient with a disease, for example cystic fibrosis, inflammatory bowel disease (such as Crohn's disease), carcinoma, adenoma, adenocarcinoma, colon cancer, diabetes (such as type I or type II), Barrett's esophagus Gaucher's disease, alpha-1-antitrypsin deficiency, Lesch-Nyhan syndrome, anaemia, Schwachman-Bodian-Diamond syndrome, polycythaemia vera, primary myelofibrosis, glycogen storage disease, familial hypercholestrolaemia, Crigler-Najjar syndrome, hereditary tyrosinanaemia, Pompe disease, progressive familial cholestasis, Hreler syndrome, SCID or leaky SCID, Omenn syndrome, Cartilage-hair hypoplasia, Herpes simplex encephalitis, Scleroderma, Osteogenesis imperfecta, Becker muscular dystrophy, Duchenne muscular dystrophy, Dyskeratosis congenitor, etc. In some embodiments of the invention, disease organoids can be obtained by culturing stem cells taken from a human or animal with a disease. Disease organoids still have characteristics of the tissue from which they were obtained. Therefore, a cystic fibrosis small intestinal organoid grown from a small intestinal crypt falls within the definition of a small intestinal organoid. Similarly, a colon carcinoma organoid falls within the definition of a colon organoid.

There is some confusion in the literature as to the definition of a cancer stem cell. Here, we follow the consensus reached at a recent AACR workshop (Clarke et al., 2006. Cancer Res. 66:9339-44), which states that the cancer stem cell "is a cell within a tumor that possesses the capacity to self-renew and to cause the heterogeneous lineages of cancer cells that comprise the tumor. Cancer stem cells can thus only be defined experimentally by their ability to recapitulate the generation of a continuously growing tumor". Alternative terms in the literature include tumor-initiating cell and tumorigenic cell. Assays for cancer stem cell activity need to address the potential of self-renewal and of tumor propagation. The gold-standard assay currently is serial xeno-transplantation into immunodeficient mice. In addition, cancer stem cells in the context of this invention normally express Lgr5. However, in some embodiments, cancer initiating/propagating/stem cells that do not express Lgr5 can also be cultured by the culture media and methods of the invention.

### Genomic and phenotypic integrity of stem cells and organoids comprising said stem cells

Clinical and research applications for stem cells and their differentiated progeny require reproducible stem cell culture methods that provide populations of cells of suitable quality. Generally, *in vitro* expansion of stem cells aims to provide a population of cells which resemble their *in vivo* counterparts as closely as possible. This property is herein referred to as the "genomic and phenotypic integrity" of the cells. Organoids obtained by culturing diseased cells, such as cancer cells or cystic fibrosis cells, also resemble their *in vivo* counterparts i.e. they maintain their disease genotype and/or phenotype and therefore, also maintain their "genomic and phenotypic integrity" in that sense i.e. they maintain the genetic or phenotypic instability characteristic of the disease that is remincent of the *in vivo* situation. Therefore, in some embodiments, the invention provides "normal" organoids obtained from healthy tissue. In other embodiments, the invention provides "disease" organoids, such as cancer organoids (for example, colon carcinoma organoids or adenocarcinoma organoids) or cystic fibrosis small intestinal organoids obtained from diseased tissue.

For the first time, the inventors have discovered that it is possible to expand human epithelial stem cells **in** culture, with minimal loss of genomic and phenotypic integrity, for at least 3 months, preferably at least 4 months, at least 5 months, at least 6 months, at least 7 months, at least 9 months, or at least 12 months or more (see Example 1). Under the improved culture conditions of the invention, human intestinal organoids displayed budding organoid structures, rather than the cystic structures seen under previous culture conditions. Metaphase spreads of organoids more than 3 months old consistently revealed 46 chromosomes in each of the 20 cells taken from three different donors. Furthermore, microarray analysis revealed that the stem cells in culture possessed similar molecular signatures to intestinal crypt cells including intestinal stem cell genes.

Therefore, in some embodiments the invention provides organoids that have been grown for at least 3 months, preferably at least 4 months, at least 5 months, at least 6 months, at least 7 months, at least 9 months, or at least 12 months or more with minimal loss of genomic and phenotypic integrity.

In some embodiments, the invention provides human intestinal organoids comprising budding structures. In some embodiments of the invention, human intestinal organoids do not comprise cystic structures. In some embodiments of the invention, human intestinal organoids comprise more budding structures than cystic structures.The inventors also demonstrated that the human intestinal organoids generated by media and methods of the present invention, mimicked *in vivo* cell fate decisions in response to external factors. For example, it has previously been shown that Notch inhibition **in** intestinal stem cells, terminates intestinal epithelial proliferation and induces goblet cell hyperplasia *in vivo.* The inventors were able to show that the intestinal organoids of the invention, when treated with a Notch inhibitor, ceased proliferation and most cells converted into goblet cells within 3 days.

These results show the dramatic improvement **in** the genomic and phenotypic integrity of the stem cells and organoids produced by the methods and media of the present invention compared to previous methods and media.

The genomic integrity of stem cells of the invention can be confirmed by karyotype analysis. Stem cells and their progeny can be karyotyped using known methods as described **in** Sato, T et al., Single Lgr5 stem cells build crypt-villus structures in vitro without a mesenchymal niche. Nature 459, 262-265, 2009.

A "normal karyotype" is one where all chromosomes are present (i.e. euploidy) with no noticeable alterations. Accordingly, in preferred embodiments of the invention more than 50%; more than 70%; more than 80%; more than 90%; more than 95%; or more than 99% of the stem cells and differentiated cells in an expanded population exhibit normal karyotypes after 1, 2, 3, 4, 5, 6, 9, 12 or more months. The term "expanded population" encompasses organoids.

A "normal phenotype" refers to cells which display, to a first approximation, the same visual characteristics, gene expression and behaviour as the average *in vivo* counterpart cell. In preferred embodiments of the invention more than 50%; more than 70%; more than 80%; more than 90%; more than 95%; or more than 99% of the stem cells in an expanded population cultured according to the invention exhibit normal phenotypes after 1, 2, 3, 4, 5, 6, 9, 12 or more months.

For example, visually a normal phenotype may be judged by the number of dead cells outside the organoid, the amount of 'budding' of the organoid compared to cystic growth (budding structures are preferred), and the overall integrity of the single layer of epithelial cells (e.g. columnar squamous phenotype). In addition the cell types present may help to judge whether an organoid is visually "normal".

Preferred properties of the stem cells and organoids of the invention are outlined below.

### Stem cell markers

When mouse genes are referred to herein, a human organoid of the invention may have a similar gene profile but wherein the human gene counterparts are substituted for the mouse genes. Thus, also provided by the invention is a human organoid having a gene expression profile as described herein, but in respect of the corresponding human genes. The human counterparts of the mouse genes listed herein will be readily available to the skilled person.

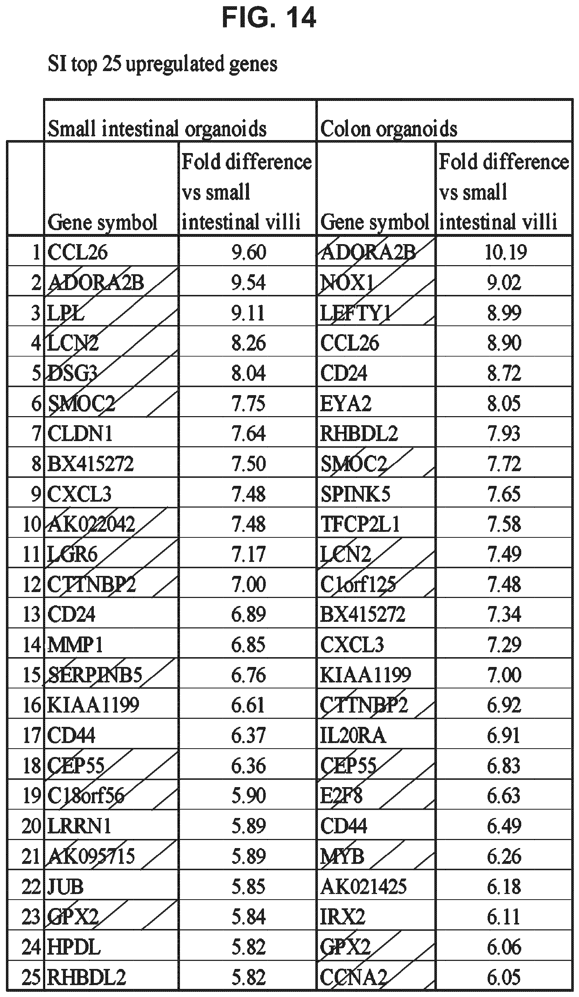

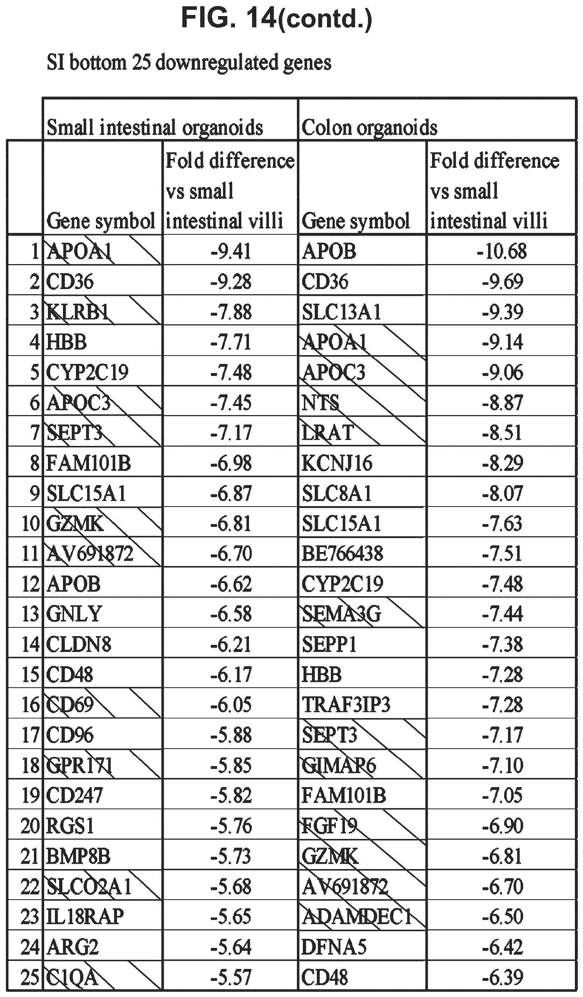

In one embodiment, the invention provides a population of adult stem cells characterised by natural expression of Lgr5. In a preferred embodiment, the invention provides a population of adult stem cells characterised by natural expression of at least Lgr5 and one or more (e.g. 1, 2, 3, 4, 5, 6, 7, 8, 9, 10, 11, 12, 13, 14, 15, 16, 17, 18, 19, 20, 21, 22 or 23) of stem cell markers from the group consisting of: LGR4, epcam, Cd24a, Cdca7, Axin, CK19, Nestin, Somatostatin, CXCR4⁺, CD133⁺, DCAMKL-1, CD44, Sord, Sox9, CD44, Prss23, Sp5, Hnf1α, Hnf4a, Sox9, KRT7 and KRT19, Tnfrsf19. The stem cell markers may be tissue specific. For example, pancreatic stem cells or organoids may be characterised by natural expression of one or more (for example 1, 2, 3, 4, 5, 6, 7, 8, 9, 10, 11, 12, 1314, or 15 for example, 1, 2, 3 or 4) of: CK19, Nestin, Somatostatin, insulin, glucagon, CXCR4⁺, Ngn3, Pdx1, NeuroD, Nkx2.2, Nkx6.1, Pax6, Mafa, Hnf1b, optionally Tnfrsf19 at a significant level; gastric organoids may be characterised by natural expression of one or more (for example 1, 2, 3 or 4) of: CD133⁺, DCAMKL-1, CD44, optionally Tnfrsfl9 at a significant level; and crypt-villus organoids may be characterised by expression of one or more or all (for example 1 or 2) of: Sord and/or Prss23, at a significant level or all genes of table/figure 14, for example, at a significant level.

The term "significant level" as used herein in the context of marker expression is used synonymously with the term "detectable level", as described below.

Small intestinal and gastric organoid cell populations also express markers of progenitor populations common to the small intestine and stomach, such as one or both of Cd44 and Sox9 (Barker & Huch et al Cell stem cell 2010). These are highly expressed in the stem cells according to the invention. Cells according to this aspect of the invention may also up-regulate Wnt target genes, including for example, one, two or all of MMP7, Sp5 Tnfrs19 and axin2. This provides strong evidence of the requirement for an active and robust canonical Wnt signalling activity to maintain the self renewing capacity of these cultures.

The inventors have observed that expression of the 'stem cell' genes is present in the early organoids at a level significantly higher then the differentiated cells that become the offspring of these stem cells. For example, the genes LGR5, LGR4, Epcam, CD44, Tnfrsf19, Sox9, Cd24a, Sp5, Prom1/CD133, Cdca7, are preferably expressed in the organoids of the invention but are preferably significantly downregulated upon differentiation of the pancreas, liver, small intestine and colon organoids. In addition, the genes RNF43 and ZNRF3 are preferably expressed in the organoids of the invention.

By "natural expression" is meant that the cells have not been manipulated recombinantly in any way, i.e., the cells have not been artificially induced to express these markers or to modulate these markers' expression by introduction of exogenous genetic material, such as introduction of heterologous (non-natural) or stronger promoters or other regulatory sequences operably linked to either the endogenous genes or exogenously-introduced forms of the genes. Natural expression is from genomic DNA within the cells, including introns between the exon coding sequences where these exist. Natural expression is not from cDNA. Natural expression can if necessary be proven by any one of various methods, such as sequencing out from within the reading frame of the gene to check that no extraneous heterogenous sequence is present. "Adult" means post-embryonic. With respect to the stem cells of the present invention, the term "adult stem cell" means that the stem cell is isolated from a tissue or organ of an animal at a stage of growth later than the embryonic stage.

This stem cell population can also be characterised by a lack of natural expression of certain markers at any significant level, many of which are associated with cellular differentiation. Specifically, the cells of the isolated adult stem cell population do not naturally express one or more of Cd11b, CD13, CD14, AFP, Pdx1, any CYP member (e.g. CYP3A11, CYP 11A1) at a significant level. As defined herein, these markers are said be to be negative markers.

### Detecting markers and isolating cells

The term "expressed" is used to describe the presence of a marker within a cell. In order to be considered as being expressed, a marker must be present at a detectable level. By "detectable level" is meant that the marker can be detected using one of the standard laboratory methodologies such as PCR, blotting or FACS analysis. A gene is considered to be expressed by a cell of the population of the invention if expression can be reasonably detected after 30 PCR cycles, which corresponds to an expression level in the cell of at least about 100 copies per cell. The terms "express" and "expression" have corresponding meanings. At an expression level below this threshold, a marker is considered not to be expressed. The comparison between the expression level of a marker in a cell of the invention, and the expression level of the same marker in another cell, such as for example an embryonic stem cell, may preferably be conducted by comparing the two cell types that have been isolated from the same species. Preferably this species is a mammal, and more preferably this species is human. Such comparison may conveniently be conducted using a reverse transcriptase polymerase chain reaction (RT-PCR) experiment.

In some embodiments, a population of cells or an organoid of the invention is considered to express a marker if at least about 5%, (for example, at least 10%, at least 20%, at least 30%, at least 40%, at least 60%, at least 70%, at least 80%, at least 90%, at least 95%, at least 97%, at least 98%, at least 99% or 100%) of the cells in the cell population or organoid according to the invention show expression of the marker.

In some embodiments, the cells express a cell marker at a significant level if they comprise between 1 x 10² to 1 x 10⁵, for example 5 x 10² to 1 x 10⁴ or 1 x 10³ to 1 x 10⁴ fold more copies of the mRNA encoding the cell marker relative to the number of mRNA copies of the housekeeping gene GADPH.

In some embodiments, the expression of a gene in an organoid or cell of the invention when cultured in expansion medium is several fold (e.g. at least 1.5 fold, 2 fold, 3 fold, 4 fold, 5 fold) higher than when the organoid or cell is cultured in differentiation medium or in the fully differentiated adult tissue. In some embodiments, a cell or organoid of the invention when cultured under differentiation conditions, exhibits an increase in expression of genes that are known as differentiation genes compared to a cell or organoid of the invention when cultured under expansion conditions and also may show a decrease in the level of expression of at least one or more stem cell/progenitor genes compared to a cell or organoid of the invention when cultured in expansion medium.

Any one of a number of physical methods of separation known in the art may be used to select the cells of this aspect of the invention and distinguish these from other cell types. Such physical methods may involve FACS and various immuno-affinity methods based upon makers specifically expressed by the cells of the invention. As described above, Lgr5, CD44 and Sox9 are three of the cell markers expressed at high levels in the stem cells of the invention. Therefore, by way of illustration only, the stem cells of the invention may be isolated by a number of physical methods of separation, which rely on the presence of these.

In one embodiment, the cells of the invention may be isolated by FACS utilizing an antibody, for example, against one of these markers. Fluorescent activated cell sorting (FACS) can be used to detect markers characteristic of a particular cell type or lineage. As will be apparent to one skilled in the art, this may be achieved through a fluorescent labeled antibody, or through a fluorescent labeled secondary antibody with binding specificity for the primary antibody. Examples of suitable fluorescent labels includes, but is not limited to, FITC, Alexa Fluor^{®} 488, GFP, CFSE, CFDA-SE, DyLight 488, PE, PerCP, PE-Alexa Fluor^{®} 700, PE-Cy5 (TRICOLOR^{®}), PE-Cy5.5, PI, PE-Alexa Fluor^{®} 750, and PE-Cy7. This list is provided by way of example only, and is not intended to be limiting.

It will be apparent to a person skilled in the art that FACS analysis using an anti-Lgr5 antibody will provide a purified stem cell population. However, in some embodiments, it may be preferable to purify the cell population further by performing a further round of FACS analysis using one or more of the other identifiable markers.

Immunohistochemistry may also be used to understand the distribution and localisation of biomarkers and differentially expressed proteins in different parts of a cell population or organoid. Visualising an antibody-antigen interaction can be accomplished in a number of ways that are well known in the art, such as those that are described in described in Barker et al, Identification of stem cells in small intestine and colon by marker gene Lgr5.Nature, 2007 Oct 25;449(7165):1003-7.

In another embodiment, the cells of the invention may be isolated by immuno-affinity purification, which is a separation method well known in the art. By way of illustration only, the cells of the invention may be isolated by immuno-affinity purification directed towards c-kit. As will be apparent to one skilled in the art, this method relies upon the immobilisation of antibodies on a purification column. The cell sample is then loaded onto the column, allowing the appropriate cells to be bound by the antibodies, and therefore bound to the column. Following a washing step, the cells are eluted from the column using a competitor which binds preferentially to the immobilised anti-c-kit antibody, and permits the cells to be released from the column.It will be apparent to a person skilled in the art that immuno-affinity purification using an immobilised antibody will provide a purified cell population. However, in some embodiments, it may be preferable to purify the cell population further by performing a further round of immuno-affinity purification using one or more of the other identifiable markers, and use an aliquot of the isolated clones to ascertain the expression of other relevant intracellular markers.

It will be apparent to a person skilled in the art that LGR5 or stem cell purification can be preceded by any number of purification steps,such as purification of the epithelium with methods known in the art, for example EDTA purification or Epcam FACS sorting of the epithelium.

It will be apparent to a person skilled in the art that the sequential purification steps are not necessarily required to involve the same physical method of separation. Therefore, it will be clear that, for example, the cells may be purified through a FACS step using an anti-Lgr5 antibody, followed by an immuno-affinity purification step using a SSEA-1 affinity column. In certain embodiments, the cells may be cultured after isolation for at least about 15, at least about 20 days, at least about 25 days, or at least about 30 days. In certain aspects, the cells are expanded in culture longer to improve the homogeneity of the cell phenotype in the cell population.

Mircroarray analysis, cluster analysis and comparative gene expression profiling can be used to compare population phenotype with phenotype of the original parent cells or of the appropriate *in vivo* counterparts (Sato T et al., Paneth cells constitute the niche for Lgr5 stem cells in intestinal crypts. Nature 469 415-418).

Lineage tracing of Lgr5+ stem cells shows preservation of crypt-villus characteristics in organoids.

In another embodiment, high content analysis may be used to assess phenotypic integrity of stem cells of the invention. For example, a number of high content screening kits and platforms exist, such as point scanning 4 color ImageXpress ULTRA (Molecular Devices, Union City, USA), the spinning disk (nipkow disk) Pathway 855 and 435 from BD Biosciences (formerly Atto Biosciences, Rockville, Maryland), Opera (PerkinElmer Inc., Waltham, MA) and the slit scanning IN Cell 3000 (GE/Amersham Biosciences, Cardiff, UK), Arrayscan VTI (Cellomics (Cellomics)), IN Cell Analyzer 2000 (GE Healthcare Piscataway, New Jersey, USA), Acumen eX3 (TTP LabTech Ltd (Acumen eX3)), Scanalyzer (Scanalyzer LemnaTec, Aachen Germany) and ImageXpress MICRO (Molecular Devices, Union City, USA), IN Cell 1000 (GE/Amersham Biosciences Piscataway, New Jersey, USA), the Pathway HT (Becton Dickinson Biosciences) and the ImageXpress MICRO (Molecular Devices, Union City, USA), Scan^R (Olympus Soft Imaging Solutions, Germany).

### Plating density

In some embodiments of the invention, single-cell suspensions or small clusters of cells (2-50 cells/cluster) will normally be seeded, rather than large clusters of cells, as is known in the art. As they divide, such cells will be seeded onto a support at a density that promotes cell proliferation. Typically, when single cells are isolated the plating density of at least 1-500 cells/well is used, the surface of the well being 0.32 cm². When clusters are seeded the plating density is preferably 250-2500 cells/cm². For replating, a density of between about 2500 cells/ cm² and about 5,000 cells/ cm² may be used. During replating, single-cell suspensions or small cluster of cells will normally be seeded, rather than large clusters of cells, as in known in the art.

### Further differentiation

In some embodiments of the invention, certain components of the expansion medium can be withdrawn to change the cell fate of the cultured cells towards differentiation. Any components of the culture medium that are responsible for maintaining an undifferentiated state and/or activating stem cell or progenitor genetic programs may be withdrawn from the culture medium.

In some embodiments of the invention, withdrawal of the inhibitors of the invention can enable cells of the organoid to differentiate to mature cells, such as mature goblet and enteroendocrine cells in crypt-villus organoids. Thus in some embodiments, the invention provides a method for further differentiating the organoids using a second culture medium which does not comprise an inhibitor of the invention. For example, see Example 1.

For example, in some embodiments, the inhibitor of TGF-beta and/or the inhibitor of p38 are withdrawn from the cell culture medium to allow the cells to differentiate. By "withdrawn" or "withdrawal" of a component from the cell culture medium is meant that when the cells are replated and the medium is changed, the component is not added to the fresh medium.

In some embodiments, Wnt is present in the expansion medium but not in the differentiation medium. For example, some embodiments comprise withdrawal of Wnt for differentiation of colon organoids to mature enterocytes. Wnt may also be withdrawn to enable differentiation of crypt-villus organoids.

In some embodiments, Rspondin is present in the expansion medium but not in the differentiation medium. For example, some embodiments comprise withdrawal of Rspondin for differentiation of colon organoids to mature enterocytes. Rspondin may also be withdrawn to enable differentiation of crypt-villus organoids. In some embodiments Rspondin and Wnt may withdrawn to enable differentiation of crypt-villus organoids.

In some embodiments, nicotinamide is present in the expansion medium but not in the differentiation medium. Thus, in some embodiments, nicotinamide and SB202190 (or another p38 inhibitor) are withdrawn from the cell culture medium to enable differentiation of the cells, for example, into crypt-villus organoids or colon organoids.

Thus, a method of obtaining differentiated cells or organoids may comprise culturing epithelial cells in a culture method of the invention which comprises a TGF-beta and/or p38 inhibitor to enable the cells to survive and/or proliferate (i.e. expansion medium) and then continuing to culture the cell and replenish the media, wherein the replenished media does not comprise a TGF-beta inhibitor and/or p38 inhibitor (i.e. differentiation medium).

In some embodiments, the differentiation medium comprises additional components. For example in some embodiments the differentiation medium comprises a gamma secretase inhibitor, for example DAPT or DBZ. In some embodiments the differentiation medium comprises RANK ligand (also referred to herein as RANKL). As mentioned above, the addition of a gamma secretase inhibitor can direct the differentiation of intestinal organoids cells, such as small intestinal organoid cells, towards secretory cells, such as goblet cells. The addition of RANKL to the culture medium can direct differentiation intestinal organoid cells such as small intestinal organoid cells, towards M cells.

In some embodiments the invention provides a culture medium for differentiating stem cells from a tissue of interest, wherein the culture medium comprises or consists of the components of the culture medium used for expanding the stem cells from the tissue type of interest but wherein one or more of the following are excluded from the medium for differentiating stem cells: Wnt, Rspondin, BMP inhibitor, TGF-beta inhibitor, receptor tyrosine kinase ligand, p38 inhibitor and nicotinamide.

Furthermore, the invention provides a method for expanding a single stem cell or a population of stem cells, preferably to generate an organoid, wherein the method comprises culturing the single stem cell or population of stem cells in a culture medium according to the invention, wherein the method comprises:
culturing the stem cell, population of stem cells or tissue fragments in a first expansion medium;
continuing to culture the stem cell, population of stem cells or tissue fragments and replenishing the medium with a differentiation medium, wherein the differentiation medium does not comprise one or more of, preferably all of the factors selected from: a TGF-beta inhibitor, a p38 inhibitor, nicotinamide and Wnt.

In general, where a component is described as being "removed" from a medium, it is meant that that component is not added when the medium is replenished i.e. the component is excluded from the replenished medium. When the medium is "replenished" this can mean that the medium is physically removed from the extracellular matrix and then replaced with fresh medium.

For the colon, liver and pancreas, very few differentiated cells are present in the expansion medium. Only once the expansion medium is replaced with a differentiation medium, do the cells begin to differentiate. At this stage, the organoids also start to lose their stem cells. Differentiated organoids may be appropriate for certain uses, such as (but not limited to) transplantation, drug screening of metabolic diseases, toxicology (for example using liver organoids comprising hepatocytes) and for studying antibacterial functions of the small intestine. Expanding organoids may generally be more appropriate for other uses, such as (but no limited to) regenerative medicine and drug screening, for example for cancer or cystic fibrosis. Expanding organoids generally have more growth potential (and thus greater longevity) than differentiated organoids. In some embodiments, the colon, liver and pancreatic organoids are not further differentiated.

The small intestine and prostate organoids differ from the colon, liver and pancreatic organoids, in that they maintain an expanding stem cell population whilst also differentiating at the same time. They do not need to be cultured in a separate differentiation medium in order for differentiated cell types to be present. They can be considered to have the properties of both an expanding and a differentiated organoid. However, to achieve full differentiation of small intestinal organoids, they can be cultured in a separate differentiation medium that preferably does not comprise Wnt3a and that preferably comprises a gamma secretase inhibitor and/or a RANK ligand (also referred to herein as RANKL). By "full" differentiation it is meant that all differentiated cell types are present including goblet cells, neuroendocrine cells, tuft cells, M-cells, enterocytes and paneth cells. Some of these differentiated cell types, for example paneth cells, are also present (sometimes in smaller quantities) in the expanding organoids.

### Organoids

The cells described above grow into organoids. Accordingly, an organoid obtainable by a method of the invention is a further aspect of the invention. Also provided is an organoid as described herein. The organoid is preferably a human organoid. To the best of our knowledge, this is the first time that human organoids have been obtained that are functional and alive after such an extended period of time (i.e at least 3 months, preferably at least 4 months, at least 5 months, at least 6 months, at least 7 months, at least 9 months, or at least 12 months or more of culture; see examples included herein). Functionality is preferably characterized by the presence of tissue-specific markers and/or by the structure of said organoid as defined herein. Since the final amount of organoids obtained correlates with the duration of culture, the skilled person will understand that the invention is a pioneer invention and potentially opens new possibilities in for example regenerative medicine. Thus, there is provided an organoid as described herein that is functional and alive after at least 3 months (e.g. at least 4, 5, 6, 7, 8 or more months) of culture. For example, there is provided an organoid as described herein that retains at least one or more (e.g. 1, 2 or 3) of its structure, marker expression and function after at least 3 months (e.g. at least 4, 5, 6, 7, 8 or more months) of culture.

For example, an organoid according to the present invention may comprise a population of cells of at least 1x10³ cells, at least 1 x 10⁴ cells, at least 1x10⁵ cells, at least 1x10⁶ cells, at least 1x10⁷ cells or more. Each organoid comprises between approximately 1x10³ cells and 5x10³ cells. The inventors have shown that it is possible to grow organoids from single Lgr5+ stem cells into organoids comprising a population of cells as described above or comprising a population of cells of approximately 10⁴ cells. For example, it has now been shown for mouse that it is possible to start growth of an organoid from single stem cells. Thus, the invention provides a method for generating an organoid from a single stem cell. In some embodiments, the organoid comprises approximately 10⁴ cells. In some embodiments, 10-20, or 20-30 or 30-40 or 40-50 organoids may be grown together in one well of a 24 well plate.

In some embodiments, the invention provides an organoid or population of cells, which is capable of surviving in culture for at least 3 months, for example at least 4 months, at least 5 months, at least 6 months, at least 7 months, at least 9 months, or at least 12 months or more, when cultured in a culture medium of the invention.

In some embodiments, the invention provides an organoid or population of cells, wherein the organoid or population of cells expands at a rate of at least 3 fold, at least 4 fold, at least 5 fold, at least 6 fold, at least 7 fold, at least 8 fold, at least 9 fold or at least 10 fold per week.

Preferably the population of cells or organoids will expand at a rate of about 4-5 fold per week or more than two population doublings a week. Therefore, in some embodiments, the population of cells or organoids will expand at a rate of at least 3 fold, at least 4 fold, at least 5 fold, at least 6 fold, at least 7 fold, at least 8 fold, at least 9 fold or at least 10 fold per week.

Organoids of the invention may be obtained using cells isolated from any suitable source. Generally, the cells used to generate an organoid will be isolated from the same tissue type as the organoid which is generated. The organoids are preferably mammalian, for example, murine, bovine, porcine or human. Most preferably, the organoids are human.

In some embodiments, the invention provides an organoid or population of cells, wherein the organoid or population of cells is a normal (healthy) organoid or population of cells or a disease organoid or population of cells, for example obtained by culturing stem cells taken from a human or animal with a disease.

In some embodiments, the invention provides an organoid or population of cells which is frozen and stored at below -5°C, below -10°C, below -20°C, below -40°C, below -60°C, or below -80°C, below -100 °C, below -150 °C or at approximately -180°C. The organoid or population of cells of the invention may be stored in liquid nitrogen. Therefore, in some embodiments, the invention provides an organoid or population of cells which is stored in liquid nitrogen.

In some embodiments, the invention provides an organoid of the invention, wherein the organoid is a small intestine organoid, a colon organoid, a gastric organoid, a pancreatic organoid, a liver organoid or a prostatic organoid.

### Organoid structure and morphology

Organoids of the invention, obtainable by expansion of stem cells, provide a population of cells which resemble their *in vivo* counterparts.

Image analysis may be used to assess characteristics of cells in culture such as cell morphology; cell structures; evidence for apoptosis or cell lysis; and organoid composition and structure. Many types of imaging analysis are well known in the art, such as electron microscopy, confocal microscopy, stereomicroscopy, fluorescence microscopy. Histological analysis can reveal basic architecture and cell types.

Illustrative examples of organoids generated according to the invention are given in the accompanying figures. It can be seen that organoids according to the invention may possess a layer of cells with at least one bud and a central lumen. The organoids in the outside of the matrigel tend to be larger than the organoids in the center of the matrigel, perhaps because they have better access to the necessary growth factors. Structurally, organoids according to the invention are often elongated in shape. They may include one or more budding structure - a single cell epithelial layer with similarities to ducts or islets. Under confocal microscopy, the structures may stain positive for keratin. They may include cells with polarised nuclei and small cytoplasm. The organoids may have a section which is formed of multiple layers; such cells often tend to have their nuclei more central to the cells, i.e. not polarized. The cells in the multilayer section may organise themselves to include a gap, or lumen between the cells. In some embodiments the organoids of the invention comprise or consist of epithelial cells. In some embodiments, the organoids comprise or consist of a single layer of epithelial cells. In some embodiments non-epithelial cells are absent from the organoids. In some embodiments, the organoids of the invention comprise all the differentiated cell types that exist in their corresponding *in vivo* tissue counterpart.

In some embodiments human intestinal organoids displayed budding organoid structures, rather than the cystic structures seen under previous culture conditions. Metaphase spreads of organoids more than 3 months old consistently revealed 46 chromosomes in each of the 20 cells taken from three different donors.

In some embodiments the organoids of the invention comprise a single monolayer of cells. In some embodiments the organoids of the invention have a section which is formed of multiple layers. Multiple layers of cells are also referred to herein as regions of "stratified" cells. By "stratified" it is meant that there are multiple (more than one) layers of cells. In some embodiments the organoids of the invention comprise single monolayers that are folded (or invaginated) to form two or more layers. It can sometimes be difficult to distinguish between folded (or invaginated) monolayers and regions of stratified cells. In some embodiments an organoid comprises both regions of stratified cells and regions of folded monolayers. In some embodiments the organoids of the invention have a section which is formed of multiple layers and a section comprising a single monolayer of cells. Morphologically, the cells appear like their corresponding *in vivo* tissue counterpart.

Therefore, in some embodiments the invention provides an organoid, preferably obtainable using the culture media and methods of the invention, which is a three-dimensional organoid comprising epithelial cells surrounding a central lumen, wherein optionally the epithelial cells exist in distinct dividing domains and differentiating domains. In some embodiments the organoid of the invention is a three-dimensional organoid comprising epithelial cells arranged in regions of monolayers, optionally folded monolayers and regions of stratified cells. In some embodiments, non-epithelial cells are absent from said organoid. In some embodiments, all differentiated cell types of the normal *in vivo* tissue are present in said organoid.

### Crypt-villus organoids

In small intestinal crypt-villus organoids the structural arrangement of the organoids is very similar to the structure of *in vivo* crypt-villi: the Lgr5+ stem cell and their niche cells (Paneth cells) are next to each other at the base of the crypt, followed by the transit amplifying cells, just above the base of the crypt and leading into the sides of the villi and finally the differentiated cells, such as enterocytes that make up the rest of the villi and become more and more differentiated towards the top of the villi. It can be seen that organoids according to the invention may possess a layer of cells with at least one bud and a central lumen. The organoids in the outside of the matrigel tend to be larger than the organoids in the center of the matrigel, perhaps because they have better access to the necessary growth factors. Structurally, organoids according to the invention are often elongated in shape. Under confocal microscopy, the structures may stain positive for keratin. They may include cells with polarised nuclei and small cytoplasm. The crypt-villus organoids are generally single-layered.

In some embodiments, for example for a mouse crypt-villus organoid, a crypt-villus organoid is a three-dimensional organoid, comprising crypt-like domains surrounding a central lumen lined by villus-like epithelial domains, which are epithelial domains comprising differentiated cell types. In some embodiments, non-epithelial cells are absent from said organoid.

In some embodiments, for example for a human crypt-villus organoid, a crypt-villus organoid is a three-dimensional organoid, comprising crypt-like domains surrounding a central lumen. In some embodiments, dividing cells are confined to the budding structures. No or few differentiated cells are present. Under differentiation conditions the differentiated cells of the intestine are formed. In some embodiments, non-epithelial cells are absent from said organoid. In some embodiments, when the organoid is expanding, for example when it is in an expansion culture medium according to the invention, the organoid has few or no differentiated cells.

In some embodiments, a small intestinal organoid of the invention cultured in a culture medium of the invention comprising RANKL, comprises M-cells. In some embodiments of the invention, a small intestinal organoid of the invention cultured in a culture medium of the invention comprising a gamma-secretase inhibitor, comprises goblet cells. In some embodiments, a small intestinal organoid cultured in a differentiation medium (for example wherein the differentiation medium comprises a basal medium, Noggin, EGF, a TGF-beta inhibitor and a p38 inhibitor, a gamma-secretase inhibitor and a RANKL) comprises all differentiated cell types including, for example, goblet cells, neuroendocrine cells, tuft cells, M-cells, enterocytes and paneth cells. Some of these differentiated cell types, for example paneth cells, are also present (sometimes in smaller quantities) in the expanding organoids.

Human intestinal organoids display budding organoid structures, rather than the cystic structures seen under previous culture conditions. The upper opening of freshly isolated crypts becomes sealed and this region gradually balloons out and becomes filled with apoptotic cells, much like apoptotic cells are pinched off at the villus tip. Thus, in some embodiments, the crypt-villus organoids have a crypt-like structure surrounding a central lumen lined by a villus-like epithelium and filled with apoptotic cell bodies. In some embodiments, the lumen is opened at consecutive time intervals to release the content into the medium.

In some embodiments, the crypt region undergoes continuous budding events which create additional crypts, a process reminiscent of crypt fission.

The inventors also demonstrated that the human intestinal organoids generated by media and methods of the present invention, mimicked *in vivo* cell fate decisions in response to external factors. For example, it has previously been shown that Notch inhibition in intestinal stem cells, terminates intestinal epithelial proliferation and induces goblet cell hyperplasia *in vivo.* Thus in some embodiments, when a crypt-villus organoid of the invention is treated with a Notch inhibitor, proliferation ceases and most cells (for example more than 50%, more than 60%, more than 70%, more than 80%, more than 90%, more than 95%, more than 98%) convert into goblet cells within 3 days.

Metaphase spreads of organoids more than 3 months old consistently revealed 46 chromosomes in each of the 20 cells taken from three different donors. Furthermore, microarray analysis revealed that the stem cells in culture possessed similar molecular signatures to intestinal crypt cells including intestinal stem cell genes.

### Colon organoids

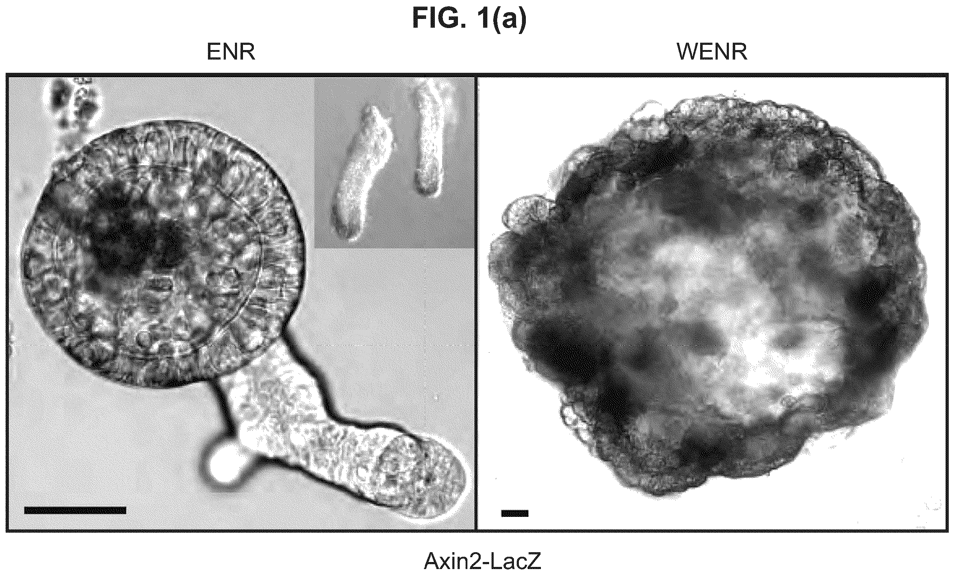

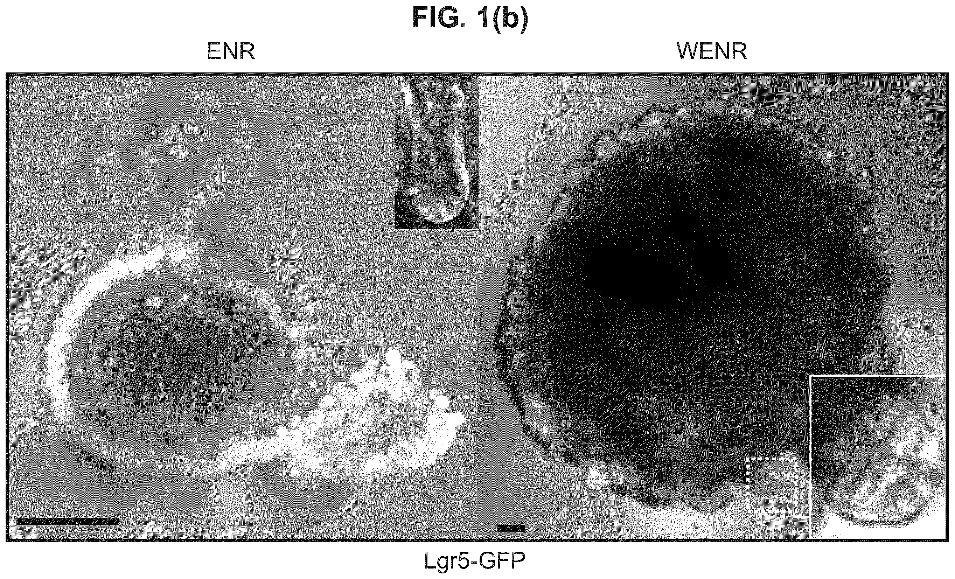

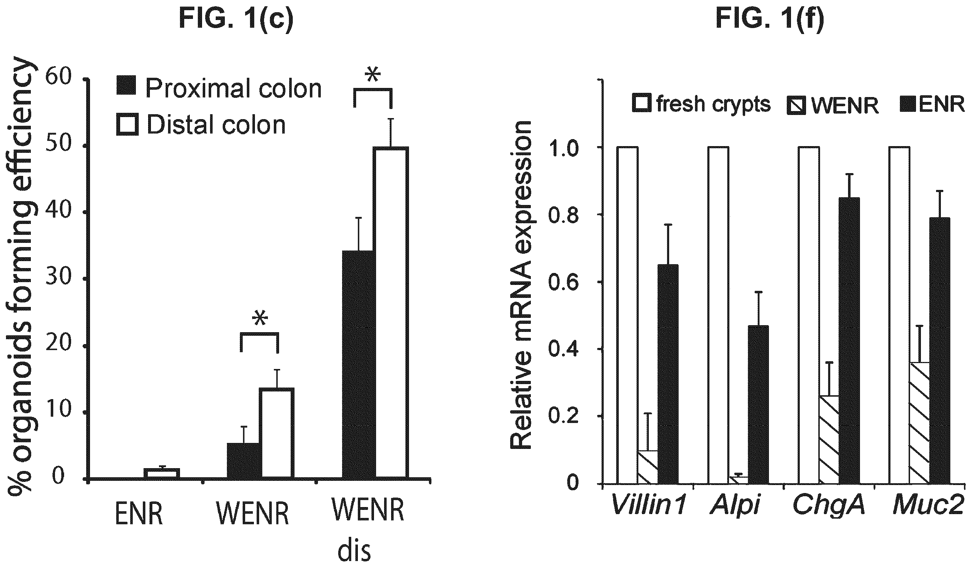

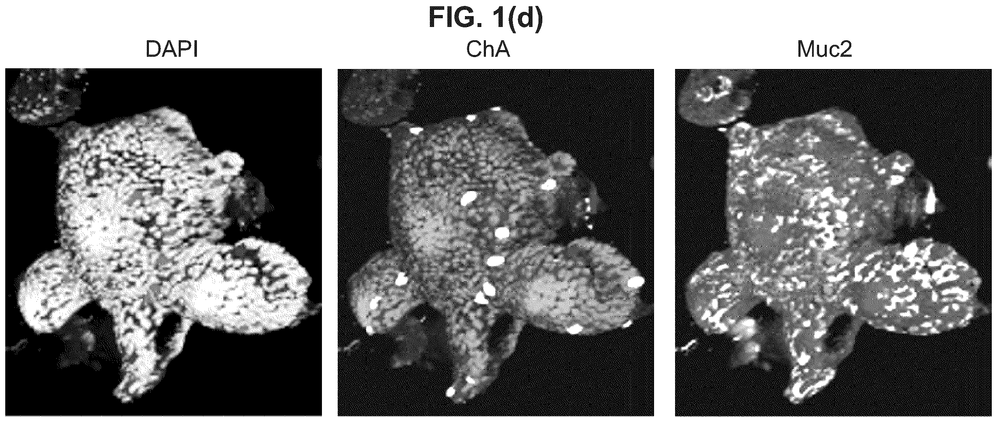

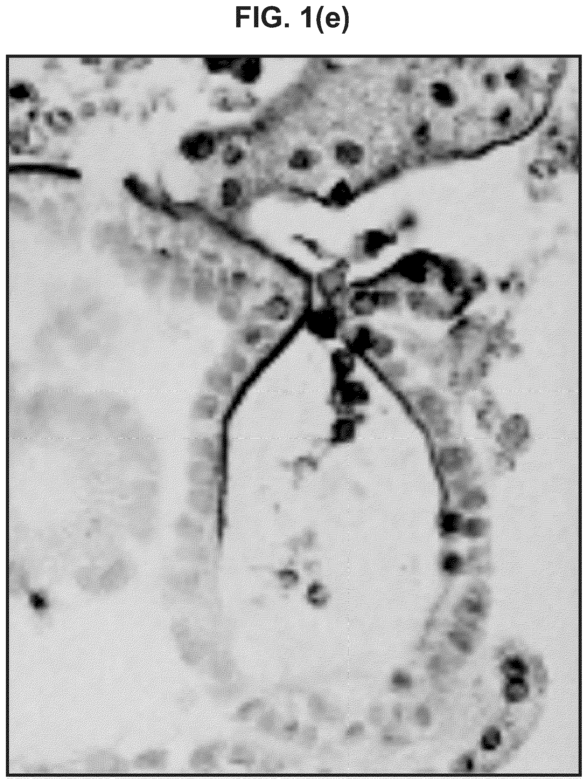

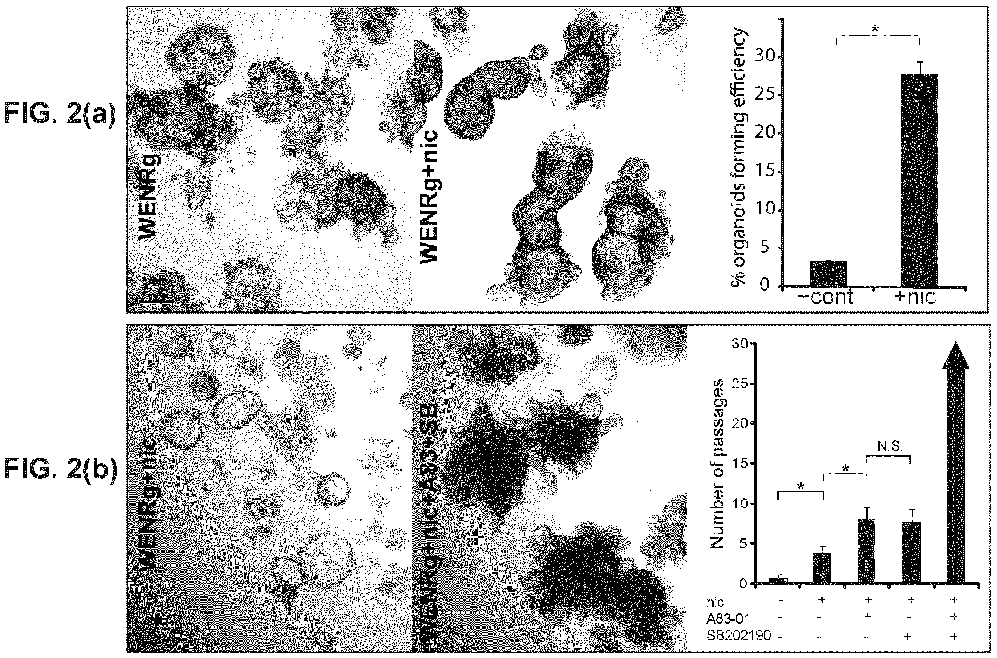

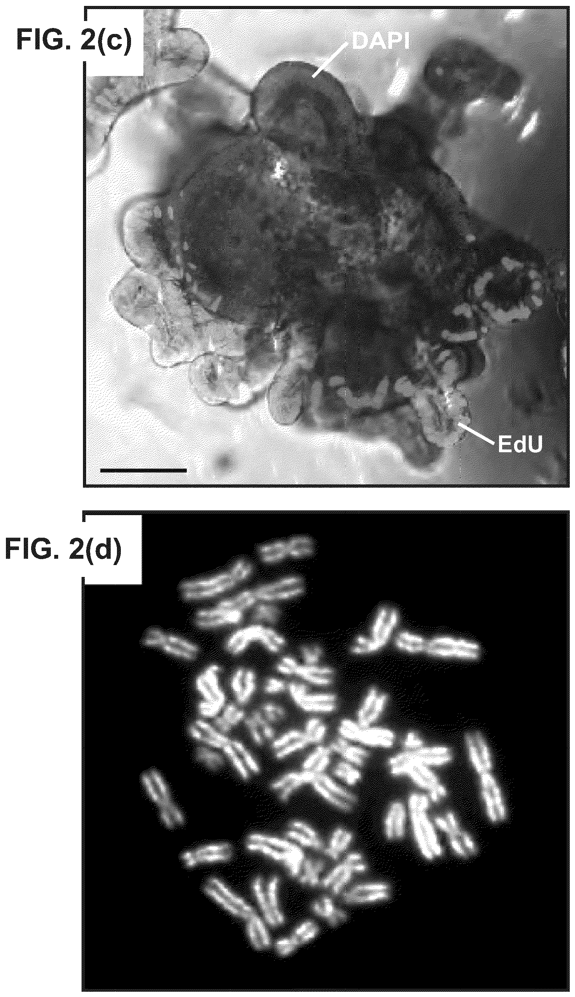

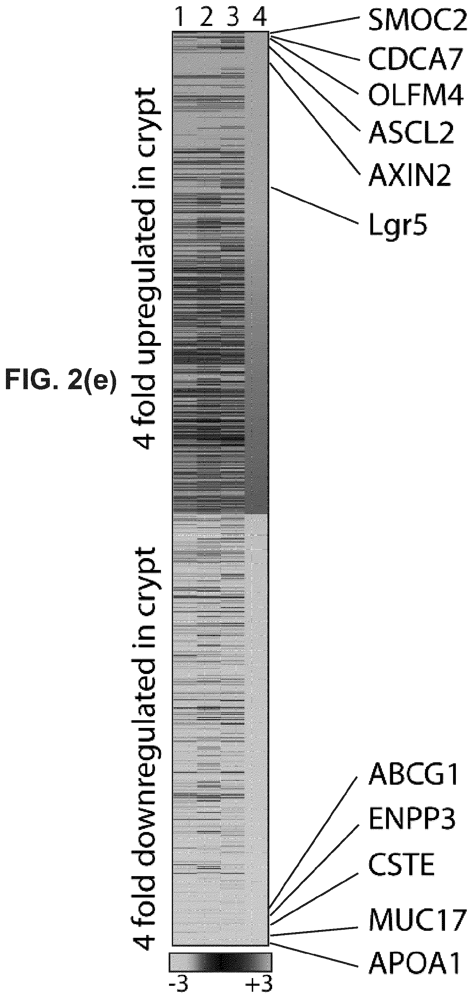

Colon organoids exhibit a similar cell composition to crypt-villus organoids. Thus, the comments for crypt-villus organoids above apply to colon organoids mutatis mutandis. For example, see figures 1 and 2.

Typically, the difference between the colon and small intestinal organoids is that the crypts are shallower in the colon making it look a little like a "football" rather than a sphere with protrusions. Both small intestinal and colon organoids have domains that contain stem cells and transit amplifying (TA) cells, and other domains containing differentiating and/or differentiated cells. For the small intestinal organoids the differentiated domains are sometimes referred to as "villus-like". The differentiated domains of the colon organoids are typically similar in cell composition to the "villus-like" domains of the small intestine but the colon itself does not have villi.

The amount of Wnt present can influence the size of the budding structures (i.e. the depth of the crypts) in the organoids. More Wnt reduces budding. The colon produces more Wnt than the small intestine and so requires less additional Wnt in the culture medium and typically has shallower crypts than the small intestine. The same difference is seen in the organoids.

In some aspects, colon organoids are provided by the invention. The inventors have found that mouse colon organoids can be obtained by culturing colon crypts in an ENR + Wnt3A (WENR) cell culture media. Thus, in some embodiments, the invention provides a colon organoid obtained by culturing colon crypts in WENR media.

The inventors have also surprisingly found that human colon organoids can be maintained using a culture medium comprising WENR plus gastrin plus nicotinamide. In some embodiments, a human colon organoid of the invention is obtainable by using a media comprising WENR plus gastrin plus nicotinamide and also comprising an inhibitor of TGF beta. For example, in some embodiments, the following cell culture media may be used to obtain a human colon organoid: WENR+gastrin+nicotinamide+A8301+SB202190. In other embodiments, the following cell culture medium may be used to obtain a human colon organoid: WENR+Nicotinamide+A83-01

In some embodiments, a mouse colon organoid has a maximal diameter of approximately 200-700um, for example 250-600 um, 300-500 um, 320-450 um, 340-400 um, 300-380 um, for example approximately 360um. In some embodiments, a colon organoid has a minimal diameter of approximately 100-400um, for example 150-350 um, 170-300 um, 190-280 um, 195-250 um, for example, approximately 235um. In a further embodiment, the organoids can have a diameter of up to 1mm. In some embodiments, a human colon organoid has a maximal diameter of approximately 300-800um, for example 350-700 um, 400-600 um, 450-550 um, 475-540 um, 500-530 um, for example approximately 500um. In some embodiments, a colon organoid has a minimal diameter of approximately 200-500um, for example 250-450 um, 300-415 um, 350-400 um, 325-380 um, for example, approximately 375um. In a further embodiment, the organoids can have a diameter of up to 1mm. In some embodiments, a colon organoid of the invention comprises budding structures. These may be visible by using EdU stain to visualize proliferating cells.

Human colon organoids retain their characteristic budding structure under the Human Intestinal Stem Cell Culture ("HISC") condition (WENRg+nicotinamide+TGF-beta inhibitor (e.g. A83-01)+p38 inhibitor (e.g. SB202190)).In some embodiments, a colon organoid is a three-dimensional organoid, comprising budding structures which are proliferating and contain stem cells. These stem cell domains surround a central lumen. Dividing cells are generally confined to the budding structures. In some embodiments, no or few differentiated cells are present. Under differentiation conditions the differentiated cells of the intestine are formed, for example mature enterocytes. In some embodiments, non-epithelial cells are absent from said organoid.

### Pancreatic organoids

Pancreatic organoids of the invention preferably exhibit budding. In some embodiments, the pancreatic organoids are from 100-1000 micrometers in diameter, for example, 200-900 micrometers, 300-1000 micrometers, 400-700 micrometers. The pancreatic organoids are preferably single layered. There are only the very beginnings of islet or ductal structures. Budding structure are indicative of a healthy proliferation status and stem cell maintenance.

In some embodiments, for example when pancreatic organoids are grown in a culture medium of the invention (and in the absence of TGF-beta inhibitors), pancreatic organoids are mainly cystic structures with few budding structures or duct-like domains. The cystic structures comprise mainly monolayers but some regions of stratified cells may be present. The cells express stem cell and progenitor (ductal) markers. No differentiated cells, such as beta-cells, are present in the organoids. The cyst is mainly formed by a monolayer, but stratified parts exist. Cell types resemble stem cells / progenitor (duct cell gene expression). There are no differentiated cells (β-cells).

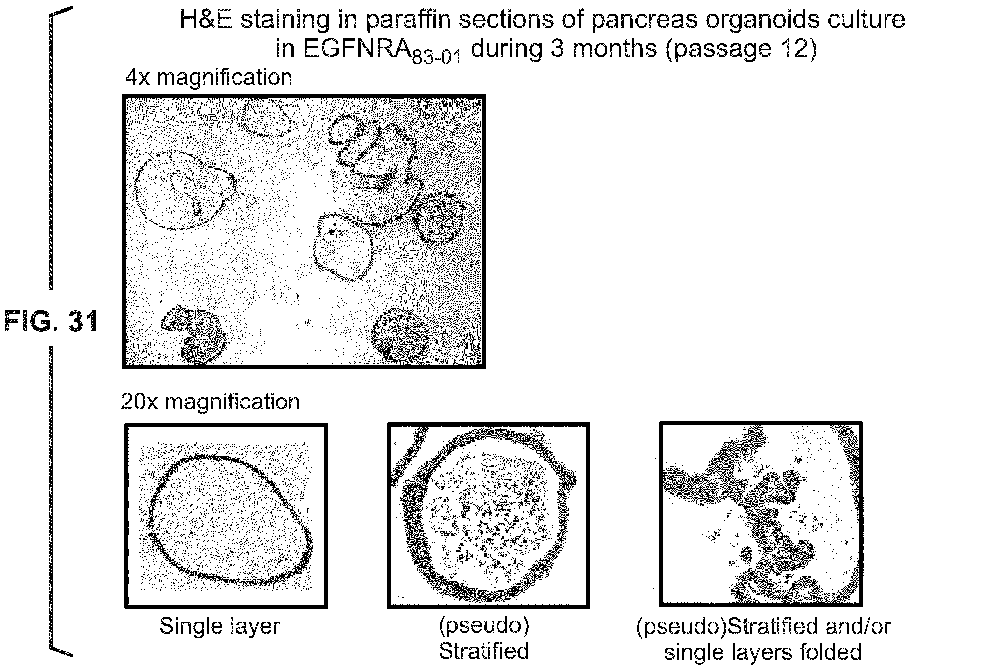

In other embodiments, for example when pancreatic organoids are grown in the presence of a TGF-beta inhibitor, such as A83-01, for example in a culture medium of the invention, the pancreatic organoids comprise more budding structures/ductal-like domains (this means cells are duct-like cells more than the structure is like a duct), as shown by Krt19 staining (for example, see figure 31). Monolayers of polarized cells can be identified, but also areas with stratified cells.

### Adenocarcinoma and colon cancer organoids

Adenocarcinoma and colon cancer organoids generally form cystic structures instead of budding structures. This is reminiscent of the absence of good cell niche support. Adenoma crypts cultured with EFG+Noggin show approximately 16x expansion in the first 10 days. Adeno(carcino)ma and colon cancer organoids may provide useful research tools and drug screening models.

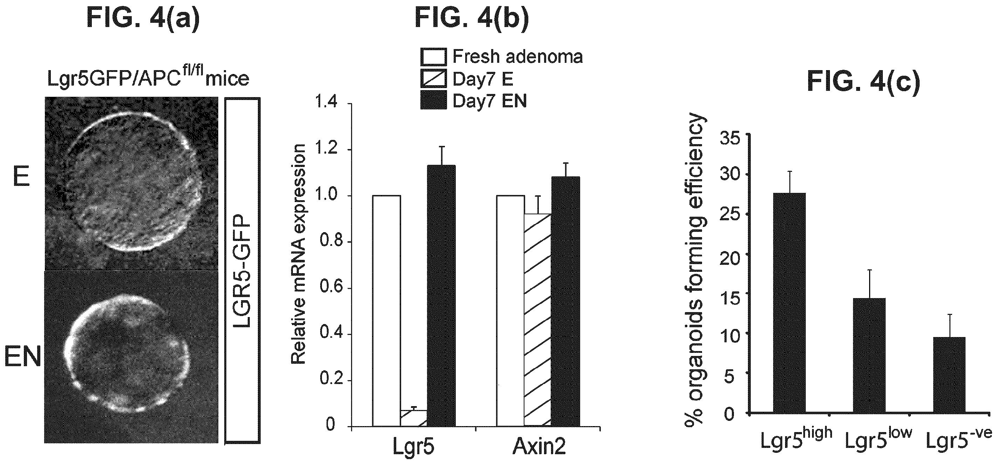

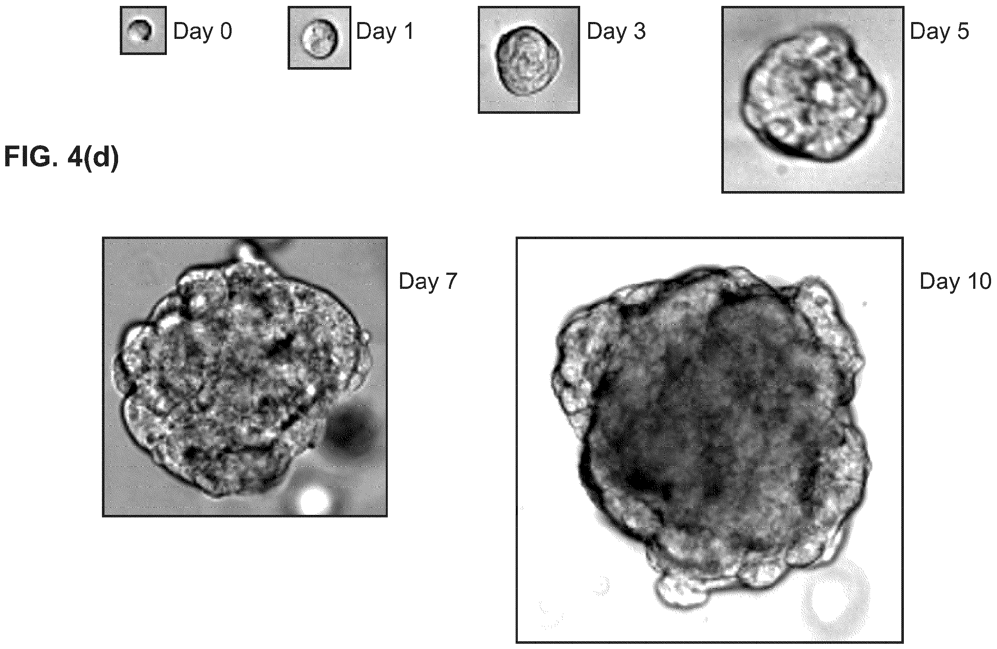

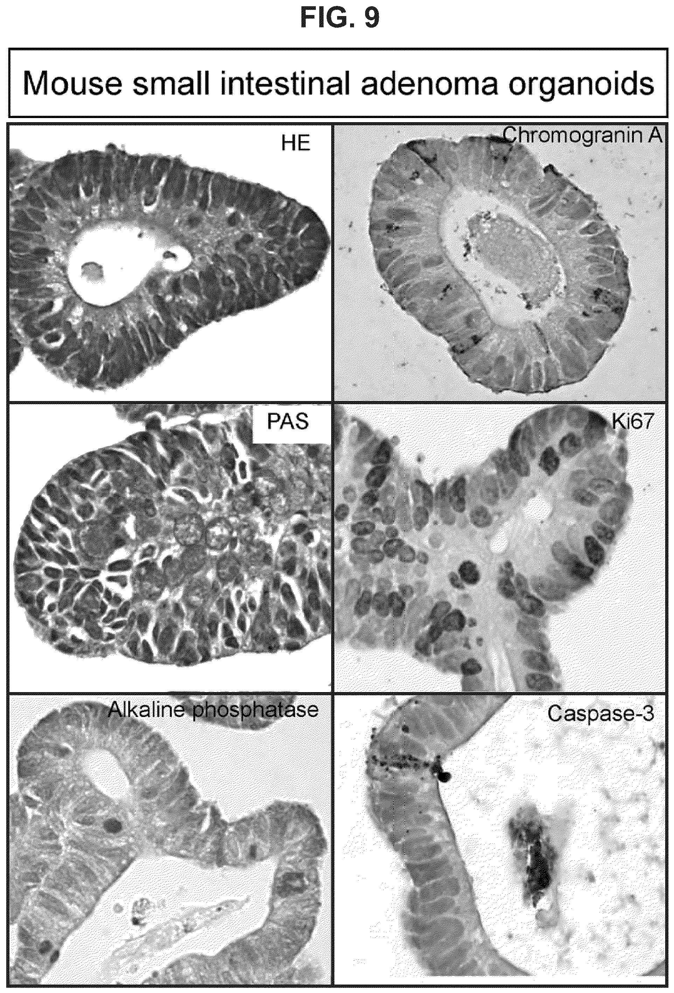

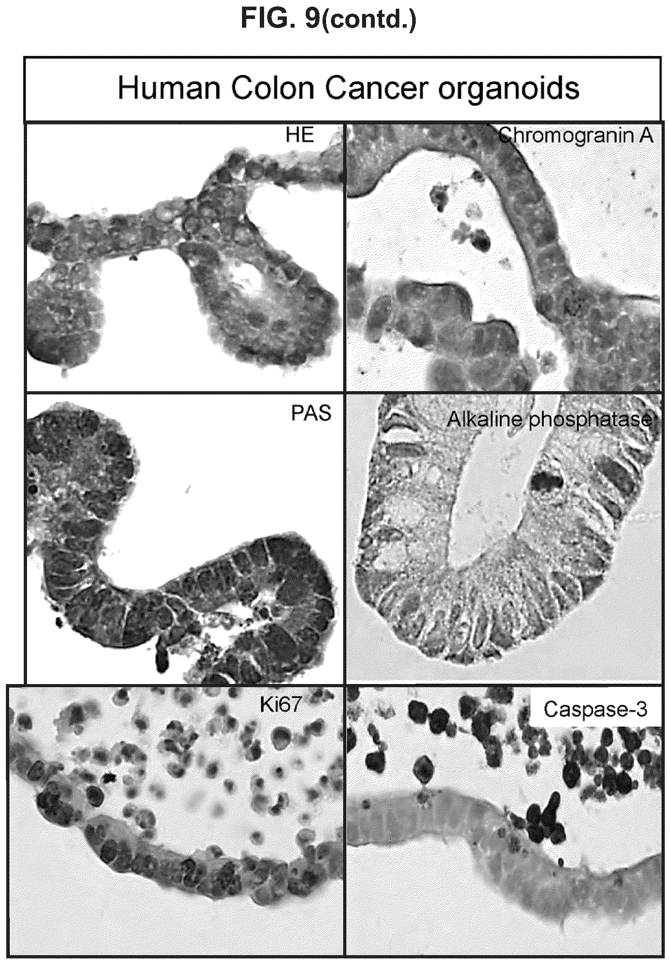

Carcinoma, adenoma and adenocarcinoma organoids are largely cystic (for example, see figures 4 and 9). However, in some embodiments, they may also comprise structures that resemble their normal tissue organoid counterparts.

### Barrett's Esophagus (BE) organoids

A BE organoid of the invention comprises budding structures (for example, see figure 5).

Morphologically, the cells in the organoids of the invention appear like their corresponding *in vivo* tissue counterpart.

Barrett's Esophagus is a disease marked by the presence of columnar epithelium in the lower esophagus, replacing the normal squamous cell epithelium as a result of metaplasia. The histological hallmark of Barrett's esophagus is the presence of intestinal goblet cells in the esophagus. Exploiting the similarity between Barrett's Esophagus and the intestinal epithelium, the inventors showed that the culture medium and methods of the invention could be used to maintain Barrett's Esophagus epithelium for up to 1 month. The inventors also demonstrated, for the first time, that addition of FGF10 to the culture medium of the invention enabled the Barrett's Esophagus organoids to form budding structures and significantly prolonged the culture duration to more than three months. Thus, a Barrett's Esophagus organoid is an example of an organoid of the invention. In some embodiments, a Barrett's Esophagus organoid has a cystic structure. In some embodiments, a Barrett's Esophagus organoid of the invention comprises Paneth cells. In some embodiments, a Barrett's Esophagus organoid of the invention expresses lysozyme.

The inventors, therefore, also describe a culture medium according to the invention, comprising FGF10, for the culture of Barrett's Esophagus epithelium.

In some embodiments of the invention, Barrett's Esophagus organoids may be grown using a culture medium according to invention also comprising FGF10. In some embodiments, these Barrett's Esophagus organoids express Ki67 and have a minimal number, preferably less than 10%, less than 5% or less than 1% PAS-positive cells and Mucin-positive cells. In some embodiments, the Barrett's Esophagus organoids comprise lysozyme-positive Paneth cells.

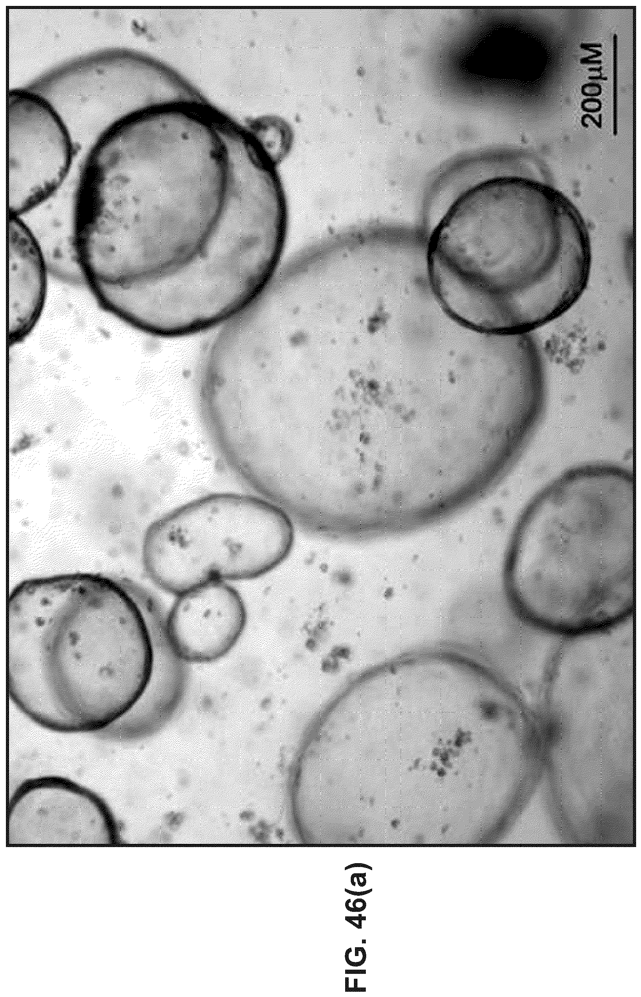

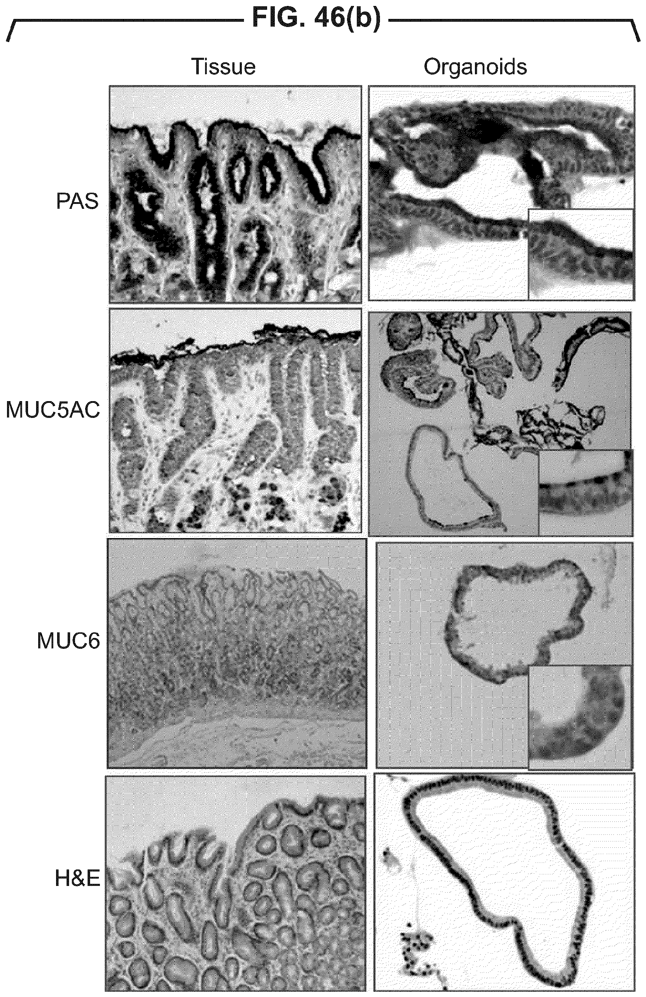

### Stomach (gastric) organoids (for example, see figure 46)

Mouse gastric organoids grown in a culture medium of the invention are three-dimensional organoids, comprising or consisting of a single layer epithelia, that comprises a gastric gland base like domains (formed by stem and progenitor cells) surrounding a central lumen lined by epithelial domains comprising differentiated cell types, and optionally wherein non-epithelial cells are absent from said organoid.

Human gastric organoids grown in a culture medium of the invention comprise cystic structures. The cystic structure is a monolayer of polarized cells. These human gastric organoids grown in the presence of a TGF-beta inhibitor resemble mouse gastric organoids much more closely than human organoids grown in the absence of TGF-beta inhibitor.

### Prostatic organoids (see figures 41 to 43)

Under culture conditions comprising EGF, Noggin, Rspondin, murine prostatic organoids form three dimensional cystic structures with a lumen. In time the layers fold inward forming 3-4 layers of (stratified) epithelial cells. The outer layer is mostly composed of CK5+ basal epithelial cells whereas the inner layers are mostly composed of CK8+ luminal epithelial cells. No stem cell compartment has been identified; all domains contain dividing cells. Therefore, in some embodiments, a prostate organoid grown in the absence of testosterone comprises stratified layers of dividing epithelial cells. In a further embodiment, the prostate organoid comprises an outer layer of cells comprising CK5+ basal epithelial cells and inner layers comprising CK8+ luminal epithelial cells. In some embodiments, a prostate organoid grown in the absence of testosterone does not contain any stem cells.

### Addition of testosterone to the prostate culture medium

The inventors have shown that the addition of (DiHydro) testosterone to the culture conditions for the prostatic organoids, results in the majority of cells differentiating into CK8+ luminal cells which form a single layer of epithelium that folds onto itself into two layers. Prostate organoids grown in the presence of testosterone consist of mostly luminal cells with or without a second layer of basal cells. The structure resembles the *in vivo* structure. Both differentiated and dividing cells are present, as well as stem cells and progenitors. Therefore, in some embodiments, for example when cultured in a medium comprising testosterone, a prostate organoid is a three-dimensional organoid comprising cystic structures and a lumen. In some embodiments the prostate organoid comprises CK8+ luminal cells which form a monolayer of epithelium. In some embodiments the monolayer is folded into two or more layers. In other embodiments, the organoid may comprises regions of stratified cells. In some embodiments, the prostate organoid comprises differentiated cells while maintaining the dividing stem cell population.In some embodiments, the shape of the organoid is determined by the origin of the cellular or tissue starting material (i.e. the position in the prostate before isolation). The prostate consists of different lobes or regions which display the different epithelial structures described above (stratified and folded), After *in vitro* culturing the organoids appear to some extent to maintain the different macroscopic structure (stratified or folded) of the part of the prostate from which it originated.

### Liver organoids

Structurally, mouse liver organoids according to the invention are often elongated in shape. They may include one or more budding structure - a single cell epithelial layer which has a structure not unlike a bile duct. Under confocal microscopy, the structures may stain positive for keratin. They may include cells with polarised nuclei and small cytoplasm. The organoids may have a section which is formed of multiple layers; such cells often tend to have their nuclei more central to the cells, i.e. not polarized. The cells in the multilayer section may organise themselves to include a gap, or lumen between the cells. Human liver organoids of the invention, in some embodiments have a generally cystic structure.

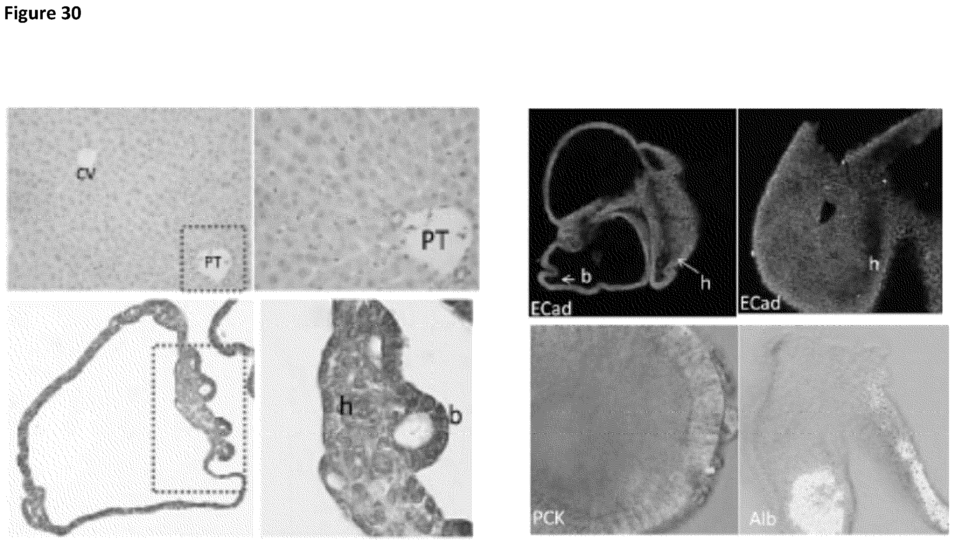

In some embodiments, a liver organoid is a three-dimensional organoid, with a cystic structure (for example, see figure 30). Under expansion conditions the organoid may consist of stem cells and progenitor cells where two domains are defined: (1) A duct-like domain, formed by a single-layer cubical epithelia (positive for the ductal marker Krt19) with cells lining a central lumen; and (2) a pseudo-stratified epithelial domain where krt19 positive cells and scattered albumin positive cells are detected. This architecture (areas with single layer epithelia together with areas of pseudostratified epithelia) resembles the embryonic liver bud. Under expansion conditions fully differentiated cells are not present, although expression of hepatocyte/hepatoblast-specific markers can in some embodiments be detected. Differentiation conditions result in the formation of a cystic organoid where the duct-like domain (single layer epithelia) is lost and the entire structure becomes a pseudo-stratified epithelia containing >50% polarized hepatocytes.

A liver organoid, preferably comprises a hepatocyte and a cholangiocyte cell (although hepatocytes are especially seen following differentiation in DM and are not required for expansion), more preferably wherein at least one of the following markers could be detected: at least one hepatocyte marker such as albumin, transthyretrin, B-1 integrin and Glutamine synthetase and/or at least one of CYP3A11, FAH, tbx3, TAT and Gck and/or at least one cholangiocyte maker such as Keratin 7 and 19. The skilled person knows how to detect each of these markers (i.e. RT-PCR and/or immunofluorescence). Preferably the expression of each of these markers is assessed as carried out in the experimental part. Each of these markers is usually expressed after at least two weeks, three weeks or one month of culture using a method of the invention. Microarray analysis of the organoids in both culture conditions showed that liver organoids resemble adult liver tissue.

Preferably all cells in a liver organoid express hepatocyte surface markers. For example, in some embodiments, at least 50% (for example 50-60%), at least 60%, at least 70%, at least 80%, at least 90%, at least 99% or 100% of cells in a liver organoid express hepatocyte markers. In some embodiments, approximately 35% of the cells in a liver organoid express a hepatocyte surface marker, for example, 25-45%, 30-40%, 33-37%, 35% or less, or 15-35% of cells. In some embodiments, the expansion phase would have less hepatocytes, for example less than 20%, less than 10%, less than 5% of the cells, less than 2%, less than 1%, preferably 0% of the cells. Preferably, cells and organoids generated according to the invention also possess hepatocyte functions, such as expressing or staining positive for the mature hepatic markers albumin, B-1 integrin,, CK-8, CK-18, transthyretin (TTR), glucose 6P, Met, Glutamine synthase (Glul), transferrin, Fahd1, Fahd2a, K7, K19 and cytochrome P450 isoforms 3A13 (CYP3A13), 51 (CYP51) 2D10 (CYP2D10), 2j6 (CYP2j6), 39A1 (CYP39A1), 4A10 (CYP4A10), 4F13 (CYP4F13) 4F16 (CYP4F16), CYP4B1and 20A1(CYP20A1). Also, embryonic liver gene AFP is in some embodiments not detected in neither of both culture conditions, as in adult liver. In some embodiments, the expression of alpha fetal protein is just above the background gene expression.

Also, the well-known liver transcription factors as HNF1a, HNF1b and HNF4a are highly expressed in both conditions.

Since liver and pancreas are closely related organs, we investigated whether our liver cultures also expressed pancreas-specific genes. The pancreas is functionally divided into endocrine and exocrine pancreas. The endocrine pancreas is mainly characterized for expressing insulin, glucagon and somatostatin. The expression of these hormones is tightly regulated by a set of endocrine pancreas-specific transcription factors, the most important being Pdx1 and NeuroD. The exocrine pancreas is formed by acinar and ductal compartments responsible of producing the digestive enzymes amylase, pancreatic lipase and chymotrypsin, among others. The expression of these genes is also regulated by specific exocrine pancreatic genes as Ptfl.

The pancreas specific genes Ptfla, pancreatic amylase (Amy2a4), pancreatic lipase (Pnlip), insulin (ins1 and ins2), glucagon (Gcg), chymotrypsin (cela1), Pdx1 and NeuroD were absent in the liver cultures here described.

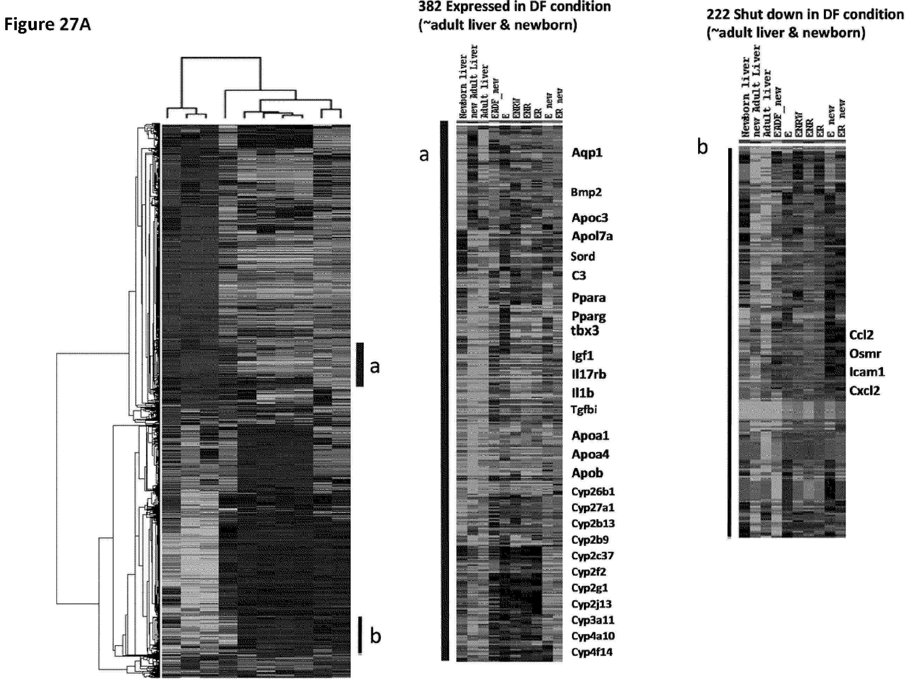

In some embodiments, one or more or all of the following genes are expressed in the liver organoids at a similar level to the corresponding gene in adult liver hepatocytes: Aqp1, Bmp2, Apo3, Apol7a, Sord, C3, Ppara, Pparg, tbx3, lgf1, ll17rb, ll1b, Tgfbi, Apoa1, Apoa4, Apob, Cyp26b1, Cyp27a1, Cyp2b13, Cyp2b9, Cyp2c37, Cyp2f2, Cyp2g1, Cyp2j13, Cyp3a11, Cyp4a10 and Cypf14. For example, see Figure 27A.

In some embodiments, one or more of the following genes is expressed in the liver organoids at a similarly shut down level compared to the corresponding gene in adult liver hepatocytes: Ccl2, Osmr, Icam1 and Cxcl2.

In some embodiments, one or both of the following genes is differentially expressed in both a liver organoid and newborn liver: mKi67 and cdkn3, meaning that the expression of these genes is higher in the organoids than in the differentiated organoids or whole organ.

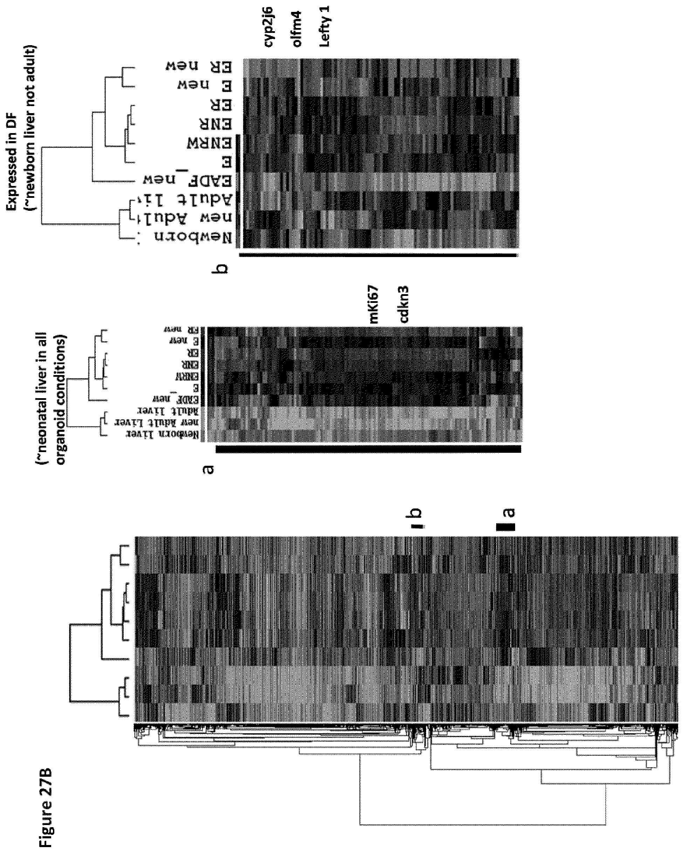

In some embodiments, one, two or all of the following genes are expressed at a similar level in a liver organoid and a newborn liver: cyp2j6, olfm4 and Lefty 1. For example, see Figure 27B.

In some embodiments, a liver organoid of the invention has a ductal phenotype when cultured in expansion medium of the invention (e.g. EM1 or EM2).

In some embodiments, a liver organoid of the invention expresses adult liver markers when cultured in a differentiation medium of the invention.

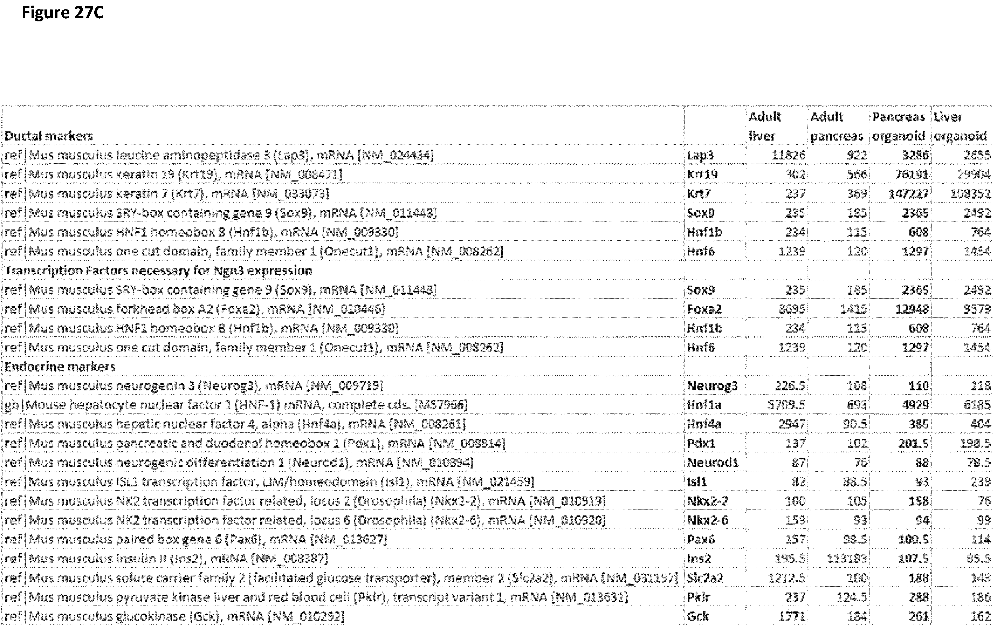

In one embodiment, a liver organoid of the invention has a gene expression profile as shown in Figure 27C.

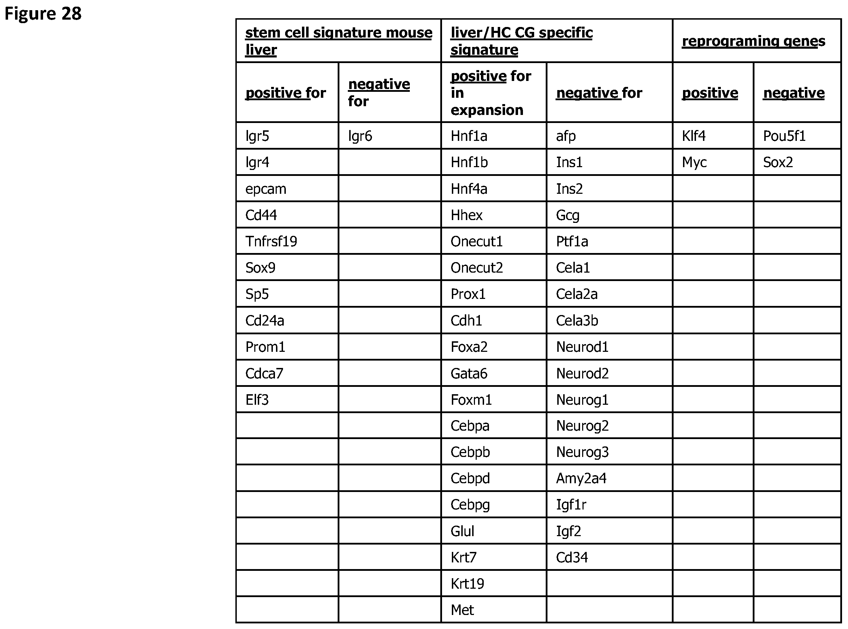

In a particularly preferred embodiment, a mouse liver cell population or organoid of the invention has the gene expression profile as shown in Figure 28. For example, in one preferred embodiment, a mouse liver cell population or organoid of the invention:
a) expresses at least one (e.g. 1, 2, 3, 4, 5, 6, 7, 8, 9, 10, 11), preferably all of the following stem cell markers: lgr5, lgr4, epcam, Cd44, Tnfrsfl9, Sox9, Sp5, Cd24a, Prom1, Cdca7 and Elf3; and/or
b) does not express the following stem cell marker: lgr6; and/or
c) expresses at least one (e.g. 1, 2, 3, 4, 5, 6, 7, 8, 9, 10, 11, 12, 13, 14, 15, 16, 17, 18, 19), preferably all of the following hepatocyte or cholangiocyte markers when grown in expansion medium of the invention: Hnf1α, Hnf1b, Hnf4a, Hhex, Onecut1, Onecut2, Prox1, Cdh1, Foxa2, Gata6, Foxm1, Cebpa, Cebpb, Cebpd, Cebpg, Glul, Krt7, Krt19 and Met; and/or
d) does not express at least one (e.g. 1, 2, 3, 4, 5, 6, 7, 8, 9, 10, 11, 12, 13, 14, 15, 16, 17 ) of the following genes when grown in expansion medium of the invention: afp, Ins1, Ins2, Gcg, Ptf1a, Cela1, Cela2a, Cela3b, Neurod1, Neurod2, Neurog1, Neurog2, Neurog3, Amy2a4, Igf1r, Igf2 and Cd34; and/or
e) expresses at least one (e.g. 1, 2 or 3) of the following reprogramming genes: Klf4, Myc and Pou5fl and/or
f) does not express the following reprogramming gene: Sox2.
wherein the expression of the genes is preferably detected by measuring expression at the mRNA level, for example, using a microarray.

More preferably a mouse liver cell population or organoid of the invention has all of features a) to f) above.

In some embodiments, the gene expression profile described above for a mouse liver cell population or liver organoid of the invention is for a mouse cell population or organoid cultured in liver expansion medium of the invention.

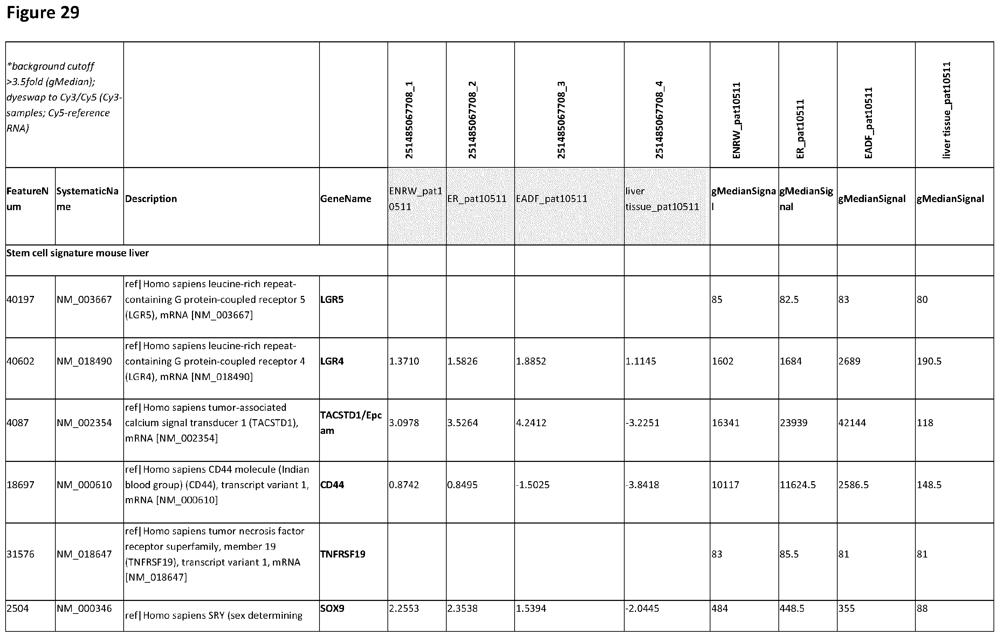

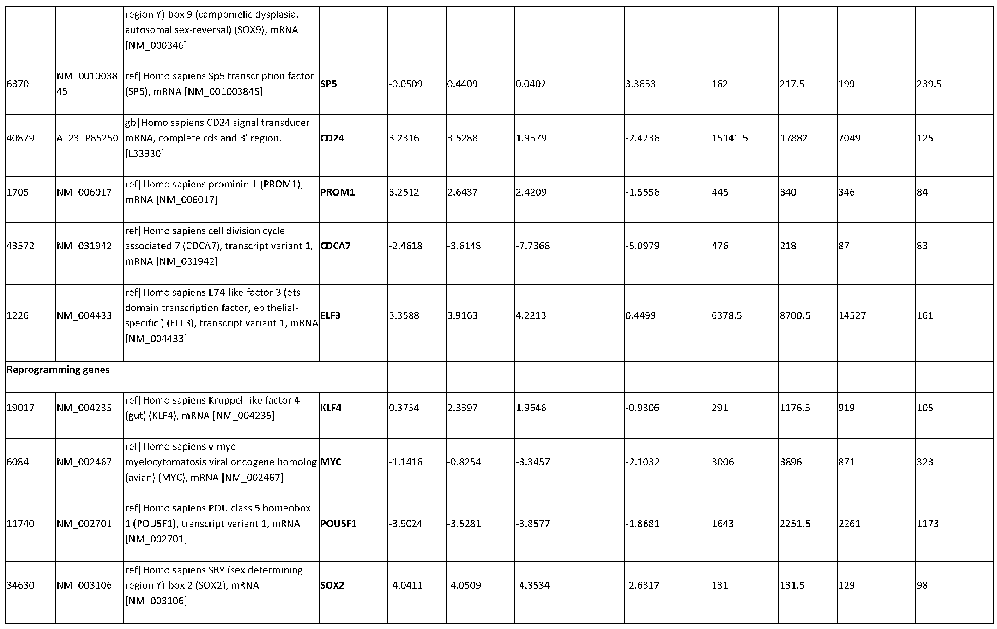

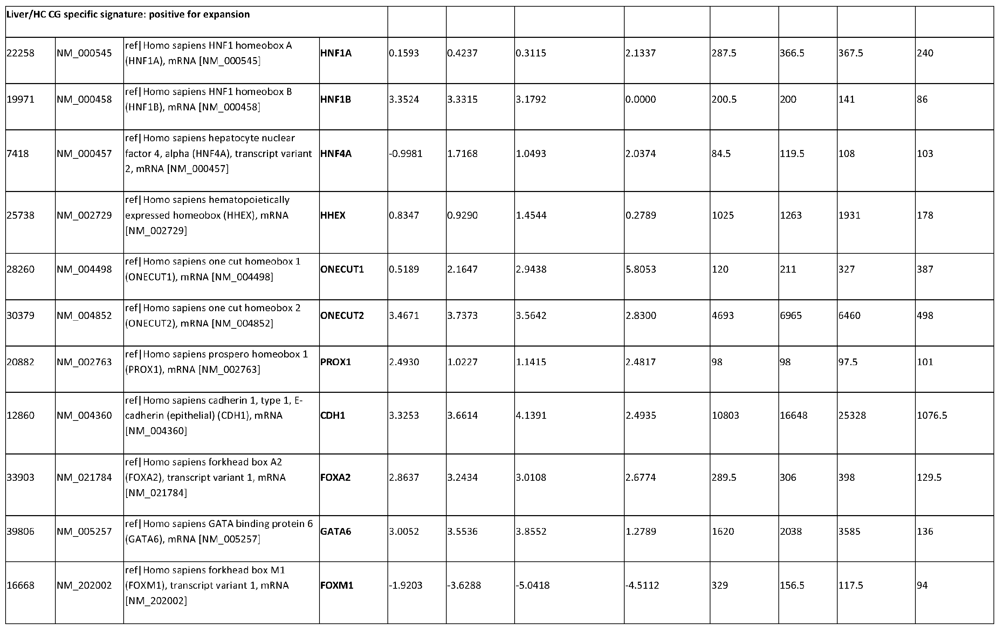

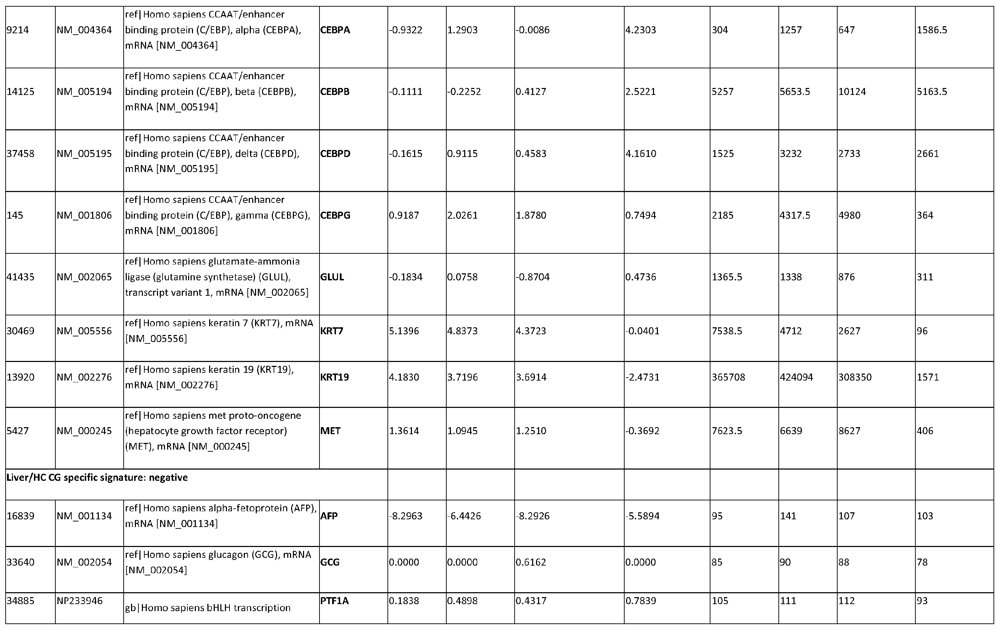

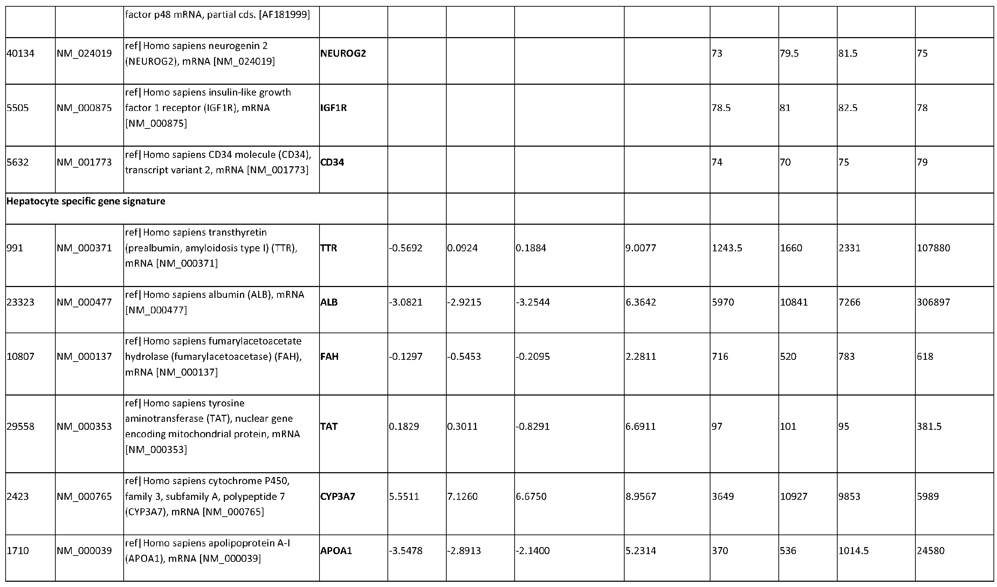

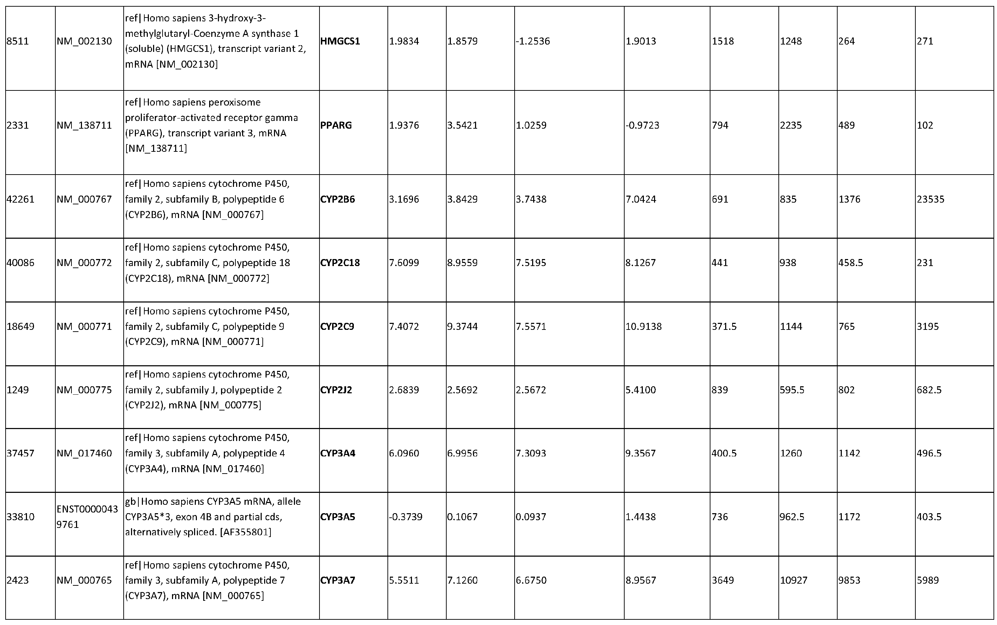

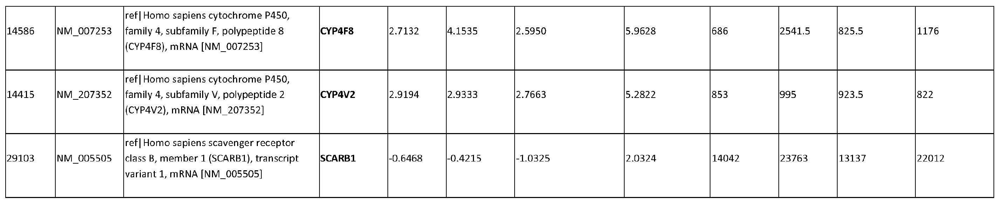

In some embodiments, there is provided a human liver cell population or organoid of the invention that has the gene expression signature shown in Figure 29. For example, a human liver cell population or organoid cultured in EM1 of the invention preferably expresses the genes indicated in Figure 29 as being expressed in EM1 cell culture medium. For example, a human liver cell population or organoid cultured in EM2 of the invention preferably expresses the genes indicated in Figure 29 as being expressed in EM2 cell culture medium. For example, a human liver cell population or organoid cultured in DM of the invention preferably expresses the genes indicated in Figure 29 as being expressed in DM cell culture medium.

For example, in one preferred embodiment, a human liver cell population or organoid of the invention:
a) expresses at least one (e.g. 1, 2, 3, 4, 5, 6, 7, 8, 9), preferably all of the following stem cell signature genes: LGR4, TACSTD1/Epcam, CD44, SOX9, SP5, CD24, PROM1, CDCA7 and ELF3; and/or
b) expresses at least one (e.g. 1, 2, 3, 4), preferably all of the following reprogramming genes: KLF4, MYC, POU5F1 and SOX2; and/or
c) expresses at least one (e.g. 1, 2, 3, 4, 5, 6, 7, 8, 9, 10, 11, 12, 13, 14, 15, 16, 17, 18, 19), preferably all of the following hepatocyte/cholangiocyte specific genes: HNF1A, HNF1B, HNF4A, HHEX, ONECUT1, ONECUT2, PROX1, CDH1, FOXA2, GATA6, FOXM1, CEBPA, CEBPB, CEBPD, CEBPG, GLUL, KRT7, KRT19 and MET; and/or
d) does not express at least one (e.g. 1, 2, 3, 4, 5, 6), preferably all of the following hepatocyte/cholangiocyte specific genes: NEUROG2, IGF1R and CD34, AFP, GCG and PTF1A, for example, it does not express NEUROG2, IGF1R and CD34; and/or
e) expresses at least one (e.g. 1, 2, 3, 4, 5, 6, 7, 8, 9, 10, 11, 12, 13, 14, 15, 16, 17, 18), preferably all of the following hepatocyte specific genes: TTR, ALB, FAH, TAT, CYP3A7, APOA1, HMGCS1, PPARG, CYP2B6, CYP2C18, CYP2C9, CYP2J2, CYP3A4, CYP3A5, CYP3A7, CYP4F8, CYP4V2 and SCARB1;
wherein the expression of the genes is preferably detected by measuring expression at the mRNA level, for example, using a microarray.

More preferably a human liver cell population or organoid of the invention has all of features a) to e) above.

In some embodiments, the genes in a human liver cell population or organoid of the invention are upregulated or downregulated relative to expression of a reference RNA as shown in Figure 29. Preferably, the reference RNA is Universal Human Reference RNA (Stratagene, Catalog #740000). In some embodiments, a gene is upregulated or downregulated relative to the reference RNA if it is also shown in Figure 29 as being upregulated or downregulated relative to the reference RNA but the extent of upregulation or downregulation need not be the same. In other embodiments, the extent of upregulation or downregulation is +/-35%, +/-30%, +/-25%, +/-20%, +/-20%, +/-15%, +/-10%, +/-5%, +/-3%, or more preferably +/-1.5-fold, +/-2-fold, +/-3-fold, +/- 5-fold or approximately the same as shown in Figure 29. In other embodiments, the absolute level of expression of the genes in a human organoid of the invention is +/-35%, +/-30%, +/-25%, +/-20%, +/-15%, +/-10%, +/-5%, +/-3%, or +/-1.5-fold, +/-2-fold, +/-3-fold, +/- 5-fold or approximately the same as shown in Figure 29.

The human liver cell population or organoids of the invention also preferably express Lgr5 and/or Tnfrsf19, preferably both. In some embodiments, the human liver cell population or organoids, when cultured in expansion medium of the invention express Lgr5 and/or Tnfrsf19, preferably both. Preferably, expression of Lgr5 and/or Tnfrsfr19 is detected by RT PCR. In some embodiments, Lgr5 and/or Tnfrsf19 are present at much lower levels of expression in organoids or cells when cultured in the differentiation medium compared to their level of expression organoids or cells when cultured in the expansion medium (for example at least 2-fold, at least 3-fold, at least 4-fold, at least 5-fold, at least 10-fold, at least 15-fold lower).

Liver cells and organoids according to the present invention may preferably be capable of secreting albumin, for example, at a rate of between approximately 1µg per hour per 10⁶ cells and 10µg per hour per 10⁶ cells, preferably between 2µg and 6µg per hour per 10⁶ cells.

Furthermore, such liver cells and organoids may secrete urea. For example, in a 35mm dish of cells, the activity of urea synthesis may be between 1µg and 50µg in 48 hours, preferably between 5µg and 30µg.

Liver cells and organoids according to the invention may show visible glycogen stores, for example, when stained. The capacity for cells and organoids according to the invention to synthesize glycogen actively can be tested by switching the culture media from low-glucose differentiation media to high-glucose DMEM supplemented with 10% FBS and 0.2µM dexamethasone for two days.

Liver cells and organoids according to the invention may possess inducible cytochrome P450 activity (e.g. CYP1A). Such activity may be tested, for example, using an ethoxyresorufin-O-deethylase (EROD) assay (Cancer Res, 2001, 61: 8164-8170). For example, cells or organoids may be exposed to a P450 substrate such as 3-methylcholanthrene and the levels of EROD activity compared to control cells.

Morphologically, the liver organoid cells appear hepatocyte-like.

A preferred liver organoid comprises or consists of a cystic structure with on the outside a layer of cells with buds and a central lumen as depicted in Figure 30. This liver organoid may have one or more (e.g. 2, 3, or all 4) of the following characteristics: (a) having a cell density of >5×10⁵ cells/cm³, preferably >10×10⁵ cells/cm³; (b) having a thickness equivalent to 2-30 layers of cells, preferably a thickness equivalent to 2-15 layers of cells; (c) the cells mutually contact in three dimensions, (d) demonstrate a function inherent to healthy liver tissue, (e) have an elongated shape, with 2 defined domains, i.e. a single layered epithelial domain where highly polarized cells are detected and keratin markers are expressed (this domain resembles the bile duct domain) and the other domain constitutes the main body of the organoid and is formed by a multilayered epithelia with non-polarized cells wherein albumin expression may be detected. It is clear to the skilled person that such a liver organoid is preferably not a liver fragment and/or does not comprise a blood vessel, and/or does not comprise a liver lobule or a bile duct.

Within the context of the invention, a liver fragment is a part of an adult liver, preferably a human adult liver. Preferably a liver organoid as identified herein is therefore not a liver fragment. A liver organoid is preferably obtained using a cell from an adult liver, preferably an epithelial stem cell from an adult liver, more preferably an epithelial stem cell from an adult liver expressing Lgr5. A liver organoid may also be obtained from any cell which upon damage or culturing expresses Lgr5 and is therefore an Lgr5-expressing cycling stem cell.

In some embodiments, a liver organoid comprises cells that express Lgr5. For example, in some embodiments, at least 2%, more preferably at least 5%, at least 10%, at least 20%, at least 30%, at least 40%, at least 50%, at least 60%, at least 70%, at least 80%, at least 90%, at least 95% of the cells in the liver organoid express Lgr5. Similarly, the invention provides a cell or a population of cells which express Lgr5, wherein said cells are obtained from a liver organoid of the invention. The progeny of such cells is also encompassed by the invention.

In an embodiment, a liver organoid is a liver organoid which is still being cultured using a method of the invention and is therefore in contact with an extracellular matrix. Preferably, a liver organoid is embedded in a non-mesenchymal or mesenchymal extracellular matrix. Within the context of the invention, "in contact" means a physical or mechanical or chemical contact, which means that for separating said liver organoid from said extracellular matrix a force needs to be used.

In a preferred embodiment, a liver organoid could be cultured during at least 2, 3, 4, 5, 6, 7, 8, 9, 10 weeks or 1, 2, 3, 4, 5, 6, 7, 8, 9, 10 months or longer. In some embodiments, the liver organoid is expanded or maintained in culture for at least 3 months, preferably at least 4 months, at least 5 months, at least 6 months, at least 7 months, at least 9 months, or at least 12 months or more. Preferably, a liver organoid cultured using expansion media of the invention comprising a TGF beta inhibitor may be cultured for at least 4 weeks, more preferably at least 5 weeks at 5 fold expansion a week or two or more population doublings per week (e.g.. for at least 10 doublings, at least 20 doublings, more preferably at least 25 doublings, for example, at least 30 doublings). Preferably, a liver organoid cultured using expansion media of the invention comprising a prostaglandin pathway activator in addition to a TGF beta inhibitor may be cultured for at least 7 weeks, more preferably at least 8 weeks at 2 or more doublings (e.g. 2-3 doublings) per week (i.e. at least 15 doublings, at least 25 doublings, at least 30 doublings, at least 32 doublings, at least 35 doublings, e.g. 32-40 doublings or at least 40 doublings, for example, at least 50 doublings). Thus, preferably, a liver organoid of the invention, for example a human liver organoid, is obtained using expansion media of the invention.

In another preferred embodiment, a liver organoid originates from a single cell, preferably expressing Lgr5, more preferably wherein the single cell comprises a nucleic acid construct comprising a nucleic acid molecule of interest.

### Organoid composition and gene expression

The crypt-villus, colon crypt and pancreatic organoids typically comprise stem cells and/or progenitor cells and, therefore, these organoids share certain patterns of gene expression. In some embodiments, one or more (for example, 1, 2, 3, 4, 5, 6 or 7) or all of the following markers can be detected: LGR5, LGR4, epcam, Cd44, Sox9, Cd24a, and CD133/Prom1 and optionally Tnfrsfl9. In another embodiment, the expression of one or two or all of the following progenitor genes can be detected: Pdx1, Nkx2.2, and Nkx6.1. After differentiation, gene expression patterns of the crypt-villus, colon crypt and pancreatic organoids are expected to diverge as the differentiated organoids express tissue-specific adult markers, such as insulin in the pancreas for example.

### Crypt villus organoids

In some embodiments of the invention, the organoids comprise crypt-villus like extensions which comprise all differentiated epithelial cell types, including proliferative cells, Paneth cells, enterocytes and goblet cells. In some embodiments, the crypt-villus organoids of the invention do not contain myofibroblasts or other non-epithelial cells. A crypt-villus organoid of the invention preferably comprises enterocytes, including absorptive enterocytes, goblet cells, enteroendocrine cells, and Paneth cells in a crypt-villus-like structure. Preferably at least one (for example, 2, 3, 4, 5 or 6) of the following markers could be detected: SMOC2, CDCA7, OLFM4, ASCL2, AXIN2 and/or Lgr5 Tnfrsf19, CD24a, Sox9, CD44, Prom1 (see Figure 2e and Figure 14). In some embodiments, the markers RNF43 and ZNRF3 can be detected. In some embodiments, one or more (for example 1, 2, 3, 4 or 5) or all of SMOC2, CDCA7, OLFM4, ASCL2, AXIN2 and/or Lgr5 are at least 2-fold, 3-fold, or 4-fold upregulated in crypts, whereas markers that are at least 2-fold, 3-fold, or 4-fold downregulated in crypts include at least one or more (for example 1, 2, 3 or 4) or all of ABCG1, ENPP3, CSTE, MUC17 and/or APOA1. In this context "upregulation" is relative to the villus of the intestine or to the top section of the colon crypt. Microarray analysis, comparing the gene expression of differentiated organoid cells to stem cells, revealed that the small intestinal crypt-villus and colonic organoids possess comparable molecular signatures of intestinal crypts including the expression of intestinal stem cell genes. Thus, the invention also provides a colonic organoid having the molecular signature described above for crypt-villus organoids. Organoids cultured *in-vitro* clearly exhibit a similar expression profile to freshly isolated small intestinal crypts and express known stem cell markers.

In some embodiments, the mRNA encoding one or more genes (e.g. 1, 2, 3, 4, 5, 6, 7, 8, 9, 10, 11, 12, 13, 14, 15, 16, 17, 18, 19, 20, 21, 22, 23, 24 or 25) listed in Figure 14 (for example all of the genes shaded in Figure 14) as being upregulated in crypt-villus organoids or colon organoids respectively is upregulated in a crypt-villus organoid or colon organoid of the invention compared to a freshly isolated small intestinal villi, as determined by microarray. In some embodiments, the mRNA encoding one or more (e.g. 1, 2, 3, 4, 5, 6, 7, 8, 9, 10, 11, 12, 13, 14, 15, 16, 17, 18, 19, 20, 21, 22, 23, 24 or 25) genes listed in Figure 14 (for example all of the genes shaded in Figure 14) as being downregulated in crypt-villus organoids or colon organoids respectively is downregulated in a crypt-villus organoid or colon organoid of the invention compared to a freshly isolated small intestinal villi, as determined by microarray. In some embodiments, the fold upregulation or downregulation is as indicated in Figure 14 + /- 25%, for example, +/ - 20%, +/ - 15%, + /- 10%, +/-5%, +/-3% or approximately as quoted in Figure 14. For example, a crypt-villus organoid of the invention may have ADORA2B upregulated 9.54 fold +/- 25% compared to freshly isolated small investinal villi. The same applies, mutatis mutandis, to the other genes listed in Figure 14.

In some embodiments, the crypt villus organoids show natural expression of Lgr5. In some embodiments, the crypt villus organoids show natural expression of at least Lgr5 and one or more (for example 1, 2, 3, 4, 5, 6, 7, 8, 9, 10, 11, 12, 13, 14, 15, 16) or all of stem cell markers from the group consisting of: CK19, Nestin, Somatostatin, CXCR4⁺, CD133⁺, DCAMKL-1, CD44, Sord, Sox9, CD44, Prss23, Sp5, Hnf1α, Hnf4a, Sox9, KRT7 and KRT19. In addition or alternatively, crypt-villus organoids may be characterised by expression of one or more or all (for example 1 or 2) of: Sord and/or Prss23. In addition or alternatively, crypt-villus organoids may be characterised by expression of CD44 and/or Sox9. In another embodiment, the crypt-villus organoids show expression of one or more (for example 1, 2, 3, 4, 5, 6, 7, 8, 9) or all of the markers from the group consisting of: lgr5, lgr4, epcam (tacstd1), Cd44, Tnfrsfl9, Sox9, Sp5, Cd24a, Prom1, and Cdca7.

In some embodiments, a crypt villus organoid comprises Paneth cells expressing lysozyme.

### Colon organoids

In some embodiments, a colon organoid contains enteroendocrine cells (e.g. as detectable using chromagranin A stain), goblet cells (as detectable using mucin 2 stain). In some embodiments, less than 10% of the cells in the colon organoid are enteroendocrine cells (e.g. 0.01-5%, 0.1-3%). In some embodiments, less than 30% of the cells in the colon organoid are goblet cells (e.g. 1-25%, 1-15%, 5-10%). In some embodiments, the distribution of the enteroendocrine cells and/or the goblet cells is as shown in the Figure 1d.

In some embodiments, a colon organoid contains mature enterocytes (e.g. as visualised by alkaline phosphatise staining). In some embodiments, less than 10% of the cells in the colon organoid are mature enterocytes (e.g. less than 5%, less than 3%, 0.01-5%, 0.1-3%, 0.1-5%).

In preferred embodiments, a colon organoid does not comprise Paneth cells because there are no Paneth cells in a naturally occurring *in vivo* colon.

In some embodiments, the colon organoids show natural expression of Lgr5.

In some embodiments, a colon organoid expresses one or more (e.g. 1, 2, 3 or 4) of Villin1, Alpi, ChgA and Muc2. In some embodiments, the relative amount of Villin1 mRNA expressed by a colon organoid of the invention compared to a freshly isolated colon crypt is at least 3% (e.g. at least 5%, at least 8%, at least 10%), for example between 5-15%. In some embodiments, the relative amount of Alpi mRNA expressed by a colon organoid of the invention compared to a freshly isolated colon crypt is at least 0.5% (e.g. at least 1%, at least 2%), for example, between *0.5-5%.* In some embodiments, the relative amount of ChgA mRNA expressed by a colon organoid of the invention compared to a freshly isolated colon crypt is at least 15% (e.g. at least 20%, at least 22%), for example, between 15-30%. In some embodiments, the relative amount of Muc2 mRNA expressed by a colon organoid of the invention compared to a freshly isolated colon crypt is at least 20% (e.g. at least 25%, at least 30%, at least 35%), for example, between 25-37%.

In some embodiments, a human colon organoid of the invention expresses known stem cell markers.

### Pancreatic organoids

The pancreas contains three classes of cell types: the ductal cells, the acinar cells, and the endocrine cells. The endocrine cells produce the hormones glucagon, insulin somatostatin and pancreatic polypeptide (PP), which are secreted into the blood stream and help the body regulate sugar metabolism. The acinar cells are part of the exocrine system, which manufactures digestive enzymes, and ductal cells from the pancreatic ducts, which connect the acinar cells to digestive organs. During development, Islets of Langerhans are thought to descend from progenitor endocrine cells which emerge from the pancreatic duct and after differentiation aggregate to form Islets of Langerhans. Islets of Langerhans comprise α cells, β cells, δ cells, and PP cells.

Pancreatic organoid cells may have an expression pattern that resembles ductal cell markers, such as one or more (e.g. 1, 2 or all) of K7, K19 and Hnflb and/or one or more general stem cell markers such as Sox9 and/or Onecut1. This is likely to be part of their stem cell signature. Generally, fewer differentiation markers are seen. In some embodiments in which a cell is isolated from a pancreatic duct in order to generate a pancreatic organoid of the invention, the cell type that gives rise to a pancreatic organoid of the invention is not a ductal cell (meaning the epithelial cells positive for keratin 7 and keratin 19 that form the ductal tube), but it is a cell attached to the pancreatic duct, meaning a cell that is located in the next layer of cells after the duct in contact with the pancreatic tissue (i.e. not facing the lumen of the duct.) Thus, in embodiments in which the cell type that gives rise to a pancreatic organoid is not a ductal cell, the pancreatic organoid will not express K7 or K19. However, such a pancreatic organoid will still preferably express one or more general stem cell progenitor markers such as Sox9.

A pancreatic organoid of the invention preferably comprises α cells, β cells, δ cells, and PP cells. In a further preferred embodiment, a pancreatic organoid comprises beta-cells. For example, a pancreatic organoid may comprise more than 1%, more than 5%, more than 10%, more than 15%, or more than 20% beta-cells. Expression of insulin may be used as a marker for beta cells.

In an alternative embodiment, the pancreatic organoid comprises progenitor cell types, optionally with a ductal origin, that can give rise to differentiated cell-types upon transplantation into a human or animal. In a preferred embodiment, the progenitor cell types can give rise to insulin-secreting beta-cells upon transplantation into a human or animal. The inventors have shown that human pancreatic organoids, grown according to the media and methods of the invention, can be transplanted into mice and stimulate insulin-secreting cells within one month (see example 4). It can be easily understood that this could lead to revolutionary treatments for patients with diabetes and insulin-deficiencies.

In some embodiments, a pancreatic organoid of the invention may comprise ductal cells, acinar cells and endocrine cells. In some embodiments, K19 is used as a marker for ductal cells.

In some embodiments, a beta-cell exists within pancreatic islands or Islets of Langerhans. An islet generally comprises around 1500 cells *in vivo,* for example, 1300-1700 cells. In one embodiment, a pancreatic organoid comprises at least 0.5%, at least 1%, at least 1.5%, at least 2%, at least 3%, at least 5%, at least 10%, at least 15%, at least 20%, at least 25%, at least 30% or more Islets of Langerhans by mass. In some embodiments, the Islets of Langerhans of the pancreatic organoid are composed of approximately 65 to 90% beta cells, approximately 15 to 20% alpha-cells, approximately 3 to 10% delta cells, and approximately 1% PP cells. However, this is by no means exclusive. For example, in some embodiments, it is desirable to have many beta cells in an organoid of the invention. Alternatively, an organoid may comprise progenitor cells that may be transplanted so that they differentiate *in vivo.*

In some embodiments, a pancreatic organoid expresses one, two or all three of Pdx1, Nkx2.2 and Nkx6.1. A pancreatic organoid may express one, two, three or all four of NeuroD, Pax6, Pax4 and Mafa. Pax4 serves as a marker for the presence of insulin producing cells because it is an essential transcription factor for the differentiation of insulin producing cells from endocrine progenitor cells during embryonic development. A pancreatic organoid may express Ngn3.

In some embodiments, at least one (for example 1, 2, 3, 4, 5) of the following markers can be detected in a pancreatic organoid of the invention: insulin (ins1 and/or ins2), glucagon (Gcg), somatostatin, Pdx1 and NeuroD. In some embodiments, at least one (for example 1, 2, 3, 4, 5) of the following markers can be detected in a pancreatic organoid of the invention: insulin (ins1 and/or ins2), glucagon (Gcg), somatostatin, Pdx1 and NeuroD and the following markers are not detected: ptfla, amy2a4, Pnlip and cela1. In some embodiments, at least one (for example 1, 2, 3, 4, 5, 6, 7, 8 or 9) of the following markers can be detected in a pancreatic organoid of the invention: Ptfla, pancreatic amylase (Amy2a4), pancreatic lipase (Pnlip), insulin (ins1 and/or ins2), glucagon (Gcg), somatostatin, chymotrypsin (cela1), Pdx1 and NeuroD.

In some embodiments, the pancreatic organoids show natural expression of Lgr5. In some embodiments, the pancreatic organoids show natural expression of at least Lgr5 and one or more (e.g. 1, 2, 3, 4, 5, 6, 7, 8, 9, 10, 11, 12, 13, 14, 15, 16, 17, 18, 19 or 20) stem cell markers selected from the group consisting of: CK19, Nestin, CXCR4⁺, CD133⁺, DCAMKL-1, CD44, Sord, Sox9, CD44, Prss23, Sp5, Hnf1α, Hnf4a, Sox9, KRT7 and KRT19, prom1, Cd24a, Lgr4, epcam. Alternatively or additionally, in some embodiments, pancreatic organoids may be characterised by natural expression of one or more (for example 1, 2, 3 or 4) of: CK19, Nestin, (insulin, glucagon) and CXCR4⁺.

In some embodiments, the pancreatic organoids or cells of the invention express Somatostatin. Somatostatin is a hormone expressed in differentiated delta cells and so may serve as a marker for delta cells.

Alternatively or additionally, in some embodiments, pancreatic organoids show natural expression of one or more early endocrine markers, for example at least one or more (e.g. 1, 2, 3, 4, 5, 6 or 7) of the following early endocrine markers: Sox9, Hnflb, Hnf6, Hnfla, Nkx2.2, Nkx6.1 and Pdx1.

Alternatively or additionally, in some embodiments, pancreatic organoids show natural expression of one or more early endocrine markers, for example at least one or more (e.g. 1, 2, 3 or 4) of the following endocrine markers: Foxa2, Hnf6, Hnflb and Sox9. In some embodiments, although the pancreatic organoids show natural expression of one or more (e.g. 1, 2, 3 or 4) of the following endocrine markers: Foxa2, Hnf6, Hnflb and Sox9, they do not show expression of Ngn3.

Alternatively or additionally, in some embodiments, pancreatic organoids show natural expression of one or more ductal markers, for example, one or both of keratin 7 and keratin 19. In some embodiments, the pancreatic organoids show natural expression of one or more ductal markers at a significant or detectable level. Thus, in some embodiments, the pancreatic organoids have a ductal phenotype. In some embodiments, pancreatic organoids show expression of one or more (for example, 1, 2, 3, 4, 5, 6, 7, 8, 9, 10, 11, 12, 13, 14, 15) or all of the following markers, selected from the group: Hnf1A, Hnf1B, Hnf4A, HHEX, ONECUT1, ONECUT2, CDH1, FOXA2, GATA6, CEBPB, CEBPD, CEBPG, Glul, Krt7, Krt19 and MET.

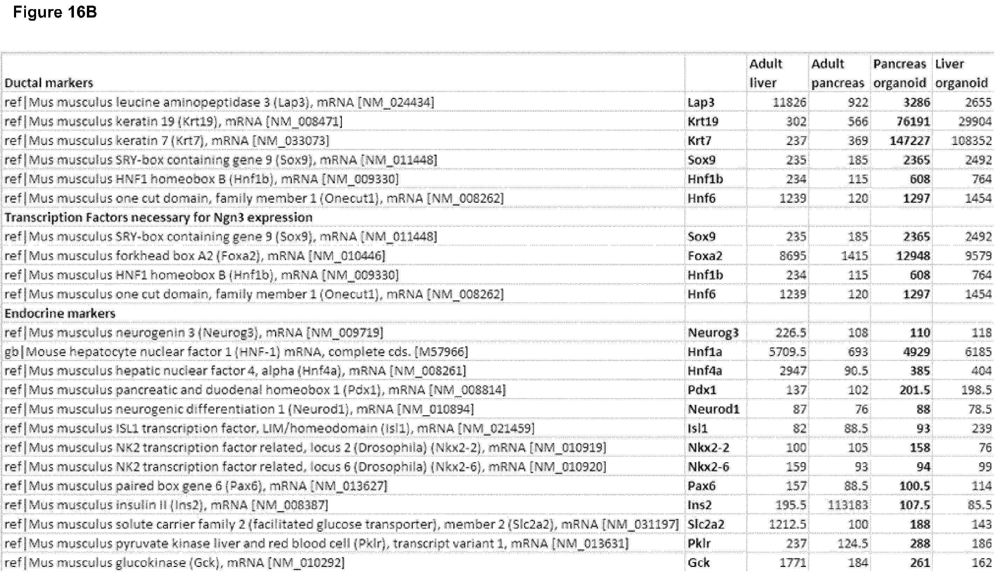

However, the pancreatic organoids may have some ductal features in combination with features of insulin-producing precursor cells. For example, they may express one or more ductal markers as shown in Figure 16B. In some embodiments, a pancreatic organoid exhibits a gene expression profile relative to adult pancreas or liver organoids approximately as shown in Figure 16B. For example, in some embodiments, these genes are upregulated or down regulated in pancreatic organoids compared to adult pancreas liver organoids to approximately the same fold ratio as in figure 16B, for example, less than +/- 3%, less than +/- 5%, less than +/- 10%, less than +/-20%,

In some embodiments, insulin-positive cells appear from the ductal lining in the pancreatic organoids.

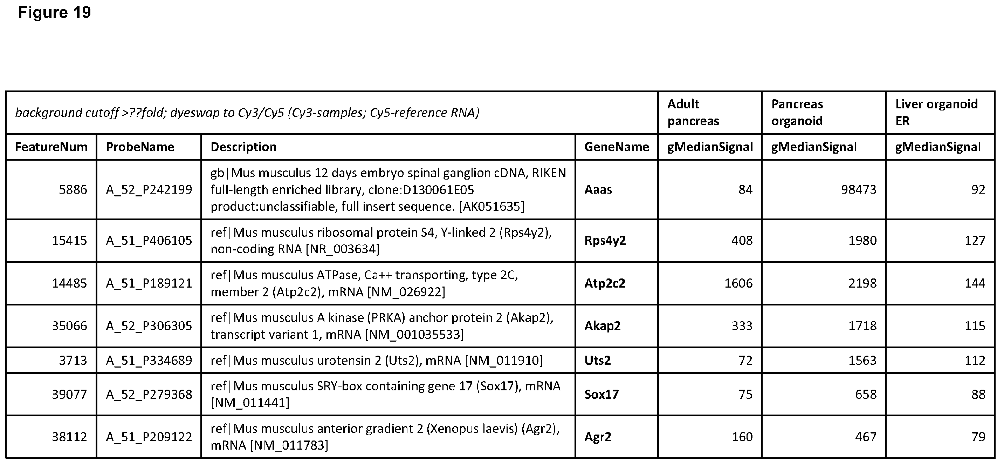

In some embodiments, one or more (e.g. 1, 2, 3, 4, 5, 6 or 7), preferably all of the following genes are upregulated in pancreas organoids compared to liver organoids: Aaas, Rps4y2, Atp2c2, Akap2, Uts2, Sox17, Agr2. For example, in some embodiments, these genes are upregulated in pancreatic organoids compared to liver organoids to approximately the same fold ratio as in figure 19, for example, less than +/- 3%, less than +/- 5%, less than +/- 10%, less than +/- 20%.

In one embodiment, a pancreatic organoid comprises at least 10³, at least 10⁴, at least 10⁵ or more cells in total. In one embodiment, a pancreatic organoid comprises more than 50%, more than 60%, more than 70% or more than 80% ductal-like endocrine progenitor cells However, lower percentages of ductal-like endocrine progenitor cells are also envisaged.

### Barrett's Esophagus (BE) organoids

A BE organoid of the invention is Ki67+.

Preferably a BE organoid has a minimal number (e.g. less than 25%, less than 20%, less than 10%, less than 5%, less than 2%, less than 1% cells) of PAS+ and Mucin+ cells 4 days after withdrawal of Nicotinamide and SB202190 from the expansion medium to covert it to the differentiation medium.

In some embodiments, a BE organoid comprises goblet cells. These may be induced by treatment of the differentiation medium with a gamma-secretase inhibitor such as DBZ (e.g. at 10uM), for example, for 4 days.

In some embodiments, a Barrett's Esophagus organoid of the invention comprises Paneth cells.

In some embodiments, a Barrett's Esophagus organoid of the invention expresses lysozyme.

### Gastric organoids

In some embodiments, the gastric organoids of the invention show natural expression of Lgr5. In some embodiments, gastric organoids of the invention show natural expression of at least Lgr5 and one or more (e.g. 1, 2, 3, 4, 5, 6, 7, 8, 9, 10, 11, 12, 13, 14, 15, 16 or 17) of stem cell markers from the group consisting of: CK19, Nestin, Somatostatin, CXCR4⁺, CD133⁺, DCAMKL-1, CD44, Sord, Sox9, CD44, Prss23, Sp5, Hnf1α, Hnf4a, Sox9, KRT7 and KRT19. Alternatively or additionally, in some embodiments gastric organoids may be characterised by natural expression of one or more (for example 1, 2 or 3) of: CD133⁺, DCAMKL-1 and CD44. Alternatively or additionally, gastric organoids may be characterised by CD44 and Sox9.

### Prostate organoids

In some embodiments, the prostate organoids of the invention, such as mouse prostates, show natural expression of Lgr5. In some embodiments, the prostate organoids show natural expression of luminal prostate markers, such as Cytokeratin 18 (CK18) and Cytokeratin 8 (CK8). In some embodiments, the prostate organoids of the invention show natural expression of Androgen Receptor (AR). In some embodiments, the prostate organoids express basal markers, such as p63 and/or Cytokeratin 5 (CK5). In some embodiments, when testosterone (e.g. DHT) is added to the medium, the expression of basal markers, Lgr5 and Tnfrsf19 are downregulated compared to organoids grown in the absence of testosterone (e.g. DHT). The prostate specific transcription factor NKX3.1 is expressed in all conditions. Therefore, in some embodiments the prostate organoids of the invention show natural expression of the prostate specific marker Nkx3.1.

In some embodiments, the prostate organoids of the invention, for example normal or cancer human prostate organoids, show natural expression of luminal markers, such as CK18, CK8 and/or B-MSP. In some embodiments, the prostate organoids show natural expression of AR. In some embodiments, basal epithelial markers, such as CK14, CK5 and/or p63 are expressed. In some embodiments, TNFRSF19 is expressed. The prostate specific transcription factor NKX3.1 is expressed in all conditions. Therefore, in some embodiments the prostate organoids of the invention show natural expression of the prostate specific marker Nkx3.1.

The addition of testosterone (e.g. DHT) to a culture medium according to the invention allows prostate organoids to grow that maintain a stem cell population allowing up to 3-fold faster growth (than without testosterone) and most (if not all) differentiated cell types of the prostate (both basal and luminal cells) are also present. These conditions allow unlimited cell expansion (so far 9 months at 2.5 population doublings a week). Therefore, in some embodiments, a prostate organoid comprises all differentiated cell types of the prostate, for example both basal and luminal cells. In a preferred embodiment, a prostate organoid comprises all differentiated cell types, for example both basal and luminal cells, and stem cells.

In normal tissue, addition of testosterone (e.g. DHT) increases AR expression in all culture conditions. In some embodiments, prostate organoids have upregulated AR expression compared to prostate cells grown in the absence of testosterone. In tumour tissue AR expression is not influenced by testosterone (e.g. DHT) addition. Therefore, in some embodiments, a prostate cancer organoid does not have increased AR expression relative to *in vivo* prostate cancer cells. The stem cell marker LGR5 is expressed under ENRF conditions in prostate organoids from normal tissue. In prostate organoids obtained from tumour tissue, LGR5 expression is induced with the addition of testosterone (e.g. DHT). In some embodiments, prostate organoids express LGR5.

### Organoid functions

In some embodiments, organoids generated by media and methods of the present invention, mimic *in vivo* cell fate decisions in response to external factors. Preferably, cells and organoids generated according to the invention also possess tissue-specific functions.

### Pancreatic organoids

A pancreatic organoid preferably possesses endocrine and exocrine pancreatic functions, such as expressing one or more (for example 1, 2 or all 3) of insulin, glucagon and somatostatin. The expression of these hormones is tightly regulated by a set of endocrine pancreas-specific transcription factors, the most important being Pdx1 and NeuroD. The exocrine pancreas is formed by acinar and ductal compartments responsible of producing the digestive enzymes amylase, pancreatic lipase and chymotrypsin, among others. The expression of these genes is also regulated by specific exocrine pancreatic genes as Ptfla.

Pancreatic cells and organoids according to the present invention may preferably be capable of secreting insulin, for example, at a rate of between approximately 1 µg per hour per 10⁶ cells and 10µg per hour per 10⁶ cells, for example, between 2µg and 6µg per hour per 10⁶ cells. The level of insulin secretion can be detected by methods well known in the art, for example, by Western Blot compared to a reference or by C-peptide Elisa. The preferred method to demonstrate that pancreatic organoids can secrete insulin is by testing productin of C-peptide. Proinsulin C-peptide serves as an important linker between the A- and the B- chains of insulin and facilitates the efficient assembly, folding, and processing of insulin in the endoplasmic reticulum. Equimolar amounts of C-peptide and insulin are then stored in secretory granules of the pancreatic beta cells and both are eventually released to the portal circulation. Thus, C-peptide is a preferred marker of insulin secretion.

Thus, in one embodiment there is provided a pancreatic organoid that secretes insulin following transplantation *in vivo.* In some embodiments, following transplantation in vivo, the pancreatic organoid secretes insulin at a rate of at least 1 µg per hour per 10⁶ cells, for example, at least 2µg per hour per 10⁶ cells, at least 4µg per hour per 10⁶ cells, at least 6µg per hour per 10⁶ cells, at least 8µg per hour per 10⁶ cells or at least 10µg per hour per 10⁶ cells, In some embodiments, the cells in the pancreatic organoid are not capable of secreting insulin and/or do not express insulin as a marker when cultured in vitro. However, cells from a pancreatic organoid of the present invention are preferably capable of secreting insulin in vivo when transplanted into a patient, for example, into the patient's pancreas. In some embodiments, the ability to secrete insulin may not be present immediately upon transplantation, but is present by about one month after transplantation, for example, by 6 weeks, 2 months or 3 months after transplantation.

If an enriched endocrine cell sample is obtained from a pancreatic organoid of the invention, in some embodiments, 75-85% of the cells in the enriched endocrine cell sample would be insulin-secreting cells.

In some embodiments, the invention provides pancreatic organoids for use in treating diabetes. In some embodiments the pancreatic organoids are expanding organoids, whereas in other embodiments they may be differentiated organoids. In some embodiments one or more (e.g. 1, 2, 3, 4, 5, 6, 7 etc) whole organoids are transplanted into an animal or patient, whereas in other embodiments a sample of cells is transplanted into a patient.

### Crypt-villus organoids

A crypt-villus organoid preferably possesses secretory and self-renewal functions. For example, a crypt-villus organoid preferably secretes mucin, enzymatic and hormonal secretions, such as lysozyme, cholecystokinin, secretin and gastric inhibitory peptide, and other glycoproteins.

### Gastric organoids

The human stomach is anatomically and functionally divided into two major regions. The pyloric antrum close to the intestine mainly produces protective mucus and secretes hormones such as gastrin. The gastric corpus secretes hydrochloric acid and gastric enzymes such as pepsinogen. The gastric epithelium of the both regions is organized in invaginations called glands. These glands harbor the gastric stem cells, progenitor cells and differentiated cells. The precise composition of the differentiated cells varies according to the function of the anatomic region. In the pyloric antrum, glands are mainly composed of mucin 6 producing cells and hormone producing endocrine cells. In the corpus, pepsinogen-producing chief cells and acid-secreting parietal cells are dispersed between the mucus producing cells and sparse endocrine cells. The surface region between gastric glands is occupied by mucus producing cells that mainly produce the surface mucin 5.

Gastric organoids resemble the gastric epithelium in structure and function. Although they are mostly spheric, they can have domains with invaginations that most likely resemble glandular structures. Staining of mucins and pepsinogen shows that the most abundant cell types in the gastric organoids are mucin 6 producing mucus cells and pepsinogen producing chief cells (and/or their progenitors). Accordingly, RT-PCRs indicate the expression of pepsinogen and mucin 6. Further, expression of gastrin indicates the presence of endocrine cells and the expression of Lgr5 indicates the presence of stem cells.

In some embodiments, gastric organoids have natural expression of one or more (e.g. 1, 2, 3 or 4) of gastrin, pepsinogen, mucin 6 and/or Lgr5. In some embodiments, gastric organoids comprise mucin 6 producing mucus cells and pepsinogen producing chief cells and optionally Lgr5+ stem cells. In some embodiments, gastric organoids comprise endocrine cells. In some embodiments, gastric organoids are mostly spheric but have domains with invaginations that resemble glandular structures.

### Prostate organoids

In some embodiments, prostatic organoids comprise or consist of two distinct epithelial lineages, basal cells and luminal cells. In some embodiments basal cells and luminal cells secrete prostatic fluids.

*In vivo* the prostatic epithelium is strongly folded, ensuring maximum surface area. The two epithelial lineages form a simple stratified epithelium with the basal epithelial cells forming the basal/outer layer and the strongly polarized luminal epithelial cells situated on top forming the inner/luminal layer. The luminal compartment is essential for the secretory function of the prostate. The prostatic fluid is alkaline and is composed of several proteins, such as Prostate Specific Antigen (PSA), Human Kallikrein 2 (KLK2) and β-microseminoprotein (β-MSP). The primary functions of prostatice fluid are: 1) preparing the milieu of the uturus for the semen, which is performed by the alkalinity of the fluid and the paracrine functions of β-MSP, and 2) increasing the fluidity of the seminal fluid, allowing the spermatozoa to swim freely, which is performed by the proteases PSA and KLK2 which breakdown seminogelins.

The expression of secretory proteins is tightly regulated by the androgen receptor (AR), which binds to testosterone and subsequently translocates to the nucleus and activates transcription. Disruptions in AR function show a strong downregulated of secretory proteins on a transcriptional and protein level.

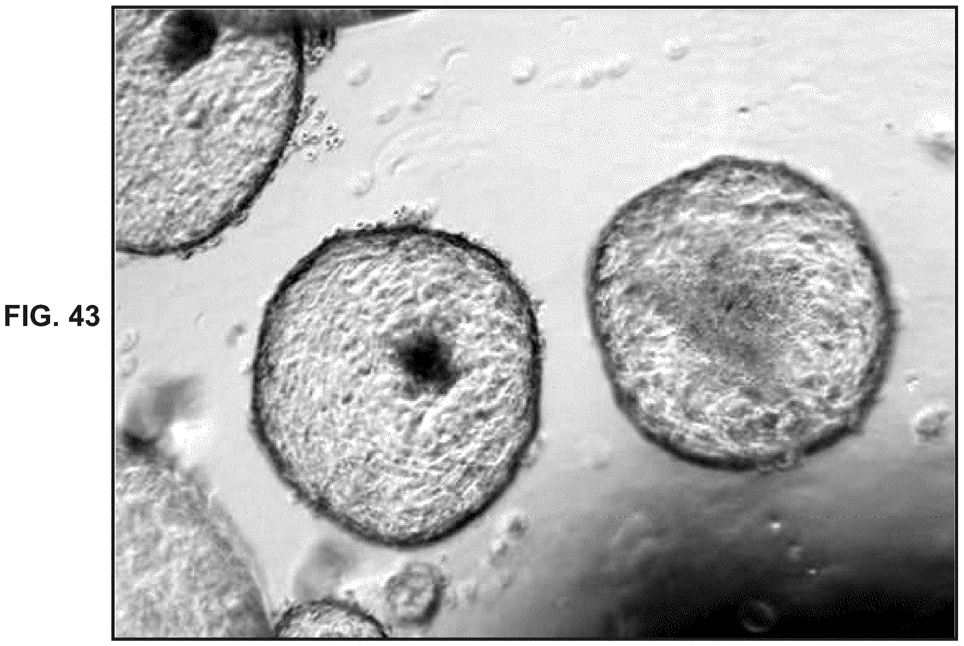

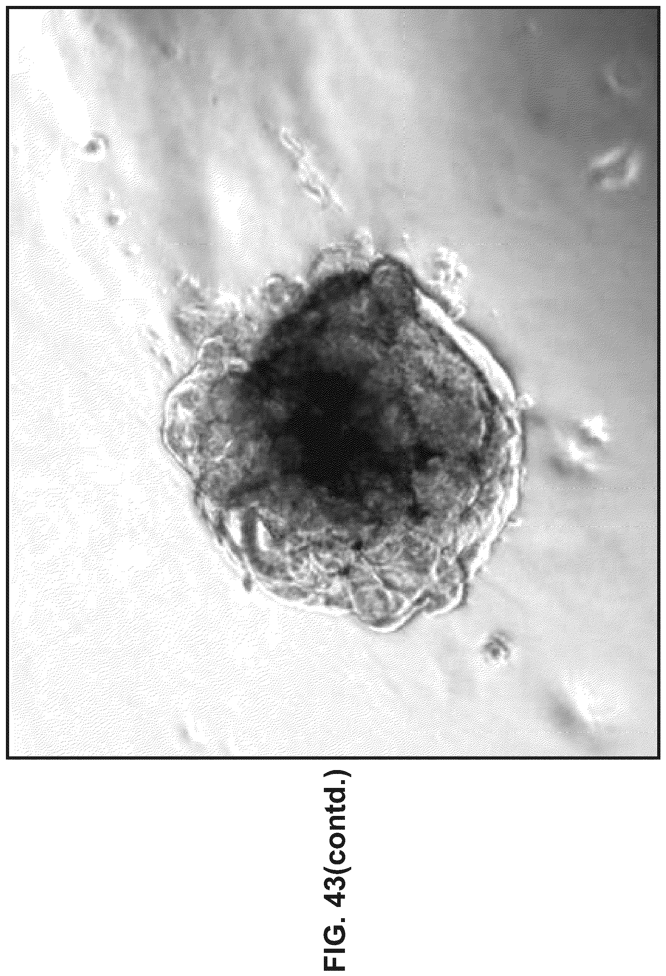

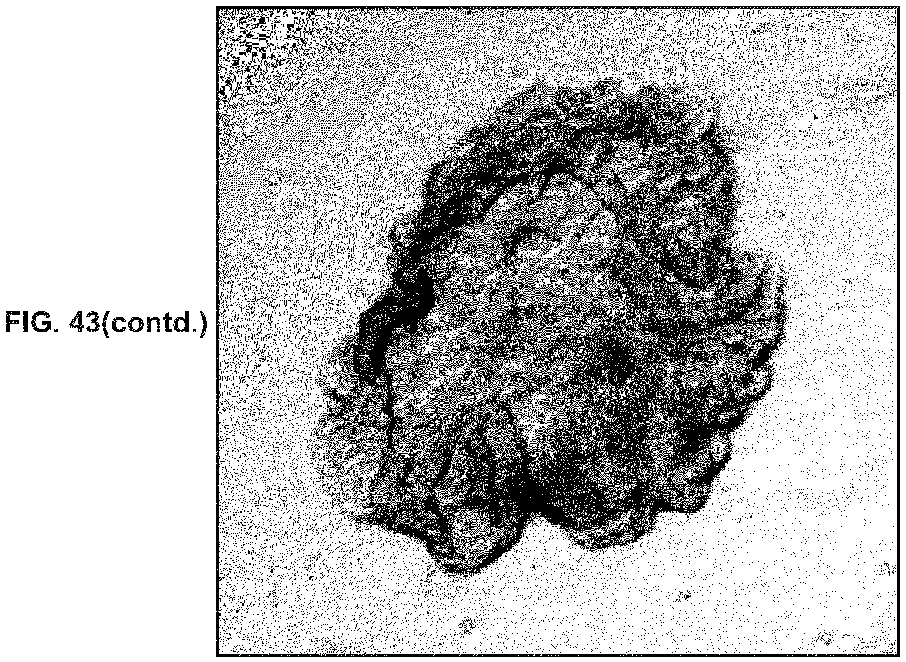

Figure 43 shows the stratification of the prostate organoids grown under ENR+Dihydrotestosterone (DHT) conditions, clearly showing Cytokeratin 5+ basal cells forming an outer layer of cells and the Cytokeratin 8+ luminal cells forming a strongly polarized inner layer. In some embodiments, a prostate organoid comprises cytokeratin 5+ basal cells and cytokeratin 8+ luminal cells, optionally wherein the Cytokeratin 5+ basal cells forming an outer layer of cells and the Cytokeratin 8+ luminal cells forming a strongly polarized inner layer. In some embodiments, a prostate organoid comprises folded layers of cells, optionally comprising strong folding. Such folding maximizes the surface area of secretory cells, showing that on a morphological level prostate organoids resemble the *in vivo* prostate. In some embodiments, the morphology of a prostate organoid resembles the *in vivo* morphology of the prostate.

The prostate organoids cultured in ENR conditions do not any secrete prostatic fluid into the lumen. By contrast, addition of testosterone (e.g. DHT) to the medium results in secretion of fluids, for example prostatic fluid, in the organoid lumen. This is due to the activation of the AR-dependent transcriptional program in prostatic organoids by testosterone (e.g. DHT), which results in secretion of fluids by CK8+ luminals cells. The data show that prostatic organoids both morphologicaly and functionally resemble the *in vivo* prostatic epithelium.

Accordingly, in some embodiments, for example wherein the prostate organoids are cultured in a culture medium comprising testosterone (e.g. DHT), a prostate organoid secretes fluid, for example prostatic fluid into the lumen of the organoid. In some embodiments, for example wherein the prostate organoids are cultured in a culture medium comprising testosterone (e.g. DHT), the functionality of a prostate organoid resembles the *in vivo* functionality of the prostate.

### Tissue fragments

Within the context of the invention, a tissue fragment is a part of an adult tissue, preferably a human adult tissue, such as part of a human adult small intestine, colon or pancreas. Further examples of human adult tissue in the context of this invention include stomach, liver and prostate. The tissue may be normal (healthy) tissue or it may be diseased or infected tissue. Preferably an organoid as identified herein is therefore not a tissue fragment. An organoid is preferably obtained using a cell from an adult tissue, preferably an epithelial stem cell from an adult tissue, optionally from an adult tissue fragment, more preferably an epithelial stem cell from an adult tissue or adult tissue fragment expressing Lgr5. Therefore, within the context of this invention, a tissue fragment preferably comprises Lgr5+ stem cells.

In an embodiment, an organoid is an organoid which is still being cultured using a method of the invention (preferably using a culture medium of the invention) and is therefore in contact with an extracellular matrix. Preferably, an organoid is embedded in a non-mesenchymal extracellular matrix. Within the context of the invention, "in contact" means a physical or mechanical or chemical contact, which means that for separating said organoid from said extracellular matrix a force needs to be used. In some embodiments, the extracellular matrix is a gelatinous protein mixture secreted by Engelbreth-Holm-Swarm (EHS) mouse sarcoma cells, such as Matrigel (BD Biosciences). In other embodiments of the invention, organoids may be removed from culture and used for transplantation or regenerative purposes. Thus the invention provides an organoid of the invention for use in transplantation into a mammal, preferably into a human.

### Survival rate

The inventors show here, for the first time, that addition of an inhibitor of ALK4, ALK5, ALK7 or p38 kinase, to the previously described stem cell culture medium, improved culture plating efficiency by at least 50% and by more than 100% in some cases (see table 2). The inventors have also shown that including both inhibitors (an ALK inhibitor and a p38 inhibitor e.g. A83-01 and SB-202190) in the culture medium synergistically prolongs the culture period.

Accordingly, in one embodiment of the invention, the stem cells survive for at least 3 months, preferably at least 4 months, at least 5 months, at least 6 months, at least 7 months, at least 9 months, or at least 12 months or more.

### Speed of proliferation

The speed of proliferation may be assessed in terms of the cell population doubling level. The population doubling level refers to the total number of times the cells in the population have doubled since their primary isolation *in vitro.* The population doubling level can be determined by cell counting. Alternatively, the speed of proliferation can be assessed by a cellular proliferation assay, for example in which specific fluorescent probes measure DNA synthesis activity by BrdU incorporation and cell proliferation state by Ki67 expression (Thermo Scientific* Cellomics, Millipore).

Further examples of cellular proliferation assays for stem cells are readily available can be found online or in journals such as Current Protocols. One example of many is: http://products.invitrogen.com/ivgn/en/US/adirect/invitrogen?cmd=catDisplayStyle&catKey=10 1&filterDispName=Cellular Proliferation Assays for Stem Cells&filterType=1&OP=filter&filter=ft_1101%2Ff_494303*&_bcs_=H4sIAAAAAAAAAH2 NsQrDMAxEv0ZTsEkdKFmzZC70C4IjakFsGVuOfz%2FK0I6F4x284c48YJxfhffm%0ApQ7gn sMby0ke6x8fRDJMC7hV03u3lE6Swh9M1nNUWUlQq1UFJkXgeIvvotnSbn6Lbl1yPshvQpyq %0ADRIPfQE33RlnKQ21Lvuql7CrAAAA.

The inventors have observed that using the culture media of the invention cells can expand by up to an average of 5 times a week. For example, growing a single cell for two weeks would give approximately 25 cells on average. The skilled person will understand that the average population doubling time of the stem cells of the invention may vary according to several factors, such as passage number, culture conditions, seeding density etc.

In one embodiment, the average population doubling time may be 6 to 48 hours, 12 to 36 hours, 18 to 30 hours, or approximately 24 hours. For example, a stem cell population cultured using a culture medium of the invention may be expected to double approximately 4-7 times, or approximately 5 times per week.

In another embodiment, the average population doubling time is 12 to 96 hours, 24 to 72 hours, or approximately 72 hours. In another embodiment, the cell population doubles on average more than once, more than twice, more than three times, more than four times or more than five times a week.

### Other properties of organoids of the invention

In a preferred embodiment, an organoid could be cultured during at least 2, 3, 4, 5, 6, 7, 8, 9, 10 weeks or 1, 2, 3, 4, 5, 6 months or longer. In a preferred embodiment, an organoid could be cultured during at least 2, 3, 4, 5, 6, 7, 8, 9, 10 weeks or 1, 2, 3, 4, 5, 6, 7, 8, 9, 10, 11, 12 months or longer.In another preferred embodiment, an organoid originates from a single cell, preferably expressing Lgr5, more preferably wherein the single cell comprises a nucleic acid construct comprising a nucleic acid molecule of interest.

The invention further provides an organoid, preferably comprising at least 50% viable cells, more preferred at least 60% viable cells, more preferred at least 70% viable cells, more preferred at least 80% viable cells, more preferred at least 90% viable cells. Viability of cells may be assessed using Hoechst staining or Propidium Iodide staining in FACS.

The viable cells preferably possess tissue-specific functions, or characteristics of tissue-specific functions, as described above.

The inventors have also shown that organoids generated by media and methods of the present invention can be frozen and stored at -80°C or below, such as in liquid nitrogen. Frozen organoids can be thawed and put into culture without losing their 3D structure and integrity and without significant cell death. Therefore, in one embodiment, the invention provides frozen organoids stored at below -5°C, below -10°C, below -20°C, below -40°C, below -60°C, or below -80°C.

The cells and organoids of the present invention differ from any cells and organoids that have been made previously (WO2009/022907 and WO2010/016766) in that they have better phenotypic (better differentiation profile including goblet cell conversion upon addition of gamma secretase inhibitors for the crypt-villus organoids) and karyotypic integrity, as determined by the methods outlined above, better survival rates and faster speeds of cellular proliferation. Accordingly, for intestinal, colon and pancreatic embodiments, an organoid of the present invention clearly represents the human intestinal, colon or pancreas epithelium, with full preservation of phenotypic and karyotypic integrity and maintenance of proliferation and differentiation.

### Uses of stem cells or organoids of the invention

The invention provides the use of an organoid or expanded population of cells of the invention for use in drug screening, (drug) target validation, (drug) target discovery, toxicology and toxicology screens, personalized medicine, regenerative medicine and/or as *ex vivo* cell/organ models, such as disease models.

Cells and organoids cultured according to the media and methods of the invention are thought to faithfully represent the *in vivo* situation. This is true both for expanded populations of cells and organoids grown from normal tissue and for expanded populations of cells and organoids grown from diseased tissue. Therefore, as well as providing normal *ex vivo* cell/organ models, the organoids or expanded population of cells of the invention can be used as *ex vivo* disease models.

Organoids of the invention can also be used for culturing of a pathogen and thus can be used as *ex vivo* infection models. Examples of pathogens that may be cultured using an organoid of the invention include viruses, bacteria, prions or fungi that cause disease in its animal host. Thus an organoid of the invention can be used as a disease model that represents an infected state. In some embodiments of the invention, the organoids can be used in vaccine development and/or production.

Diseases that can be studied by the organoids of the invention thus include genetic diseases, metabolic diseases, pathogenic diseases, inflammatory diseases etc, for example including, but not limited to: cystic fibrosis, inflammatory bowel disease (such as Crohn's disease), carcinoma, adenoma, adenocarcinoma, colon cancer, diabetes (such as type I or type II), Barrett's esophagus, Gaucher's disease, alpha-1-antitrypsin deficiency, Lesch-Nyhan syndrome, anaemia, Schwachman-Bodian-Diamond syndrome, polycythaemia vera, primary myelofibrosis, glycogen storage disease, familial hypercholestrolaemia, Crigler-Najjar syndrome, hereditary tyrosinanaemia, Pompe disease, progressive familial cholestasis, Hreler syndrome, SCID or leaky SCID, Omenn syndrome, Cartilage-hair hypoplasia, Herpes simplex encephalitis, Scleroderma, Osteogenesis imperfecta, Becker muscular dystrophy, Duchenne muscular dystrophy, Dyskeratosis congenitor etc.

Traditionally, cell lines and more recently iPS cells have been used as *ex vivo* cell/organ and/or disease models (for example, see Robinton et al. Nature 481, 295, 2012). However, these methods suffer a number of challenges and disadvantages. For example, cell lines cannot be obtained from all patients (only certain biopsies result in successful cell lines) and therefore, cell lines cannot be used in personalised diagnostics and medicine. iPS cells usually require some level of genetic manipulation to reprogramme the cells into specific cell fates. Alternatively, they are subject to culture conditions that affect karotypic integrity and so the time in culture must be kept to a minimum (this is also the case for human embryonic stem cells). This means that iPS cells cannot accurately represent the *in vivo* situation but instead are an attempt to mimic the behaviour of *in vivo* cells. Cell lines and iPS cells also suffer from genetic instability.

By contrast, the organoids of the invention provide a genetically stable platform which faithfully represents the *in vivo* situation. The organoids of the invention can also be expanded continuously, providing a good source of genetically stable cells. In particular, an expanding population can be "split", meaning that the organoid is split apart and all cells of the organoid are divided into new culture dishes or flasks. The divided cells are removed from the organoid and can then themselves be cultured and expanded to produce new organoids containing further expanded populations that can then be split again. Splits are also referred to herein as "passages". An organoid of the invention may be cultured for 1 or more passages, for example, 1, 2, 3, 4, 5, 6, 7, 8, 9, 10, 15, 20, 25, 30 or more passages, for example, 20-30 passages, 30-35 passages, 32-40 passages or more. In some embodiments, an expanding cell population or organoid is split once a month, once every two weeks, once a week, twice a week, three times a week, four times a week, five times a week, six times a week or daily. Thus the organoids of the invention can provide an ongoing source of genetically stable cellular material. In some embodiments, the expanding organoids of the invention comprise all differentiated cell types that are present in the corresponding *in vivo* situation. In other embodiments, the organoids of the invention may be differentiated to provide all differentiated cell types that are present *in vivo.* Thus the organoids of the invention can be used to gain mechanistic insight into a variety of diseases and therapeutics, to carry out *in vitro* drug screening, to evaluate potential therapeutics, to identify possible targets (e.g. proteins) for future novel (drug) therapy development and/or to explore gene repair coupled with cell-replacement therapy.

The organoids of the invention can be frozen and thawed and put into culture without losing their genetic integrity or phenotypic chrateristics and without loss of proliferative capacity. Thus the organoids can be easily stored and transported.

For these reason the organoids or expanded populations of cells of the invention can be a tool for drug screening, target validation, target discovery, toxicology and toxicology screens and personalized medicine.

Accordingly, in a further aspect, the invention provides the use of the expanded stem cell population or organoid, such as intestinal crypt-villus organoids or pancreatic organoids according to the invention in a drug discovery screen, toxicity assay or in medicine, such as regenerative medicine. For example, any one of the small intestinal, colon, pancreatic, gastric, liver or prostate organoids may be used in a drug discovery screen, toxicity assay or in medicine, such as regenerative medicine.

### Mucosal vaccines

An additional important use of the organoids is in the development of mucosal vaccinations. Mucosal vaccines are vaccines that are administered via the mucosa. This can be any mucosal surface such as via the nose, mouth, or rectum. They can be administered via an inhaler, a spray or other external aids. This has several clear benefits over injections such as that no medical staff are needed for administering the vaccine, which may be important, for example in developing countries.

In the intestine, M cells (or "microfold cells") are cells found in the follicle-associated epithelium of the aggregated lymphoid nodules of the ileum. They transport organisms and particles from the gut lumen to immune cells across the epithelial barrier, and thus are important in stimulating mucosal immunity. They have the unique ability to take up antigen from the lumen of the small intestine via endocytosis or phagocytosis, and then deliver it via transcytosis to dendritic cells (an antigen presenting cell) and lymphocytes (namely T cells) located in a unique pocket-like structure on their basolateral side.

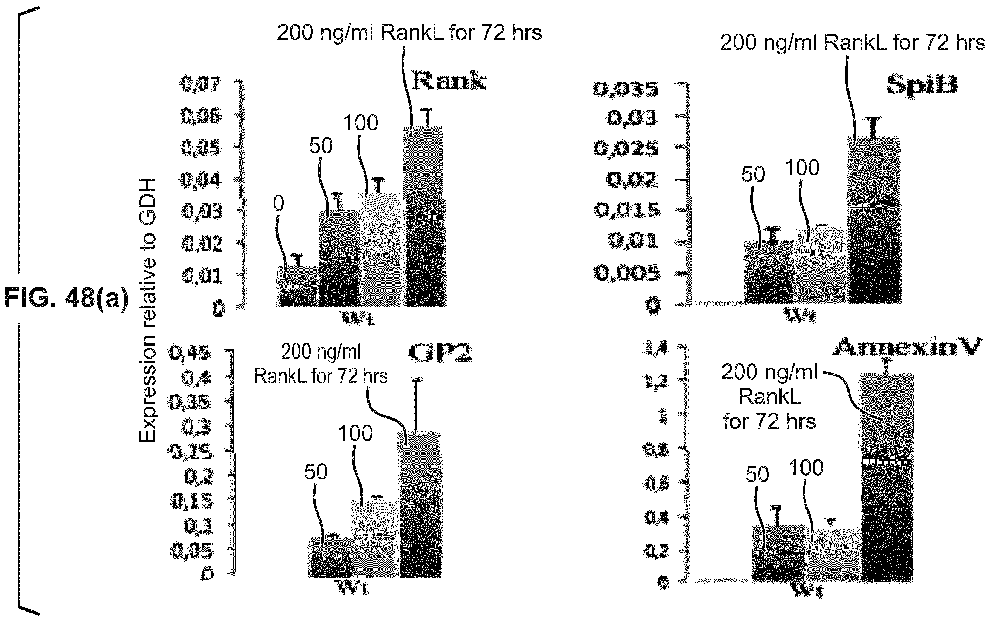

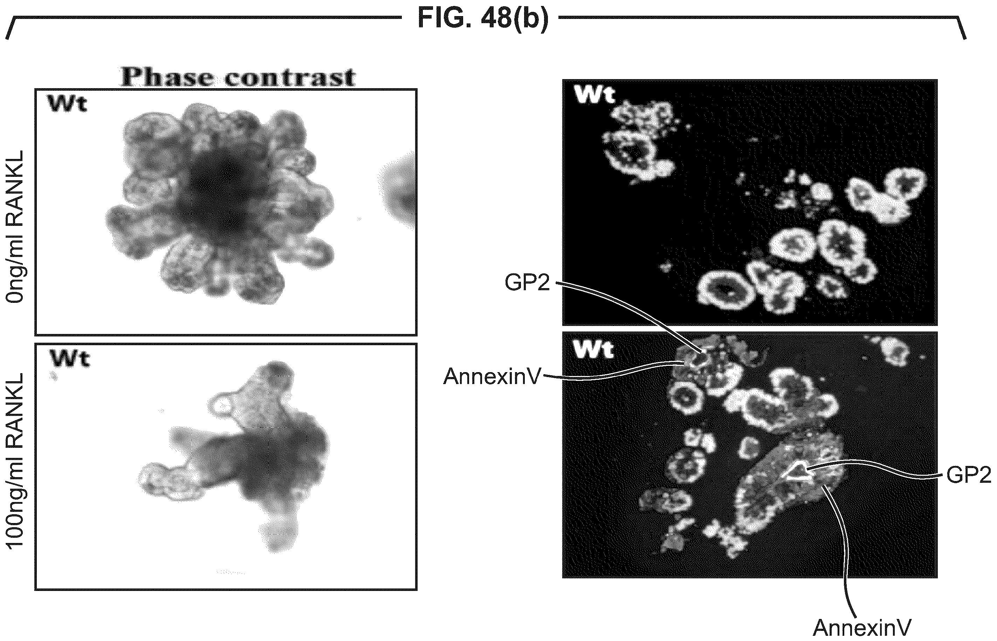

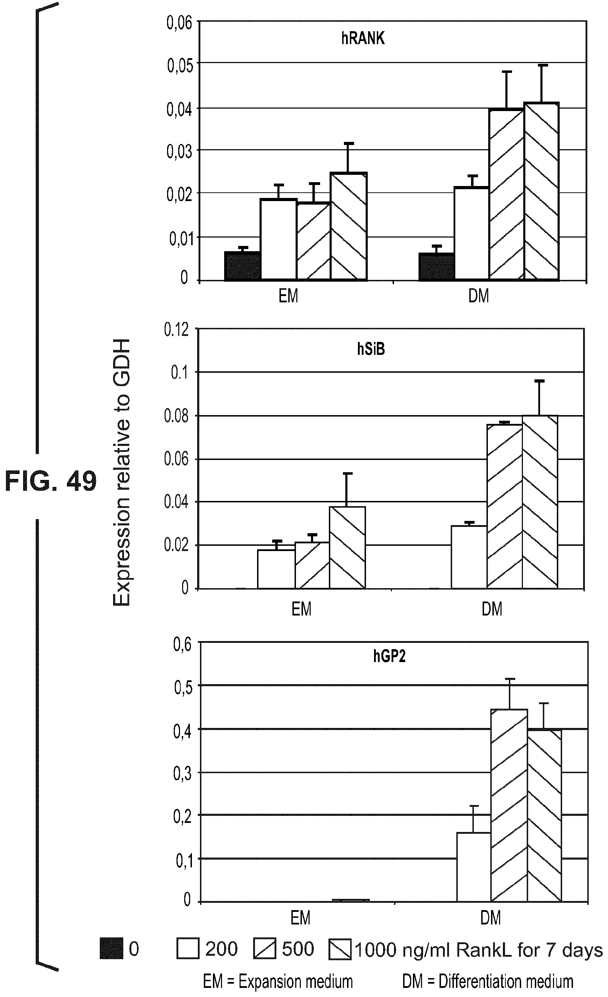

Figure 48 shows that mouse organoids can develop into M cells when stimulated with RANK ligand. Figure 49 shows that it is also possible to generate M cells in human intestinal organoids. Therefore, in some embodiments of the invention, the expanded cell population comprises M cells. In some embodiments of the invention, an organoid, for example a small-intestinal organoid, comprises M cells.

The efficiency of mucosal vaccines can be substantially increased when they are targeted to M cells. Therefore, the expanded stem cell population or organoid of the invention can be used for testing the ability of M cells to take up pathogens or antigens and to present them to the immune system. Therefore, in some embodiments the invention provides the use of the expanded stem cell population or organoid of the invention in drug screening, for example in vaccine development and/or vaccine production. For example, in some embodiments the expanded stem cell population or organoid may be used for the development or production of vaccines against viral, bacterial, fungal or other parasitic infections, for example (but not limited to) cholera, Respiratory syncytial virus (RSV), Rotavirus and HIV. In a particular embodiment, the invention provides small intestinal organoids that have been differentiated in a culture medium of the invention comprising RANKL, for use in mucosal vaccine development.

### Drug screening

For preferably high-throughput purposes, said expanded stem cell population or organoid of the invention, such as crypt-villus organoids or pancreatic organoids, are cultured in multiwell plates such as, for example, 96 well plates or 384 well plates. Libraries of molecules are used to identify a molecule that affects said organoids. Preferred libraries comprise antibody fragment libraries, peptide phage display libraries, peptide libraries (e.g. LOPAP^{™}, Sigma Aldrich), lipid libraries (BioMol), synthetic compound libraries (e.g. LOP AC^{™}, Sigma Aldrich) or natural compound libraries (Specs, TimTec). Furthermore, genetic libraries can be used that induce or repress the expression of one of more genes in the progeny of the stem cells. These genetic libraries comprise cDNA libraries, antisense libraries, and siRNA or other non-coding RNA libraries. The cells are preferably exposed to multiple concentrations of a test agent for a certain period of time. At the end of the exposure period, the cultures are evaluated. The term "affecting" is used to cover any change in a cell, including, but not limited to, a reduction in, or loss of, proliferation, a morphological change, and cell death. Said expanded stem cell population or organoid of the invention such as crypt-villus organoids or pancreatic organoids can also be used to identify drugs that specifically target epithelial carcinoma cells, but not said expanded stem cell population or organoid of the invention, such as crypt-villus organoids or pancreatic organoids.

The inventors have shown that it is possible to take a biopsy from the small intestine and expand it for just 7-14 days and obtain an organoid which is ready for carrying out a drug screen. The ability to obtain a useful organoid of the invention **in** such a short time shows that the organoids would be highly useful for testing individual patient responses to specific drugs and tailoring treatment according to the responsiveness. In some embodiments, wherein the organoid is obtained from a biopsy from a patient, the organoid is cultured for less than 21 days, for example less than 14 days, less than 13 days, less than 12 days, less than 11 days, less than 10 days, less than 9 days, less than 8 days, less than 7 days (etc).

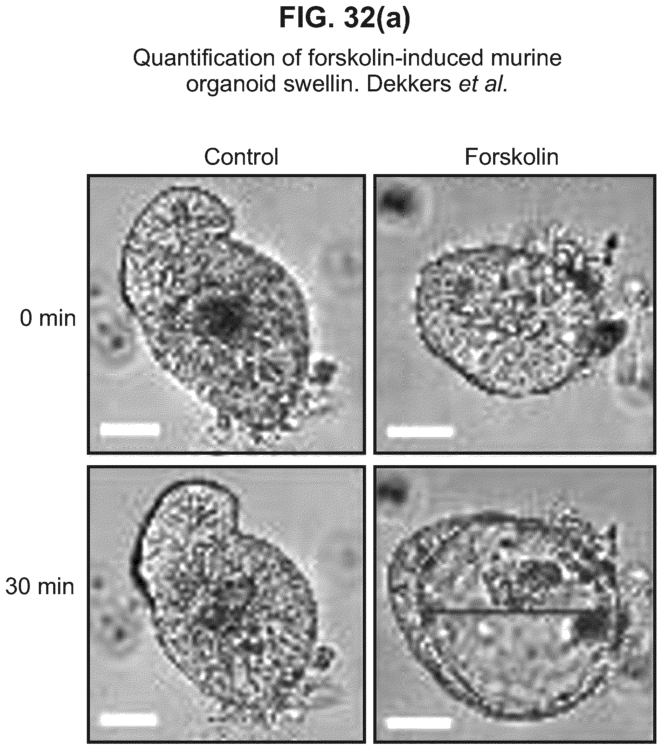

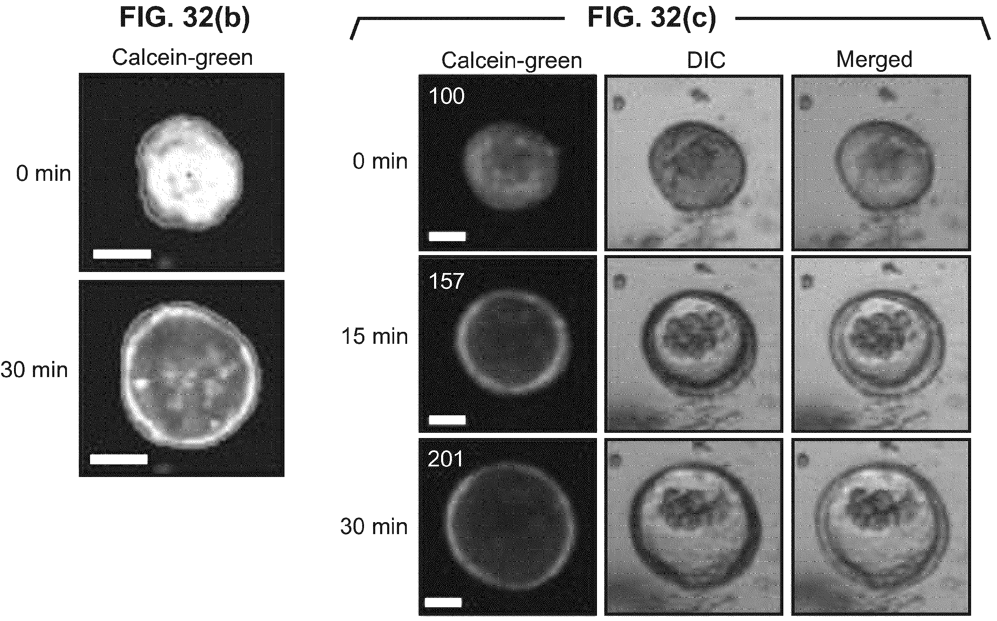

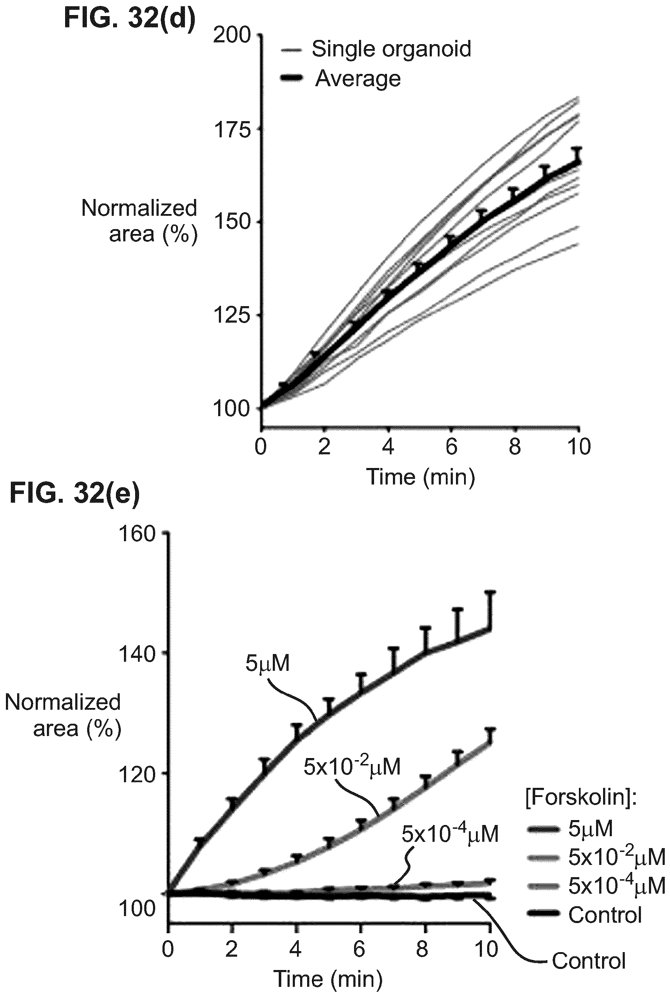

The organoids are also useful for wider drug discovery purposes. For example, Figures 32 to 40 show that small intestinal organoids taken from healthy patients and from cystic fibrosis patients can be used to test drugs against cystic fibrosis. Specifically, Figures 32 to 40 show that forskolin-induced swelling of normal small intestinal organoids is dependent upon the cystic fibrosis transmembrane conductance regulator (CFTR), and thus it is possible to test for correction of CFTR function using forskolin-induced swelling as a positive read out. Therefore, in some embodiments, the organoids of the invention could be used for screening for cystic fibrosis drugs. However, it will be understood by the skilled person that the organoids of the invention would be widely applicable as drug screening tools for infectious, inflammatory and neoplastic pathologies of the human gastrointestinal tract and other diseases of the gastrointestinal tract and infectious, inflammatory and neoplastic pathologies and other diseases of other tissues described herein including pancreas, liver and prostate. **In** some embodiments the organoids of the invention could be used for screening for cancer drugs.

In some embodiments, the expanded cell populations, for example the organoids of the invention or organoids obtained using media and methods of the invention can be used to test libraries of chemicals, antibodies, natural product (plant extracts), etc for suitability for use as drugs, cosmetics and/or preventative medicines. For instance, **in** some embodiments, a cell biopsy from a patient of interest, such as tumour cells from a cancer patient, can be cultured using culture media and methods of the invention and then treated with a a chemical compound or a chemical library. It is then possible to determine which compounds effectively modify, kill and/or treat the patient's cells. This allows specific patient responsiveness to a particular drug to be tested thus allowing treatment to be tailored to a specific patient. Thus, this allows a personalized medicine approach.

The added advantage of using the organoids for identifying drugs in this way is that it is also possible to screen normal organoids (organoids derived from healthy tissue) to check which drugs and compounds have minimal effect on healthy tissue. This allows screening for drugs with minimal off-target activity or unwanted side-effects.

Drugs for any number of diseases can be screened in this way. For example the organoids of the invention can be used for screening for drugs for cystic fibrosis, Barrett's esophagus, carcinomas, adenocarcinomas, adenomas, inflammatory bowel disease (such as Crohn's disease), liver disease etc. The testing parameters depend on the disease of interest. For example, when screening for cancer drugs, cancer cell death is usually the ultimate aim. For cystic fibrosis, measuring the expansion of the organoids in response to the drugs and stimuli of CFTR is of interest. In other embodiments, metabolics or gene expression may be evaluated to study the effects of compounds and drugs of the screen on the cells or organoids of interest.

Therefore, the invention provides a method for screening for a therapeutic or prophylactic drug or cosmetic, wherein the method comprises:
culturing an expanded cell population (for example, an organoid) of the invention, for example with a culture medium of the invention, optionally for less than 21 days;
exposing said expanded cell population (for example, an organoid) of the invention to one or a library of candidate molecules;
evaluating said expanded cell populations (for example, organoids) for any effects, for example any change in the cell, such as a reduction in or loss of proliferation, a morphological change and/or cell death;
identifying the candidate molecule that causes said effects as a potential drug or cosmetic.

In some embodiments, computer- or robot-assisted culturing and data collection methods are employed to increase the throughput of the screen.

In some embodiments, expanded cell population (for example, an organoid) is obtained from a patient biopsy. In some embodiments, the candidate molecule that causes a desired effect on the cultured expanded cell population (for example, an organoid) is administered to said patient.

Accordingly, in one aspect, there is provided a method of treating a patient comprising:
(a) obtaining a biopsy from the diseased tissue of interest in the patient;
(b) screening for a suitable drug using a screening method of the invention; and
(c) treating said patient with the drug obtained in step (b).

In some embodiments, the drug or cosmetic is used for treating, preventing or ameliorating symptoms of genetic diseases, metabolic diseases, pathogenic diseases, inflammatory diseases etc, for example including, but not limited to: cystic fibrosis, inflammatory bowel disease (such as Crohn's disease), carcinoma, adenoma, adenocarcinoma, colon cancer, diabetes (such as type I or type II), Barrett's esophagus, Gaucher's disases, alpha-1-antitrypsin deficiency, Lesch-Nyhan syndrome, anaemia, Schwachman-Bodian-Diamond syndrome, polycythaemia vera, primary myelofibrosis, glycogen storage disease, familial hypercholestrolaemia, Crigler-Najjar syndrome, hereditary tyrosinanaemia, Pompe disease, progressive familial cholestasis, Hreler syndrome, SCID or leaky SCID, Omenn syndrome, Cartilage-hair hypoplasia, Herpes simplex encephalitis, Scleroderma, Osteogenesis imperfecta, Becker muscular dystrophy, Duchenne muscular dystrophy, Dyskeratosis congenitor etc.

### Target discovery

In some embodiments, the organoids of the invention or cells grown using the culture media and methods of the invention can be used for target discovery. Cells of the organoids originating from healthy or diseased tissue may be used for target identification. The organoids of the invention may be used for discovery of drug targets for cystic fibrosis, inflammatory bowel disease (such as Crohn's disease), carcinoma, adenoma, adenocarcinoma, colon cancer, diabetes (such as type I or type II), Barrett's esophagus Gaucher's disease, alpha-1-antitrypsin deficiency, Lesch-Nyhan syndrome, anaemia, Schwachman-Bodian-Diamond syndrome, polycythaemia vera, primary myelofibrosis, glycogen storage disease, familial hypercholestrolaemia, Crigler-Najjar syndrome, hereditary tyrosinanaemia, Pompe disease, progressive familial cholestasis, Hreler syndrome, SCID or leaky SCID, Omenn syndrome, Cartilage-hair hypoplasia, Herpes simplex encephalitis, Scleroderma, Osteogenesis imperfecta, Becker muscular dystrophy, Duchenne muscular dystrophy, Dyskeratosis congenitor etc. Cells and organoids cultured according to the media and methods of the invention are thought to faithfully represent the *in vivo* situation. For this reason they can be a tool to find novel (molecular) targets in specific diseases.

To search for a new drug target, a library of compounds (such as siRNA) may be used to transduce the cells and inactivate specific genes. In some embodiments, cells are transduced with siRNA to inhibit the function of a (large) group of genes. Any functional read out of the group of genes or specific cellular function can be used to determine if a target is relevant for the study. A disease-specific read out can be determined using assays well known in the art. For example, cellular proliferation is assayed to test for genes involved in cancer. For example, a Topflash assay as described herein, may be used to detect changes in Wnt activity caused by siRNA inhibition. Where growth reduction or cell death occurs, the corresponding siRNA related genes can be identified by methods known in the art. These genes are possible targets for inhibiting growth of these cells. Upon identification, the specificity of the identified target for the cellular process that was studied will need to be determined by methods well known in the art. Using these methods, new molecules can be identified as possible drug targets for therapy.

### Target and drug validation screens

Patient-specific organoids obtained from diseased and/or normal tissue can be used for target validation of molecules identified in high throughput screens. The same goes for the validation of compounds that were identified as possible therapeutic drugs in high throughput screens. The use of primary patient material expanded in the organoid culture system can be useful to test for false positives, etc from high throughput drug discovery cell line studies.

In some embodiments, the expanded stem cell population (for example, organoid of the invention), such as crypt-villus organoids or pancreatic organoids can be used for validation of compounds that have been idenfitied as possible drugs or cosmetics in a high-throughput screen.

### Toxicity assay

Said expanded stem cell population (for example, organoid of the invention), such as crypt-villus organoids or pancreatic organoids, can further replace the use of cell lines such as Caco-2 cells in toxicity assays of potential novel drugs or of known or novel food supplements.

Toxicology screens work in a similar way to drug screens (as described above) but they test for the toxic effects of drugs and not therapeutic effects. Therefore, in some embodiments, the effects of the candidate compounds are toxic.

### Culturing pathogens

Furthermore, said expanded stem cell population (for example, organoid of the invention), such as crypt-villus organoids or pancreatic organoids, can be used for culturing of a pathogen such as a norovirus which presently lacks a suitable tissue culture or animal model.

### Regenerative medicine and transplantation

Cultures comprising the expanded stem cell population (for example, organoid of the invention), such as crypt-villus organoids or pancreatic organoids are useful in regenerative medicine, for example in post-radiation and/or post-surgery repair of the intestinal epithelium, in the repair of the intestinal epithelium in patients suffering from inflammatory bowel disease such as Crohn's disease and ulcerative colitis, and in the repair of the intestinal epithelium in patients suffering from short bowel syndrome. Further use is present in the repair of the intestinal epithelium in patients with hereditary diseases of the small intestine/colon. Cultures comprising pancreatic organoids are also useful in regenerative medicine, for example as implants after resection of the pancreas or part thereof and for treatment of diabetes such as diabetes I and diabetes II.

In an alternative embodiment, the expanded epithelial stem cells are reprogrammed into related tissue fates such as, for example, pancreatic cells including pancreatic beta-cells. Thus far, it has not been possible to regenerate pancreatic cells from adult stem cells. The culturing methods of the present invention will enable to analyse for factors that trans-differentiate the closely related epithelial stem cell to a pancreatic cell, including a pancreatic beta-cell.

It will be clear to a skilled person that gene therapy can additionally be used in a method directed at repairing damaged or diseased tissue. Use can, for example, be made of an adenoviral or retroviral gene delivery vehicle to deliver genetic information, like DNA and/or RNA to stem cells. A skilled person can replace or repair particular genes targeted in gene therapy. For example, a normal gene may be inserted into a nonspecific location within the genome to replace a nonfunctional gene. In another example, an abnormal gene sequence can be replaced for a normal gene sequence through homologous recombination. Alternatively, selective reverse mutation can return a gene to its normal function. A further example is altering the regulation (the degree to which a gene is turned on or off) of a particular gene. Preferably, the stem cells are *ex vivo* treated by a gene therapy approach and are subsequently transferred to the mammal, preferably a human being in need of treatment.

Since small biopsies taken from adult donors can be expanded without any apparent limit or genetic harm, the technology may serve to generate transplantable epithelium for regenerative purposes. The fact that organoids can be frozen and thawed and put into culture without losing their 3D structure and integrity and without significant cell death further adds to the applicability of organoids for transplantation purposes. Furthermore, in some embodiments, organoids embedded in, or in contact with, an ECM can be transplanted into a mammal, preferably into a human. In another embodiment, organoids and ECM can be transplanted simultaneously into a mammal, preferably into a human.

The skilled person would understand that an ECM can be used as a 3D scaffold for obtaining tissue-like structures comprising expanded populations of cells or organoids according to the invention. Such structures can then be transplanted into a patient by methods well known in the art. An ECM scaffold can be made synthetically using ECM proteins, such as collagen and/or laminin, or alternatively an ECM scaffold can be obtained by "decellularising" an isolated organ or tissue fragment to leave behind a scaffold consisting of the ECM (for example see Macchiarini et al. The Lancet, Volume 372, Issue 9655, Pages 2023 - 2030, 2008). In some embodiments, an ECM scaffold can be obtained by decellularising an organ or tissue fragment, wherein optionally said organ or tissue fragment is from the pancreas, liver, intestine, stomach or prostate.

As mentioned above, the invention provides an organoid or population of cells of the invention for use in transplantation into a mammal, preferably into a human. Also provided is a method of treating a patient in need of a transplant comprising transplanting an organoid or population of cells of the invention into said patient, wherein said patient is a mammal, preferably a human.

Advantageously, the invention enables a small biopsy to be taken from an adult donor and expanded without any apparent limit or genetic harm and so the technology provided herein may serve to generate transplantable epithelium for regenerative purposes.

Significantly, the inventors have found that when human pancreatic organoids of the invention are transplanted under the peri-renal capsule in mice, these cells differentiate to form mature beta cells that secrete insulin. This is significant as it means that even if the population of cells or organoid of the invention does not secrete insulin at a detectable level whilst the cells or organoids are being cultured in vitro, these cells may be useful for transplantation into a patient for the treatment of an insulin-deficiency disorder such as diabetes.

Thus the invention comprises a method of treating an insulin-deficiency disorder such as diabetes, or a patient having a dysfunctional pancreas, comprising transplanting a pancreatic organoid of the invention or cells from a pancreatic organoid of the invention into the patient.

In some embodiments, the cells or organoid do not express or secrete insulin upon transplantation into the patient but differentiate within the patient such that they secrete insulin. For example, the ability to secrete insulin may not be detectable immediately upon transplantation, but may be present by about one month after transplantation, for example, by 6 weeks, 2 months or 3 months after transplantation.

The patient is preferably a human, but may alternatively be a non-human mammal, such as a cat, dog, horse, cow, pig, sheep, rabbit or mouse.

Thus, included within the scope of the invention are methods of treatment of a human or non-human animal patient through cellular therapy. Such cellular therapy encompasses the application of the stem cells or organoids of the invention to the patient through any appropriate means. Specifically, such methods of treatment involve the regeneration of damaged tissue. In accordance with the invention, a patient can be treated with allogeneic or autologous stem cells or organoids. "Autologous" cells are cells which originated from the same organism into which they are being re-introduced for cellular therapy, for example **in** order to permit tissue regeneration. However, the cells have not necessarily been isolated from the same tissue as the tissue they are being introduced into. An autologous cell does not require matching to the patient in order to overcome the problems of rejection. "Allogeneic" cells are cells which originated from an individual which is different from the individual into which the cells are being introduced for cellular therapy, for example in order to permit tissue regeneration, although of the same species. Some degree of patient matching may still be required to prevent the problems of rejection.

Generally the cells or organoids of the invention are introduced into the body of the patient by injection or implantation. Generally the cells will be directly injected into the tissue in which they are intended to act. Alternatively, the cells will be injected through the portal vein. A syringe containing cells of the invention and a pharmaceutically acceptable carrier is included within the scope of the invention. A catheter attached to a syringe containing cells of the invention and a pharmaceutically acceptable carrier is included within the scope of the invention.

The skilled person will be able to select an appropriate method and route of administration depending on the material that is being transplanted (i.e. population of cells, single cells in cell suspension, organoids or fragments of organoids) as well as the organ that is being treated.

As discussed above, cells of the invention can be used in the regeneration of tissue. In order to achieve this function, cells may be injected or implanted directly into the damaged tissue, where they may multiply and eventually differentiate into the required cell type, **in** accordance with their location **in** the body. Alternatively, the organoid can be injected or implanted directly into the damaged tissue. Tissues that are susceptible to treatment include all damaged tissues, particularly including those which may have been damaged by disease, injury, trauma, an autoimmune reaction, or by a viral or bacterial infection. In some embodiments of the invention, the cells or organoids of the invention are used to regenerate the colon, small intestine, pancreas, esophagus or gastric system.

For example, **in** one embodiment, the cells or organoids of the invention are injected into a patient using a Hamilton syringe.

The skilled person will be aware what the appropriate dosage of cells or organoids of the invention will be for a particular condition to be treated.

In one embodiment the cells or organoids of the invention, either in solution, in microspheres or in microparticles of a variety of compositions, will be administered into the artery irrigating the tissue or the part of the damaged organ in need of regeneration. Generally such administration will be performed using a catheter. The catheter may be one of the large variety of balloon catheters used for angioplasty and/or cell delivery or a catheter designed for the specific purpose of delivering the cells to a particular local of the body. For certain uses, the cells or organoids may be encapsulated into microspheres made of a number of different biodegradable compounds, and with a diameter of about 15 µm. This method may allow intravascularly administered cells or organoids to remain at the site of damage, and not to go through the capillary network and into the systemic circulation in the first passage. The retention at the arterial side of the capillary network may also facilitate their translocation into the extravascular space.

In another embodiment, the cells or organoids may be retrograde injected into the vascular tree, either through a vein to deliver them to the whole body or locally into the particular vein that drains into the tissue or body part to which the cells or organoids are directed. For this embodiment many of the preparations described above may be used.

In another embodiment, the cells or organoids of the invention may be implanted into the damaged tissue adhered to a biocompatible implant. Within this embodiment, the cells may be adhered to the biocompatible implant *in vitro,* prior to implantation into the patient. As will be clear to a person skilled in the art, any one of a number of adherents may be used to adhere the cells to the implant, prior to implantation. By way of example only, such adherents may include fibrin, one or more members of the integrin family, one or more members of the cadherin family, one or more members of the selectin family, one or more cell adhesion molecules (CAMs), one or more of the immunoglobulin family and one or more artificial adherents. This list is provided by way of illustration only, and is not intended to be limiting. It will be clear to a person skilled in the art, that any combination of one or more adherents may be used.

In another embodiment, the cells or organoids of the invention may be embedded in a matrix, prior to implantation of the matrix into the patient. Generally, the matrix will be implanted into the damaged tissue of the patient. Examples of matrices include collagen based matrices, fibrin based matrices, laminin based matrices, fibronectin based matrices and artificial matrices. This list is provided by way of illustration only, and is not intended to be limiting.

In a further embodiment, the cells or organoids of the invention may be implanted or injected into the patient together with a matrix forming component. This may allow the cells to form a matrix following injection or implantation, ensuring that the cells or organoids remain at the appropriate location within the patient. Examples of matrix forming components include fibrin glue liquid alkyl, cyanoacrylate monomers, plasticizers, polysaccharides such as dextran, ethylene oxide-containing oligomers, block co-polymers such as poloxamer and Pluronics, nonionic surfactants such as Tween and Triton'8', and artificial matrix forming components. This list is provided by way of illustration only, and is not intended to be limiting. It will be clear to a person skilled in the art, that any combination of one or more matrix forming components may be used.

In a further embodiment, the cells or organoids of the invention may be contained within a microsphere. Within this embodiment, the cells may be encapsulated within the centre of the microsphere. Also within this embodiment, the cells may be embedded into the matrix material of the microsphere. The matrix material may include any suitable biodegradable polymer, including but not limited to alginates, Poly ethylene glycol (PLGA), and polyurethanes. This list is provided by way of example only, and is not intended to be limiting.

In a further embodiment, the cells or organoids of the invention may be adhered to a medical device intended for implantation. Examples of such medical devices include stents, pins, stitches, splits, pacemakers, prosthetic joints, artificial skin, and rods. This list is provided by way of illustration only, and is not intended to be limiting. It will be clear to a person skilled in the art, that the cells may be adhered to the medical device by a variety of methods. For example, the cells or organoids may be adhered to the medical device using fibrin, one or more members of the integrin family, one or more members of the cadherin family, one or more members of the selectin family, one or more cell adhesion molecules (CAMs), one or more of the immunoglobulin family and one or more artificial adherents. This list is provided by way of illustration only, and is not intended to be limiting. It will be clear to a person skilled in the art, that any combination of one or more adherents may be used.

### Methods of the invention

The invention also provides a method for expanding a population of stem cells, wherein the method comprises:
a) providing a population of stem cells;
b) providing a culture medium according to the invention;
c) contacting the stem cells with the culture medium; and
d) culturing the cells under appropriate conditions.

Furthermore, the invention provides a method for expanding isolated tissue fragments, wherein the method comprises:
a) providing an isolated tissue fragment;
b) providing a culture medium according to the invention;
c) contacting the isolated tissue fragment with the culture medium; and
d) culturing the cells under appropriate conditions.

A method for 'expanding' a population of cells or isolated tissue fragments is one that involves maintaining or increasing the number of stem cells in an initial population to generate an expanded population of stem cells which retain their undifferentiated phenotype and self-renewing properties. However, it may also include the production of differentiating progeny, which may, for example, form tissue-like structures contributing to organoid formation. Hence, there are herein provided methods for obtaining an organoid, such as a small intestinal (crypt-villus) organoid, a colon organoid, a pancreatic organoid, a gastric organoid, a prostatic organoid, a liver organoid, an adenocarcinoma organoid, a carcinoma organoid or a Barrett's Esophagus organoid, comprising culturing stem cells or tissue fragments comprising said stem cells in a culture medium of the invention. The invention provides a method for expanding a single stem cell or a population of stem cells, preferably to generate an organoid, wherein the method comprises culturing the single stem cell, population of stem cells or tissue fragment in a culture medium according to the invention. In some embodiments, the method for obtaining an organoid comprises culturing the stem cells or tissue fragments with a first "expansion" medium, followed by culturing the stem cells or tissue fragments with a second "differentiation" medium. In some embodiments, the differentiation medium does not comprise certain components of the expansion medium, for example, the differentiation medium does not comprise Wnt, Rspondin, nicotinamide, a TGF-beta inhibitor and/or a p38 inhibitor.

In some embodiments, the method for expanding a single stem cell or a population of stem cells, preferably to generate an organoid, comprises expanding the single stem cell, population of stem cells or tissue fragment in a first culture medium according to the invention, and optionally, differentiating the expanded cells or tissue fragments in a second culture medium according to the invention.

Thus the invention provides a method for expanding a single stem cell or a population of stem cells, preferably to generate an organoid, wherein the method comprises:
providing a stem cell, a population of stem cells or an isolated tissue fragment;
providing a culture medium according to the invention;
contacting the stem cells with the culture medium;
culturing the cells under appropriate conditions.

In some embodiments, the method comprises bringing the stem cell, the population of stem cells or the isolated tissue fragment and the culture medium into contact with an extracellular matrix or a 3D matrix that mimics the extracellular matrix by its interaction with the cellular membrane proteins such as integrins, for example a laminin-containing extracellular matrix such as Matrigel^{™} (BD Biosciences). In some embodiments, the culture medium is diffused into the extracellular matrix.

In some embodiments, the invention provides a method for expanding a single stem cell or a population of stem cells or tissue fragment, preferably to generate an organoid, wherein the method comprises:
culturing the stem cell, population of stem cells or tissue fragment in a first expansion medium;
continuing to culture the stem cell, population of stem cells or tissue fragment and replenishing the medium with a differentiation medium, wherein the differentiation medium does not comprise one or more of, preferably all of the factors selected from: a TGF-beta inhibitor, a p38 inhibitor, nicotinamide and Wnt.

In some embodiments, the invention provides a method for expanding a single stem cell or a population of stem cells, preferably to generate an organoid of a tissue of interest, comprising:
expanding stem cells or tissue fragments from said tissue of interest in a culture medium of the invention that is suitable for said tissue of interest; and optionally differentiating the expanded stem cells or tissue fragments in a culture medium of the invention that is suitable for said tissue of interest.

Isolated stem cells are preferably cultured in a microenvironment that mimics at least in part a cellular niche in which said stem cells naturally reside. Said cellular niche is mimicked by culturing said stem cells in the presence of biomaterials, such as matrices, scaffolds, and culture substrates that represent key regulatory signals controlling stem cell fate. Said biomaterials comprise natural, semi-synthetic and synthetic biomaterials, and/or mixtures thereof. A scaffold provides a two-dimensional or three dimensional network. Suitable synthetic materials for said scaffold comprise polymers selected from porous solids, nanofibers, and hydrogels such as, for example, peptides including self-assembling peptides, hydrogels composed of polyethylene glycol phosphate, polyethylene glycol fumarate, polyacrylamide, polyhydroxyethyl methacrylate, polycellulose acetate, and/or co-polymers thereof (see, for example, Saha et al, 2007 Curr Opin Chem Biol 1 1(4) 381-387, Saha et al, 2008 Biophysical Journal 95 4426-4438, Little et al , 2008 Chem Rev 108, 1787-1796). As is known to a skilled person, the mechanical properties such as, for example, the elasticity of the scaffold influences proliferation, differentiation and migration of stem cells. A preferred scaffold comprises biodegradable (co)polymers that are replaced by natural occurring components after transplantation in a subject, for example to promote tissue regeneration and/or wound healing. It is furthermore preferred that said scaffold does not substantially induce an immunogenic response after transplantation in a subject. Said scaffold is supplemented with natural, semi-synthetic or synthetic ligands, which provide the signals that are required for proliferation and/or differentiation, and/or migration of stem cells. In a preferred embodiment, said ligands comprise defined amino acid fragments. Examples of said synthetic polymers comprise Pluronic^{®} F 127 block copolymer surfactant (BASF), and Ethisorb^{®} (Johnson and Johnson).

A cellular niche is in part determined by the stem cells and surrounding cells, and the extracellular matrix (ECM) that is produced by the cells in said niche. In one method of the invention, isolated crypts or epithelial stem cells are attached to an ECM. ECM is composed of a variety of polysaccharides (mostly heparin sulphate proteoglycans), water, elastin, and glycoproteins, wherein the glycoproteins comprise collagen, entactin (nidogen), fibronectin, and laminin. ECM is secreted by connective tissue cells. Different types of ECM are known, comprising different compositions including different types of glycoproteins and/or different combination of glycoproteins. Said ECM can be provided by culturing ECM-producing cells, such as for example fibroblast cells, in a receptacle, prior to the removal of these cells and the addition of isolated crypts or epithelial stem cells. Examples of extracellular matrix-producing cells are chondrocytes, producing mainly collagen and proteoglycans, fibroblast cells, producing mainly type IV collagen, laminin, interstitial procollagens, and fibronectin, and colonic myofibroblasts producing mainly collagens (type I, III, and V), chondroitin sulfate proteoglycan, hyaluronic acid, fibronectin, and tenascin-C. Alternatively, said ECM is commercially provided. Commercially provided ECMs are typically synthetic ECMs. Examples of commercially available extracellular matrices are extracellular matrix proteins (Invitrogen) and Matrigel^{™} (BD Biosciences). The use of an ECM for culturing stem cells enhanced long-term survival of the stem cells and the continued presence of undifferentiated stem cells.

An example of an ECM for use in a method of the invention comprises at least two distinct glycoproteins, such as two different types of collagen or a collagen and laminin. Said ECM can be a synthetic hydrogel extracellular matrix or a naturally occurring ECM. A most preferred ECM is provided by Matrigel^{™} (BD Biosciences), which comprises laminin, entactin, and collagen IV. Therefore, in some embodiments, the ECM for use in a method of the invention is a 3D matrix that mimics the extracellular matrix by its interaction with the cellular membrane proteins such as integrins.

Thus in some embodiments, a method of the invention comprises bringing the stem cell, the population of stem cells or the isolated tissue fragment and the culture medium into contact with an extracellular matrix, for example a laminin-containing extracellular matrix such as MatrigelTM (BD Biosciences). In some embodiments, the culture medium is diffused into the extracellular matrix.

### Compositions and other forms of the invention

The invention provides a composition comprising a culture medium according to the invention and stem cells. The invention also provides a composition comprising a culture medium according to the invention and organoids. Furthermore, the invention provides a composition comprising a culture medium according to the invention and an extracellular matrix.

The invention also provides a composition comprising a culture medium of the invention, an extracellular matrix and stem cells of the invention. The invention also provides a composition comprising a culture medium of the invention, an extracellular matrix and one or more organoids of the invention. The invention also provides a culture medium supplement that can be used to produce a culture medium as disclosed herein. A 'culture medium supplement' is a mixture of ingredients that cannot itself support stem cells, but which enables or improves stem cell culture when combined with other cell culture ingredients. The supplement can therefore be used to produce a functional cell culture medium of the invention by combining it with other cell culture ingredients to produce an appropriate medium formulation. The use of culture medium supplements is well known in the art.

The invention provides a culture medium supplement that comprises an inhibitor according to the invention. The supplement may contain any inhibitor (or combination of inhibitors) disclosed herein. The supplement may also contain one or more additional cell culture ingredients as disclosed herein, *e.g*. one or more cell culture ingredients selected from the group consisting of amino acids, vitamins, inorganic salts, carbon energy sources and buffers.

A culture medium or culture medium supplement may be a concentrated liquid culture medium or supplement (*e.g*. a 2x to 250x concentrated liquid culture medium or supplement) or may be a dry culture medium or supplement. Both liquid and dry culture media or supplements are well known in the art. A culture medium or supplement may be lyophilised.

A culture medium or supplement of the invention will typically be sterilized prior to use to prevent contamination, *e.g*. by ultraviolet light, heating, irradiation or filtration. A culture medium or culture medium supplement may be frozen (*e.g*. at -20°C or -80°C) for storage or transport. In some embodiments, the culture medium may be stored as a liquid (e.g. at approximately 4°C). In some embodiments, the culture medium may be split and stored as two components: a frozen component (e.g. at between approximately -20°C and approximately - 80°C) and a liquid component (e.g. at approximately 4°C). In particular, temperature-sensitive or time-sensitive degradable material is preferably included in the frozen component, whereas less sensitive material (for example DMEM or FCS) can be storred in the liquid form and thus included in the liquid component for storage and shipping.

The invention also provides a hermetically-sealed vessel containing a culture medium or culture medium supplement of the invention. Hermetically-sealed vessels may be preferred for transport or storage of the culture media or culture media supplements disclosed herein, to prevent contamination. The vessel may be any suitable vessel, such as a flask, a plate, a bottle, a jar, a vial or a bag.

The invention also provides a kit comprising a culture medium, culture medium supplement and/or a composition of the invention. In some embodiments, the kit further comprises at least one other additional component, for example selected from the list comprising: an ECM (for example, Matrigel^{™}), a population of cells and an organoid.

### General

"GI" numbering is used above. A GI number, or "GenInfo Identifier", is a series of digits assigned consecutively to each sequence record processed by NCBI when sequences are added to its databases. The GI number bears no resemblance to the accession number of the sequence record. When a sequence is updated (*e.g.* for correction, or to add more annotation or information) then it receives a new GI number. Thus the sequence associated with a given GI number is never changed.

The term "comprising" encompasses "including" as well as "consisting" e.g. a composition "comprising" X may consist exclusively of X or may include something additional e.g. X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value *x* is optional and means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Various aspects and embodiments of the invention are described below in more detail by way of example. It will be appreciated that modification of detail may be made without departing from the scope of the invention.

### DESCRIPTION OF THE DRAWINGS

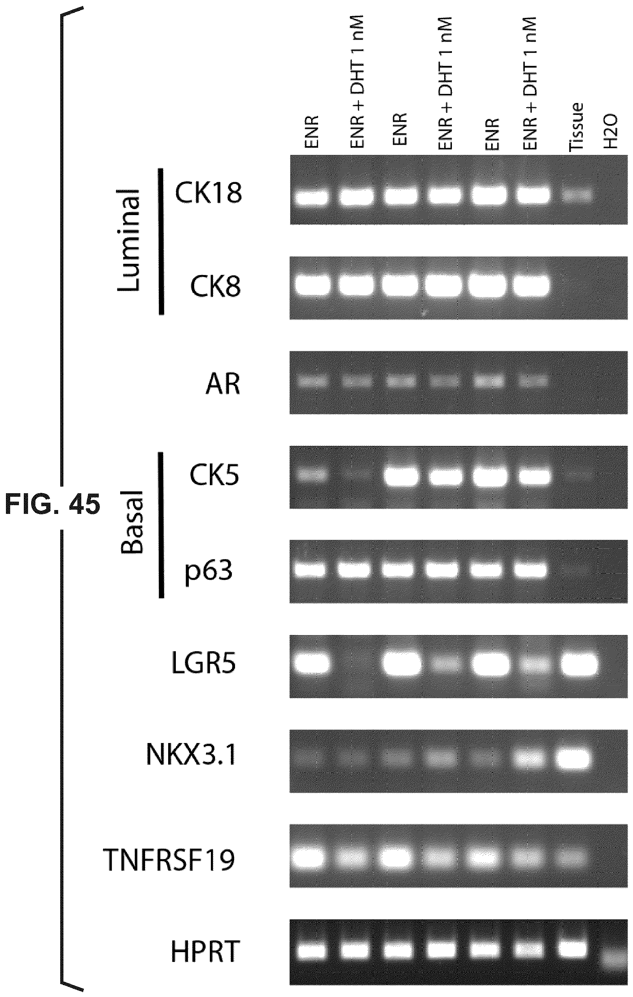

**Figure 1** **Mouse colon culture**
   **a.** left: Axin2 expression is under the control of the Wnt signaling pathway. Colon crypt organoids of Axin2-LacZ reporter mice cultured with EGF, Noggin, and R-spondin (ENR) for 3 days. Absence of LacZ stain indicates that no active Wnt signal is present in the colon organoids under ENR growth condition. Inset shows active Wnt signalling visualized by LacZ expression (dark stain) in freshly isolated colon crypts from the Axin2-LacZ reporter mice. right: Axin2-LacZ mice derived colon crypts cultured with ENR + Wnt3A (WENR) for 10 days. Dark stain indicates LacZ expression in these organoids.
   **b.** left: Lgr5-GFP-ires-CreER colon crypts cultured with ENR for 3 days. Absence of GFP fluorescence indicates loss of Lgr5 expression in the colon organoids under ENR growth condition. Inset shows Lgr5-GFP expression in freshly isolated colon crypts from Lgr5-GFP-ires-CreER mice. right: Lgr5-GFP-ires-CreER colon crypt cultured with WENR for 10 days demonstrates the presence of Lgr5 stem cells.
   **c.** Culture efficiency is determined under three different conditions: ENR, WENR full crypts, and WENR crypts after mild enzymatic digestion (WENR digested). Colon crypts were isolated from proximal colon (black columns) or distal colon (white columns). *:p<0.05.
   **d, e:** 4 days after removal of Wnt3A from the WENR culture medium results in organoid differentiation. **d**. Chromogranin A (ChA) in enteroendocrine cells;Mucin2 (muc2) in Goblet cells and the counter stain with DAPI can be seen. e. Mature enterocytes are visualized by Alkaline phosphatase staining.
   **f.** Relative mRNA expression of mature epithelial cell markers (Vil1 (Villin1), Alpi (Alkalin phosphatase), Chga (Chromogranin A), Muc2 (Mucin2)) are shown. WENR cultured colon crypt organoids are cultured for 4 days in WENR (hatched pattern) or ENR (black) condition. Freshly isolated colon crypts (white) are used for control. Scale bar in a, b, d, e: 50 µm. Error bars indicate s.e.m. n=3.
**Figure 2** **Human colon culture**
   **a.** The effect of nicotinamide on human colon crypt organoids. The majority of human colon crypt organoids die within a few days in WENR+gastrin (WENRg) condition (left panel). Addition of nicotinamide (middle panel: WENRg+nic) improves culture efficiency and lifespan of human colon organoids. * p<0.001. nic: nicotinamide.
   **b.** The effect of small molecule inhibitor for Alk4/5/7 (A83-01) and for the MAP kinase p38 (SB202190) on human colon crypt organoids. Left panel: Human colon organoids cultured in WENRg + nicotinamide containing medium form cystic structures 3-4 weeks after culture. Middle panel: Human colon organoids retain their characteristic budding structure under the Human Intestinal Stem Cell Culture ("HISC") condition (WENRg+nicotinamide+A83-01+SB202190). Right panel: A83-01 and SB202190 synergistically increase number of passages of the human colon organoids. * p<0.001. N.S.=statistically not significant. Error bars indicate s.e.m. n=5.
   **c.** Proliferating cells visualized by the incorporation of EdU are confined to the budding structures. DAPI is used as a counterstain
   **d.** Representative picture of a karyotype from a 3-month-old human colon crypt organoid. Scale: 100 µm.
   **e.** Heat-map of the expression profile of cultured human intestinal organoids. The heat-map is a comparison of human small intestinal crypts and human small intestinal villi. Genes more highly expressed in the crypt are dark grey (top-half of heat-map), genes more highly expressed in the villus are light grey (bottom-half of the heat-map). Organoids cultured in-vitro clearly exhibit a similar expression profile to freshly isolated small intestinal crypts and express known stem cell markers. (lane 1: human small intestinal organoids #1, lane 2: human small intestinal organoids #2, lane 3: human colon organoids, lane 4: freshly isolated human small intestinal crypts. The four samples are compared to human smallintestinal villus).

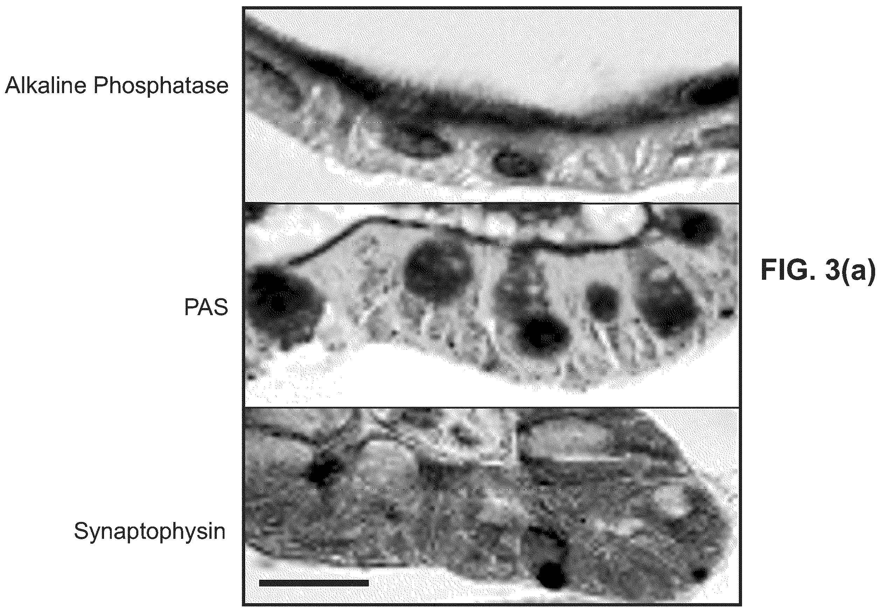

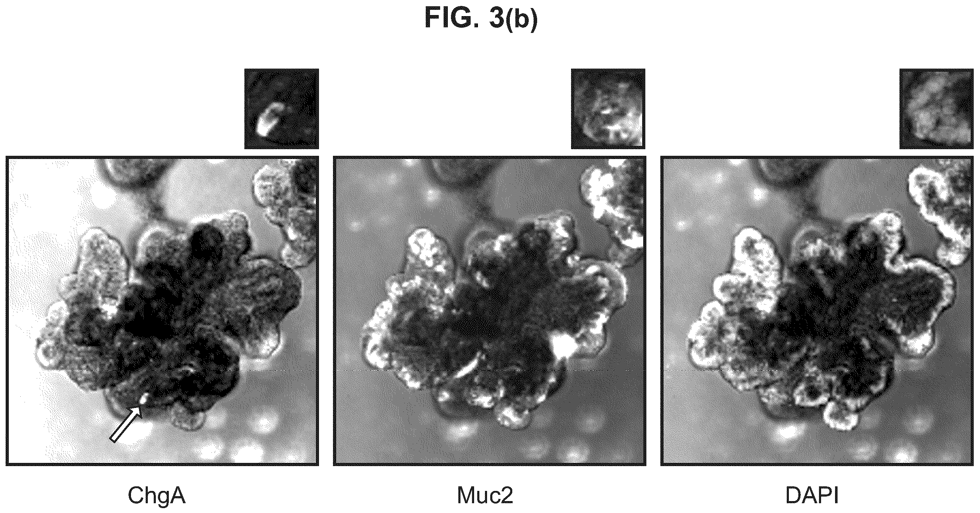

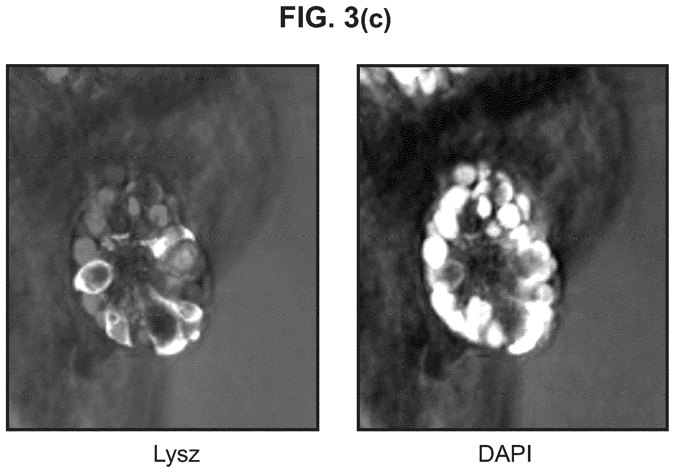

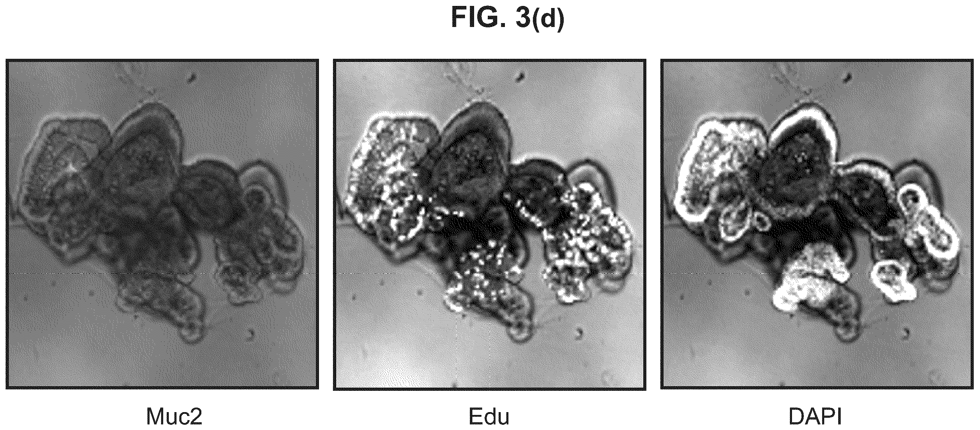

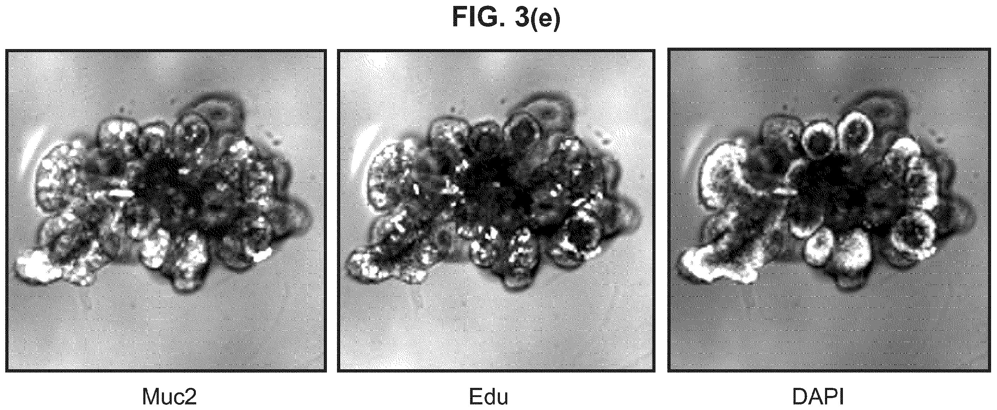

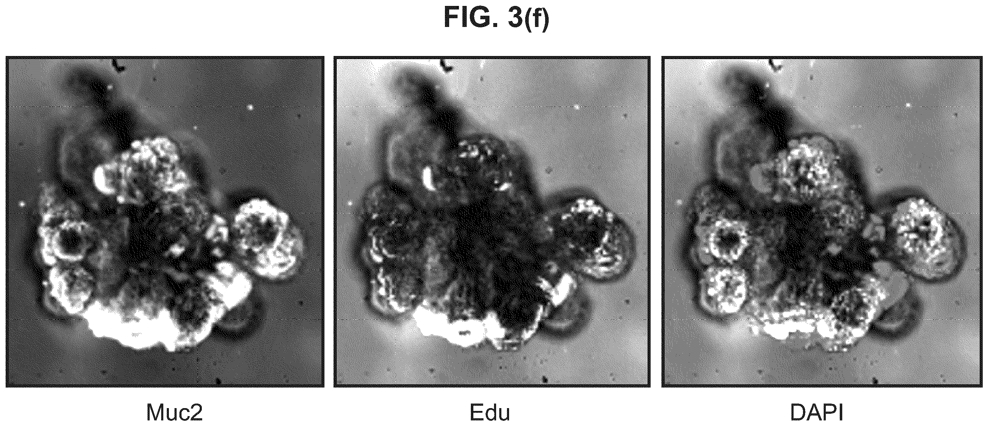

**Figure 3** **Human intestinal organoid cell type composition**
   **(a-c)** Human organoids differentiate into the different cell types of the intestine after withdrawal of Nicotinamide and SB202190. Markers of the different cell types were used to demonstrate differentiation. **(a)** Top panel: Alkaline phosphatase staining for mature enterocytes, Middle panel: PAS staining for goblet cells, Bottom panel: Synaptophysin staining for enteroendocrine cells. **(b)** In each case, the light areas indicate staining. Mucin2 (Muc2) staining in the middle panel represents goblet cells and Chromogranin A (ChgA) in the left-hand panel represents enteroendocrine cells (see arrow and inset). DAPI is used as a counterstain (right panel). **(c)** Lysozyme (Lysz) is stained in the left-hand panel to show Paneth cells. DAPI is used as a counterstain (right panel).
   (**d-f)** Goblet cell differentiation (Muc2) is blocked by SB202190 treatment of organoids **(d),** while the Notch inhibitor DBZ increases goblet cell number in the human organoids **(f).** Proliferating cells are represented by EdU incorporation (middle panel) are increased in SB202190 treated organoids **(d)** or decreased in DBZ treated organoids **(f)**.
   Organoids are cultured under the following conditions for 5 days: a) top: ENRg+A83-01+SB202190+Nicotinamide, a) middle and bottom, b), c) WENRg+A83-01, d)WENRg+A83-01+SB202190, e) WENRg+A83-01, f) WENRg+A83-01+DBZ.
   Scale bar:20 µm (a), 50 µm (b-f). a, b, d-f: human colon crypt organoids, c: human small intestinal organoids.
**Figure 4** **Adeno(carcino)ma cultures**
   **a.** Lgr5-GFP-ires-CreER/APCfl/fl crypts cultured with EGF (E) (top) or EGF+Noggin (EN) (bottom) for 10 days. **b.** Relative mRNA expression of Lgr5 and Axin2. Freshly isolated adenoma cells (white) were cultured with EGF (hatched) or EGF+Noggin (black).
   **c.** Culture efficiency of organoids from sorted Lgr5-GFPhi, Lgr5-GFPlo, Lgr5-GFP-ve cells. *p<0.01. one way ANOVA. Error bars indicate s.e.m. n=3
   **d.** Time course culture of human colon adenocarcinoma cells.
**Figure 5** **Culture of Barrett's esophagus and treatment with Notch inhibitor.**
   **a.** Isolated epithelium from Barrett's esophagus (BE) cultured with HISC condition for 7 days forms cystic structures. **b.** Addition of FGF10 significantly increases the number of passages for BE organoids. Error bars indicate s.e.m. n=3 **c.** Representative time course of a BE organoid. **d.** Paraffin sections from BE organoids. Nicotinamide and SB202190 are withdrawn for 4 days with **(right)** or without **(left)** the Notch inhibitor DBZ added to the medium. Proliferating cells (Ki67 stain) disappear and PAS+ goblet cells increase with DBZ treatment.
**Figure 6** **Axin2 mRNA expression is recovered in mouse colon**
   **organoids under the presence of Wnt-3A**
   Isolated colonic crypts are analysed for Axin2 mRNA expression after 3 days or 7 days culture with ENR (hatched) or WENR (black). Freshly isolated colon crypts were used as control. Error bars indicate s.e.m. n=3
**Figure 7** **Relative mRNA expression of mature epithelial cell markers**
   Freshly isolated small intestinal crypts (white) are cultured in HISC condition for 14 days, followed by a culture with the indicated culture condition for 4 days. mRNA expression of ALPI (Alkaline phosphatase), VIL1 (Villin 1), LYZ (Lysozyme), CHGB (Chromogranin B) and MUC2 (Mucin2) was analysed. Culture condition: HISC (black), ENRg+A83-01+SB202190+Nicotinamide, WENRg+A83-01, ENRg+A83-01, ENRg. Freshly isolated small intestinal crypts were used as control (set as 1.0 for ALPI, VIL1 and LYZ, as 5.0 for CHGB and MUC2. Error bars indicate s.e.m. n=3.

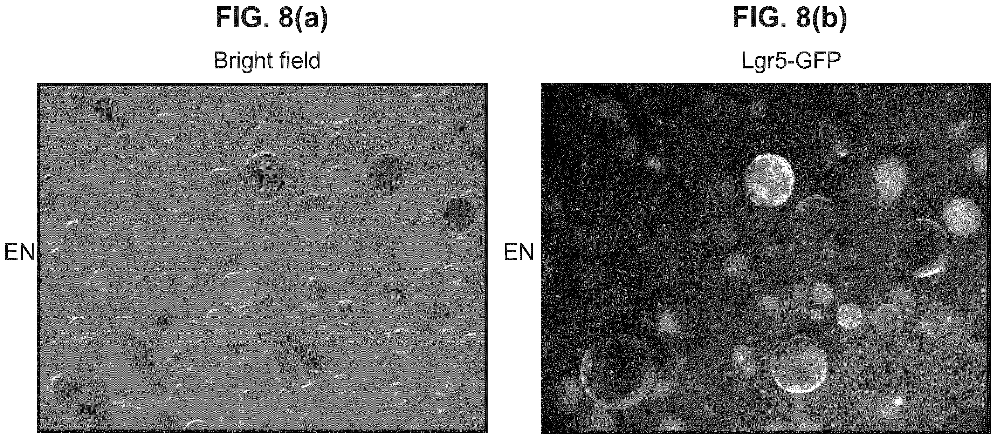

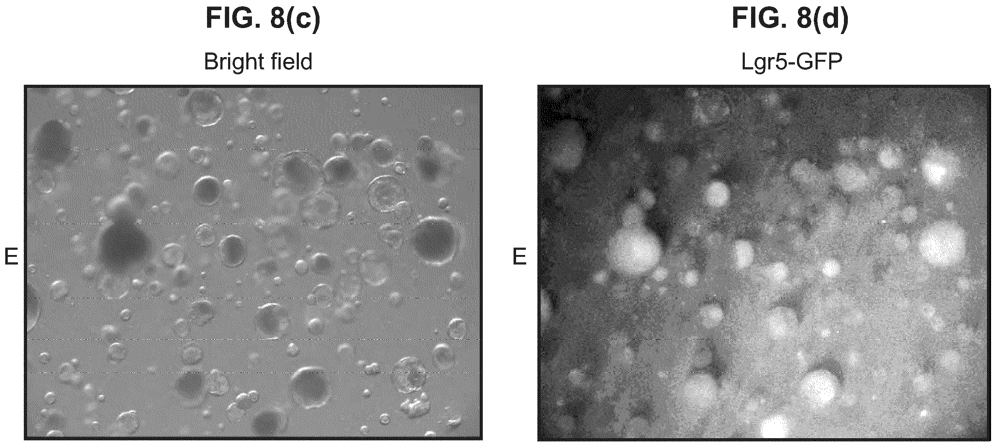

**Figure 8** **Sorted Lgr5-GFP- cells form Lgr5-GFP+ organoids**
   Single sorted Lgr5-GFP- APCfl/fl adenoma cells are cultured with EGF+Noggin (EN) or EGF (E) for 7 days. Adenoma organoids derived from Lgr5-GFP- cells recovered Lgr5-GFP expression under EN condition but not under E condition (a, c: bright, b, d: GFP autofluorescence).
**Figure 9** **Histochemical analysis of adenoma/colon cancer organoids**
   Mouse small intestinal adenoma organoids (Left panel) and human colon cancer organoids (Right panel) were analyzed with indicated histochemical (HE, PAS and Alkaline phosphatase) or immunohistochemical (Chromogaranin A, Ki67 and Caspase3) stainings.

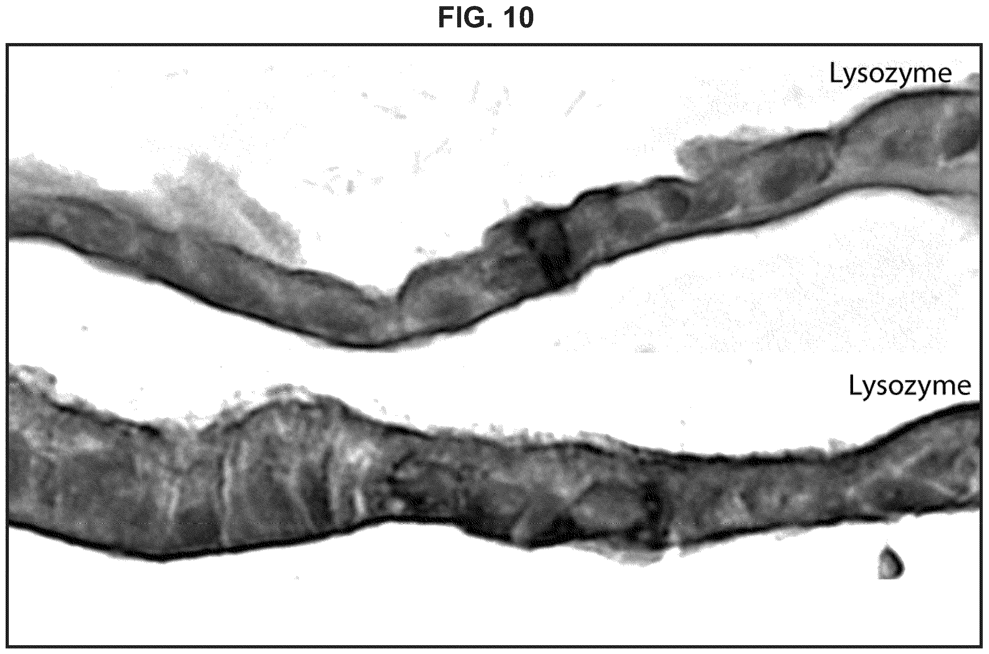

**Figure 10** **Paneth cells in BE organoids**
   Lysozyme+ Paneth cells were observed in differentiated BE organoids.

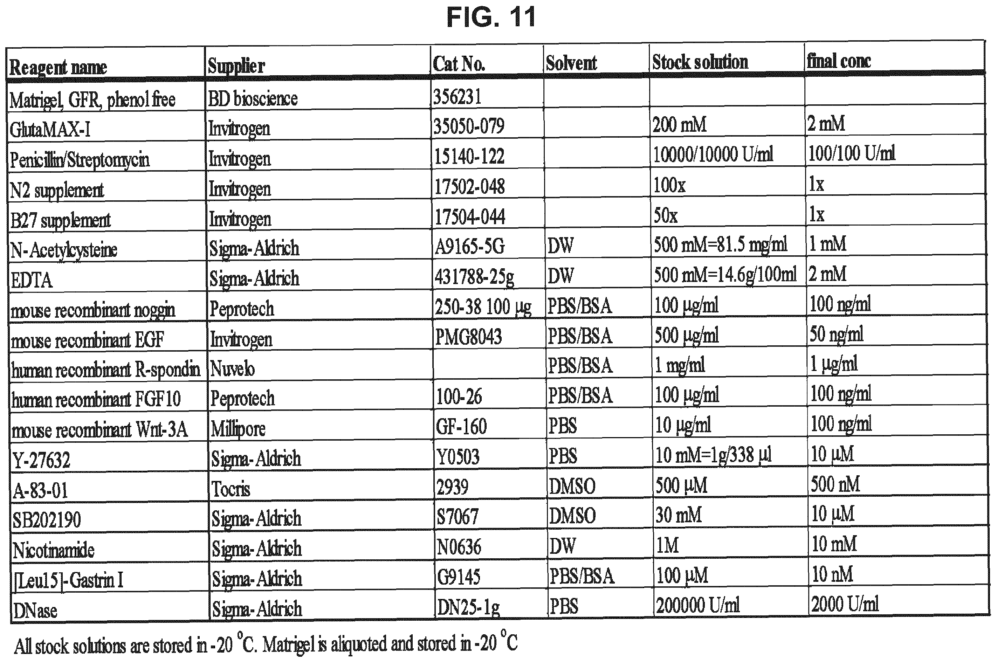

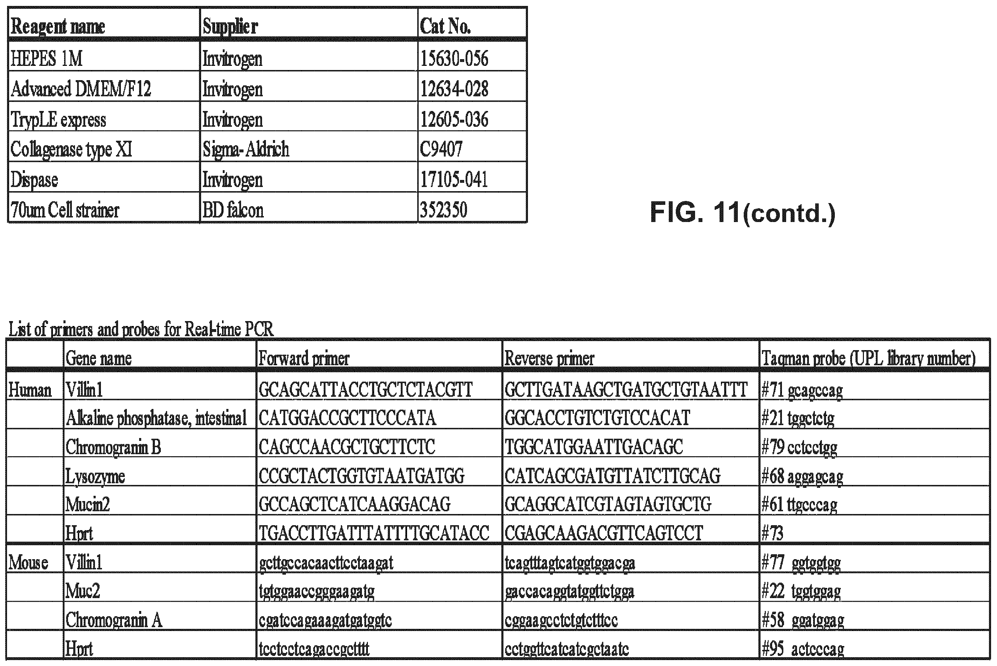

**Figure 11** **List of reagents used for organoid culture**

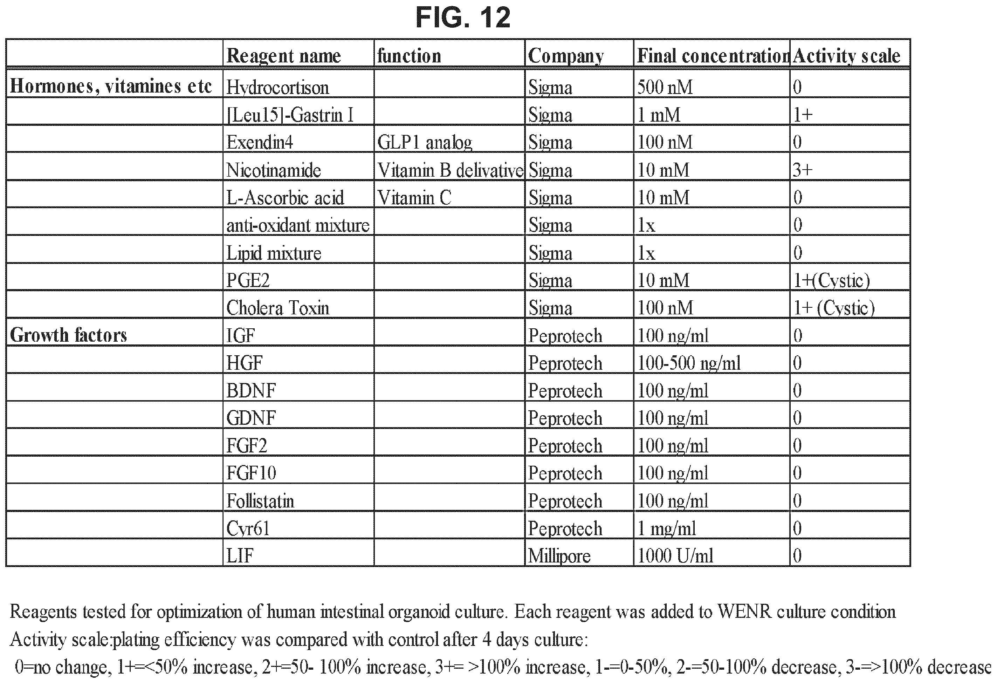

**Figure 12** **List of reagents used for optimization of human intestinal organoid culture**

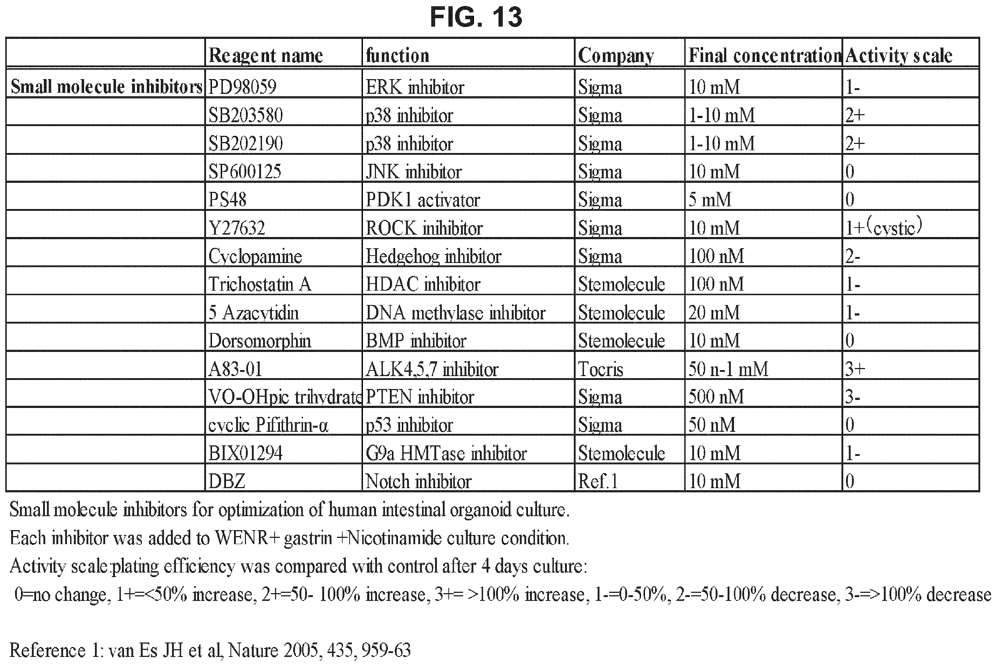

**Figure 13** **List of small molecule inhibitors used for optimization of human intestinal organoids culture**
**Figure 14** **List of the 25 most up- and down-regulated genes**
   mRNA from human small intestinal organoids or colon organoids are compared with that from freshly isolated small intestinal villi by microarray. The 25 most upregulated and downregulated genes are shown. Hatched lines highlight genes which were in the top 70 most upregulated and downregulated genes in freshly isolated human small intestinal crypts vs. villi.

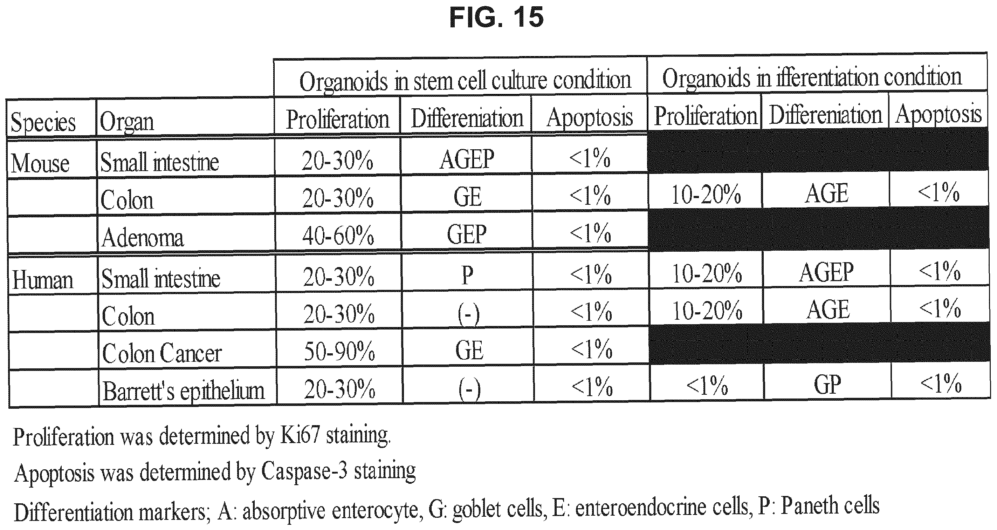

**Figure 15****. Summary of proliferation, differentiation and apoptosis status of each organoid culture condition**

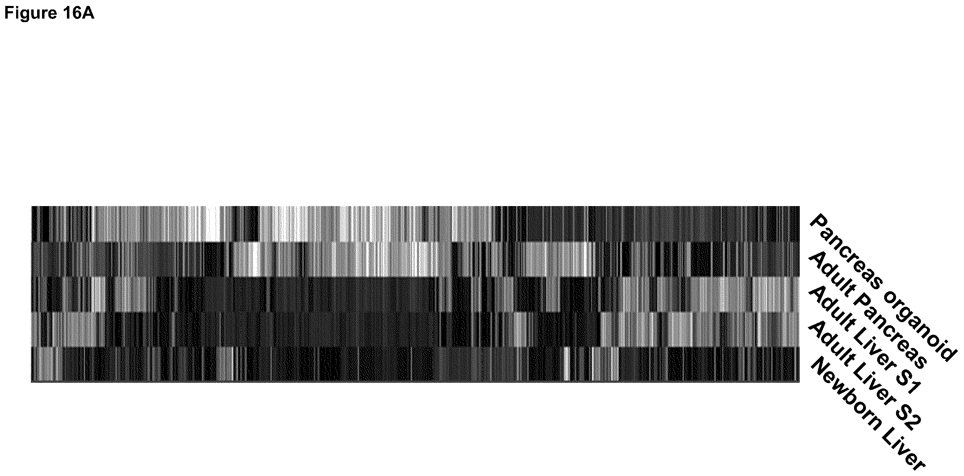

**Figure 16****: Microarray comparison of mouse pancreatic organoids**
   A - Microarray clustering analysis, comparing RNA from the pancreas organoids (cultured in the conditions described in Example 2) with adult pancreas, adult liver and newborn liver. From left to right: i) pancreas organoid; ii) adult pancreas; iii) adult liver (sample 1 [S1] and sample 2 [2]); iv) adult liver S2; and v) newborn liver.
   B - Raw signal data from the microarray analysis, comparing the expression levels of selected ductal markers, transcription factors necessary for Ngn3 expression and endocrine markers in adult liver, adult pancreas, pancreas organoids and liver organoids in expansion media.

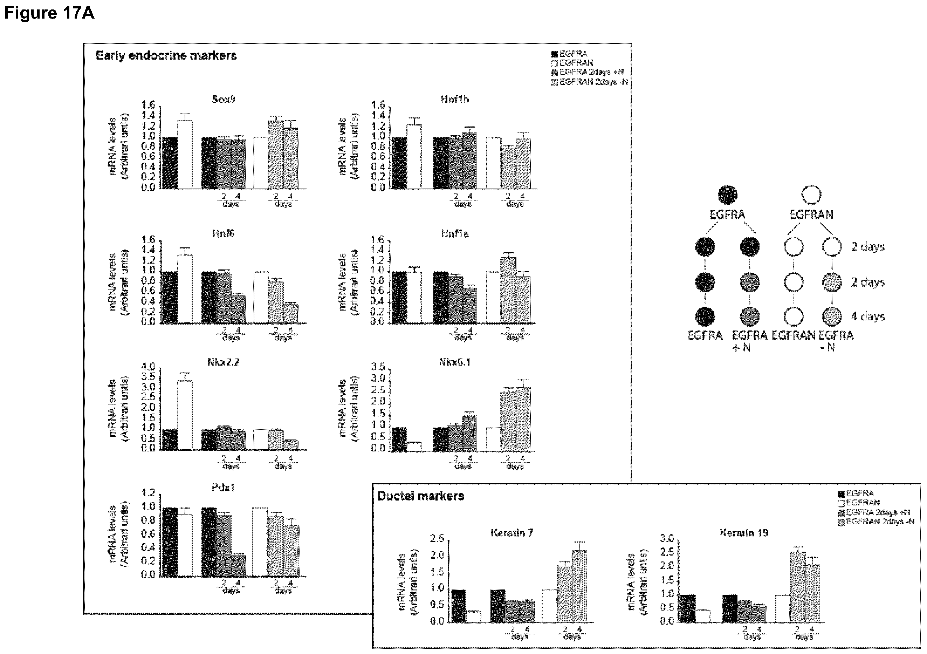

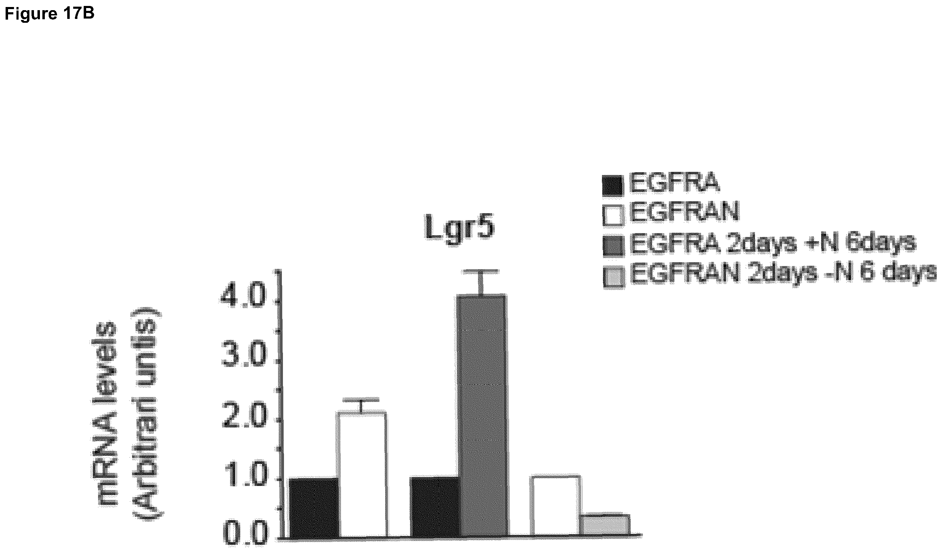

**Figure 17****: The effect of Noggin on the expansion of pancreatic organoids**
   A - Bar charts showing gene expression analysis of pancreatic organoids cultured in EGFRA so, that have never been cultured with Noggin (black) with organoids cultured in EGFRAN , so have always been cultured with Noggin (white). The effect of culturing the pancreatic organoids in EGFRA for 2 days and then withdrawing Noggin and culturing for a further 2 or 4 days (light grey) and the effect of culturing the pancreatic organoids in EGFRA for 2 days and then adding Noggin and culturing for a further 2 or 4 days (dark grey) on gene expression is also shown. mRNA levels (arbitrary units) are presented on the Y axis. mRNA of the following early endocrine markers is analysed in the main figure: Sox9, Hnf1b, Hnf6, Hnf1a, Nkx2.2, Nkx6.1 and Pdx1. mRNA of the following ductal markers in analysed in the inset part: keratin 7 (Krt7) and keratin 19 (Krt19).
   B - Bar chart showing the effect of Noggin on the expression of Lgr5 in pancreatic organoids in the expansion culture medium. Data are provided for pancreatic organoids cultured in EGFRA that have never been cultured with Noggin (black) with organoids cultured in EGFRAN and so have always been cultured with Noggin (white). The effect of culturing the pancreatic organoids in EGFRAN and then withdrawing Noggin and culturing for a further 6 days (light grey) and the effect of culturing the pancreatic organoids in EGFRA and then adding Noggin and culturing for a further 6 days (dark grey) on Lgr5 gene expression is also shown. mRNA levels (arbitrary units) are presented on the Y axis.

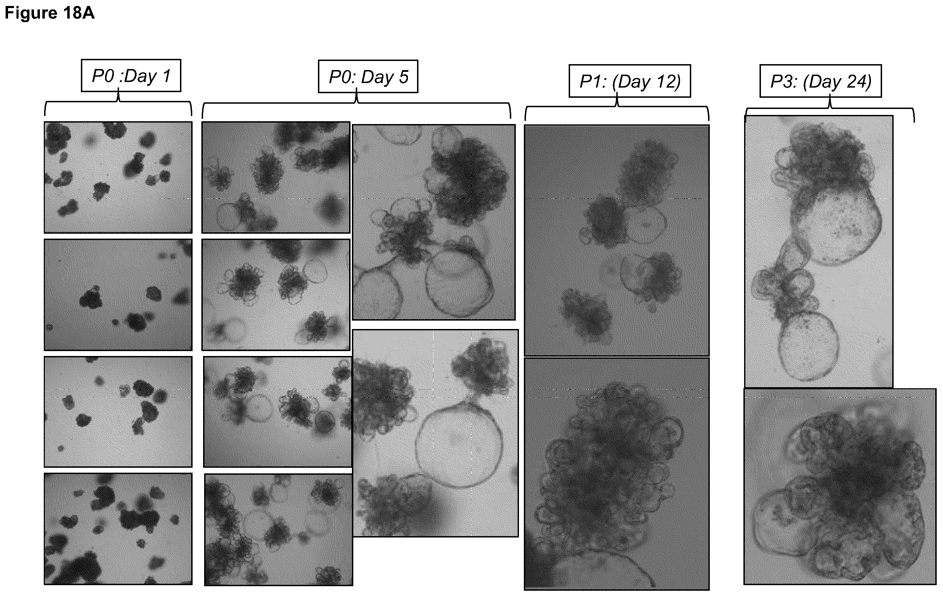

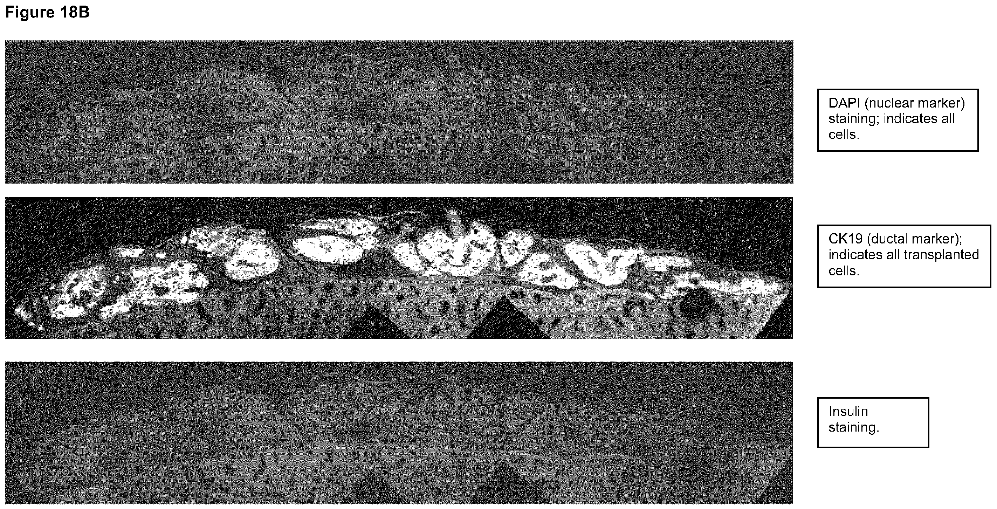

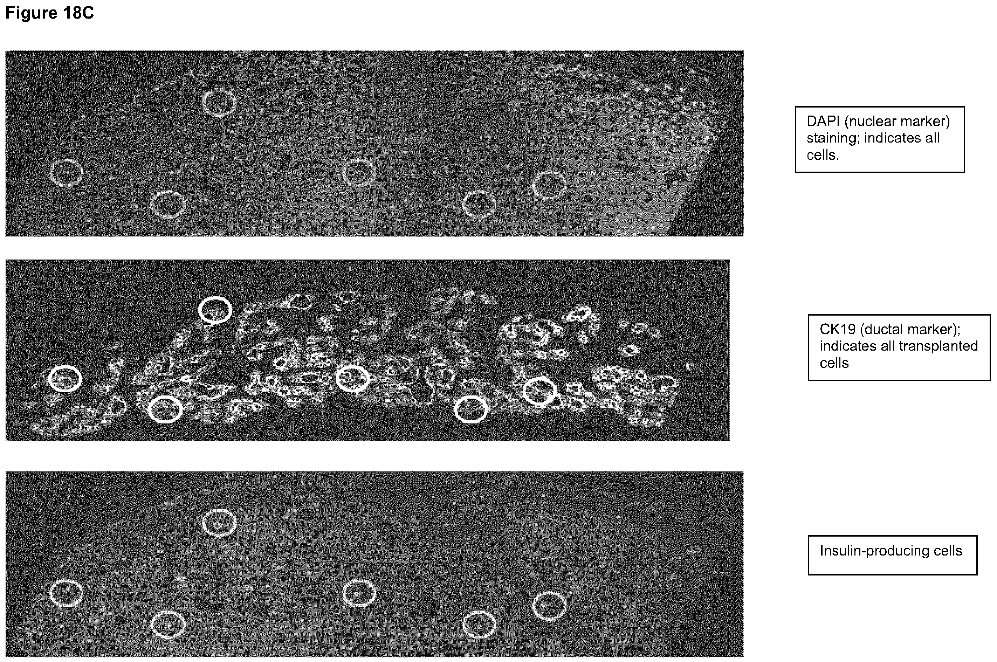

**Figure 18****: Human insulin producing cells develop from *ex vivo* expanded, *in vivo* transplanted progenitor cells**
   A - Growth of human pancreas tissue from progenitor cells (pancreas stem cells) at P0: (Day 1); P0: (Day 5); P1: (Day 12) and P3: (Day 24), where "P" refers to the number of passages. Figures 18B and C show transplantation of human pancreatic organoids under the murine peri-renal capsule.
   B - 3 hours after transplantation of the pancreatic organoid cells into the recipient mice: DAPI (nuclear marker) staining in the upper picture indicates all cells; K19 (ductal marker) staining in the lower picture shows all transplanted cells and insulin (beta cell marker) in the lower picture indicates insulin-producing cells.
   C - 1 month after transplantation of the pancreatic organoid cells into the recipient mice: DAPI (nuclear marker) staining in the upper picture (in blue) indicates all cells; CK19 (ductal marker) expression in the middle picture (in green) indicates all transplanted cells and insulin (beta cell marker) in the lower picture (in red) indicates insulin-producing cells. A selection of the insulin-producing cells are encircled but all clearly stained cells are thought to be insulin positive.
**Figure 19****: Pancreatic organoid gene expression**
   This table shows the pancreatic gene expression of the most upregulated genes when compared to liver organoids.

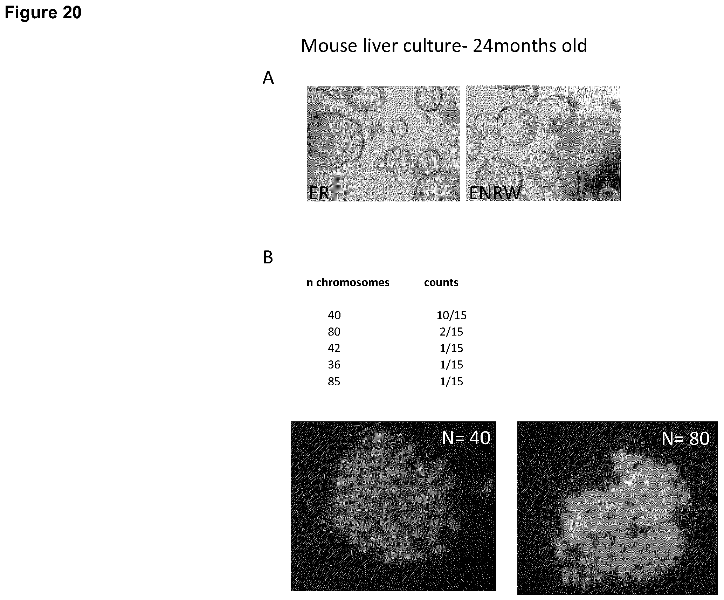

**Figure 20****: Mouse liver organoid culture shows stable karyotyping after long-term culture.**
   A - DIC images of liver organoids maintained in EGF (E) and R-spondin 1 (R), supplemented with FGF10, HGF and Nicotinamide (left figure, ER) or maintained in the same combination supplemented with Noggin (N) and Wnt3Aconditioned media (W) (right figure, ENRW) for a period of 24 months.
   B - Karyotype analysis of mouse liver organoids after 8 months in culture. Normal chromosomal counts (n=40, left panel figure) and polyploidy, a typical hepatocyte feature, were found (n=80, right panel figure)

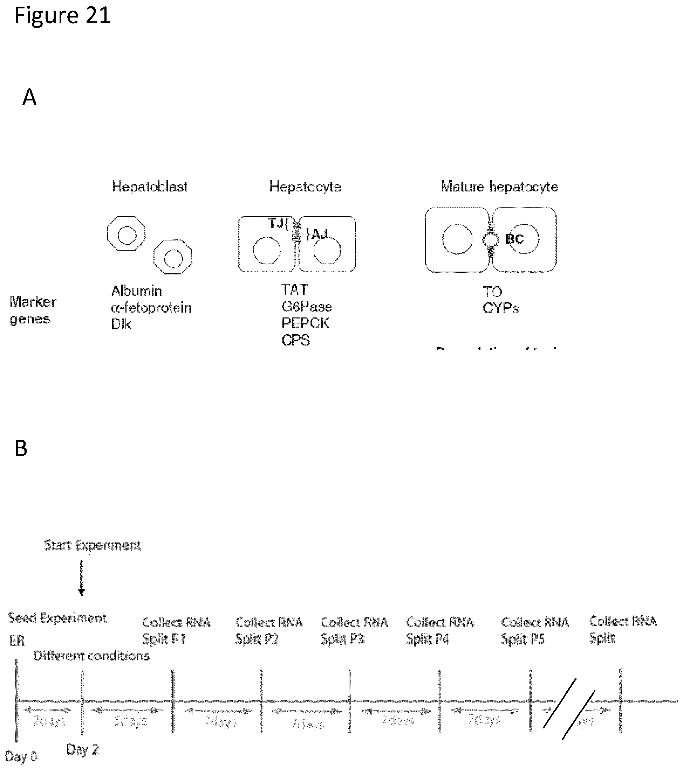

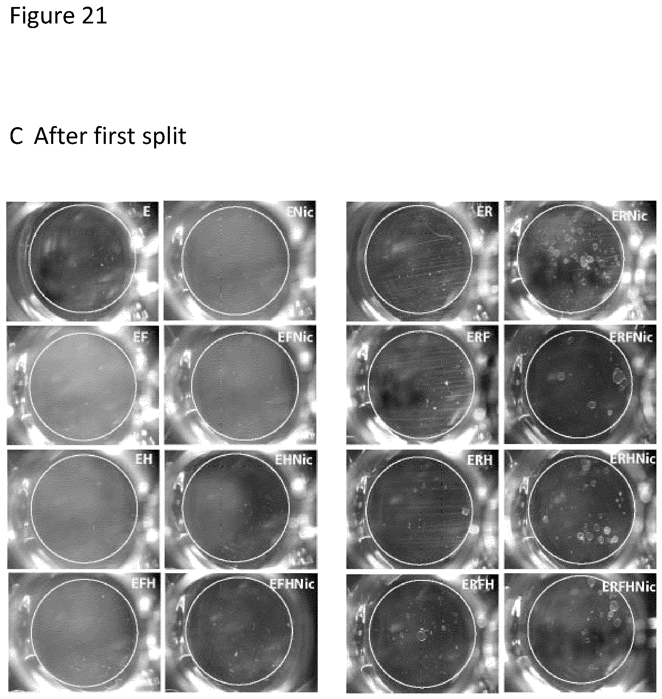

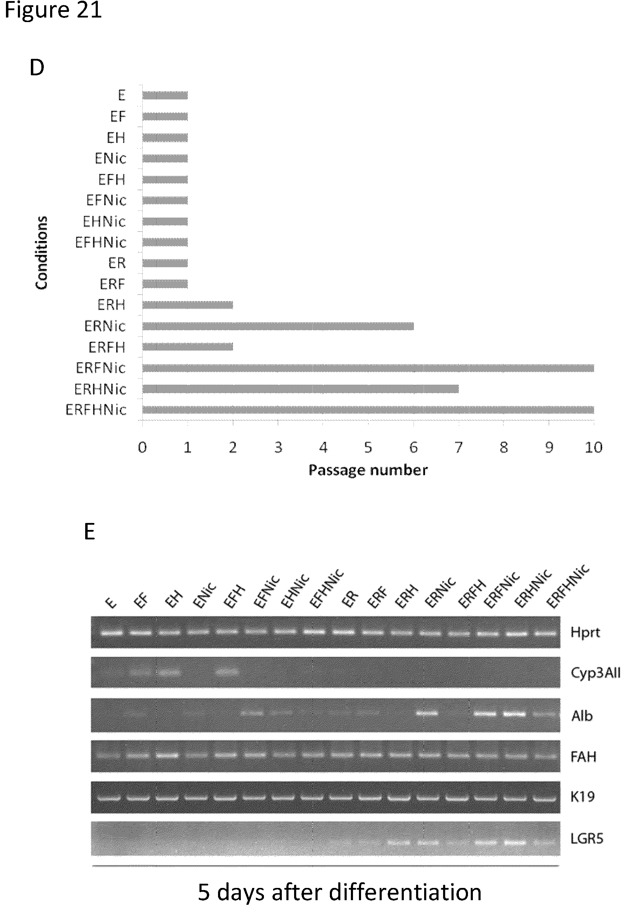

**Figure 21****: Supplemental factors FGF10, HGF and Nicotinamide; effect on liver organoid growth and differentiation.**
   A - Diagram depicting the genes differentially expressed during the 3 stages of liver development, from hepatoblast to mature hepatocyte.
   B - Scheme showing the protocol used. Cultures were seeded in expansion medium (ERFHNic: EGF (E) and R-spondin 1 (R), supplemented with FGF10, HGF and Nicotinamide; ERFHNic is indicated as 'ER' in Figure 8B) 2 days prior the experiment. Two days later, culture media was changed to either EGF (E) alone or EGF supplemented with R-spondin 1 (ER) with or without additional supplements chosen from FGF10 (F) or HGF (H) or Nicotinamide (Nic) or a combination of these at the concentrations stated in the text. Five days later cultures were split and replated at 1:4 ratio for each condition. Under these conditions, cultures have been split and replated every 7 days for a total period of 10 weeks
   C - First day after first split in each of the culture conditions tested. Results shows that EGF and Rspondin 1 combined with FGF10 or HGF or Nicotinamide or a combination of these are essential to achieve at least 1 passage.
   D - After long-term culture, the combination of ER supplemented with FNic or ER supplemented with FHNic, both result in high passage numbers. After passage 10, the growth rate is better for the culture condition including the 3 supplemental factors; ERFHNic.
   E - RT-PCR analysis showing the expression of different hepatocyte markers (CYP3A11, Alb, FAH) and cholangiocyte marker (K19) 5 days after the withdrawal of certain factors (starting point was ERFHNic). Note that only the condition EF showed expression of all hepatocyte markers tested. HPRT was used as a housekeeping gene to normalize for gene expression.
**Figure 22****: Table showing the quantification of different hepatocyte and cholangiocyte specific transcription factors in cells from three different liver culture conditions and in adult liver tissue.** Also shown is the expression of the key components of the Notch and TGF-beta signalling pathways. E=EFHNic, ER=ERFHNic, ENRW=ENRWFHNic.

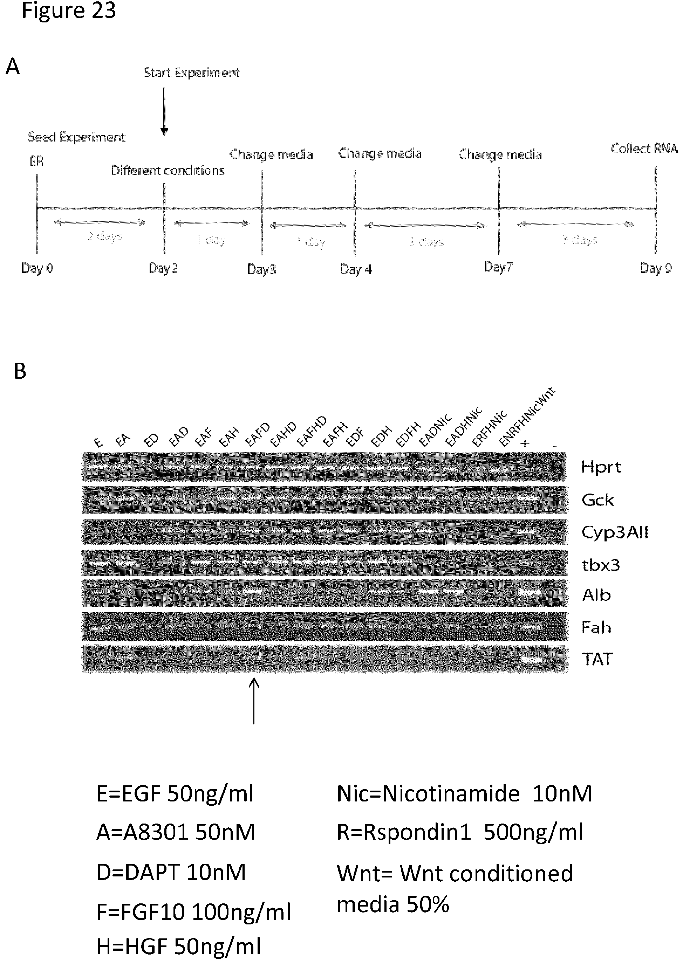

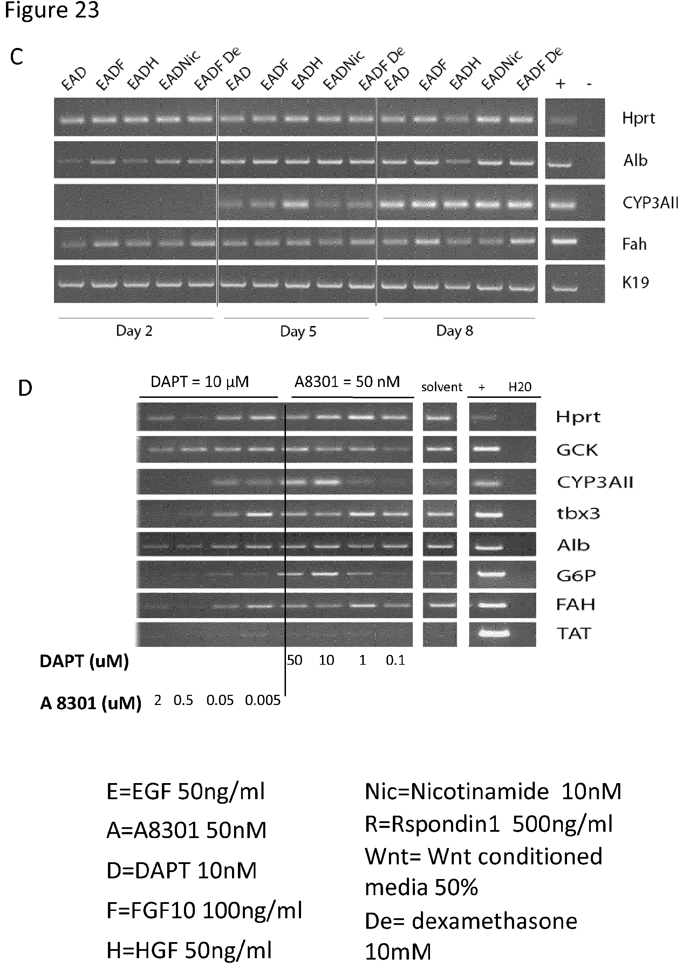

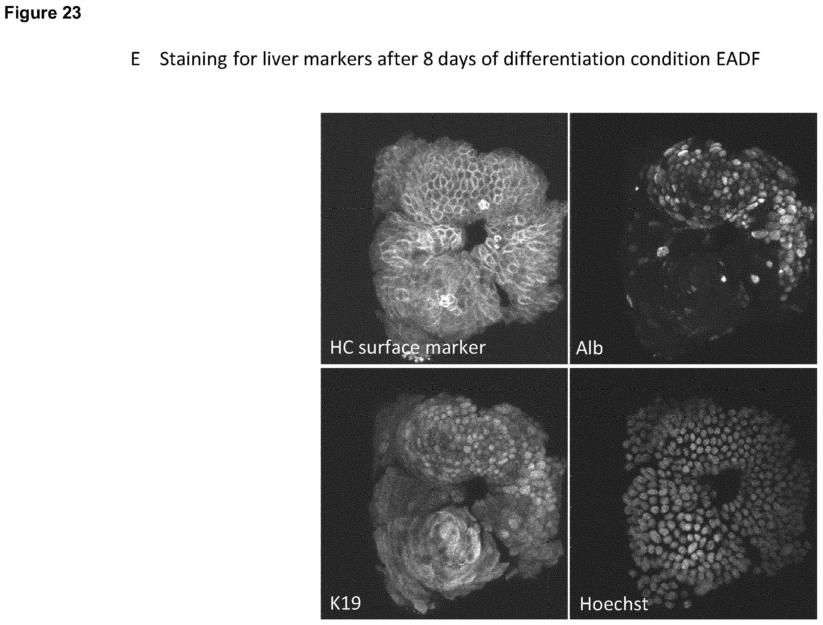

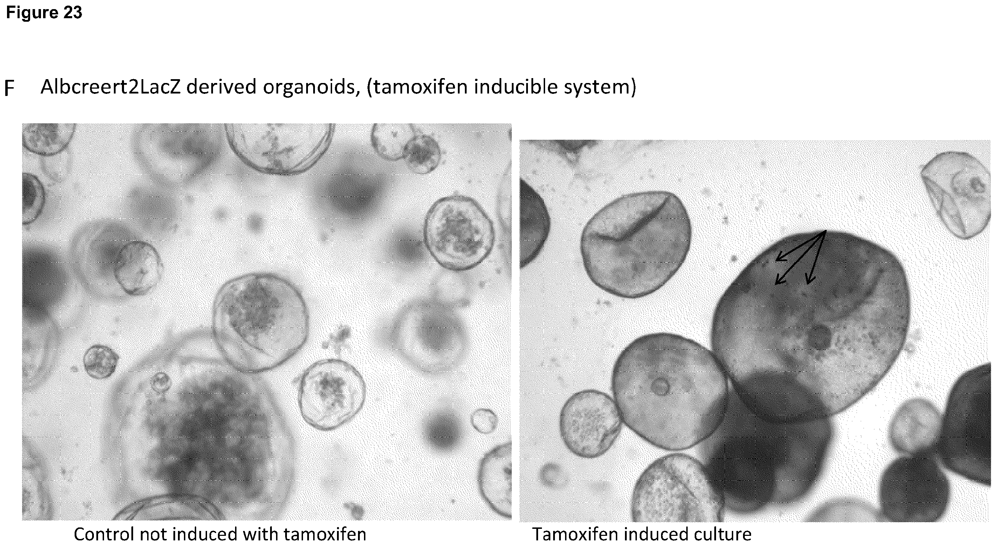

**Figure 23****: Differentiation protocol**
   A - Scheme showing the protocol used. Cultures were seeded in expansion medium (ERFHNic: EGF (E) and R-spondin 1 (R), supplemented with FGF10, HGF and Nicotinamide; ERFHNic is indicated as 'ER' in Figure 10A) 2 days prior to the experiment. Two days later, culture media was changed to EGF (E) supplemented with either A8301 (A), or DAPT (D), or FGF10 (F) or HGF (H) or Nicotinamide (Nic) or R-spondin 1 (R) or Wnt3A or Noggin (N) or a combination of these at the concentrations shown. RNA was isolated at several time points. Mouse liver tissue was taken as positive control (+) whereas water was taken as negative control (-).
   B - RT-PCR analysis showing the expression of the hepatocyte markers CYP3A11, Alb, FAH, tbx3, TAT and Gck 7 days after differentiation conditions. Note that only the condition EADF showed an expression of all hepatocyte markers tested. HPRT was used as a housekeeping gene to normalize for gene expression.
   C - Time course expression analysis after differentiation conditions. At days 2, 5 and 8 days after differentiation, the expression of the hepatocyte markers CYP3A11, Alb, FAH, and the cholangyocyte marker K19, was analysed by RTPCR. Note that the expression of the liver markers CYP3A11 and FAH starts at day 5 and peaks at day 8 after. HPRT was used as a housekeeping gene to normalize for gene expression. A; A8301, D; DAPT, F; FGF10, H; HGF, De; Dexamethasone
   D - Titration experiment showing the expression of the hepatocyte markers CYP3A11, Alb, FAH, tbx3, TAT, G6P and Gck 7 days after different concentrations of the differentiation compounds A and D. HPRT was used as a housekeeping gene to normalize for gene expression.
   E - Immunofluorescent staining for the liver markers K19, Albumin and hepatocyte surface marker
   F - Xgal staining on Albcreert2LacZ mice liver-derived organoids. Albumin positive cells (arrows) were detected after EADF differentiation in tamoxifen induced Albcreert2LacZ derived cultures.
**Figure 24****: Prostaglandin signalling pathway** (Antagonism of the prostaglandin D₂ receptors DP₁ and CRTH2 as an approach to treat allergic diseases. Roy Pettipher, Trevor T. Hansel & Richard Armer Nature Reviews Drug Discovery 6, 313-325 (April 2007)).

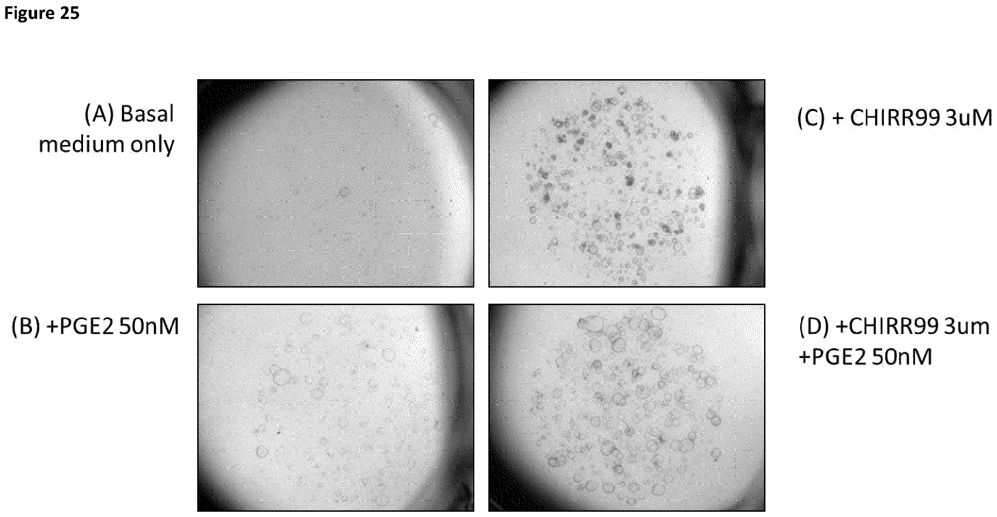

**Figure 25****: Liver organoids** cultured in (A) basal medium comprising hEGF (100ng/ml, Invitrogen); human noggin (hnoggin) (25ng/ml, peprotech); gastrin (10nM, sigma); hFGF10 (peprotech); nicotinamide (10mM, sigma); A8301 (500nM, Tocris); hHGF (50ng/ml, peprotech); Rspo conditioned media (10%); (B) basal medium + PGE2 (50nM); (C) basal medium + CHIR99021 (3uM); (D) basal medium + CHIR99021 (3uM) + PGE2 (50 nM).

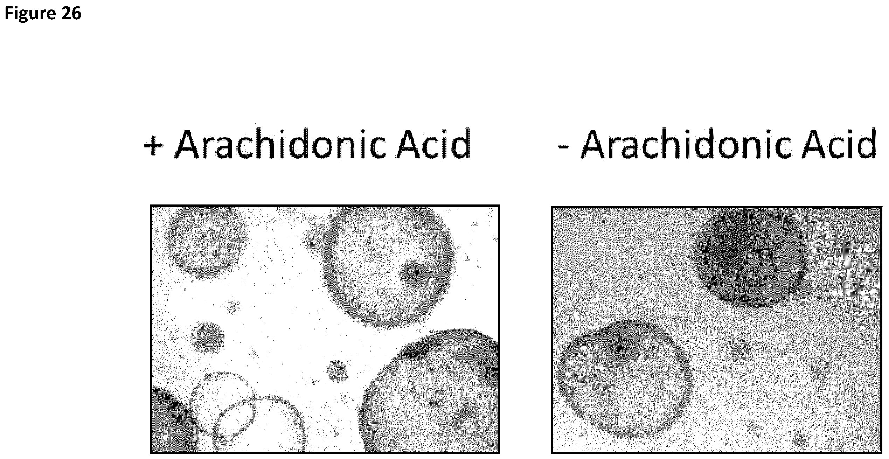

**Figure 26****: Liver organoids** cultured in basal medium (as described for Figure 25) with and without Arachidonic acid.
**Figure 27****: Gene expression profile of mouse liver organoids under differentiation conditions resemble the adult and newborn liver profile**
   A - Gene clusters showing the genes similarly expressed (a) or similarly shut down (b) between the differentiation condition EADF and adult or newborn liver.
   B - Gene clusters showing the genes differentially expressed between the liver organoids and adult or newborn liver (a) and the genes similarly expressed between EADF and newborn liver (b).
   C - Raw signal data from a microarray analysis, comparing the expression levels of selected ductal markers, transcription factors necessary for Ngn3 expression and endocrine markers in adult liver, adult pancreas, pancreas organoids and liver organoids in expansion media.
**Figure 28****: Mouse liver signature genes**
   Table showing a) markers expressed in mouse liver stem cells; b) markers not expressed in mouse liver stem cells; c) hepatocyte and cholangiocyte markers expressed in mouse liver stem cell signature for mouse liver organoids in expansion media; d) hepatocyte and cholangiocyte markers not expressed in mouse liver stem cell signature for mouse liver organoids in expansion media; e) reprogramming genes expressed in mouse liver organoids; f) reprogramming genes not expressed in mouse liver organoids. The results were obtained using a liver microarray using the Universal Mouse Reference RNA (Strategene, Catalog #740100) as a reference RNA. If the absolute figures detected were less than 100, the gene was consider as undetected.
**Figure 29****: Human liver signature genes**
   Table showing results of liver mircroarray of human organoids. From left to right, the results are shown for a) expansion medium EM1, b) expansion medium EM2, c) differentiation medium, d) adult liver.
   The numbers (log2) in the left four columns are the result of a comparison between the sample and a reference (commercial) RNA sample which is used for all arrays. The relative expression of mRNA in each sample compared to the RNA present in the reference sample is shown.. The reference RNA used was Universal Human Reference RNA (Stratagene, Catalog #740000). Thus, negative numbers in these columns do not relate to real expression levels it just means there is less of that RNA then in the Reference sample. The 4 columns on the right are absolute figures. If they are below 100, they are considered as undetected.
**Figure 30****: Morphology of liver organoids.** (A) Upper panels: paraffin section of a mouse liver showing the different domains (PT= portal triad, CV= central vein). Lower panels: Paraffin section of a liver organoid showing different domains b (single layered epithelia) and h (stratified epithelia) (B) Right pannel: Ecadherin staining in the liver organoids. Two different domains can be identified. Domain b, formed by a single layered epithelia that resembles the bile duct structures in the liver. This bile duct domain is formed by highly polarized cells that shows positive staining for pancytokeratin (PCK) (lower panel). Left panels show the presence of a second domain within the liver organoids. This h domain is formed by a stratified epithelia with non-polarized cells. The cells are organized around a central lumen and express the hepatocyte marker Alb. Magnification 10 x.
**Figure 31****: H&E staining of pancreas organoids**
   Mouse pancreas organoids were cultured in expansion conditions EGFNRA83-01 [(EGF(50ng/ml), Gastrin (50nM), Noggin (10%), Rspondin (5%), FGF10 (100ng/ml) and A8301 (50nM)] during 8 passages (~10 weeks). The organoids were removed from the matrigel using BD Cell Recovery Solution following manufacturer's instructions and fixed with 4% paraformaldehyde at room temperature during 1h. Then, the organoids were washed three times with cold PBS, dehydrated with increased concentration of alcohol and embedded in paraffin. 3um parafine sections were stained with Hematoxyline-Eosine to analyze the histology of the pancreas organoids.
   A strong variability in the shape and structure of the organoids was observed. Some of the organoids are cystic structures formed by a monolayer of polarized epithelial cells. Other organoids show the same monolayer of epithelial cells and some stratified areas where cells are smaller in size and with a round shape. In some organoids invaginations occupying the inner space of the cystic structure were observed.
   The stainings show that some organoids comprise mostly monolayers of epithelial cells (left bottom), whereas other organoids comprise stratified regions and/or pseudo stratified regions and/or folded monolayers (right bottom). Most pancreatic organoids comprise regions of stratified cells and monolayers (sometimes folded).
**Figure 32****. Quantification of forskolin-induced murine small intestine organoid swelling. (a)** Light microscopy analysis of organoids stimulated with forskolin or DMSO. Representative examples for the indicated time points after start of stimulation are shown. The red line indicates the internal organoid lumen. **(b)** Fluorescence confocal image of a calcein-green-labeled organoid with object recognition (green line) by image analysis software. **(c)** Representative example of a forskolin-stimulated calcein-green-labeled organoid. Differential interference contrast (DIC) and fluorescence was imaged using live cell confocal microscopy. Surface area relative to t=0 is indicated in the top-left corner. **(d)** The surface area relative to t=0 (normalized area) of 11 individual organoids in a single well. The average is indicated in black (mean ± s.e.m.). **(e)** Dose-dependent increase of surface area by forskolin (5µM (n=4 number of organoids analyzed), 5x10⁻²µM (n=11), 5x10⁻⁴µM (n=10), DMSO n=9)). Scale bars (a-c) 30µm. All results are representative for at least three independent experiments.

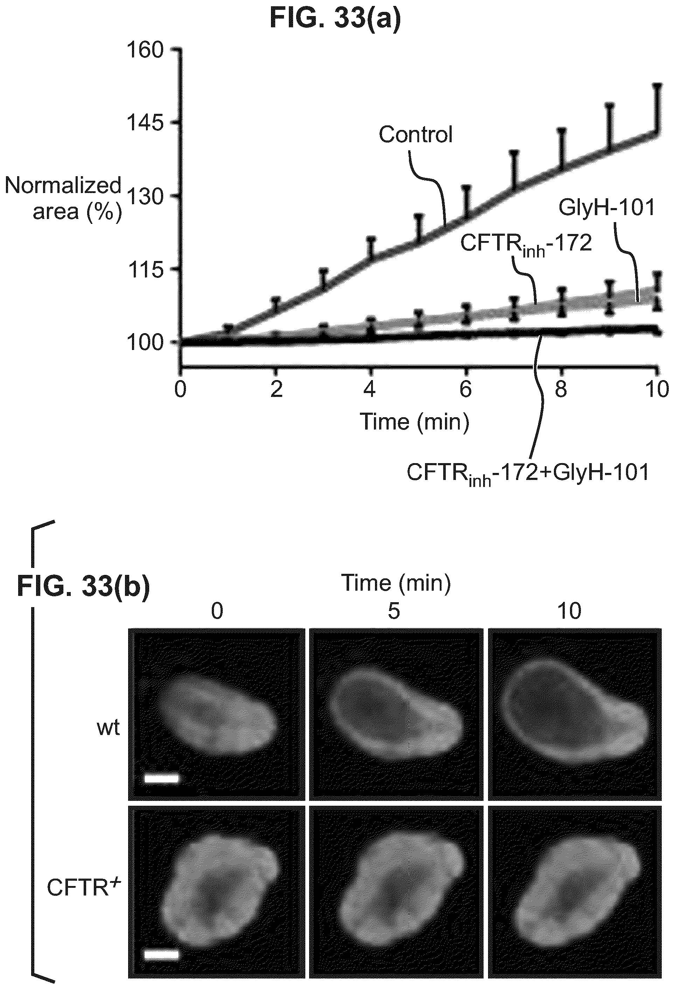

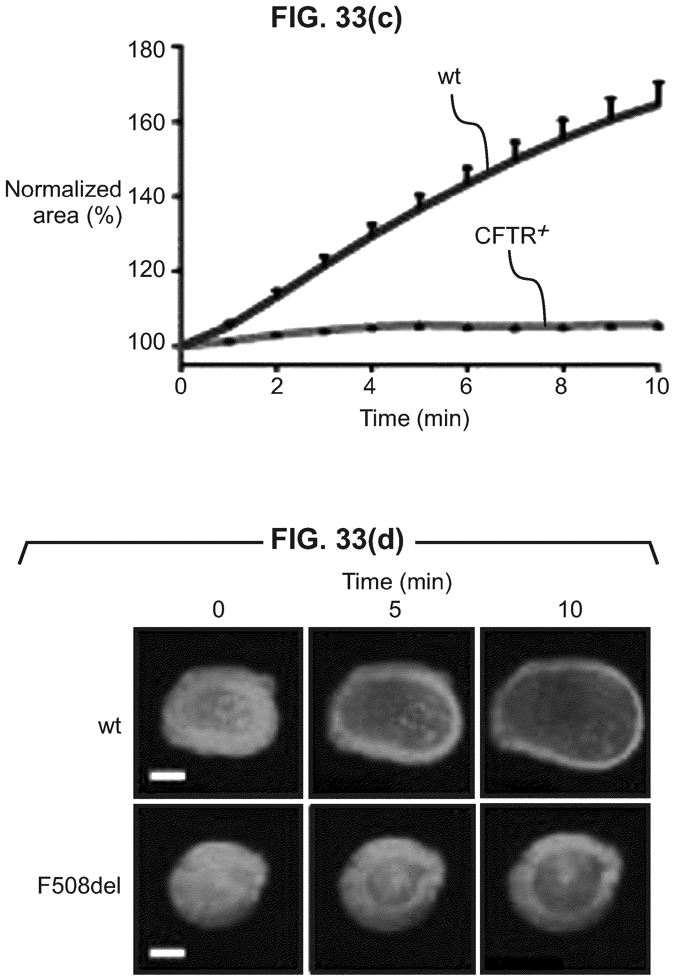

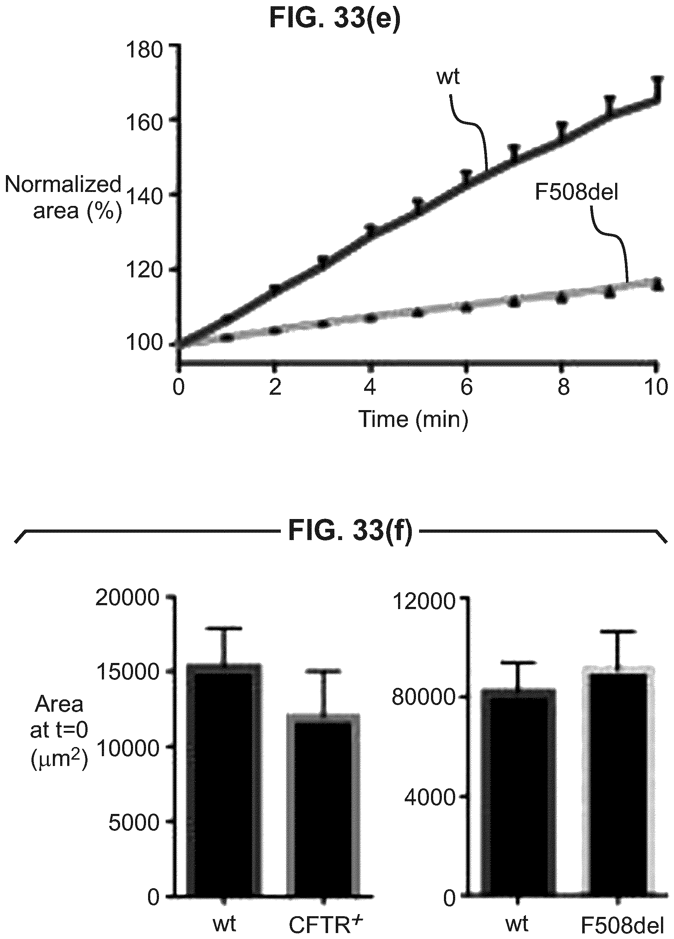

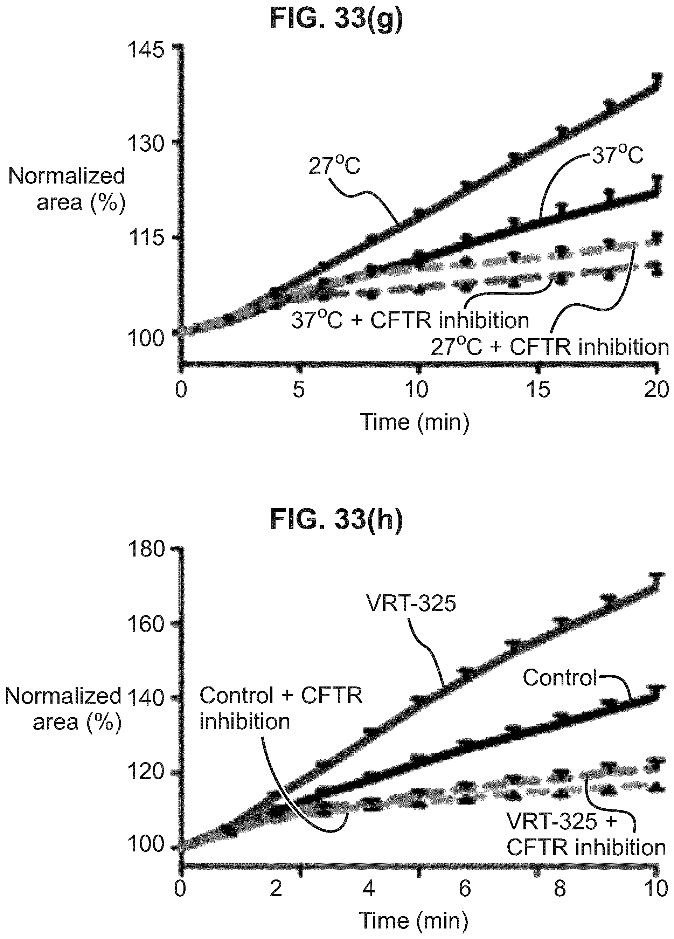

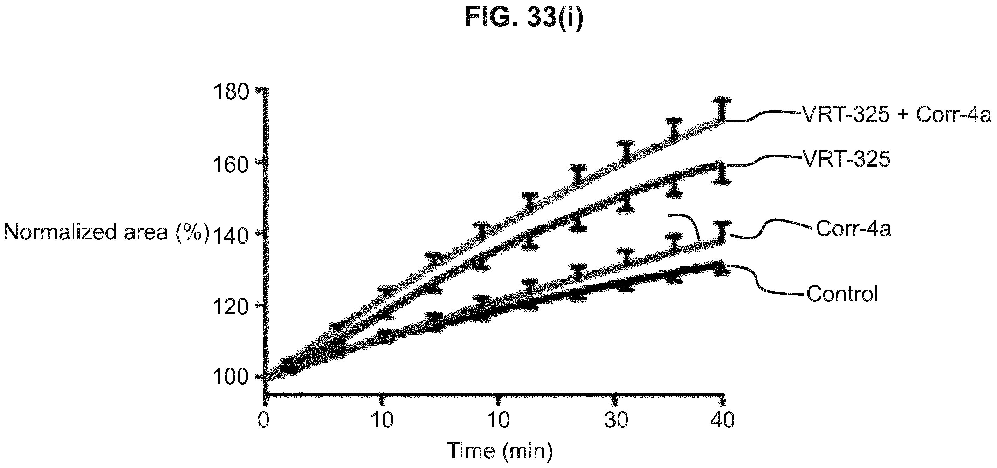

**Figure 33****. Forskolin-induced swelling of murine organoids is CFTR dependent. (a)** Normalized swelling curves of forskolin-stimulated calcein-green-labeled organoids pre-incubated with DMSO (n=8), CFTR-ᵢₙₕ172 (n=7), GlyH-101 (n=9) or both CFTR-ᵢₙₕ172 and GlyH-101 (n=11) (mean ± s.e.m.). (**b**) Representative confocal microscopy images of calcein-green labeled wild type or CFTR-deficient organoids in response to forskolin. Scale bars 50µm. **(c)** Quantification of forskolin-induced swelling in wild type (n=6) or CFTR-deficient (n=11) organoids (mean ± s.e.m.) (**d,e**) Similar to b,c but for wild type (n=8) and CFTR-F508del (n=12) organoids. Scale bars 30µm. **(f)** Absolute size of wild type or CFTR-deficient organoids quantified in (c) at t=0 (mean ± s.e.m.). **(g)** Forskolin-stimulated swelling of calcein-green labeled CFTR-F508del organoids cultured at 37°C with (n=20) or without (n=15) CFTR inhibition or cultured at 27°C for 24 hours with (n=31) or without (n=27) CFTR inhibition (mean ± s.e.m.). **(h)** Forskolin-induced swelling of calcein-green labeled CFTR-F508del organoids pre-incubated for 24 hours with DMSO with (n=15) or without (n=18) CFTR inhibition or pre-incubated with the CFTR corrector compound VRT-325 with (n=14) or without (n=26) CFTR inhibition (mean ± s.e.m.). **(i)** Normalized forskolin-induced swelling of CFTR-F508del organoids pre-treated for 24 hours with DMSO (n=16), VRT-325 (n=18), Corr-4a (n=20) or both correctors (n=20) (mean ± s.e.m.). All results are representative for at least three independent experiments.

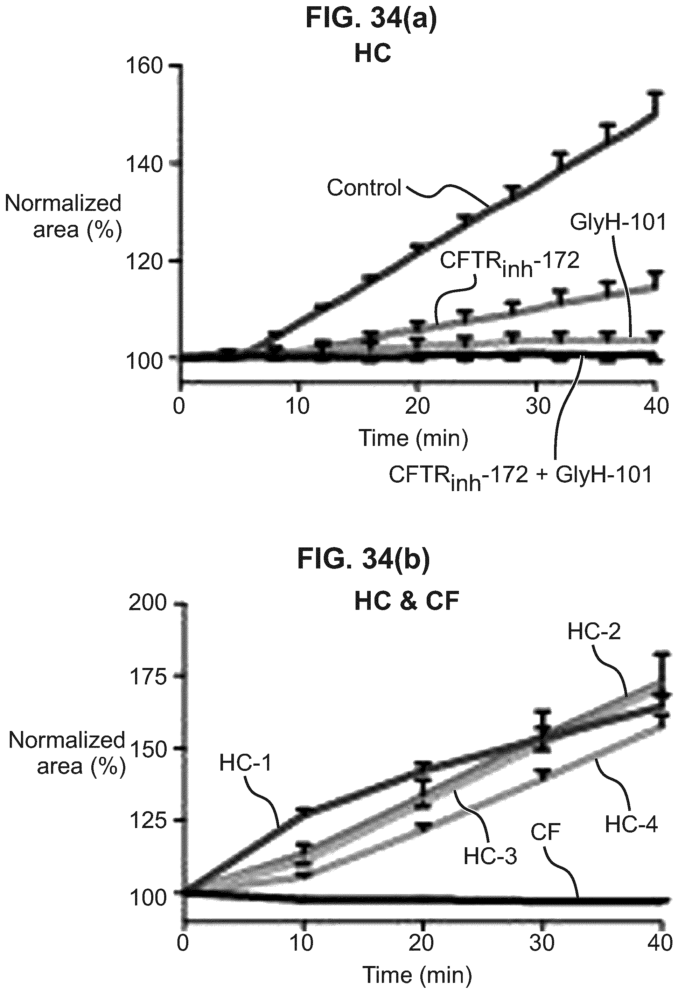

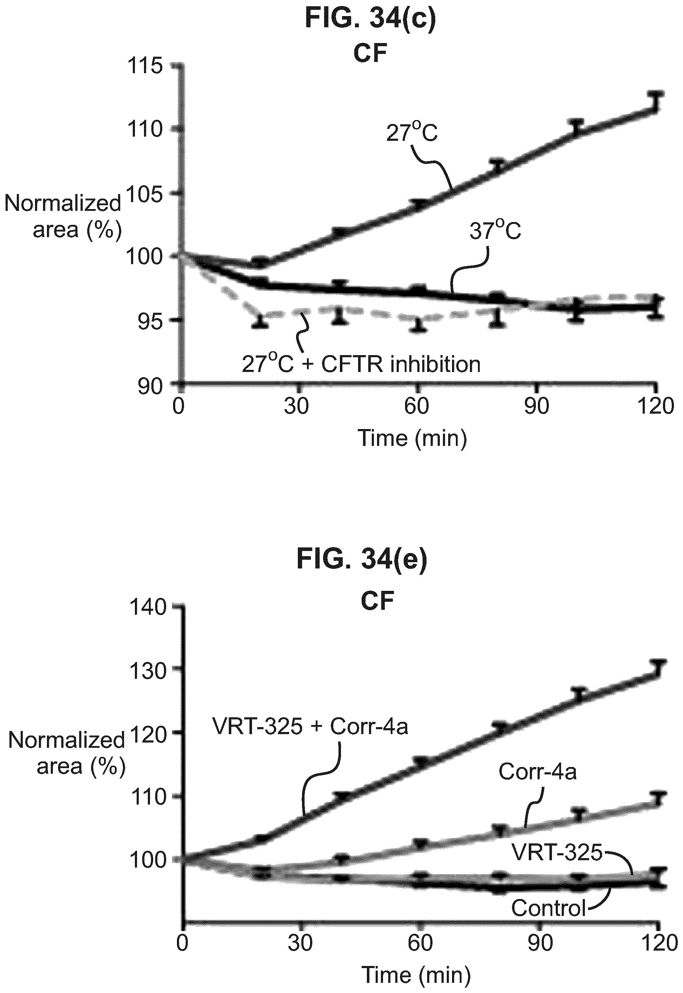

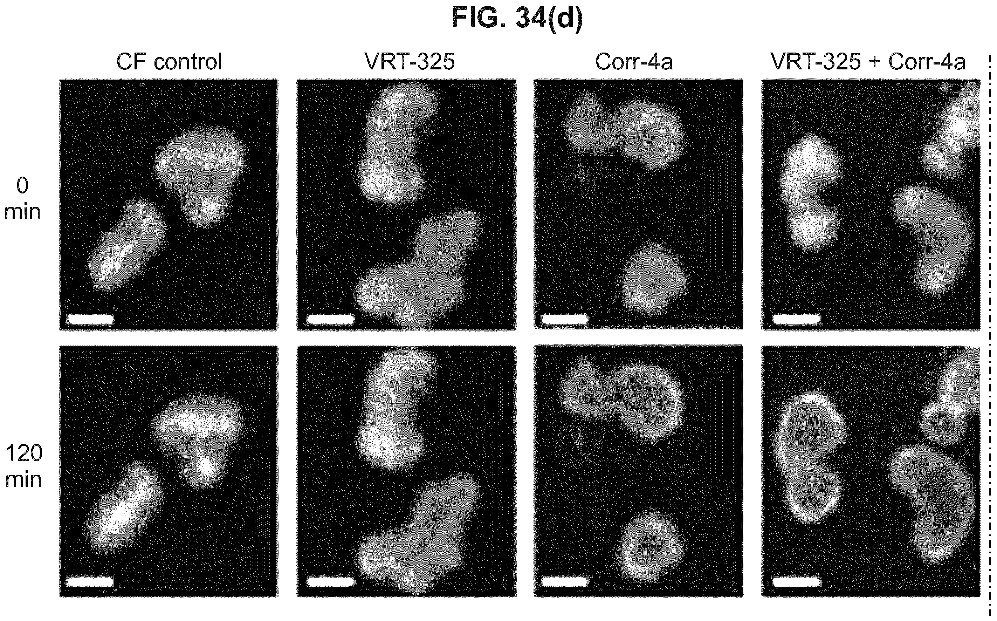

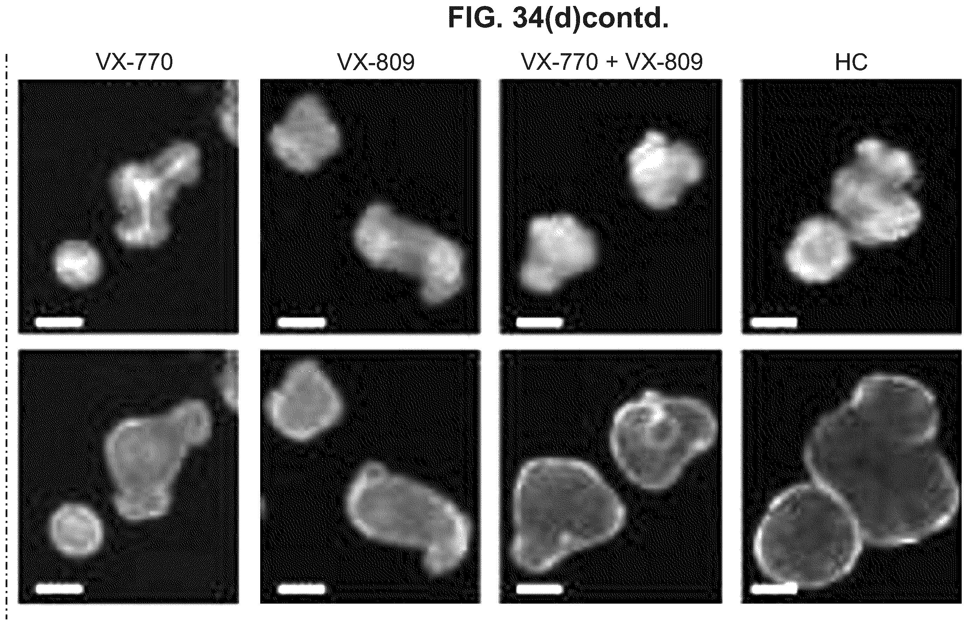

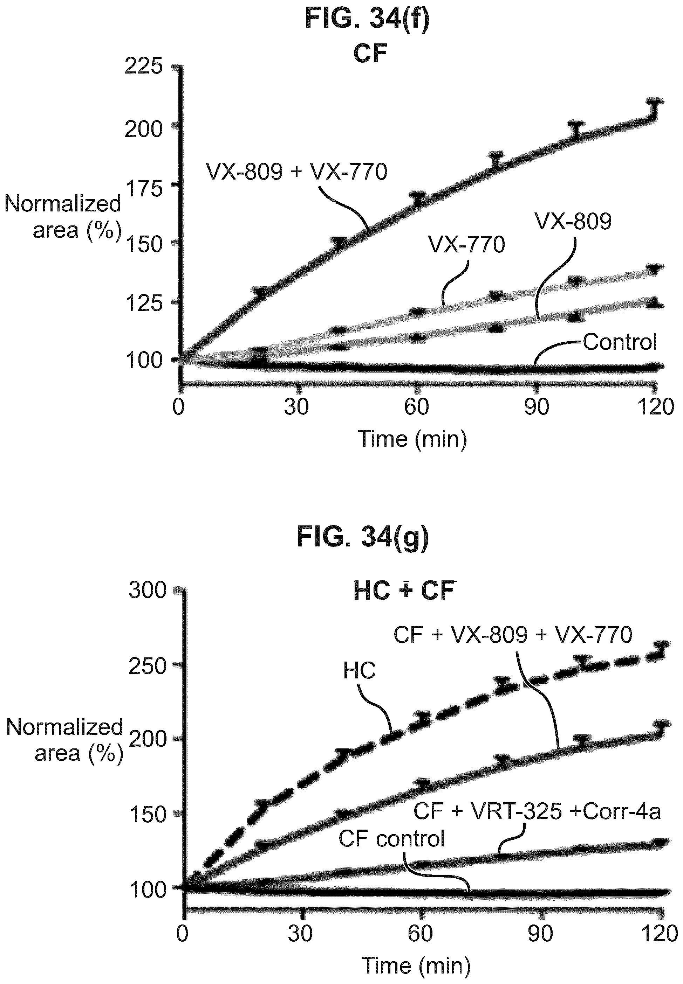

**Figure 34****. Forskolin-induced swelling in human healthy control or cystic fibrosis organoids. (a)** Quantification of forskolin-induced organoid swelling pre-incubated with DMSO, CFTRᵢₙₕ-172, GlyH-101 or both CFTRᵢₙₕ-172 and GlyH-101 (n=5, n=7, n=8, n=10) (mean ± s.e.m.). **(b)** Forskolin-stimulated swelling of organoids derived from 4 individual healthy controls (1-4: n=30, n=18, n=13, n=42) and 1 CF CFTR-F508del homozygous patient (n=30) (mean ± s.e.m.). **(c)** Normalized swelling of forskolin-induced calcein-green labeled CFTR-F508del organoids cultured for 24 at 37°C, or at 27°C with or without CFTR inhibition (n=10 for all conditions) (mean ± s.e.m.). **(d)** Representative confocal microscopy images of calcein-green labeled HC-derived or CF patient-derived organoids in response to forskolin upon pharmalogical manipulation of CFTR. Scale bars 60µm. **(e)** Normalized forskolin-induced swelling of CFTR-F508del organoids pre-treated for 24 hours with DMSO, VRT-325, Corr-4a, or both correctors (n=10 for all conditions) (mean ± s.e.m.). **(f)** CF patient-derived organoid swelling in response to forskolin with or without 24 hour pre-treatment of corrector VX-809, VX-770 stimulation (simultaneous with forskolin) or combined VX-809 and VX-770 treatment (n=10 for all conditions) (mean ± s.e.m.). **(g)** Forskolin-induced swelling of organoids upon DMSO treatment (control) or combined compound treatment from e and f, compared to HC organoids (n=10 for all conditions) (mean ± s.e.m.). Each line in (d) represents organoid swelling averaged form at least three independent experiments per individual. Results from all other figures are representative for at least three independent experiments.

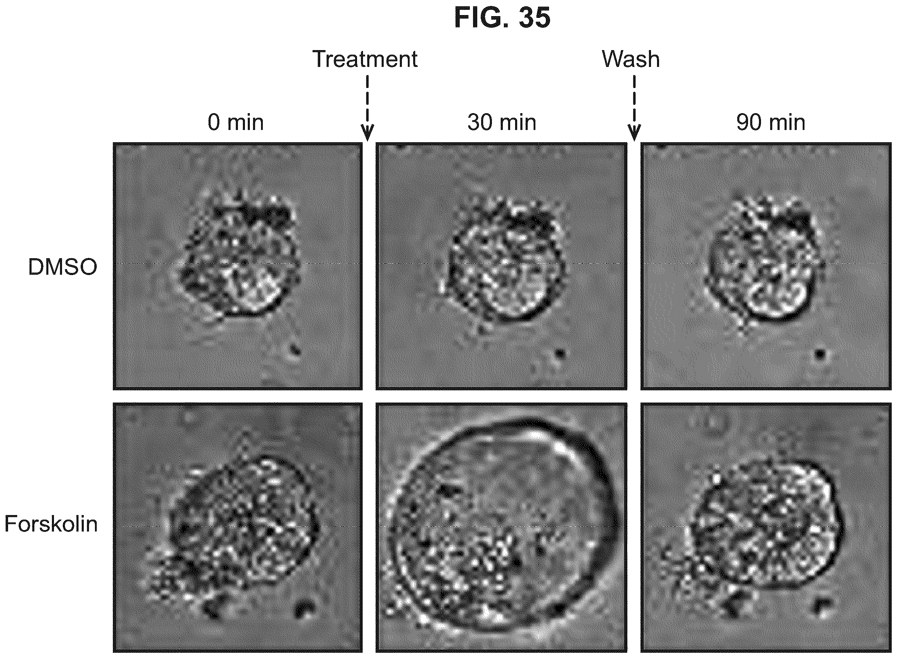

**Figure 35****.** Light microscopy analysis of wild type murine organoids stimulated with forskolin or DMSO. Representative examples for the indicated timepoints after start of stimulation are shown. The forskolin-induced swelling (FIS) of organoids was reversed upon removal of forskolin by washing.

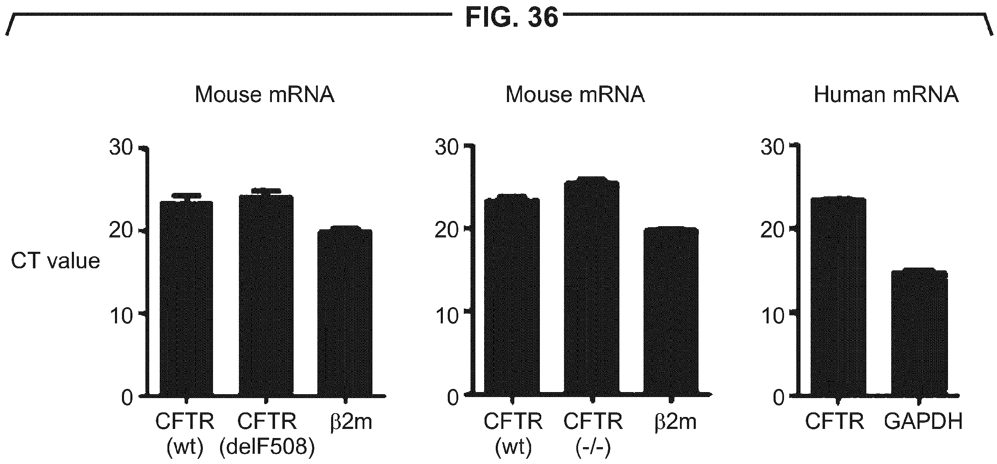

**Figure 36****.** CFTR mRNA is expressed in mouse and human organoids. The bars show real-time PCR cycle threshold (CT) values representing mRNA levels of CFTR, β2m or GAPDH isolated from CFTR-F508del (left graph) or CFTR-/- (middle graph) organoids and their corresponding wild types, or human organoids.

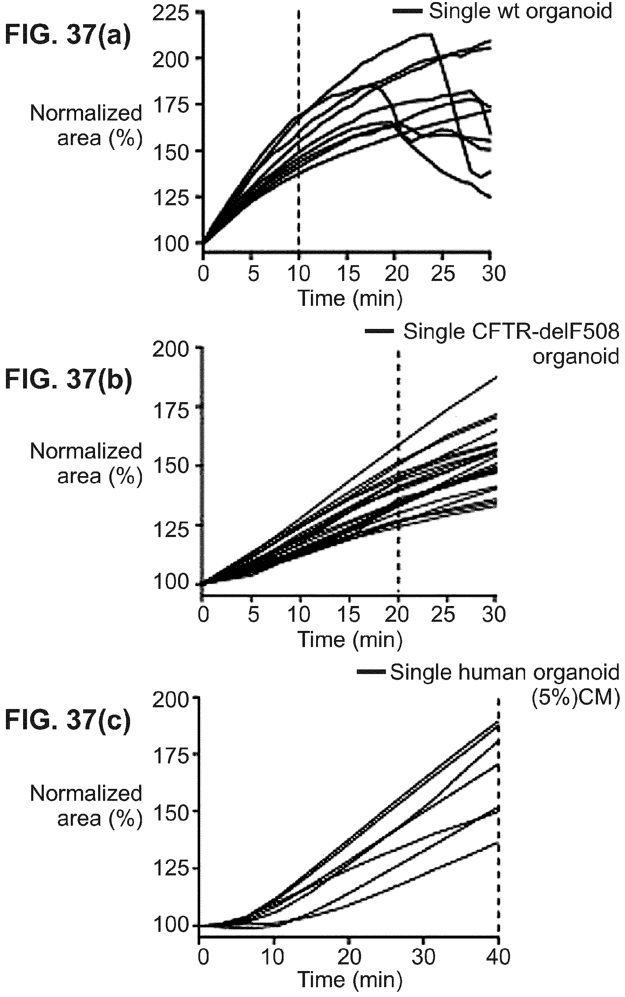

**Figure 37****.** Gradual forskolin-induced swelling prevents organoid collapse. Normalized surface area increase of individual forskolin-stimulated **(a)** wild type, **(b)** CFTR-F508del (temperature-rescued) and **(c)** human (5% Wnt3a-conditioned medium, WCM) organoids. The averaged forskolin-induced swelling of different organoid types was analyzed up to 10 (Fig. 1d,e + 2a,c,e), 20 (Fig. 2g) or 40 (Fig. 2h,i + 2a-c) minutes (dashed line).

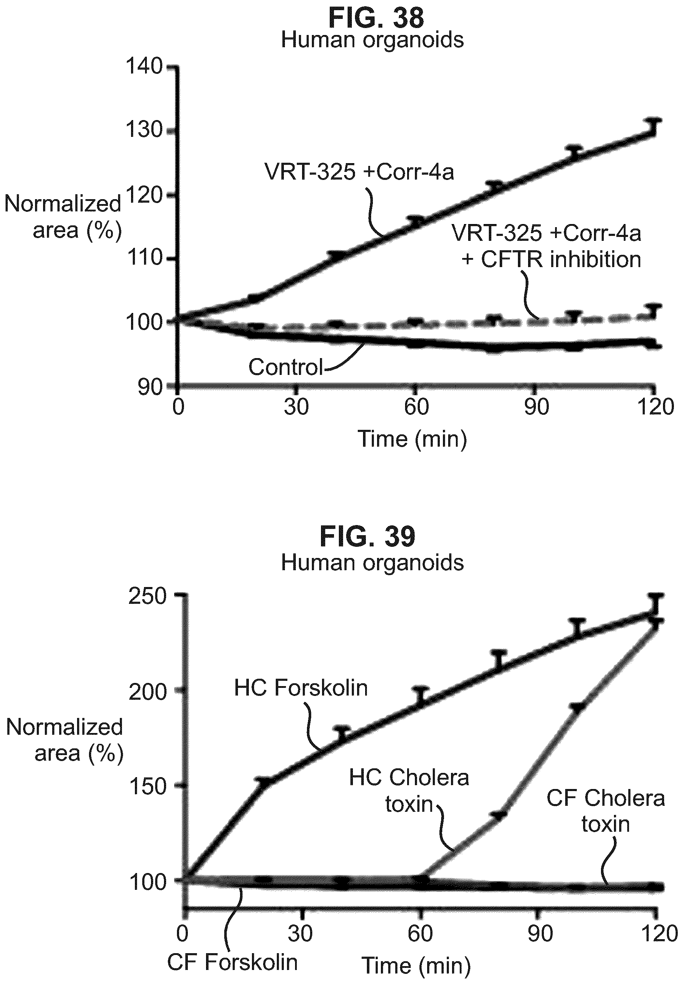

**Figure 38****.** Increased FIS by treatment of corrector compounds is CFTR dependent. Forskolin-induced swelling of calcein-green labeled human CFTR-F508del organoids pre-incubated for 24 hours with DMSO, or with both VRT-325 and Corr-4a with or without CFTR inhibition (n=10 for all conditions) (mean ± s.e.m.). Results are representative for at least three different experiments.
**Figure 39****.** Cholera toxin-induced organoid swelling in human organoids is CFTR dependent. Forskolin and cholera toxin induce swelling of HC-derived organoids, but not of CFTR-F508del organoids. The cholera toxin response is delayed compared to forskolin because of its apical extracellular function. (n=10 for all conditions) (mean ± s.e.m.). Results are representative for at least three different experiments.

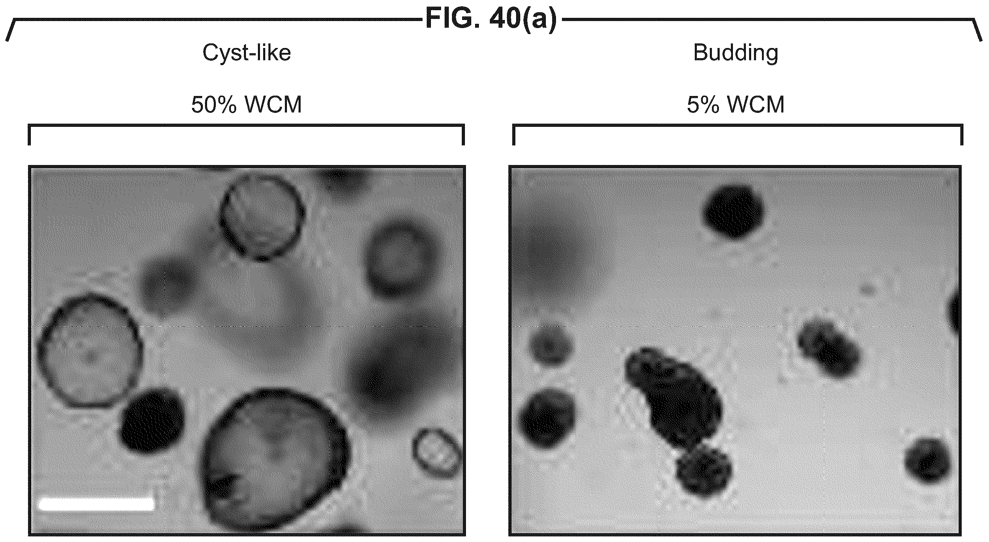

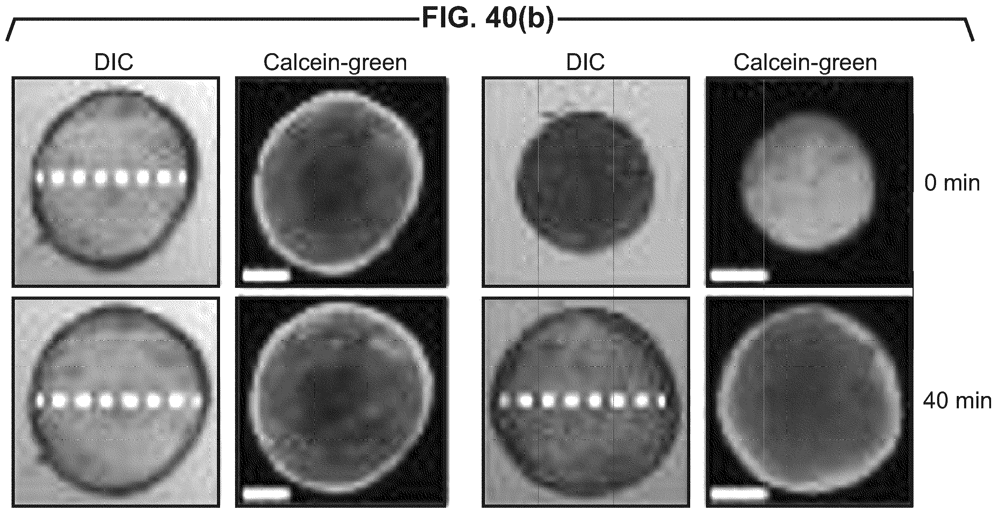

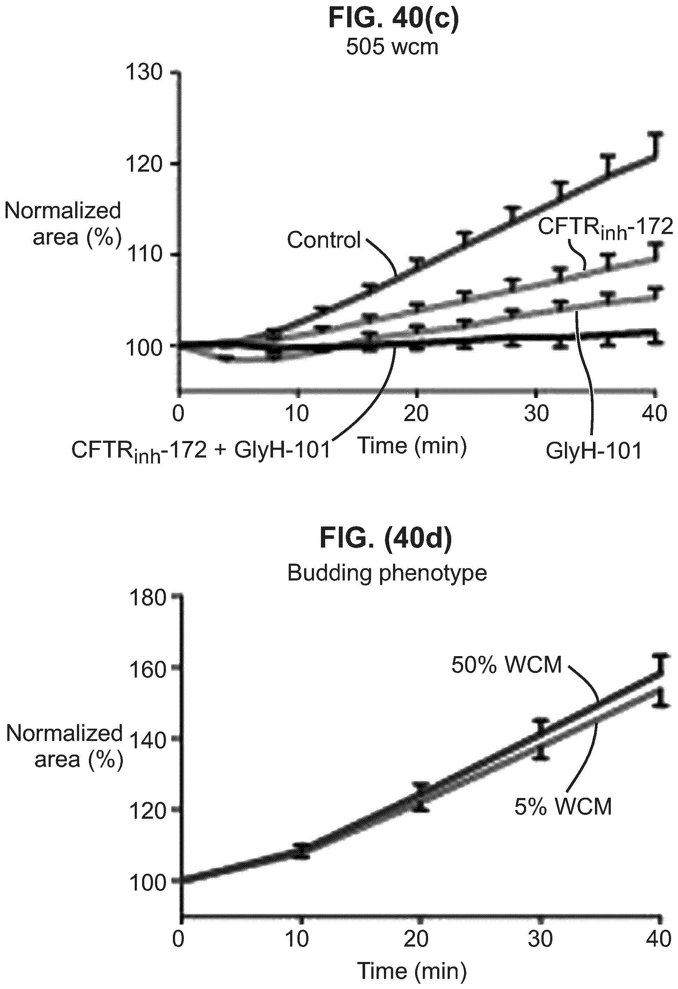

**Figure 40****.** Human organoids in normal or reduced Wnt3a culture conditions. **(a)** Light microscopy images of human organoids cultured at normal (50%, left panel) or reduced (5%, right panel) Wnt3a conditioned medium (WCM) concentrations. Scale bars 400 µm. **(b)** Representative examples of forskolin-induced swelling at normal or reduced Wnt3a conditions. Scale bars 50 µm. The dashed line depicts the internal organoid lumen **(c)** Quantification of forskolin-induced organoid swelling at normal Wnt3a levels pre-incubated with DMSO, CFTRᵢₙₕ-172, GlyH-101 or both CFTRᵢₙₕ-172 and GlyH-101 (n=29, n=41, n=26, n=15) (mean ± s.e.m.). (d) Quantification of forskolin-induced swelling of low-passage budding organoids cultured at 50% (n=9) or 5% (n=12) Wnt3a conditioned medium (WCM) concentrations averaged from two independent experiments (mean ± s.e.m.). All results are representative for at least three independent experiments.
**Figure 41****. H&E stains of prostate organoids**
   Tissue fragments of mouse prostate epithelium were embedded in matrigel. The expanding cells were split weekly. The culture can be maintained for extended period of time without loosing genetic stability or proliferation capacity. Figure 41 shows a comparison of the prostate epithelium of the prostate itself and the organoid cultures after periods of three months. The mouse prostate organoids grow in media containing ENR (EGF, Noggin and Rspondin) in the presence or absence of testosterone. Human culture requires the addition of a TGF-beta inhibitor. The H&E of fixation and embedding in paraffin demonstrates the different levels of stratification and folding of the epithelium in vivo. The cultured prostate organoids show similar diversity in folding and stratification.

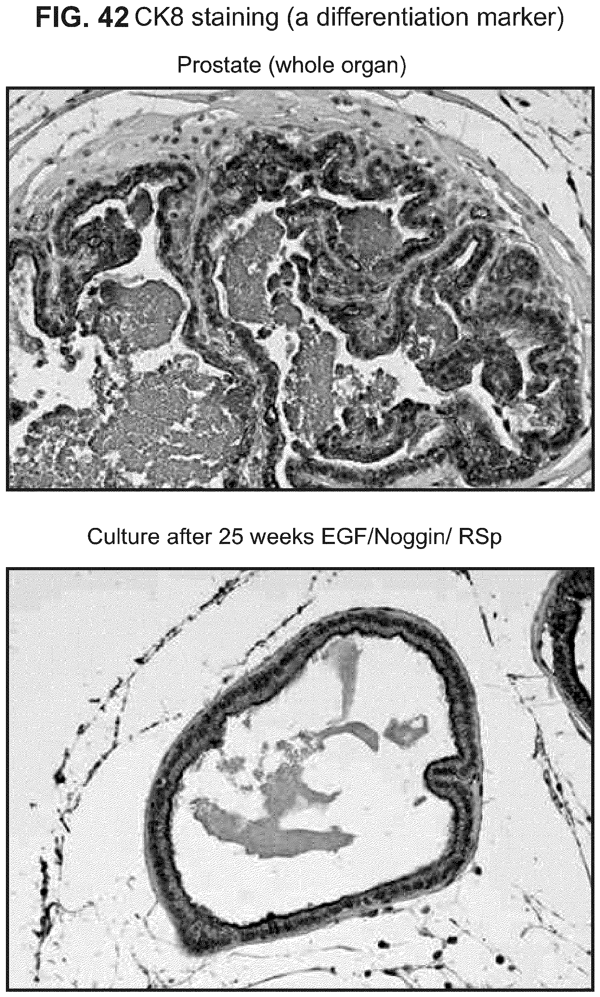

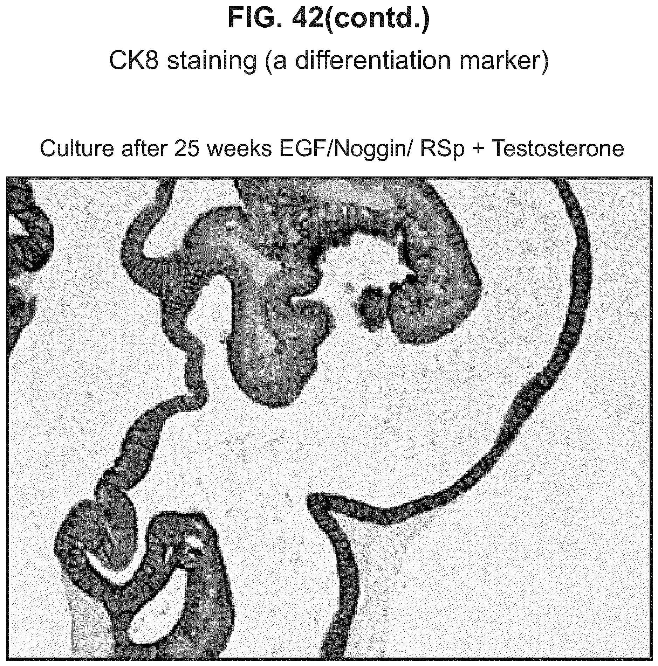

**Figure 42****. CK8 (a differentiation marker) stains of prostate organoids**
   Tissue fragments of mouse prostate epithelium were embedded in matrigel. The expanding cells expand were split weekly. The culture can be maintained for extended period of time without loosing genetic stability or proliferation capacity. Figure 42 shows the presence of CK8 expressing luminal cells. The cultures are grown in media containing ENR (EGF, Noggin and Rspondin) in the presence or absence of testosterone. Human culture requires the addition of a TGF-beta inhibitor. The addition of testosterone allows for the differentiation into CK8 positive luminal cells while at the same time stimulating stem cell maintenance and expansion. Testosterone also increases the stratification and folding of the epithelium.
**Figure 43****. Mouse prostate after 25 weeks in culture.**
   Prostate organoids grown in the EGF, Noggin, Rspondin, NAC, B27, Glutamin/max, pen/strep, Ad-DMEM/F12 + testosterone. The shape of the organoid is determined by origin of tissue (position in the prostate before isolation). The prostate consists of different lobes or regions. The different regions display specific epithelial structures (stratification and folding), After *in vitro* culturing the organoids appear to maintain the different macroscopic structure (stratified or folded) of the part of the prostate from which it originated.

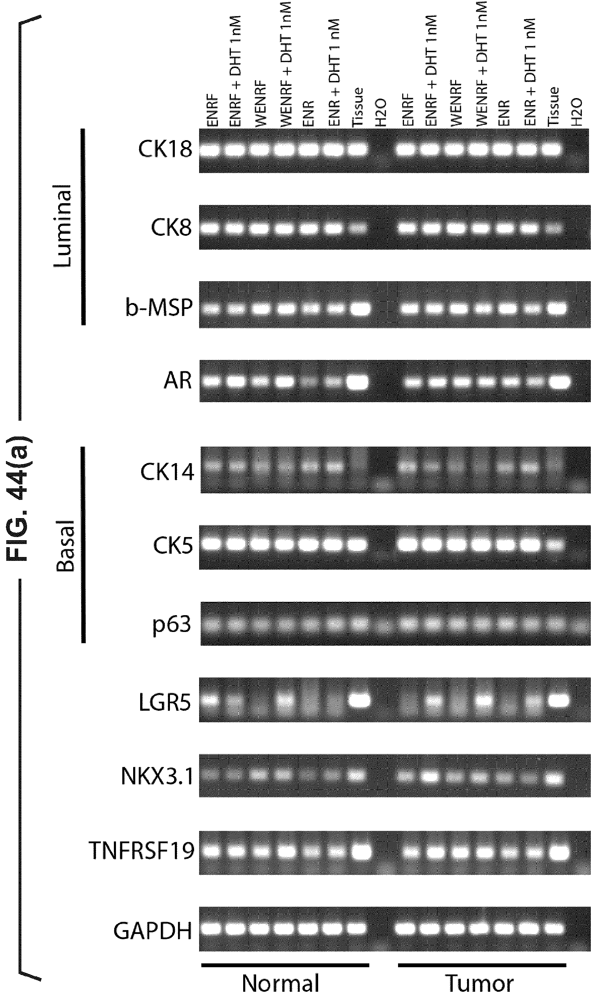

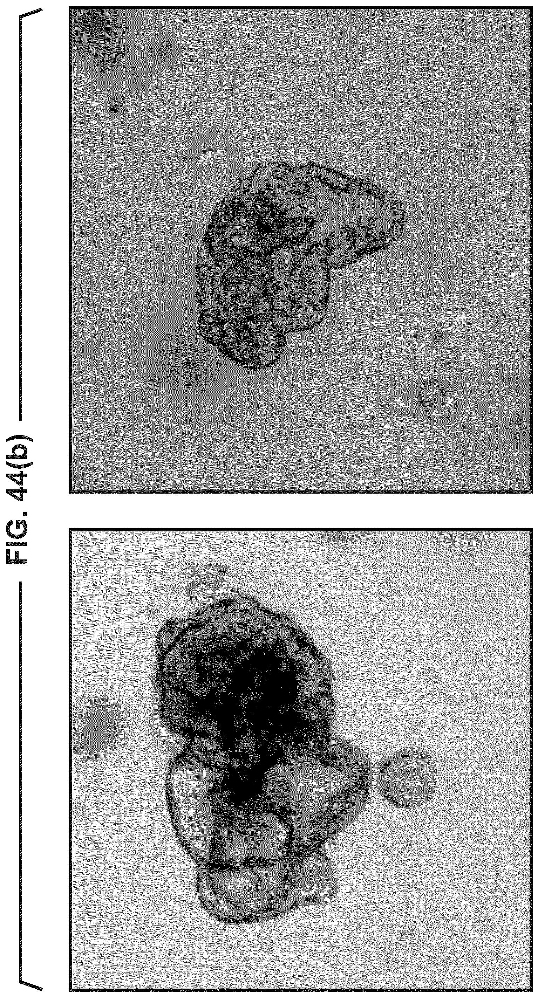

**Figure 44****. PCR of 3 week human prostate culture**
   Normal and cancerous prostatic epithelium was isolated and grown for three weeks in ENR FGF10, ENRF+DHT, WENRF, WENRF + DHT, ENR, ENR + DHT culture conditions. All culture conditions contained A83, P38i and Nicotinamide.
   Fig 44(a): RNA was isolated and RT-PCR was performed for markers of prostatic epithelium. In both normal and tumor tissue the luminal markers CK18, CK8 and B-MSP are expressed. All culture conditions express the AR. In normal tissue addition of DHT increases AR expression in all culture conditions. In tumor tissue AR expression is not influenced by DHT addition. In all culture conditions basal epithelial markers CK14, CK5 and p63 are expressed. Putative stem cell marker LGR5 is expressed under ENRF conditions in normal tissue. In tumor tissue LGR5 expression is induced with the addition of DHT in all culture conditions. TNFRSF19, also a putative stem cell marker, is expressed in all conditions in normal and tumor tissue. The prostate specific transcription factor NKX3.1 is expressed in all conditions. Addition of testosterone increases growth/ doublings while maintaining markers for the different cell type of the prostate (basal and luminal).
   Fig 44(b): Two representative pictures of human organoids grown under ENRF+ 1nM DHT conditions
      Lane 1: Line 1 ENRF Normal tissue
      Lane 2: Line 1 ENRF + 1 nM DHT Normal tissue
      Lane 3: Line 2 WENRF Normal tissue
      Lane 4: Line 2 WENRF + 1 nM DHT Normal tissue
      Lane 5: Line 3 ENR Normal tissue
      Lane 6: Line 3 ENR + 1 nM DHT Normal tissue
      Lane 7: Whole prostate Normal tissue
      Lane 8: H₂O
      Lane 9: Line 1 ENRF Tumor tissue
      Lane 10: Line 1 ENRF + 1 nM DHT Tumor tissue
      Lane 11: Line 2 WENRF Tumor tissue
      Lane 12: Line 2 WENRF + 1 nM DHT Tumor tissue
      Lane 13: Line 3 ENR Tumor tissue
      Lane 14: Line 3 ENR + 1 nM DHT Tumor tissue
      Lane 15: Whole prostate Tumor tissue
      Lane 16: H₂O
**Figure 45****. PCR of mouse prostate organoid**
   Three biologically independent lines were cultured under ENR or ENR + 1 nM DHT conditions. RNA was isolated and RT-PCR was performed for markers of prostatic epithelium. In both culture conditions luminal prostate markers Cytokeratin 18 (CK18) and Cytokeratin 8 (CK8) are broadly expressed. Androgen Receptor (AR) is expressed in both conditions. Basal markers p63 and Cytokeratin 5 (CK5) are expressed in both culture conditions, but upon addition of DHT basal markers are downregulated. Putative stem cell markers Lgr5 and Tnfrsf19 are downregulated upon addition DHT. However under these conditions stemness is maintained while differentiated cells are also present. These conditions allow unlimited cell expansion (for now 9 months at population doublings 2,5 a week). All cultures are positive for the prostate specific marker Nkx3.1. Addition of testosterone increase growth/ doublings up to 3 fold while maintain markers for the different cell type of the prostate (basal and luminal).
   Lane 1: Line 1 ENR
   Lane 2: Line 1 ENR + 1 nM DHT
   Lane 3: Line 2 ENR
   Lane 4: Line 2 ENR + 1 nM DHT
   Lane 5: Line 3 ENR
   Lane 6: Line 3 ENR + 1 nM DHT
   Lane 7: Whole mouse prostate
   Lane 8: H₂O
**Figure 46****: Stomach (gastric) organoids**
   Human stomach organoids. Tissue was isolated from the corpus. The cells were culture in the stomach organoid medium (EGF, Noggin, Rspondin, Wnt, Nicotinamide, FGF10, Gastrin, TGF-beta inhibitor (A8301). Cells are split weekly.
   Fig 46(a): This picture was taken after 2 months of culturing.
   Fig 46(b): H&E and different antibody staining after fixation and paraffin sectioning of a culture after 2 weeks of culturing. It shows the presence of the following cells: PAS for Mucin producing cells; Muc5Ac for Surface mucous pit cells; Muc6 for mucous neck cells. The H&E stain shows a single layer of polarized epithelium.

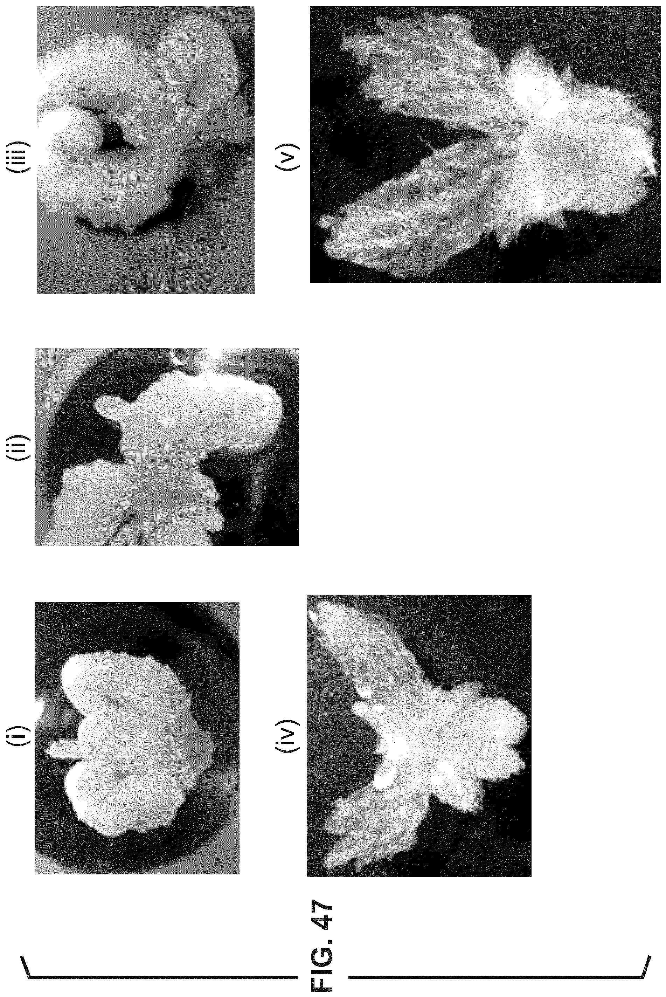

**Figure 47****: Isolation of prostatic tissue**
   See example 8 (Photos from UCSF)
**Figure 48****:** A) Mouse organoids were cultured in the presence of different doses recombinant mouse RANKL for 72h. mRNA expression levels for RANK, the transciption factor SpiB and the M cell-specific markers GP2 and AnnexinV were determined by qPCR. B) Confocal analysis of GP2 (see arrow) and AnnexinV (see arrow) expression in mouse organoids cultured with 100ng/ml RANKL for 72h.
**Figure 49****:** Human organoids were cultured in the presence of different doses of recombinant human RANKL for 7 days. mRNA expression levels for RANK, SipB and the M cell-specific marker GP2 were determined by qPCR. EM: Expansion medium; DM: differentiation medium.

### EXAMPLE 1:

To address the need for improved culture media and methods for human epithelial stem cells, the inventors investigated signalling pathways that are known to be subverted in certain cancers e.g. colorectal cancer. It was hypothesised that these pathways, which affect cell fate in cancer, may also play a role in determining cell fate under *in vitro* cell culture conditions.

In a first screening experiment, a series of vitamins, hormones and growth factors were tested in combination with standard stem cell culture media. Gastrin and nicotinamide were identified as resulting in significantly improved culture conditions. Incorporating these factors into the standard culture conditions, a second screening experiment was performed, in which certain small molecule inhibitors related to relevant signalling pathways, such as ERK, p38, JNK, PTEN, ROCK, and Hedgehog, were tested. In the present state of the art, there would be no reasonable way to predict what the outcome of each of these additional compounds would be on the culture medium properties.

**Table 2: List of reagents used for optimization of human intestinal organoids culture**

| First screening (WENR**) | | | | |
|---|---|---|---|---|
| | Description | Source | Concentration | Activity* |
| Hormones, vitamins etc | | | | |
| Hydrocortison | | Sigma | 500nM | 0 |
| **Gastrin***** | | **Sigma** | **1uM** | **1+** |
| Exendin4 | GLP1 analog | Sigma | 100nM | 0 |
| **Nicotinamide** | **Vitamin B derivative** | **Sigma** | **10mM** | **3+** |
| L-Ascorbic acid | Vitamin C | Sigma | 10uM | 0 |
| anti-oxidant mixture | | Sigma | 1x | 0 |
| Lipid mixture | | Sigma | 1x | 0 |
| PGE2 | | Sigma | 10uM | 1+ (Cystic) |
| Cholera Toxin | | Sigma | 100nM | 1+ (Cystic) |

| Growth factors | | | | |
|---|---|---|---|---|
| BDNF | | Peprotech | 100ng/ml | 0 |
| GDNF | | Peprotech | 100ng/ml | 0 |
| FGF2 | | Peprotech | 100ng/ml | 0 |
| FGF10 | | Peprotech | 100ng/ml | 0 |
| Follistatin | | Peprotech | 100ng/ml | 0 |
| Cyr61 | | Peprotech | 1ug/ml | 0 |
| LIF | | Millipore | 1000U/ml | 0 |
| | | | | |

| Second screening (WENR+gastrin+Nicotinamide) | | | | |
|---|---|---|---|---|
| Small molecule inhibitors | | | | |
| PD98059 | ERK inhibitor | Sigma | 10uM | 1- |
| SB203580 | p38 inhibitor | Sigma | 1-10uM | 2+ |
| **SB202190** | **p38 inhibitor** | **Sigma** | **1-10uM** | **2+** |
| SP600125 | JNK inhibitor | Sigma | 10uM | 0 |
| PS48 | PDK1 activator | Sigma | 5uM | 0 |
| Y27632 | ROCK inihibitor | Sigma | 10uM | 1+ cystic |
| Cyclopamine | Hedgehog inhibitor | Sigma | 100nM | 1- |
| 5 Azacytidin | DNA methylase inhibitor | Stemolecule | | 1- |
| Dorsomorphin | BMP inhibitor | Stemolecule | | 0 |
| **A83-01** | **ALK4,5,7 inhibitor** | **Tocris** | **50n-1uM** | **3+** |
| VO-OHpic trihydrate | PTEN inhibitor | Sigma | 500nM | 3- |
| Pifithrin-α | p53 inhibitor | Sigma | | 0 |
| BIX01294 | G9a HMTase inhibitor | Stemolecule | | 1- |
| *Activity scale (plating efficiency was compared with control after 4 days culture): | | | | |
| 0 = no change; 1+ = <50% increase; 2+ = 50-100% increase; 3+ = >100% increase; | | | | |
| 1- = 0-50%; 2- = 50-100% decrease; 3- = >100% decrease. | | | | |
| ** WENR comprises EGF+Noggin+R-spondin+Wnt-3a | | | | |
| *** Highlighted in bold are the compounds which showed the greatest improvement to the culture medium. | | | | |

In summary, the inventors have established long-term culture conditions under which single crypts or stem cells derived from murine small intestine (SI) expand over long periods of time. Growing crypts undergo multiple crypt fission events, whilst simultaneously generating villus-like epithelial domains in which all differentiated cell types are present. The inventors have now adapted the culture conditions to grow similar epithelial organoids from mouse colon and human SI and colon. Based on the murine small intestinal culture system, the inventors optimized the murine and human colon culture system. They found that addition of Wnt3A to the growth factor cocktail allowed mouse colon crypts to expand indefinitely. Further addition of nicotinamide, a small molecule Alk inhibitor and a p38 inhibitor was preferable for long-term human SI and colon culture. The culture system also allowed growth of murine *Apc^{min}* adenomas, human colorectal cancer and human esophageal metaplastic Barrett's epithelium. The culture technology should be widely applicable as a research tool for infectious, inflammatory and neoplastic pathologies of the human gastrointestinal tract. Moreover, regenerative applications may become feasible with *ex vivo* expanded intestinal epithelia.Self-renewal of the small intestinal and colonic epithelium is driven by the proliferation of stem cells and their progenitors located in crypts. Although multiple culture systems have been described (Evans GS et al. J Cell Sci 1992;101 ( Pt 1):219-31; Fukamachi H. J Cell Sci 1992;103 ( Pt 2):511-9; Perreault N & Jean-Francois B. Exp Cell Res 1996;224:354-64; Whitehead RH et al. Gastroenterology 1999;117:858-65), only recently have long-term culture systems become available that maintain basic crypt physiology. Two different protocols were published which allow long-term expansion of murine small intestinal epithelium. Kuo and colleagues demonstrated long-term growth of small fragments containing epithelial as well as stromal elements in a growth factor-independent fashion (Ootani A et al. Nat Med 2009;15:701-6). The inventors designed a culture system for single stem cells by combining previously defined insights in the growth requirements of intestinal epithelium. Wnt signalling is a pivotal requirement for crypt proliferation (Korinek V et al. Nat Genet 1998;19:379-83; Pinto D et al. Genes Dev 2003;17:1709-13; Kuhnert F et al. Proc Natl Acad Sci U S A 2004;101:266-71) and the Wnt agonist R-spondin1 induces dramatic crypt hyperplasia *in vivo* (Kim KA et al. Science 2005;309:1256-9). Second, EGF signalling is associated with intestinal proliferation (Dignass AU & Sturm A. Eur J Gastroenterol Hepatol 2001;13:763-70). Third, transgenic expression of Noggin induces expansion of crypt numbers (Haramis AP et al. Science 2004;303:1684-6). Fourth, isolated intestinal cells undergo anoikis outside the normal tissue context (Hofmann C et al. Gastroenterology 2007;132:587-600). Since laminin (α1 and α2) is enriched at the crypt base (Sasaki T et al. Exp Cell Res 2002;275:185-), the inventors explored laminin-rich Matrigel to support intestinal epithelial growth. Matrigel-based cultures have successfully been used for growth of mammary epithelium (Stingl J et al. Breast Cancer Res Treat 2001;67:93-109). Under this culture condition (R-spondin1, EGF, and Noggin in Matrigel), the inventors obtained ever-expanding small intestinal organoids, which displayed all hallmarks of the small intestinal epithelium in terms of architecture, cell type composition and self-renewal dynamics.

Despite extensive efforts, long-term adult human intestinal epithelial cell culture has remained difficult. There have been some long-term culture models, but these techniques and cell lines have not gained wide acceptance, possibly as a result of inherent technical difficulties in extracting and maintaining viable cells (Rogler G et al. Scandinavian journal of gastroenterology 2001;36:389-98; Buset M et al. In vitro cellular & developmental biology: journal of the Tissue Culture Association 1987;23:403-12; Whitehead RH et al. In vitro cellular & developmental biology: journal of the Tissue Culture Association 1987;23:436-42; Deveney CW et al. The Journal of surgical research 1996;64:161-9; Pang G et al. Gastroenterology 1996;111:8-18; Latella G et al. International journal of colorectal disease 1996;11:76-83; Panja A. Laboratory investigation; a journal of technical methods and pathology 2000;80:1473-5; Grossmann J et al. European journal of cell biology 2003;82:262-70). Encouraged by the establishment of murine small intestinal culture, the inventors aimed to adapt the culture condition to mouse and human colonic epithelium. The inventors now report the establishment of long-term culture protocols for murine and human colonic epithelium, which can be adapted to primary colonic adenoma/adenocarcinoma and Barrett's esophagus.

### Results

### Establishment of a mouse colon culture system

In an attempt to establish a mouse colon culture system, the inventors explored our small intestinal culture condition (here termed ENR: EGF+Noggin+R-spondin). In our experience, initial growth of colon epithelium is often observed under the ENR culture condition, but is invariably abortive. Organoid formation was studied using epithelium isolated from the distal part of the mouse colon. Under ENR conditions, the plating efficiency of single distal colonic crypts was much lower than that of small intestine (1-3% vs >90%) and these organoids could not be passaged. Recently, the inventors have shown that Paneth cells produce several Wnt ligands (Gregorieff A et al. Gastroenterology 2005;129:626-38), and that the production of Wnt by these Paneth cells is essential to maintain intestinal stem cells (Sato T et al. Nature;469:415-8). To determine the Wnt signalling status in colon organoids, the inventors cultured colon crypts from *Axin2-lacZ* mice, (a faithful Wnt reporter) (Lustig B et al. Mol Cell Biol 2002;22:1184-93) or *Lgr5-GFP* knock-in mice (Lgr5 being a Wnt-dependent stem cell marker)(Barker N et al. Nature 2007;449:1003-7).

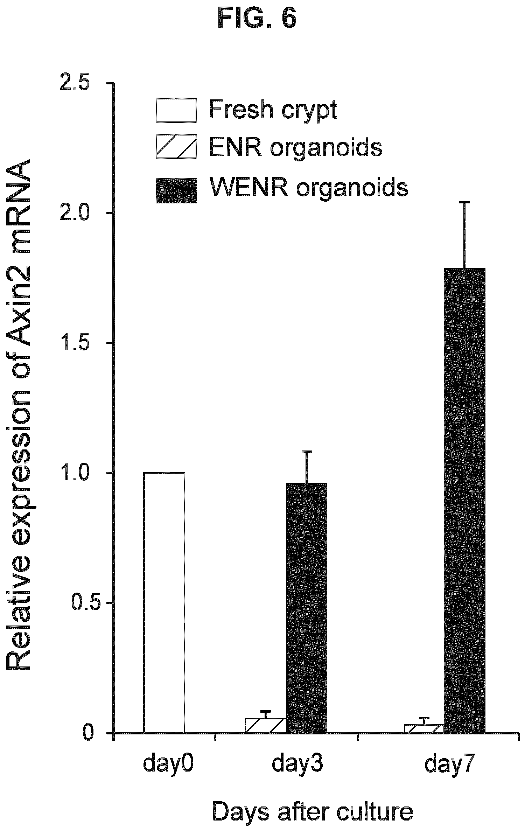

Freshly isolated colon crypts readily expressed Axin2-LacZ or Lgr5-GFP at their bottoms, but they lost expression of the Wnt reporters shortly after initiation of culture (**Figure 1a****,****b** and **Fig. 6****).** By contrast, small intestinal organoids constitutively expressed the Wnt reporters at their budding structures (Sato T et al. Nature;469:415-8; Sato T et al. Nature 2009;459:262-5). These findings suggested that colon organoids produce insufficient amounts of Wnt ligands to maintain colon stem cells. To overcome this, the inventors added recombinant Wnt3a or Wnt3a-conditioned medium to ENR culture medium (WENR medium). This increased plating efficiency of crypts in the order of 10-fold. Colon crypts formed organoids structures with numerous Axin2-LacZ (**Figure 1a**) or Lgr5-GFP+ (**Figure 1b**) buds, implying that Wnt activation was restored. Freshly isolated colon crypts contain fully mature cells in their upper parts, and the inventors reasoned that these mature cells may interfere with organoid growth. When the inventors mildly digested colon crypts into small clusters of cells, thus physically separating proliferative crypt bottoms from differentiated upper crypt regions, most of fragments derived from crypt top died, yet cell clusters from colon crypt base efficiently formed organoids (**Figure 1c**).

Mouse small intestinal epithelium grown under ENR conditions generates all differentiated epithelial cell types concomitant with stem cell self-renewal. The inventors have shown previously that the addition of Wnt3A to these cultures interferes with intestinal differentiation and yields organoids that largely consist of undifferentiated progenitors (Sato T et al. Nature;469:415-8). This is not unexpected given the central role of Wnt signalling in the maintenance of the undifferentiated crypt progenitor state (van de Wetering M et al. Cell 2002;111:241-50). Consistent with this observation, colonic organoids in WENR condition failed to differentiate properly. Upon withdrawal of Wnt-3A, the inventors observed differentiation along all epithelial lineages **(****Figure 1d****-f).** Of note, single sorted Lgr5+ colonic epithelial stem cells can form organoids when cultured in the presence of Y-27632 for the first two days.

### Establishment of human colon culture system

Encouraged by the success of the improved mouse colon crypt culture, the inventors applied the culture condition to human colon crypts. Although these crypts initially survived, most subsequently disintegrated within 7 days. To increase the plating efficiency of human colon crypts, the inventors screened candidate growth factors, hormones and vitamins (list in Fig 12).

Among these, the inventors found that gastrin and nicotinamide (Precursor of NAD⁺, and found to suppress Sirtuin activity (Denu JM. Trends Biochem Sci 2005;30:479-83)) improved culture efficiency **(****Fig 12****).** The effect of gastrin on plating efficiency was marginal. However, the hormone did not interfere with intestinal differentiation and we decided to include gastrin (hereafter shortened to 'g') in all human intestinal culture conditions. Importantly, nicotinamide (10 mM) was essential for prolongation of culture period beyond the initial 7 days **(****Figure 2a).** Under this culture condition, human colonic organoids could be expanded for at least 1 month. From 1 month onward, the colonic organoids changed their morphology from budding organoids structure into cystic structures **(****Figure 2b** **left).** Coinciding with the morphological conversion, proliferation progressively decreased. Occasionally, cystic organoids regained their proliferative potential. However, all organoids eventually arrested growth within 3 months. A two-phase growth arrest has been observed in other primary culture systems, such as mammary epithelial cells or keratinocytes, and has been referred to as mortality stage 1 (M1; senescence) and mortality stage 2 (M2; crisis) (Shay et al., 2006). Multi-lineage differentiation was not observed in the human intestinal organoids cultured in this condition even after the withdrawal of Wnt (data not shown).

The inventors assumed that growth arrest occurred because of inadequate culture conditions rather than a cell-intrinsic property of senescence/replicative aging. The inventors therefore extended our attempts to optimized the culture condition. The inventors screened various small molecule modulators of MAP kinases, of signaling molecules mutated in colon cancer, and of histone modifiers **(****Fig 12****)** under the WENR+gastrin+nicotinamide culture condition. The inventors found that two small molecule inhibitors, A83-01 (Alk4/5/7 inhibitor; nM) and SB202190 (p38 inhibitor; 10 uM) significantly improved the plating efficiency. Other TGF-beta receptor 1 (ALK 5) inhibitors that were also tested and showed the same results as A83-01 were LY364947, SB431542, SB505124. It would be expected that other ALK inhibitors would also work in the same way. Furthermore, the combination of the two compounds synergistically prolonged the culture period. The inventors demonstrated that all of ten tested samples expanded for at least 6 months with weekly 1:5 split. Under this culture condition, the human colonic organoids displayed budding organoid structures, rather than the cystic structures seen under the previous culture condition **(****Figure 2b****).** The proliferating cells were confined to the buds **(****Figure 2c****).** Metaphase spreads of organoids more than 3 months old consistently revealed 46 chromosomes in each cell (20 cells each from three different donors; **Figure 2d****).** The inventors sequenced the whole exome (all exons) of the colon organoids after two months in culture. The number of mutations in the organoids was extremely low. In fact in four parallel organoid cultures originating from one clone, only one mutation was found which was present in all cultures and therefore likely originated from the parental tissue.

These results implied that Alk receptor and p38 signalling negatively regulate long-term maintenance of human intestinal epithelial cells. The inventors refer to the optimized culture condition as the HISC (Human intestinal stem cell culture) condition.

### Human intestinal organoids mimic in vivo differentiation

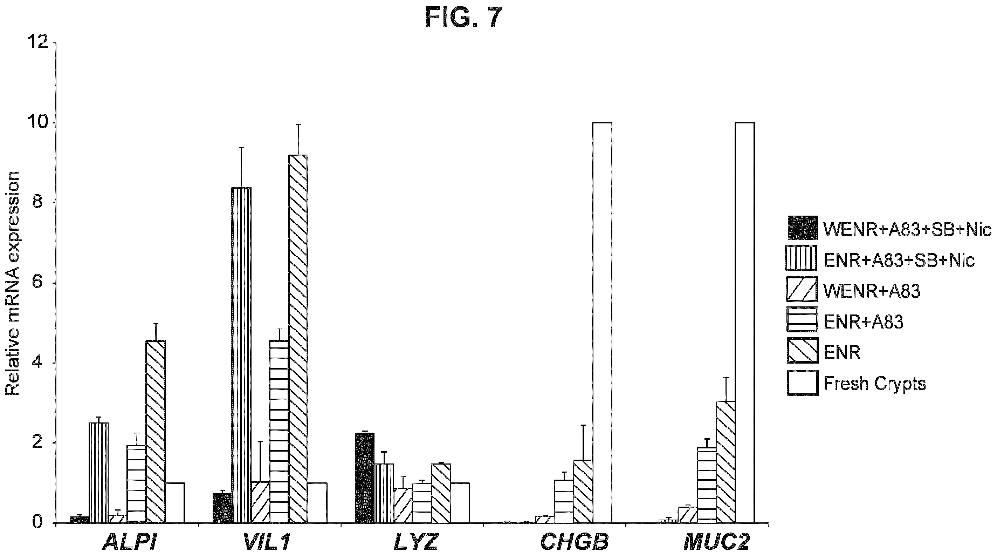

Under the HISC condition, the inventors failed to observe differentiated cells. As was seen in the mouse colon organoids, withdrawal of Wnt was required for mature enterocyte differentiation in human organoids **(****Figure 3a** **top panel and** **Fig.7****).** However, goblet and enteroendocrine cell differentiation remained blocked. We found that Nicotinamide and SB202190 strongly inhibited this differentiation, while withdrawal of the two reagents enabled the organoids to produce mature goblet and enteroendocrine cells **(****Figure 3a** **(middle and bottom panel), 3b and** **Fig. 7****.** The same differentiation inhibitory effects of Wnt, Nicotinamide and SB202190 were observed in human small intestinal organoids. Lysozyme+ Paneth cells were observed in small intestinal organoids, but not in colonic organoids **(****Figure 3d****).** It has been reported that p38 inhibitor treatment *in vivo* inhibits goblet cell differentiation and increases intestinal epithelial proliferation (Otsuka M. Gastroenterology 2010;138:1255-65, 1265 e1-9). Indeed, the inventors observed the same phenotype in the p38 inhibitor treated intestinal organoids **(****Figure 3d** **vs. e).** The inventors further examined the response of human intestinal organoids to Notch-inhibition. The inventors have previously shown that Notch inhibition with either γ-secretase inhibitors (dibenzazepine; DBZ) or by conditional targeting of the Notch pathway transcription factor CSL depleted intestinal stem cells, terminated intestinal epithelial proliferation and induced goblet cell hyperplasia *in vivo* (van Es JH et al. Nature 2005;435:959-63). Indeed, upon treatment with DBZ, the intestinal organoids ceased their proliferation and most cells converted into goblet cells within 3 days **(Figure 3g** vs **f).**

### Establishment of APC-deficient adenoma and colon adenocarcinoma

Recently, the inventors reported efficient mouse intestinal adenoma formation from Lgr5 stem cells in *Lgr5-GFP-ires-CreERT2 x APC*^{*flox*/*flox*} mice upon Tamoxifen-induced Cre activation (Barker N et al. Genes Dev 2008;22:1856-64). The inventors isolated the intestinal adenomas 10 days after induction and optimized the culture condition. The adenomas efficiently formed cystic organoid structure without budding. Since APC loss constitutively activates the Wnt pathway, the inventors expected that R-spondin1 would become dispensable for adenoma organoid growth. This was indeed observed. Furthermore, Noggin, which is essential for long-term culture of normal small intestine, was dispensable in adenoma organoids. Interestingly, the inventors observed a loss of Lgr5-GFP but not Axin2-LacZ in adenomatous organoids 7 days after withdrawal of Noggin **(****Figure 4a****,****b** and data not shown). Similar observations were made for normal intestinal organoids when grown in ER-medium (Sato T et al. Nature 2009;459:262-5). This indicated that Noggin, most likely through inhibition of BMP signals, is required to maintain Lgr5 expression, but is not required for expansion of adenoma organoids. Freshly isolated Lgr5^{hi} (but not Lgr5^{low}) cells isolated from intestinal crypts can initiate organoid growth *in vitro* (Sato T et al. Nature 2009;459:262-5). To determine the existence of a similar Lgr5-hierarchy within adenomas, the inventors isolated Lgr5-GFP^{hi} , GFP^{low} and GFP^{-ve} cells from EN-cultured organoids and examined their organoid formation ability. After a 7 day culture, Lgr5-GFP^{hi} showed the highest organoid-forming efficiency. Yet, Lgr5-GFP^{low} or ^{-ve} also formed organoids with considerable efficiency **(****Figure 4c****).** Of note, sorted GFP^{-ve} adenoma cells could give rise to Lgr5-GFP^{hi} organoids **((****Fig. 8****)).**

Many colorectal cancer cell lines have been isolated over the past four decades. Typically, such cell lines emerge as rare, clonal outgrowths after primary cultures of colon tumors enter tissue-culture crisis. Currently, no robust culture system exists which allows the consistent culture of primary human colon cancer samples without culture crisis and the consequent clonal outgrowth of culture-adapted cells. As a next step, the inventors applied intestinal adenoma culture conditions to human colorectal cancer samples. As expected, colon cancer cells required neither R-spondin nor Noggin. EGF was dispensable in most colon cancer organoids, while some colon cancer organoids decelerated their proliferation after withdrawal of EGF. Distinct from mouse intestinal adenoma, colorectal cancer organoids in the culture condition grew as irregular compact structures rather than as simple cystic structures **(****Figure 4d****).**

The inventors examined the proliferation/differentiation status of adenoma and colon cancer organoids. As expected, most of cells were Ki67+. Consistent with the strong inhibitory effect of Wnt on enterocyte differentiation **(****Figure 1f** **and** **Fig. 7****),** alkaline phosphatase staining was not observed in both types of organoids **(****Fig. 9****).** In contrast, we occasionally observed PAS+ goblet cells and chromogranin A+ endocrine cells in adenoma organoids and in some colon cancer organoids **(****Fig. 9****).**

### Culturing human metaplastic Barrett's epithelium

Barrett's Esophagus is marked by the presence of columnar epithelium in the lower esophagus, replacing the normal squamous cell epithelium as a result of metaplasia (Odze RD. Nat Rev Gastroenterol Hepatol 2009;6:478-90). The histological hallmark of Barrett's Esophagus is the presence of intestinal goblet cells in the esophagus. Exploiting the similarity between Barrett and intestinal epithelium, the inventors subjected small Barrett's epithelium (BE) biopsies to the human colon culture condition. Under these culture conditions, normal esophageal squamous cells transiently proliferated for 1 week, but the organoids could not be passaged. Barrett's Esophagus epithelium could be maintained for up to 1 month under HISC conditions **(****Figure 5a****).** The BE organoids formed cystic organoid structures indistinguishable from that of senescent human colon organoids, and typically underwent growth arrest 1 month after the culture. Addition of FGF10 to the HISC condition enabled the BE organoids to form budding structures and significantly prolonged the culture duration (> 3 months) **(****Figure 5** **b****,** **c****).** In contrast to human intestinal organoids, BE organoids remained Ki67+ with a minimal number of PAS+ and Mucin+ cells 4 days after withdrawal of Nicotinamide and SB202190. Treatment with the γ-secretase inhibitor DBZ (10 uM) for 4 days after the withdrawal blocked proliferation and induced goblet cell differentiation **(****Fig.5d-g).** This supported our previous suggestion that local delivery of such inhibitors may represent a useful therapeutic strategy for the removal of Barrett's Esophagus lesions by differentiation therapy (Menke V et al. Disease models & mechanisms 2010;3:104-10). Of note, we occasionally observed Lysozyme+ Paneth cells **(****Fig. 10****),** which indicates that BE organoids preserve multilineage differentiation.

### Discussion

The protocols developed here allow robust and long-term culture of primary human epithelial cells isolated from small intestine, colon, adeno(carcino)mas and Barrett's Esophagus (table 3).

**Table 3: List of components of the organoid culture systems**

| **Reagent name** | **Supplier** | **Cat No.** | **Solvent** | **Stock solution** | **Final conc.** |
|---|---|---|---|---|---|
| Matrigel, GFR, phenol free | BD bioscience | 356231 | | | |
| Advanced DMEM/F12 | Invitrogen | 12634-028 | | | |
| GlutaMAX-I | Invitrogen | 35050-079 | | 200 mM | 2 mM |
| HEPES 1M | Invitrogen | 15630-056 | | | 10 mM |
| Penicillin/Streptomycin | Invitrogen | 15140-122 | | 10000/10000 U/ml | 100/100 U/ml |
| N2 supplement | Invitrogen | 17502-048 | | 100x | 1x |
| B27 supplement | Invitrogen | 17504-044 | | 50x | 1x |
| N-Acetylcysteine | Sigma-Aldrich | A9165-5G | DW | 500 mM=81.5 mg/ml | 1 mM |
| EDTA | Sigma-Aldrich | 431788-25g | DW | 500 mM=14.6g/100ml | 2 mM |
| Mouse recombinant noggin | Peprotech | 250-38 100ug | PBS/BSA | 100 mg/ml | 100ng/ml |
| mouse recombinant EGF | Invitrogen | PMG8043 | PBS/BSA | 500 mg/ml | 50 ng/ml |
| human recombinant R-spondin | Nuvelo | | PBS/BSA | 1 mg/ml | 1 mg/ml |
| human recombinant FGF10 | Peprotech | 100-26 | PBS/BSA | 100 mg/ml | 100 ng/ml |
| mouse recombinant Wnt-3A | Millipore | GF-160 | PBS | 10 mg/ml | 100 ng/ml |
| Y-27632 | Sigma-Aldrich | Y0503 | PBS | 10 mM=1g/338 ml | 10 mM |
| A-83-01 | Tocris | 2939 | DMSO | 500 mM | 500 nM |
| SB202190 | Sigma-Aldrich | S7067 | DMSO | 30 mM | 10 mM |
| Nicotinamide | Sigma-Aldrich | | DW | 1M | 10 mM |
| [Leu15]-Gastrin I | Sigma-Aldrich | G9145 | PBS/BSA | 100 mM | 10 nM |
| DNase | Sigma-Aldrich | DN25-1g | PBS | 200000 U/ml | 2000 U/ml |
| TrypLE express | Invitrogen | 12605-036 | | | |
| Collagenase type XI | Sigma-Aldrich | C9407 | | | |
| Dispase | Invitrogen | 17105-041 | | | |
| 70um Cell strainer | BD falcon | 352350 | | | |

| | | | | | |
|---|---|---|---|---|---|
| All stock solutions and aliquoted Matrigel are stored in -20°C | | | | | |

In contrast to murine small intestine, murine colonic epithelial cells require Wnt ligand in the culture medium. The inventors have previously reported that CD24^{hi} Paneth cells produce Wnt-3/11, which are essential for stem cell maintenance in small intestine (Sato T, et al. Nature 2011;469:415-8). Wnt-6 and -9b mRNA are expresses at the bottom of colon crypts (Gregorieff A, et al. Gastroenterology 2005;129:626-38.). It remains undetermined whether this local Wnt production by colon crypt base cells is sufficient to activate canonical Wnt signal *in vivo* or there is another source of Wnt ligand in colon mucosa. The difference between human and mouse intestinal organoid culture conditions was unexpectedly large. A83-01 inhibits ALK4/5/7, receptors that are detected in both murine and human crypts by microarray. The inventors are currently investigating the mechanism by which ALK signal regulates human organoid growth. The inventors have not observed cellular transformation in long-term cultures and no chromosomal changes become obvious under the optimized culture conditions. Furthermore, the organoids can undergo a considerably higher number of cell division than reported for other adult human epithelial culture system (Dey D et al. PloS one 2009;4:e5329; Garraway IP et al. The Prostate 2010;70:491-501). It is generally believed that somatic cells are inherently limited in their proliferative capacity, a phenomenon called replicative aging (Walen KH. In vitro cellular & developmental biology. Animal 2004;40:150-8). Most normal human cells are believed to count the number of times they have divided, eventually undergoing a growth arrest termed cellular senescence. This process may be triggered by the shortening of telomeres, and the consequent activation of DNA damage signals (M1), or telomere attrition (M2). In the absence of the two small molecule kinase inhibitors, human intestinal organoids underwent growth arrest after 10-20 population doublings. By contrast, the replicative capacity in the optimized culture condition was extended at least up to 100 population doublings upon addition of the inhibitors, which exceeded the Hayflick limit (Hayflick L. The Journal of investigative dermatology 1979;73:8-14). This result clearly indicates that the senescent phenotype seen in the first culture system reflects inadequate growth conditions, rather than inherent replicative aging.

The culture techniques can be used to study basic aspects of stem cell biology and the control of differentiation, exemplified by depletion of stem cells and goblet cell differentiation upon Notch inhibitor treatment. Moreover, the organoid culture platform may be used for pharmacological, toxicological or microbiological studies on pathologies of the intestinal tract, as the organoids represent more closely the intestinal epithelium than often-used colon cancer cell lines such as CaCo2 or DLD1. Lastly, since small biopsies taken from adult donors can be expanded without any apparent limit or genetic harm, the technology may serve to generate transplantable epithelium for regenerative purposes.

### EXAMPLE 2 - Culturing mouse pancreatic organoids

The use of a TGF-beta inhibitor was also tested in a culture medium for mouse pancreatic organoids. The expansion medium that was used was DMEM/F12 media (supplemented with P/S, Glutamax, 10mM Hepes, B27, N2 and N-Acetylcysteine), EGF (50ng/ml), R-spondin (10%), Noggin (100ng/ml), FGF10 (100ng/ml), A8301(TGF-beta inhibitor, 500nM) and Gastrin (10µM). This differs slightly from that of the above-described HISC culture used in Example 2 in that there is no Wnt agonist (other than Rspondin) or Nicotinamide and FGF10 is added. However, these culture media share a number of key components (ENR + gastrin + TGF-beta inhibitor), the addition of the TGF-beta inhibitor being advantageous in both cases. Pancreas organoids grown in these conditions could be expanded for > 3months and passaged at least 5 times.

Microarray experiments were carried out for the pancreas organoids grown in the above-described expansion medium and the results were compared to the adult pancreas, adult liver and newborn liver (see figure 16A). The pancreas organoid clearly clusters with the adult pancreas, rather than with the liver samples, demonstrating a good phenotypic similarity with the adult pancreas.

Figure 16B shows the raw signal from the microarray experiment comparing expression levels in pancreas organoids, adult pancreas, adult liver and liver organoids for ductal markers, endocrine markers and transcription factors necessary for Ngn3 expression (Ngn3 is a transcription factor that is associated with the specification of endocrine lineages). The high levels of expression of Krt19, Krt7 and other ductal markers in the pancreas organoids, show that the pancreas organoids clearly have a ductal phenotype. These pancreatic organoids were originally grown from ductal preparations. The essential transcription factors for Ngn3 expression (Foxa2, Hnf6, Hnflb, Sox9) were all also expressed in the pancreas organoids, although expression of Ngn3 itself was not detected under expansion conditions.

The expression levels of genes important for the generation of insulin-producing cells are low. However, it is clear that in the expansion medium, proliferation and expression patterns of the pancreatic organoids closely resemble those seen in early progenitor endocrine cells.

The pancreas is mainly formed by three different cell types: acinar cells, ductal cells and endocrine cells. In a total RNA sample of adult pancreas, 90% of the RNA comes from acinar cells, so the expression levels of endocrine markers are very diluted in a total pancreas sample. Therefore, further experiments are planned for each specific cell type. For example, the inventors plan to carry out a microarray comparison between pancreas organoids, enriched acinar cell preparation, enriched ductal cell preparation and enriched endocrine cell preparation, to have a better estimation of the mRNA levels of the important genes in our pancreas organoids compared with the levels present in insulin producing cells. For example, in an enriched endocrine cell sample, 75-85% of the cells present would be insulin-secreting cells).

### EXAMPLE 3 - The effect of Noggin on the Expansion Medium

To investigate the role of the BMP inhibitor, Noggin, in the expansion medium, the inventors compared mRNA levels of early endocrine markers and ductal markers in pancreatic organoids that have always been cultured in EGFRA medium so have never been cultured in the presence of Noggin with the level of expression of the same markers in organoids that have always been cultured in EGFRAN medium (i.e. always in the presence of Noggin). The inventors also compared mRNA levels of these markers in pancreatic organoids from which Noggin was added or removed from the cultures respectively. Specifically, one sample of pancreatic organoids was cultured in EGFRA medium and then Noggin was added and the organoids were cultured for a further 2 or 4 days. Another sample of pancreatic organoids was cultured in EGFRAN medium and then Noggin was removed and the organoids were cultured for a further 2 or 4 days. The gene expression was compared and the results are shown in Figure 17A. It was found that Noggin reduces the expression of keratin 7 and keratin 19 (ductal markers) showing that Noggin blocks the differentiation towards the ductal phenotype (the keratin levels in white and dark grey samples are lower than in the black samples). Expression levels of some transcription factors essential for the generation of insulin producing cells (i.e. Sox9, Hnf6, Hnf1a, Pdx1, Nkx2.2, Nkx6.1 and Hnf1b) were unaffected by Noggin. Although Noggin prevents the cultures from acquiring a full ductal phenotype, which will likely prevent future differentiation to insulin producing cells, the inventors include Noggin in the expansion medium because it allows the cells to expand whilst maintaining some ductal features in combination with features of insulin-producing precursor cells.

The effect of the presence or absence of Noggin, or its addition or withdrawal to EGFRA medium on Lgr5 gene expression was assessed using pancreatic organoids obtained from pancreatic ducts. The results in Figure 17B show that pancreas organoids cultured with Noggin express 2 fold more Lgr5 than pancreas organoids cultured without Noggin (compare white bar second from left with black bar on left). Addition (dark grey) or withdrawal (light grey) of Noggin was also shown to affect Lgr5 levels. It is unclear whether the increase in Lgr5 gene expression in the presence of Noggin is due to an increased number of Lgr5+ cells or due to an increased level of Lgr5 expression per cell. However, the present inventors show here that BMP inhibitors, such as Noggin, promote expression of Lgr5 and, therefore, result in more proliferative organoids. Thus, BMP inhibitors are shown to be an advantageous component of the expansion media.

This is surprising, because in the literature it is described that BMP activity is useful for

differentiation culture of pancreatic cells. This conclusion is based on the observations that BMP signalling is required for the differentiation into both the ductal (see keratin7 and 19 expression) and endocrine cells. Thus, the skilled person would expect the inclusion of a BMP inhibitor, such as Noggin, to be disadvantageous in an expansion medium. However, the inventors surprisingly found that the use of a BMP inhibitor was advantageous because it resulted in more proliferative organoids and higher expression of Lgr5.

### EXAMPLE 4 - Transplantation of Human pancreatic organoids under the kidney capsule in mice

Pancreatic organoids, that had been expanded using the protocol described in example 1 (see Figure 18A), were transplanted under the renal capsule of immunodeficient mice.

Just before transplantation, organoids were treated with cell recovery solution (BD#354253, BD Biosciences) to get rid of matrigel residues. Organoids were washed several times with PBS and pelleted.

Transplantation of these organoids under the renal capsule of immunodeficient recipients was carried out using an NIH recommended procedure for islet transplantation under the kidney capsule ("Purified Human Pancreatic Islets, In Vivo Islets Function", Document No. 3104, A04, Effective Date 7th July 2008, DAIT, NIAID, NIH). A week before the transplantation, hyperglycemia was chemically induced in the recipient mice (NOD/SCID/IL2RgammaKO a.k.a. NSG) with a high dose 130mg/kg streptozotocin injection. Blood glucose levels were monitored and mice having a blood glucose above 18mmol/l were considered hyperglycaemic.

For transplantion, the hyperglycemic recipient was anesthetized and a small incision was made in the left flank to expose the left kidney. Approximately 2.5 - 3.0 mm³ of organoids were collected in a siliconized PE50 transplantation tube and transplanted under the kidney capsule using a Hamilton syringe. After cauterizing the damaged capsule the kidney was placed back into the abdominal cavity. The peritoneum and the skin were then closed with 5-0 silk sutures.

One mouse was sacrificed three hours post-transplantation and the graft was analyzed for mature beta cell and progenitor markers. In this mouse, no insulin-producing cells could be seen in the murine peri-renal capsule (Figure 18B).

A further mouse was allowed to recover in the cage with a heat pad, under close supervision. Bodyweights and blood glucose levels of the transplanted mouse were monitored for 1 month. After one month the mouse was sacrificed and the graft was analyzed for mature beta cell and progenitor markers.

1 month after transplantation, a number of insulin-producing cells could be identified. These insulin-producing cells are all the stained cells in Figure 18C, a selection of which are circled for enhanced clarity. In particular, insulin-positive cells appeared from the ductal lining, whereas no insulin-positive cells were seen in initial preparations.

The finding that the insulin producing cells are present 1 month after transplantation but are not present 3 hours after transplantation demonstrates that the insulin producing cells largely or only arise after transplantation.

These results show that cells taken from pancreatic organoids of the present invention, cultured with the media and methods of the present invention, can be transplanted into mice and can promote the growth of insulin-producing cells in the pancreas. Excitingly, human pancreatic organoids could be transplanted. This opens a number of exciting possibilities for using transplanted organoid cells to promote insulin production e.g. for treatment of diabetes.

### EXAMPLE 5 - Liver organoid culture comprising TGF-beta inhibitor

Under ER or ENRW conditions liver organoid cultures self-renew, and can be maintained and expanded in a weekly basis, for up to 1 year (figure 20A). The karyotypic analysis after 1 year shows no evidence of chromosomal aberrations. More than 66% of the cells analysed presented normal chromosomal counts and 13% of them also showed polyploidy, a characteristic trait of hepatocytes (Figure 20B).

The combination of EGF (50 ng/ml) and R-spondin 1 (1ug/ml) supplemented with FGF10 (100ng/ml), HGF (25-50ng/ml) and Nicotinamide (1-10mM), were preferable for the long term maintenance of the cultures. Under these conditions, we obtained long-lived cell cultures that express biliary duct and some hepatoblast or immature-hepatocyte markers (Glul, Albumine). However, the number of cells positive for these hepatocyte markers was very low. Under these culture conditions, no mature hepatocyte markers (e.g. p450 Cytochromes) were detected. These results suggest that the culture conditions described here facilitate the expansion of liver progenitors able to generate hepatocyte-like cells, albeit at lower numbers, but not fully mature hepatocytes (Fig.21A).

To enhance the hepatocytic nature of the cultures and obtain mature hepatocytes in *vitro,* we first determined whether the three supplemental factors (FGF10, HGF and Nicotinamide) added to EGF and Rspondin1 were exerting either a positive or negative effect on the hepatocyte expression, as well as on the self-renewal of the culture. We generated liver organoid cultures and cultured them either with EGF or EGF and Rspondin1 plus FGF10 or HGF or Nicotinamide or the combination of these, and we split the cultures once a week for a total period of 10 weeks. At each time-point we also analysed the expression of several mature hepatocyte markers (FAH, CYP3A11) and hepatoblast markers (albumin) (Figure 21B).

It was observed that Rspondin1 and Nicotinamide combined with FGF10 are essential for the growth and self-renewal of the liver cultures (Figure 21C&D). Rspondin1 and Nicotinamide both inhibit the expression of the mature marker CYP3A11 and yet promote the expression of the hepatoblast marker albumin. The addition of either FGF10 or HGF to media containing only EGF (without Rspondin1 and without nicotinamide), facilitated the expression of the mature marker CYP3A11, albeit at very low levels (figure 21E). To identify additional compounds that might facilitate hepatocyte differentiation, we used two different approaches, both based upon base conditions of: EGF + HGF and/or FGF10.

The first approach involved testing a series of compounds in addition to the EGF + FGF10 or HGF condition. A complete list of the compounds analysed is shown in table 4.

**Table 4**

| **Compounds** | **Signal** | | **Concentration** | **Result** | |
|---|---|---|---|---|---|
| | | | | ***Alb*** | ***CYP3 AII*** |
| | | | | | |
| Exendin4 | Glucagon like peptide 2 analog | **Sigma E7144** | 0.1-1uM | | |
| Retinoic Acid | RAR-RXR receptor ligand | Sigma | 25nM | | |
| Retinoic Acid + Exendin 4 | | | | | |
| Sonic Hedgehog | | Invitrogen C25II | 500-100ng/ml | | |
| BMP4 | BMP signaling | Peprotech 120-05 | 20ng/ml | | |
| DAPT | Gamma-secretase inhibitor | Sigma D5942 | 10 nM | | |
| A8301 | Alk5/4 /7 inhibitor | Tocris Bioscience 2939 | 50 nM | | |
| DAPT + A8301 | | | | +++ | +++ |
| FGF4 | FGFR1,2 ligand | Peprotech | 50ng/ml | | |
| FGF1 | FGFR1,2,3,4 ligand | Peprotech 450-33A | 100ng/ml | | |
| Dexamethasone | | Sigma D4902 25MG | 10 µM-1mM | | |
| Oncostatin M (OSM) | | **R&D systems 495-MO-025** | 10-1000 ng/ml | | |
| FGF4+OSM+Dexa | | | | | |
| IGF | | peprotech | 100ng/ml | | |
| Valproic acid | histone deacetylase inhibitor and regulator of ERK, PKC wnt/β-catenin pathways | Stemgent 04-0007 | 250 µM | | |
| Sodium Butyrate | histone deacetylase inhibitor | Stemgent 04-0005 | 250 µM | | |
| BIX01294 | G9a HMTase inhibitor | Stemgent 04-0002 | 1 µM | | |
| RG 108 | DNA methyltransferase inhibitor | Stemgent 04-0001 | 1 µM | | |
| TSA | | | 100 nM | + | - |
| Hydrocortisone | glucocorticoid | Sigma H6909 | 5nM | | |
| Oncostatin M (OSM) | | **R&D systems 495-MO-025** | 10-1000 ng/ml | | |
| ARA | | Sigma A 0937 | 500 nM | | |
| **R 59022** | Diacylglycerol kinase inhibitor | Sigma D 5919 | 500nM-50nM | + | + |
| **Arterenol bitrartre: ----** | **andrenoreceptor agonist** | **sigma A 0937** | 500nM-50nM-5nM | | |
| LIF | | | 10³ | | |
| PD 035901 | MEK1 inhibitor | Axon Medchem cat n 1386 | 500nM | | |
| CHIR99021 | GSK3 inhibitor | Axon Medchem cat n 1408 | 3uM | | |
| DMSO | | | 1% | | |
| L-Ascobic acid | | Sigma 077K13021 | 1mM | | |
| VEGF | | Peprotech | | | |
| Matrigel 50% | | | | | |
| Matrigel 20% | | | | | |
| VEGF+DEXA | | | | | |

The second approach took into account knowledge from published developmental studies regarding the expression of the transcription factors essential to achieve biliary and hepatocyte differentiation *in vivo.* A comparative analysis of the expression of transcription factors in the organoids under E or ER or ENRW conditions supplemented with FGF10, HGF and Nicotinamide is shown in figure 21. All the transcription factors required for Hepatocyte specification were present, besides tbx3 and prox1. However, we also noticed that the expression of specific biliary transcription factors was highly upregulated in the cultures containing Rspondin1 (R), indicating that the culture gene expression was unbalanced towards a more biliary cell fate.

Notch and TGF-beta signaling pathways have been implicated in biliary cell fate *in vivo.* In fact, deletion of Rbpj (essential to achieve active Notch signalling) results in abnormal tubulogenesis (Zong Y. Development 2009) and the addition of TGFb to liver explants facilitates the biliary differentiation *in vitro* (Clotman F. Genes and Development 2005). Since both Notch and TGFb signalling pathways were highly upregulated in the liver cultures (Figure 22) we reasoned that inhibition of biliary duct cell-fate might trigger the differentiation of the cells towards a more hepatocytic phenotype. A8301 was selected as an inhibitor of TGFb receptor ALK5, 4, and 7 and DAPT as inhibitor of the gamma-secretase, the active protease essential to activate the Notch pathway. We first cultured the cells for 2 days in the expansion conditions (ER media) and at day 2 (figure 23A) we started the differentiation conditions by adding the combination of the different compounds. Media was changed every other day, and the expression of differentiated markers was analysed 8-9 days later. The ER and ENRW conditions were used as negative control.

The combination of EGF + FGF10 with DAPT and A8301 resulted in surprisingly large enhancement of expression of the hepatocyte markers analysed (CYP3A11, TAT, Albumin) (figure 23B). The effect was already detectable by day 5 and peaked at days 8-9 (figure 23C). The maximal concentration efficiency was achieved at 10uM (DAPT) and 50 nM (A8301) (figure 23D) respectively. The addition of dexamethasone (a known hepatocyte differentiation molecule) did not result in any improvement in gene expression. The combination of EGF, FGF10, A8301 and DAPT not only enhances the expression but also increases the number of hepatocyte-like cells, as assessed by immunofluorescent against the hepatocyte markers albumin and 2F8, and Xgal staining on AlbCreLacZ derived organoids (figure 23E & F). Therefore, we can conclude that the aforementioned differentiation protocol facilitates the generation of hepatocyte-like cells *in vitro* from liver stem cell cultures.

### METHODS

### Reagents

Reagents used in the culture experiments are shown in Table 4.

**Mice***Lgr5-EGFP-ires-creERT2* mice (Barker N et al. Nature 2007;449:1003-7), *APC*^{*fl*/*fl*} (Sansom OJ et al. Genes Dev 2004;18:1385-90), *Axin2-lacZ* mice (Lustig B et al. Mol Cell Biol 2002;22:1184-93), C57B/6 wild type mice (6-12 week old) were genotyped as previously described and were used for experiments. *Lgr5-EGFP-ires-creERT2* mice were crossed with *APC*^{*fl*/*fl*} mice. Cre enzyme activity was induced by intraperitoneal injections of Tamoxifen (2 mg/mouse). The mice were euthanized 4 weeks after Tamoxifen induction. Murine small intestines and colons were opened longitudinally, washed with cold PBS and further processed for crypt isolation. Regions containing intestinal adenomas were identified using a stereomicroscope, cut out with a scalpel and washed with cold PBS.

### Human tissue materials

Surgically resected intestinal tissues were obtained from 30 patients from the Diaconessen Hospital Utrecht or the UMCU Hospital.

Patient material was collected from 20 patients with colon cancer (9 cecum-ascending colon, 7 sigmoid colon, 4 rectum; 33-86 years old), 5 patients with screening colonoscopy (33-63 years old) and 5 patients with Barrett's esophagus (45-78 years old). For normal tissue a distance of more than 3 cm to the tumors was kept. The intestinal tissues were washed and stripped of the underlying muscle layers. The tissue was chopped into around 5 mm pieces, and further washed with cold PBS. Endoscopic biopsies (Intestinal or esophageal) were obtained from the UMCU hospital. For each case, at least 5 biopsy samples were collected and stored in cold PBS. This study was approved by the ethical committee of DHU and UMCU, and all samples were obtained with informed consent.

### Crypt/adenoma isolation and cell dissociation

Intestinal fragments (murine normal colon, human normal small intestine and colon) were further washed with cold PBS until the supernatant was clear. Next, the tissue fragments were incubated in 2mM EDTA cold chelation buffer (distilled water with 5.6 mM Na2HPO4, 8.0 mM KH2PO4, 96.2 mM NaCL, 1.6 mM KCl, 43.4 mM Sucrose, 54.9 mM D-Sorbitiol, 0.5 mM DL-Dithiothreitol) for 30 min on ice (Gregorieff A Gastroenterology 2005(129)626-638). After removal of the EDTA buffer, tissue fragments were vigorously resuspended in cold chelation buffer using a 10-ml pipette to isolate intestinal crypts. The tissue fragments were allowed to settle down under normal gravity for 1 min and the supernatant was removed for inspection by inverted microscopy. The resuspension/sedimentation procedure was typically 6-8 times, and the supernatants not containing crypts were discarded. The supernatants containing crypts were collected in 50 ml-falcon tubes coated with bovine serum albumin. Isolated crypts were pelleted, washed with cold chelation buffer and centrifuged at 150-200 g for 3 min to separate crypts from single cells.

Murine colonic crypts were pelleted and resuspended with TrypLE express (Invitrogen) and incubated for 15 min at 37 °C. In this dissociation condition, colonic crypts were mildly digested, thereby physically separating colonic crypt bottoms from the top of the colon crypts.

Intestinal fragments containing adenomas from Tamoxifen-induced *Lgr5-EGFPires-creERT2*/*APCfl*/*fl* mice were incubated in 2 mM EDTA chelation buffer for 60 min on ice. Following washing with cold chelation buffer, most of the normal intestinal epithelial cells were detached, while adenoma cells remained attached to the mesenchyme. Next, the adenoma fragments were incubated in digestion buffer (DMEM with 2.5% fetal bovine serum, Penicillin/Stroptomycin (Invitrogen), 75 U/ml collagenase type IX (Sigma), 125 □g/ml dispase type II (Invitrogen)) for 30 min at 37 °C. The adenoma fragments were allowed to settle down under normal gravity for 1 min and the supernatant was collected in a 50ml-falcon tube, pelleted and washed with PBS. Isolated adenoma cells were centrifuged at 150-200 *g* for 3 min to separate adenoma from single cells.

Biopsy samples from Barrett's epithelium and human colon cancer samples, chopped into 5 mm pieces, were washed with PBS several times. The tissue fragments were incubated in digestion buffer for 60 min at 37 °C. After the digestion, tissue fragments were manually picked under the microscope.

For sorting experiments, isolated crypts were dissociated with TrypLE express (Invitrogen) including 2,000 U/ml DNase (Sigma) for 60 min at 37 °C. Dissociated cells were passed through 20-µm cell strainer (CellTrics) and washed with PBS. Viable epithelial single cells were gated by forward scatter, side scatter and pulse-width, and negative staining for propidium iodide or 7-ADD (eBioscience).

### Culture of intestinal crypts, adenomas, Barrett's epithelium and colon cancer

Isolated intestinal crypts, Barrett's epithelium and colon cancer cells were counted using a hemocytometer. Crypts, fragments of epithelium or single cells were embedded in matrigel on ice (growth factor reduced, phenol red-free; BD bioscience) and seeded in 48-well plates (500 crypts/fragments or 1000 single cells per 25 µl of matrigel per well). The matrigel was polymerized for 10 min at 37 °C, and 250µl /well basal culture medium (Advanced DMEM/F12 supplemented with penicillin/streptomycin, 10mM HEPES, Glutamax, 1× N2, 1× B27 (all from Invitrogen) and 1 mM *N*-acetylcysteine (Sigma)) was overlaid containing the following optimized growth factor combinations: murine EGF for murine intestinal adenomas, ENR (murine EGF, murine noggin, human R-spondin-1) for murine small intestinal crypts, WENR (recombinant human Wnt-3A or Wnt-3A conditioned medium+ENR) for murine colonic crypts, HISC (human intestinal stem cells: WENR+gastrin+nicotinamide+A83-01+SB202190) for human small intestinal/colonic crypts, HISC+human FGF10 for Barrett's epithelium. Colon cancer cells show a heterogenous behaviour and require either no addition of growth factors, murine EGF and/or A83-01 and/or SB202190. For cell sorting experiments, Y-27632 (10 µM; Sigma) was included in the medium for the first 2 days to avoid anoikis. Reagents and concentrations of each growth factor are indicated in **Fig. 12****.** An overview of the optimized combinations of growth factors and small molecule inhibitors for each organ is given in **Fig. 12****.**

### Image analysis

The images of organoids were taken by either confocal microscopy with a Leica SP5, an inverted microscope (Nikon DM-IL) or a stereomicroscope (Leica, MZ16-FA). For immunohistochemistry, samples were fixed with 4% paraformaldehyde (PFA) for 1h at room temperature, and paraffin sections were processed with standard techniques. Immunohistochemistry was performed as described previously. For whole-mount immunostaining, crypt organoids were isolated from Matrigel using Recovery solution (BD bioscience), and fixed with 4% PFA, followed by permeabilization with 0.1% Triton X-100. The primary antibodies were: mouse anti-Ki67 (1:250, Monosan), rabbit anti-Muc2 (1:100, Santa Cruz), rabbit anti-lysozyme (1:1,000, Dako), rabbit anti-synaptophysin (1:100, Dako) and anti-chromogranin A (1:100, Santa Cruz). The secondary antibodies were peroxidase-conjugated antibodies or Alexa-568-conjugated antibodies.EdU staining followed the manufacturer's protocol (Click-IT; Invitrogen). DNA was stained with DAPI (Molecular Probes). Three-dimensional images were acquired with confocal microscopy and reconstructed with Volocity Software (Improvision).

### Microarray analysis and Real-time PCR analysis

The data was deposited in the GEO database under the accession number GSE28907.

### EXAMPLE 6 - Liver organoid culture comprising Prostaglandin-2 or Arachidonic Acid

*In vitro* survival, growth and expansion of liver organoids was potently enhanced by addition of prostaglandin E2 (PGE2) or Arachidonic acid (AA) to the basal medium.

Figures 25 and 26 show that addition of PGE2 at 50nM (also seen to work in the range 10-500nM) or addition of AA at 10ug/ml (also works at 100ug/ml, though not so well), results in a greater number of larger organoids than using the basal medium alone. Importantly, the addition of PGE2 or AA allows for a longer expansion time. This means that organoids can be expanded for more population doublings before there growth decreases or slows down. Without PGE2 a growth reduction is seen after 5 weeks of culturing at 5 fold expansion per week. With PGE2 there is no growth reduction before at least 8 weeks at 5 fold expansion per week. PGE2 was seen to have a slightly greater effect than AA. The basal medium used was: hEGF (100ng/ml, Invitrogen); human noggin (hnoggin) (25ng/ml, peprotech); gastrin (10nM, sigma); hFGF10 (peprotech); nicotinamide (10mM, sigma); A8301 (500nM, Tocris); hHGF (50ng/ml, peprotech); Rspo conditioned media (10%).

PGE2 and AA are both in the same prostaglandin signalling pathway (see Figure 24), along with phospholipids, prostaglandin G2 (PGG2), prostaglandin F2 (PGF2), prostaglandin H2 (PGH2), prostaglandin D2 (PGD2). It would be expected that addition of any other activating component of this pathway would have the same beneficial effect on the culture media.

Addition of PGE2 or AA is particularly beneficial for expansion culture media. However, they may in some circumstances also be included in differentiation media.

### EXAMPLE 7 - GSK3 inhibitors are effective Wnt agonists in the culture media

CHIR99021, a GSK3 inhibitor, was shown to be an effective Wnt agonist for the culture media. In particular, it was shown to be a suitable replacement for Wnt in the culture media for colon and liver organoids.

Furthermore, as an extension to Example 6, Figure 25 shows that human liver cells grown in the presence of both CHIR99021 (Wnt agonist) and PGE2 result in more and larger organoids than cells grown with either the Wnt agonist or PGE2 alone and certainly more/larger organoids than in only the basal medium.

Therefore, GSK3 inhibitors could be used in the culture media instead of, or in addition to, other Wnt agonists, such as Wnt or Rspondin1-4.

It is surprising that CHIR99021 was such an effective Wnt replacement because GSK3 is involved in a number of different pathways, not only the Wnt pathway. This finding opens up the possibility of designing other Wnt agonists targeting GSK3, which might be useful in culture media.

### EXAMPLE 8 - Prostate

### Isolation of Prostatic epithelium (Murine protocol).

The numbered steps correspond to figure 47.
i) Sacrifice male mouse at minimally 8 weeks of age to isolate a mature prostate; isolate the urogenital sinus from the mouse.
ii) Remove seminal vesicles by breaking/cutting bloodvessels and connective tissue and making a incision at the base at the urethra
iii) Remove the bladder by breaking/cutting it near the base at the urethra
iv) Remove remaining vesicles & fat tissue by gentle tugging and cutting. What you should have left it the prostate lobes (6 of them) and a pink structure in the middle, which is the urethra;
v) Remove urethra, easily recognized by the pink color (stained dark in the picture). Carefully pull the prostate lobes, so they are no longer attached to the urethra; isolate each lobe individually, just by pulling them apart, or continue with the whole prostate.

Next, mince the prostate (lobes) in small pieces; digest the prostate in 1 ml 10 mg/ml Collagenase II (dissolved in ADMEM/F12) for 1 ½ hours at 37 °C; after collagenase digestion only "fingerlike" structures of epithelial cells should remain.
- Wash in ADMEM/F12
- Let the chunks settle down and draw off supernatant (centrifugation at low speed gets rid of most the mesenchyme)
- Centrifuge 50xG 5 min 4'C
- Resuspend in 1 ml Trypsin (TLE) and digest for approximately 30 min 37'C.

Pipette up and down every 10 minutes to ensure digestion
- Wash in ADMEM/F12
- Either start culture in ENR or ENR+ 1nM DiHydroTestosterone (seed approximately 5000 cells per well) (0.1nM-10uM) we donot know an upper limit
- Or continue with isolation of specific celltype via FACS

### Results

Prostatic epithelial cells cultured in ENR + DiHydro Testosterone, according to the methods described described above, can be maintained for 35 weeks so far. In the presence of testosterone, the cultures expand the same as without testosterone. However, with testosterone all cell types are present including stem cells, transit amplifying cells and differentiated cells i.e. there is increased differentiation whilst maintaining a stem cell population. Prostate organoids grown in the presence of testosterone also look more like the *in vivo* organ (see figures 41 and 42). Furthermore, IHC and RT-PCR shows that prostate organoids grown in the presence of testosterone contain both basal and luminal cells.

**The invention also provides the following numbered embodiments:**
1. A culture medium for expanding a population of stem cells, wherein the culture medium comprises at least one or more inhibitor that binds to and reduces the activity of one or more serine/threonine protein kinase target selected from the group comprising: TGFβ receptor kinase 1, ALK4, ALK5, ALK7, p38; and wherein the culture medium allows continual growth for at least 3 months.
2. The culture medium according to embodiment 1, wherein the at least one or more inhibitor comprises:
   a) an inhibitor that binds to and reduces the activity of ALK5; and;
   b) an inhibitor that binds to and reduces the activity of p38.
3. The culture medium of embodiment 1 or embodiment 2, wherein the inhibitor is an agent that binds to and reduces the activity of its target by more than 95%; as assessed by a cellular assay.
4. The culture medium of any one of the preceding embodiments, wherein the inhibitor has an IC₅₀ value of less than 100nM.
5. The culture medium of any one of the preceding embodiments, wherein the inhibitor acts competitively; non-competitively; uncompetitively; or by mixed inhibition.
6. The culture medium of any one of the preceding embodiments, wherein the inhibitor acts competitively and binds to the ATP-binding pocket of the serine-threonine protein kinase target.
7. The culture medium of any one of the preceding embodiments, wherein the inhibitor is: a) a small-molecule inhibitor; b) a protein or peptide; c) an antisense oligonucleotide; or d) an aptamer.
8. The culture medium of any one of the preceding embodiments, wherein the small molecule inhibitor has a molecular weight of between 50 and 800 Da.
9. The culture medium of any one of the preceding embodiments, wherein the inhibitor is a pyridinylimidazole or a 2,4-disubstituted pteridine or a quinazoline-derived inhibitor.
10. The culture medium of any one of the preceding embodiments, wherein the inhibitor is added at a concentration of between 10 nM and 10 µM.
11. The culture medium of any one of the preceding embodiments, wherein the inhibitor is selected from the group of compounds comprising: SB-202190, SB-203580, SB-206718, SB-227931, VX-702, VX-745, PD-169316, RO-4402257, BIRB-796, A83-01, LY364947 SB-431542, SB-505124, SB-525334, LY 364947, SD-093, and SJN 2511.
12. The culture medium of any one of the preceding embodiments, wherein SB-202190 or SB-203580 is added to a concentration of between 50 nM and 100 uM.
13. The culture medium according to any of the preceding embodiments, wherein the stem cells are human stem cells.
14. The culture medium according to any of the preceding embodiments, wherein the stem cells are epithelial stem cells.
15. The culture medium according to embodiment 14, wherein the human epithelial stem cells are a) pancreatic stem cells; b) intestinal stem cells; or c) colon stem cells.
16. The culture medium according to any of the preceding embodiments, wherein the stem cells form part of an organoid or isolated tissue fragment.
17. The culture medium according to any of the preceding embodiments, wherein the stem cells are cancer stem cells.
18. The culture medium of any one of the preceding embodiments, wherein the percentage of cells in the population of stem cells stem cells to have a normal karyotype, after 1, 2 or 3 or more months, is more than 90%.
19. The culture medium of any one of the preceding embodiments, wherein the percentage of cells in the population of stem cells stem cells to have a normal phenotype, after 1, 2 or 3 or more months, is more than 90%.
20. The culture medium of any one of the preceding embodiments, wherein the stem cells survive for more than three months; such as more than six months.
21. The culture medium of any one of the preceding embodiments, wherein the stem cells have an average population doubling time of 12 to 36 hours, of 18 to 30 hours, or of approximately 24 hours.
22. The culture medium according to any of the preceding embodiments, wherein the culture medium comprises a basal medium for animal or human cells and:
   a) one or more bone morphogenetic protein (BMP) inhibitor;
   b) one or more mitogenic growth factor; and
   c) one or more Wnt agonist.
23. The culture medium according to any of the preceding embodiments wherein, the culture medium comprises gastrin and/or nicotinamide.
24. A method for expanding a population of stem cells, wherein the method comprises:
   a) providing a population of stem cells;
   b) providing a culture medium according to any one of the preceding embodiments;
   c) contacting the stem cells with the culture medium; and
   d) culturing the cells under appropriate conditions.
25. A composition comprising a culture medium according to any of the preceding embodiments and stem cells.
26. A composition comprising a culture medium according to the invention and an extracellular matrix.
27. A culture medium supplement comprising the one or more inhibitor according to any of the preceding embodiments.
28. A hermetically-sealed vessel containing a culture medium according to any of the preceding embodiments or a culture medium supplement according to embodiment 27.
29. The culture medium according to any of embodiments 1 to 23 for the culture of Barrett's Esophagus epithelium, wherein the culture medium further comprises FGF10.
30. Stem cells or organoids obtained using the culture medium of any of the preceding embodiments, for use in transplantation purposes or other therapeutic applications.
31. A pancreatic organoid comprising beta-cells.
32. The pancreatic organoid of embodiment 31, further comprising α cells, δ cells and PP cells.
33. The pancreatic organoid of any one of embodiments 31 or 32, comprising α cells, β cells, δ cells and PP cells.
34. A pancreatic organoid of any one of embodiments 31 or 33 that expresses one, two or all three of Pdx1, Nkx2.2 and Nkx6.1.
35. A pancreatic organoid of any one of embodiments 31 to 34 that expresses one, two or all three of NeuroD, Pax6 and Mafa.
36. A pancreatic organoid of embodiment 35 that additionally expresses Ngn3.
37. A pancreatic organoid, for example a pancreatic organoid according to any one of embodiments 31 to 36, which is capable of secreting insulin following transplantation of the organoid into a patient.
38. A pancreatic organoid as recited in any one of embodiments 31 to 37 for use in treating a patient having an insulin-deficiency disorder such as diabetes.
39. A method of treating a patient having an insulin-deficiency disorder such as diabetes comprising transplanting a pancreatic organoid according to any one of embodiments 31 to 37 into the patient.
40. A human organoid selected from the group consisting of a crypt-villus organoid, a colon organoid, a pancreatic organoid, a gastric organoid, a Barrett's Esophagus organoid, an adenocarcinoma organoid and a colon carcinoma organoid.
41. A small-intestinal or crypt-villus organoid, obtained using the culture medium of any of embodiments 1 to 23, for use in treating damaged epithelium, for example in microvillous inclusion disease (MVID) patients.
42. A liver culture medium comprising or consisting of a basal medium for animal or human cells to which is added: one or more receptor tyrosine kinase ligand such as a mitogenic growth factor (e.g. EGF), Nicotinamide, and preferably, a Wnt agonist, preferably R-spondin 1-4 and/or CHIR99021 and one or both of a) a prostaglandin pathway activator, such as PGE2 and/or AA and b) a TGF-beta inhibitor such as A83-01.
The invention also provides the following numbered aspects:
1. A culture medium for expanding and/or differentiating a population of adult stem cells, wherein said culture medium comprises:
   i. any one of Rspondin 1-4 and/or an Rspondin mimic; and
   ii. one or more inhibitor that directly or indirectly negatively regulates TGF-beta signalling.
2. The culture medium of aspect 1, wherein the one or more inhibitor binds to and reduces the activity of one or more serine/threonine protein kinases selected from the group consisting of ALK5, ALK4, TGF-beta receptor kinase 1 and ALK7.
3. The culture medium of aspect 1 or aspect 2, wherein the one or more inhibitor that directly or indirectly negatively regulates TGF-beta signalling is selected from the group consisting of A83-01, SB-431542, SB-505124, SB-525334, SD-208, LY-36494 and SJN-2511.
4. The culture medium of any one of the preceding aspects, wherein the culture medium further comprises an inhibitor that directly or indirectly negatively regulates p38 signalling.
5. The culture medium of aspect 4, wherein the one or more inhibitor that directly or indirectly negatively regulates p38 signalling is selected from the group consisting of SB-202190, SB-203580, VX-702, VX-745, PD-169316, RO-4402257 and BIRB-796.
6. The culture medium of any one of the preceding aspects, wherein the culture medium comprises A83-01 and SB-202190 or A83-01 and SB-203580.
7. The culture medium of any one of the preceding aspects, wherein the inhibitor is added at a concentration of between 1nM and 100 µM, between 10 nM and 100 µM, between 100 nM and 10 µM, or approximately 1 µM, for example, wherein the total concentration of the one or more inhibitor is between 10 nM and 100 µM, between 100 nM and 10 µM, or approximately 1 µM.
8. The culture medium of any one of the preceding aspects, wherein the culture medium comprises one or more additional components selected from: a BMP inhibitor, a Wnt agonist, a receptor tyrosine kinase ligand, a Rock inhibitor, nicotinamide and gastrin.
9. The culture medium of any one of the preceding aspects, wherein the culture medium comprises any one of Rspondin 1-4 and/or an Rspondin mimic, a BMP inhibitor (for example, Noggin), a TGF-beta inhibitor, a receptor tyrosine kinase ligand (for example, EGF), Nicotinamide, a Wnt agonist (for example, Wnt(3a)), and optionally one or more additional components selected from: a p38 inhibitor, gastrin, FGF10, HGF and a Rock inhibitor.
10. The culture medium of aspect 8 or aspect 9, wherein the BMP inhibitor is selected from the group consisting of Noggin, Chordin, a chordin-like protein comprising a chordin domain, Follistatin, a follistatin-related protein comprising a follistatin domain, DAN, a DAN-like proteins comprising a DAN cystein-knot domain, sclerostin/SOST and alpha-2 macroglobulin.
11. The culture medium of aspect 8 or aspect 9, wherein the Wnt agonist is selected from the group consisting of Wnt-3a, a GSK-inhibitor (such as CHIR99021), Wnt 5, Wnt-6a, Norrin, and any other Wnt family protein.
12. The culture medium of aspect 8 or aspect 9, wherein the receptor tyrosine kinase ligand is a mitogenic growth factor, for example selected from a family of growth factors consisting of epidermal growth factor (EGF), Transforming Growth Factor-alpha (TGF-alpha), basic Fibroblast Growth Factor (bFGF), brain-derived neurotrophic factor (BDNF), Hepatocyte growth factor (HGF) and Keratinocyte Growth Factor (KGF).
13. The culture medium of aspect 8 or aspect 9, wherein the Rock inhibitor is selected from the group consisting of R-(+)-trans-4-(l-aminoethyl)-N-(4-Pyridyl)cyclohexanecarboxamide dihydrochloride monohydrate (Y-27632), 5-(l ,4-diazepan- l-ylsulfonyl)isoquinoline (fasudil or HA107l), and (S)-(+)-2-methyl- l-[(4-methyl-5-isoquinolinyl)sulfonyl] -hexahydro-1H- 1,4-diazepine dihydrochloride (H-1 152).
14. The culture medium of any one of the preceding aspects, wherein the culture medium additionally comprises an activator of the prostaglandin signalling pathway, for example, PGE2 and/or AA.
15. The culture medium of any one of the preceding aspects, wherein the culture medium additionally comprises testosterone, for example, (dihydro)testosterone.
16. A culture medium according to any one of aspects 1 to 15 for culturing intestinal cells, comprising or consisting of a basal medium, Wnt-3a, EGF, Noggin, any one of Rspondin 1-4, a TGF-beta inhibitor, nicotinamide, and preferably a p38 inhibitor.
17. A culture medium according to any one of aspects 1 to 15 for culturing gastric cells, comprising or consisting of a basal medium, Wnt-3a, EGF, Noggin, any one of Rspondin 1-4, a TGF-beta inhibitor, gastrin, nicotinamide, FGF-10, and preferably a p38 inhibitor.
18. A culture medium according to any one of aspects 1 to 15 for expanding liver cells, comprising or consisting of a basal medium, any one of Rspondin 1-4, Noggin, nicotinamide, EGF, FGF10, HGF, gastrin, a TGF-beta inhibitor and PGE2, and preferably Wnt-3a.
19. A culture medium according to any one of aspects 1 to 15 for expanding pancreas cells, comprising or consisting of a basal medium, any one of Rspondin 1-4, Noggin, EGF, FGF10, gastrin, a TGF-beta inhibitor, and preferably exendin 4 and Wnt-3a.
20. A culture medium according to any one of aspects 1 to 15 for culturing prostate cells, comprising or consisting of a basal medium, EGF, any one of Rspondin 1-4, Noggin, nicotinamide a TGF-beta inhibitor and preferably Wnt-3a, and FGF-10.
21. A culture medium for culturing prostate cells according to aspect 20, wherein the culture medium further comprises testosterone, for example (dihydro)testosterone.
22. A culture medium of any one of the preceding aspects for culturing cancer cells, for example cancer stem cells, such as adenocarcinoma or carcinoma cells from a tissue type of interest, wherein the culture medium comprises or consists of the components of the culture medium used for culturing the cells from the corresponding non-cancerous tissue type of interest, optionally wherein one or more of the following are excluded from the medium that is used to culture the non-cancerous cells of the tissue type of interest: Wnt-3a, EGF, Noggin, Rspondin, TGF-beta inhibitor, p38 inhibitor, nicotinamide, gastrin, FGF10 and HGF.
23. A culture medium of any one of the preceding aspects for differentiating stem cells from a tissue of interest, wherein the culture medium comprises or consists of the components of the culture medium used for expanding the stem cells from the tissue type of interest but wherein one or more of the following are excluded from the medium for differentiating stem cells: Wnt, Rspondin, BMP inhibitor, TGF-beta inhibitor, receptor tyrosine kinase ligand, p38 inhibitor and nicotinamide.
24. A culture medium according to aspect 23 for differentiating intestinal cells, comprising or consisting of a basal medium, EGF, Noggin, a TGF-beta inhibitor and a p38 inhibitor.
25. A culture medium according to aspect 23 for differentiating liver cells comprising or consisting of a basal medium, Noggin, EGF, gastrin, a TGF-beta inhibitor, a gamma-secretase inhibitor such as DAPT or DBZ, and preferably Wnt-3a.
26. A culture medium according to aspect 23 for differentiating pancreas cells comprising or consisting of a basal medium, Noggin, EGF, FGF10, gastrin, a TGF-beta inhibitor, gamma-secretase inhibitor and preferably exendin 4.
27. The culture medium according to any one of the preceding aspects, wherein the culture medium is in contact with an extracellular matrix or a 3D matrix that mimics the extracellular matrix by its interaction with the cellular membrane proteins such as integrins.
28. The culture medium of aspect 27, wherein the extracellular matrix is a laminin-containing extracellular matrix such as Matrigel^{™} (BD Biosciences).
29. A composition comprising a culture medium according to any one of aspects 1 to 26 and an extracellular matrix or a 3D matrix that mimics the extracellular matrix by its interaction with the cellular membrane proteins such as integrins, for example, a laminin-containing extracellular matrix such as Matrigel^{™} (BD Biosciences).
30. A hermetically-sealed vessel containing a culture medium or composition according to any one of the preceding aspects.
31. Use of a culture medium according to any one of aspects 1 to 28 for expanding and/or differentiating a stem cell, population of stem cells, tissue fragment or organoid.
32. Use according to aspect 31, wherein the stem cell, population of stem cells, tissue fragment or organoid is selected from the group consisting of one or more intestinal stem cells, small intestinal crypts, colonic crypts, gastric stem cells, liver stem cells, pancreas stem cells and prostate stem cells.
33. Use according to aspect 31 or aspect 32, wherein the stem cell, population of stem cells, tissue fragment or organoid is obtainable from a normal tissue.
34. Use according to aspect 31 or 32, wherein the stem cell, population of stem cells, tissue fragment or organoid is obtainable from a diseased tissue, for example from an adenoma, a carcinoma, an adenocarcinoma, an intestine of a patient having cystic fibrosis or an intestine of a patient having inflammatory bowel disease.
35. A method for expanding a single stem cell, a population of stem cells or a tissue fragment, preferably to obtain an organoid, wherein the method comprises culturing the single stem cell or population of stem cells in a culture medium according to any one of aspects 1 to 28.
36. A method according to aspect 35, wherein the method comprises:
   providing a stem cell, a population of stem cells or an isolated tissue fragment;
   providing a culture medium according to any one of aspects 1 to 28;
   contacting the stem cells with the culture medium;
   culturing the cells under appropriate conditions.
37. A method according to aspect 35, wherein the method comprises bringing the stem cell, the population of stem cells or the isolated tissue fragment and the culture medium into contact with an extracellular matrix or a 3D matrix that mimics the extracellular matrix by its interaction with the cellular membrane proteins such as integrins, for example a laminin-containing extracellular matrix such as Matrigel^{™} (BD Biosciences).
38. The method of aspect 37, wherein the culture medium is diffused into the extracellular matrix.
39. A method according to any one of aspects 35 to 38, wherein the method comprises:
   culturing the stem cell, population of stem cells or tissue fragments in a first expansion medium;
   continuing to culture the stem cell, population of stem cells or tissue fragments and replenishing the medium with a differentiation medium, wherein the differentiation medium does not comprise one or more of, preferably all of the factors selected from: a TGF-beta inhibitor, a p38 inhibitor, nicotinamide and Wnt.
40. A method according to any one of aspects 35-38 for obtaining a small intestinal or colon organoid comprising:
   expanding small intestinal or colon stem cells or tissue fragments in a culture medium according to aspect 17; and optionally
   differentiating the expanded small intestinal or colon stem cells or tissue fragments in a culture medium according to aspect 24.
41. A method according to any one of aspects 35-39 for obtaining a gastric organoid comprising culturing gastric stem cells or tissue fragments in the culture medium of aspect 16.
42. A method according to any one of aspects 35-39 for obtaining a liver organoid comprising:
   expanding liver cells or tissue fragments in a culture medium according to aspect 19; and optionally
   differentiating the expanded liver cells or tissue fragments in a culture medium according to aspect 25.
43. A method according to any one of aspects 35-39 for obtaining a pancreatic organoid comprising
   expanding pancreatic cells or tissue fragments in a culture medium according to aspect 20; and optionally
   differentiating the expanded pancreatic cells or tissue fragments in a culture medium according to aspect 26.
44. A method according to any one of aspects 35-39 for obtaining a prostate organoid comprising culturing prostate stem cells or tissue fragments in the culture medium of aspect 19.
45. A method according to any one of aspects 35-39 for obtaining an adenocarcinoma or carcinoma organoid comprising culturing adenocarcinoma or carcinoma stem cells or tissue fragments in the culture medium of aspect 22.
46. The method of any one of aspects 35-39, wherein a Rock inhibitor is added to the culture medium for the first 1, 2, 3, 4, 5, 6 or 7 days, optionally every second day.
47. The method according to any one of aspects 35-39, which comprises culturing the stem cells for 3 or more months, for example 4, 5, 6, 7, 8, 9 or more months using a culture medium according to any one of aspects 1 to 28.
48. An organoid or population of cells obtainable by the method of any one of aspects 35 to 47.
49. An organoid or population of cells according to aspect 48, which is capable of surviving in culture for at least 3 months, for example at least 4 months, at least 5 months, at least 6 months, at least 7 months, at least 9 months, or at least 12 months or more, when cultured in a culture medium according to any one of aspects 1 to 28.
50. An organoid or population of cells according to aspect 48, wherein the organoid or population of cells has been cultured for at least 3 months, for example at least 4 months, at least 5 months, at least 6 months, at least 7 months, at least 9 months, or at least 12 months or more.
51. An organoid or population of cells according to any one of aspects 48 to 50, wherein the organoid or population of cells expands at a rate of at least 3 fold, at least 4 fold, at least 5 fold, at least 6 fold, at least 7 fold, at least 8 fold, at least 9 fold or at least 10 fold per week.
52. The organoid or population of cells of any one of aspects 48 to 51, wherein the organoid or population of cells is a human organoid or a human population of cells.
53. The organoid or population of cells of any one of aspects 48 to 52, wherein the organoid or population of cells is a normal organoid or population of cells or a disease organoid or population of cells, for example obtained by culturing stem cells taken from a human or animal with a disease.
54. An organoid or population of cells according to any one of aspects 48 to 53 which is frozen and stored at below -5°C, below -10°C, below -20°C, below -40°C, below -60°C, below -80°C, below
   -100°C, or below -150°C, for example at approximately -180 °C.
55. An organoid, preferably according to any one of aspects 48 to 54, which is a three-dimensional organoid comprising epithelial cells surrounding a central lumen, wherein optionally the epithelial cells exist in distinct dividing domains and differentiating domains.
56. The organoid of aspect 55, which is a three-dimensional organoid comprising epithelial cells arranged in regions of monolayers, optionally folded monolayers and regions of stratified cells.
57. The organoid of any one of aspects 48 to 56, wherein non-epithelial cells are absent from said organoid.
58. The organoid of any one of aspects 48 to 57, wherein all differentiated cell types of the normal *in vivo* tissue are present in said organoid.
59. The organoid of any one of aspects 48 to 58, wherein the organoid is a small intestine organoid, a colon organoid, a gastric organoid, a pancreatic organoid, a liver organoid or a prostatic organoid.
60. A composition comprising:
   i) one or more organoids or population of cells according to any one of aspects 48 to 59; and
   ii) a culture medium according to any one of aspects 1 to 28 and/or an extracellular matrix.
61. An organoid according to any one of aspects 48 to 59 or a population of cells according to any one of aspects 48 to 54 or a composition according to aspect 29 or aspect 60 for use in drug screening, target validation, target discovery, toxicology, toxicology screens, personalized medicine, regenerative medicine or *ex vivo* cell/organ models, for example for use as a disease model.
62. The organoid, population of cells or composition for use according to aspect 61, wherein the regenerative medicine or personalized medicine comprises transplantation of said organoid, population of cells or composition into a mammal, preferably into a human.

## Claims

1. A method for differentiating liver epithelial stem cells using a culture medium comprising a receptor tyrosine kinase ligand, a TGF-beta inhibitor and a Notch inhibitor.

2. The method of claim 1, wherein the receptor tyrosine kinase ligand is a ligand for the EGF receptor.

3. The method of claim 2, wherein the culture medium further comprises FGF and/or HGF, optionally wherein the FGF is FGF10 or FGF2.

4. The method of any one of the preceding claims, wherein the TGF-beta is a TGF-beta inhibitor if it can inhibit TGF-beta signalling in a cellular assay in which cells are stably transfected with a reporter construct comprising the human PAI-1 promoter.

5. The method of claim 4, wherein the TGF-beta inhibitor binds to and reduces the activity of ALK5, ALK4 and/or ALK7, optionally wherein the TGF-beta inhibitor is selected from the group consisting of A83-01, SB-431542, SB-505124, SB-525334, SD-208, LY-36494 and SJN-2511.

6. The method of any one of the preceding claims, wherein the Notch inhibitor is a gamma secretase inhibitor, optionally wherein the gamma secretase inhibitor is DAPT or DBZ.

7. The method of any one of the preceding claims, wherein the culture medium further comprises:
a) dexamethasone, optionally at a concentration of between 10 nM and 10 µM;
b) a prostaglandin pathway activator, optionally wherein the prostaglandin pathway activator is PGE2 or AA; and/or
c) oncostatin M, optionally wherein the oncostatin M is at a final concentration of between 1 ng/ml and 1 mg/ml.

8. The method any one of the preceding claims, wherein the culture medium does not comprise R-spondin, a Wnt agonist, a BMP inhibitor and/or nicotinamide.

9. The method of any one of the preceding claims, wherein the liver epithelial stem cells are human liver epithelial stem cells and/or express Lgr5.

10. The method of any one of the preceding claims, wherein the method comprises bringing the liver epithelial stem cells and the culture medium into contract with an extracellular matrix.

11. An organoid or population of cells obtainable by the method of any one of the preceding claims.

12. A composition comprising:
(i) one or more organoids or populations of cells according to claim 11; and
(ii) an extracellular matrix.

13. Use of an organoid according to claim 11, a population of cells according to claim 11 or composition according to claim 12 for drug screening, target validation, target discovery, toxicology, toxicology screens, or ex vivo cell/organ models, for example for use as a disease model.

14. An organoid according to claim 11, a population of cells according to claim 11 or composition according to claim 12 for use in medicine or diagnostics, optionally wherein medicine is personalized medicine or regenerative medicine.

15. A method for screening for a therapeutic or prophylactic drug or cosmetic, wherein the method comprises:
culturing an organoid or population of cells according to claim 11;
exposing said organoid or population of cells to one or a library of candidate molecules;
evaluating said organoid
or population of cells for any effects, for example any change in a cell, such as a reduction in or loss of proliferation, a morphological change and/or cell death; and
identifying the candidate molecule that causes said effects as a potential drug or cosmetic
